(19) Europäisches Patentamt
European Patent Office
Office européen des brevets

(11) **EP 2 358 392 B1**

(12) # EUROPEAN PATENT SPECIFICATION

(45) Date of publication and mention
of the grant of the patent:
**09.01.2019 Bulletin 2019/02**

(21) Application number: **09826710.7**

(22) Date of filing: **12.11.2009**

(51) Int Cl.:
*A61K 39/395* (2006.01)     *C12P 21/08* (2006.01)
*C07K 16/00* (2006.01)

(86) International application number:
**PCT/US2009/064127**

(87) International publication number:
**WO 2010/056804 (20.05.2010 Gazette 2010/20)**

(54) **ANTIBODY FORMULATION**

ANTIKÖRPER-FORMULIERUNG

FORMULATION D'ANTICORPS

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **07.10.2009 US 249365 P**
**12.11.2008 US 113796 P**

(43) Date of publication of application:
**24.08.2011 Bulletin 2011/34**

(73) Proprietor: **MedImmune, LLC
Gaithersburg, MD 20878 (US)**

(72) Inventors:
- **SATHISH, Hasige
  Germantown, MD 20874 (US)**
- **SHAH, Ambarish
  Boyds, MD 20841 (US)**
- **CARLESSO, Gianluca
  Rockville, MD 20852 (US)**
- **DELANEY, Tracy
  Montgomery Village, MD 20886 (US)**

(74) Representative: **AstraZeneca
Milstein Building
Granta Park
Cambridge CB21 6GH (GB)**

(56) References cited:
**EP-A2- 1 158 004        WO-A2-2008/028946
WO-A2-2008/137915      US-A1- 2008 138 336**

US-B1- 6 171 586      US-B2- 7 166 283

- CHEN BEI ET AL: "Influence of histidine on the stability and physical properties of a fully human antibody in aqueous and solid forms", PHARMACEUTICAL RESEARCH, KLUWER ACADEMIC PUBLISHERS, NEW YORK, NY, US, vol. 20, no. 12, 1 December 2003 (2003-12-01), pages 1952-1960, XP002386671, ISSN: 0724-8741, DOI: 10.1023/B:PHAM.0000008042.15988.C0
- DAUGHERTY A L ET AL: "Formulation and delivery issues for monoclonal antibody therapeutics", ADVANCED DRUG DELIVERY REVIEWS, ELSEVIER BV, AMSTERDAM, NL, vol. 58, no. 5-6, 7 August 2006 (2006-08-07), pages 686-706, XP024892149, ISSN: 0169-409X, DOI: 10.1016/J.ADDR.2006.03.011 [retrieved on 2006-08-07]
- MOORE ET AL.: 'Kinetics and thermodynamics of dimer formation and dissociation for a recombinant humanized monoclonal antibody to vascular endothelial growth factor.' BIOCHEMISTRY vol. 38, no. 42, 1999, pages 13960 - 13967, XP007901441
- MORI K ET AL: "Engineering Chinese hamster ovary cells to maximize effector function of produced antibodies using FUT8 siRNA", BIOTECHNOLOGY AND BIOENGINEERING, WILEY & SONS, HOBOKEN, NJ, US, vol. 88, no. 7, 30 December 2004 (2004-12-30), pages 901-908, XP002358546, ISSN: 0006-3592, DOI: 10.1002/BIT.20326

EP 2 358 392 B1

**(Cont. next page)**

Remarks:
The file contains technical information submitted after the application was filed and not included in this specification

Remarks:
The file contains technical information submitted after the application was filed and not included in this specification

**Description**

## 1. INTRODUCTION

The present disclosure relates to liquid formulations of antibodies or fragments thereof that specifically bind to a human ICOS polypeptide, exhibit increased *in vivo* ADCC activity and undergo reversible self-association in solution, which formulations exhibit stability, low to undetectable levels of antibody fragmentation, low to undetectable levels of aggregation, and very little to no loss of the biological activities of the antibodies, even during long periods of storage. The present disclosure also relates to methods of preventing, treating, managing or ameliorating symptoms associated with an ICOS mediated disease or disorder (for example, but not limited to, systemic lupus erythematosus, myositis, multiple sclerosis, scleroderma, inflammatory bowel disease, insulin dependent diabetes mellitus, psoriasis, autoimmune thyroiditis, rheumatoid arthritis and glomerulonephritis, transplant rejection, graft versus host disease) utilizing high concentration liquid formulations of antibodies or fragments thereof that specifically bind to a human ICOS polypeptide and exhibit increased *in vivo* ADCC activity.

## 2. BACKGROUND

ICOS is a type I transmembrane protein comprising an extracellular (Ig)V-like domain. ICOS serves as the receptor for the B7h co-stimulatory molecule. ICOS expression is low on naive human T cells but becomes upregulated within hours after TCR engagement. ICOS expression persists on activated T cells subpopulations such as Th1, Th2, and Th17 $CD4^+$ cells.

Given that ICOS expression is concentrated on activated T helper cell populations, the therapeutic use of an anti-ICOS antibody with enhanced effector function holds the promise of improving the efficacy of treatment and prevention of T cell-mediated diseases and disorders, such as, but not limited to, chronic infection, autoimmune disease or disorder, inflammatory disease or disorder, graft-versus-host disease (GVHD), transplant rejection, and T cell proliferative disorder using therapeutic anti-ICOS antibodies with enhanced effector function.

EP1158004 A2 describes human antibodies which bind to AILIM (activation inducible lymphocyte immunomodulatory molecule, AILIM is also referred to as ICOS (inducible co-stimulator); an anti-hICOS antibody, JMab136 is disclosed, however, there is no disclosure of an afucosylated version of antibody JMab136 or formulations thereof.

Currently, many antibodies are provided as lyophilized formulations. Lyophilized formulations of antibodies have a number of limitations, including a prolonged process for lyophilization and resulting high cost for manufacturing. In addition, a lyophilized formulation has to be reconstituted aseptically and accurately by healthcare practitioners prior to administering to patients. Thus, a need exists for liquid formulations of antibodies, in particular, anti-human ICOS antibodies, at a concentration comparable to or higher than the reconstituted lyophilized formulations so that there is no need to reconstitute the formulation prior to administration. This allows healthcare practitioners much quicker and easier administration of antibodies to a patient.

Prior liquid antibody preparations have short shelf lives and may lose biological activity of the antibodies resulting from chemical and physical instabilities during the storage. Chemical instability may be caused by deamidation, racemization, hydrolysis, oxidation, beta elimination or disulfide exchange, and physical instability may be caused by antibody denaturation, aggregation, precipitation or adsorption. Among those, aggregation, deamidation and oxidation are known to be the most common causes of the antibody degradation (Wang et al., 1988, J. of Parenteral Science & Technology 42(Suppl):S4-S26; Cleland et al., 1993, Critical Reviews in Therapeutic Drug Carrier Systems 10(4):307-377). Thus, there is a need for a stable liquid formulation of antibodies, in particular, stable liquid anti-human ICOS antibodies.

## 3. SUMMARY

The invention provides a sterile, stable aqueous formulation comprising an antibody that specifically binds human ICOS, wherein said formulation comprises 10 mg/ml of the antibody, 80 mM NaCl, 10 mM histidine, 4% trehalose and 0.02% polysorbate 80 and wherein the pH of the formulation is 6; and wherein

a) the antibody comprises an Fc region having complex N-glycoside-linked sugar chains in which fucose is not bound to N-acetylglucosamine in the reducing end in the sugar chain,
b) the antibody undergoes reversible self-association in solution, wherein the reversible self-association does not induce aggregate formulation, and
c) at least 10 mole percent of the antibody exists as a trimer in PBS at 10 mg/ml antibody concentration at 37oC; and

wherein the antibody comprises a heavy chain sequence of SEQ ID NO: 6 and a light chain sequence of SEQ ID NO: 1.
In a preferred embodiment said formulation is isotonic.

Preferably said formulation is a pharmaceutically acceptable formulation.

More preferably said formulation is an injectable formulation and optionally wherein the formulation is suitable for intravenous, subcutaneous, or intramuscular injection.

The invention further provides a pharmaceutical unit dosage form suitable for parenteral administration to a human which comprises an antibody formulation of the invention in a suitable container.

The invention yet further provides a pre-filled syringe containing the formulation of the invention.


### 3.1. DEFINITIONS

All formulations of antibodies and/or antibody fragments that specifically bind to an antigen of interest *(e.g.,* ICOS) are herein collectively referred to as "formulations of the disclosure", "liquid formulations of the disclosure", "high concentration stable liquid formulations of the disclosure", "antibody liquid formulations of the disclosure", or "antibody formulations of the disclosure".

As used herein, the terms "antibody" and "antibodies" (immunoglobulins) encompass monoclonal antibodies (including full-length monoclonal antibodies), polyclonal antibodies, multispecific antibodies (e.g., bispecific antibodies) formed from at least two intact antibodies, human antibodies, humanized antibodies, camelised antibodies, chimeric antibodies, single-chain Fvs (scFv), single-chain antibodies, single domain antibodies, domain antibodies, Fab fragments, F(ab')2 fragments, antibody fragments that exhibit the desired biological activity, disulfide-linked Fvs (sdFv), and anti-idiotypic (anti-Id) antibodies (including, e.g., anti-Id antibodies to antibodies of the disclosure), intrabodies, and epitope-binding fragments of any of the above. In particular, antibodies include immunoglobulin molecules, biologically active fragments of the disclosed molecules and immunologically active fragments of immunoglobulin molecules, i.e., molecules that contain an antigen-binding site. Immunoglobulin molecules can be of any type (e.g., IgG, IgE, IgM, IgD, IgA and IgY), class (e.g., IgG1, IgG2, IgG3, IgG4, IgA1 and IgA2) or subclass.

Native antibodies are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Light chains are classified as either *lambda* chains or *kappa* chains based on the amino acid sequence of the light chain constant region. The variable domain of a *kappa* light chain may also be denoted herein as VK. The term "variable region" may also be used to describe the variable domain of a heavy chain or light chain. Particular amino acid residues are believed to form an interface between the light and heavy chain variable domains. Such antibodies may be derived from any mammal, including, but not limited to, humans, monkeys, pigs, horses, rabbits, dogs, cats, mice, etc.

The term "variable" refers to the fact that certain portions of the variable domains differ extensively in sequence among antibodies and are responsible for the binding specificity of each particular antibody for its particular antigen. However, the variability is not evenly distributed through the variable domains of antibodies. It is concentrated in segments called Complementarity Determining Regions (CDRs) both in the light chain and the heavy chain variable domains. The more highly conserved portions of the variable domains are called the framework regions (FW). The variable domains of native heavy and light chains each comprise four FW regions, largely adopting a β-sheet configuration, connected by three CDRs, which form loops connecting, and in some cases forming part of, the β-sheet structure. The CDRs in each chain are held together in close proximity by the FW regions and, with the CDRs from the other chain, contribute to the formation of the antigen-binding site of antibodies (see, Kabat et al., Sequences of Proteins of Immunological Interest, 5th Ed. Public Health Service, National Institutes of Health, Bethesda, MD (1991)). The constant domains are generally not involved directly in antigen binding, but may influence antigen binding affinity and may exhibit various effector functions, such as participation of the antibody in ADCC, CDC, antibody-dependent phagocytosis and/or apoptosis.

The term "hypervariable region" when used herein refers to the amino acid residues of an antibody which are associated with its binding to antigen. The hypervariable regions encompass the amino acid residues of the "complementarity determining regions" or "CDRs" (e.g., residues 24-34 (L1), 50-56 (L2) and 89-97 (L3) of the light chain variable domain and residues 31-35 (H1), 50-65 (H2) and 95-102 (H3) of the heavy chain variable domain; Kabat et al., Sequences of Proteins of Immunological Interest, 5th Ed. Public Health Service, National Institutes of Health, Bethesda, MD (1991)) and/or those residues from a "hypervariable loop" (e.g., residues 26-32 (L1), 50-52 (L2) and 91-96 (L3) in the light chain variable domain and 26-32 (H1), 53-55 (H2) and 96-101 (H3) in the heavy chain variable domain; Chothia and Lesk, J. Mol. Biol., 196:901-917 (1987)). "Framework" or "FW" residues are those variable domain residues flanking the CDRs. FW residues are present in chimeric, humanized, human, domain antibodies, diabodies, vaccibodies, linear antibodies, and bispecific antibodies.

As used herein "Fc region" includes the polypeptides comprising the constant region of an antibody excluding the first constant region immunoglobulin domain. Thus Fc refers to the last two constant region immunoglobulin domains of IgA, IgD, and IgG, and the last three constant region immunoglobulin domains of IgE and IgM, and the flexible hinge N-terminal to these domains. For IgA and IgM Fc may include the J chain. For IgG, Fc comprises immunoglobulin domains Cgamma2 and Cgamma3 (Cy2 and Cy3) and the hinge between Cgamma1 (Cγ1) and Cgamma2 (Cy2). Although the boundaries of the Fc region may vary, the human IgG heavy chain Fc region is usually defined to comprise residues C226 or P230 to its carboxyl-terminus, wherein the numbering is according to the EU index as in Kabat et al. (1991, NIH Publication 91-3242, National Technical Information Service, Springfield, VA). The "EU index as set forth in Kabat" refers to the residue numbering of the human IgG1 EU antibody as described in Kabat et al. *supra.* Fc may refer to this region in isolation, or this region in the context of an antibody, antibody fragment, or Fc fusion protein. An Fc variant protein may be an antibody, Fc fusion, or any protein or protein domain that comprises an Fc region. Particularly preferred are proteins comprising variant Fc regions, which are non-naturally occurring variants of an Fc region. The amino acid sequence of a non-naturally occurring Fc region (also referred to herein as a "variant Fc region") comprises a substitution, insertion and/or deletion of at least one amino acid residue compared to the wild type amino acid sequence. Any new amino acid residue appearing in the sequence of a variant Fc region as a result of an insertion or substitution may be referred to as a non-naturally occurring amino acid residue. Note: Polymorphisms have been observed at a number of Fc positions, including but not limited to Kabat 270, 272, 312, 315, 356, and 358, and thus slight differences between the presented sequence and sequences in the prior art may exist.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, monoclonal antibodies are advantageous in that they can be synthesized by hybridoma cells that are uncontaminated by other immunoglobulin producing cells. Alternative production methods are known to those trained in the art, for example, a monoclonal antibody may be produced by cells stably or transiently transfected with the heavy and light chain genes encoding the monoclonal antibody.

The modifier "monoclonal" indicates the character of the antibody as being obtained from a substantially homogeneous population of antibodies, and is not to be construed as requiring engineering of the antibody by any particular method. The term "monoclonal" is used herein to refer to an antibody that is derived from a clonal population of cells, including any eukaryotic, prokaryotic, or phage clone, and not the method by which the antibody was engineered. For example, the monoclonal antibodies to be used in accordance with the present disclosure may be made by the hybridoma method first described by Kohler et al., Nature, 256:495 (1975), or may be made by any recombinant DNA method (see, e.g., U.S. Patent No. 4,816,567), including isolation from phage antibody libraries using the techniques described in Clackson et al., Nature, 352:624-628 (1991) and Marks et al., J. Mol. Biol., 222:581-597 (1991), for example. These methods can be used to produce monoclonal mammalian, chimeric, humanized, human, domain antibodies, diabodies, vaccibodies, linear antibodies, and bispecific antibodies.

A "human antibody" can be an antibody derived from a human or an antibody obtained from a transgenic organism that has been "engineered" to produce specific human antibodies in response to antigenic challenge and can be produced by any method known in the art. In certain techniques, elements of the human heavy and light chain loci are introduced into strains of the organism derived from embryonic stem cell lines that contain targeted disruptions of the endogenous heavy chain and light chain loci. The transgenic organism can synthesize human antibodies specific for human antigens, and the organism can be used to produce human antibody-secreting hybridomas. A human antibody can also be an antibody wherein the heavy and light chains are encoded by a nucleotide sequence derived from one or more sources of human DNA. A fully human antibody also can be constructed by genetic or chromosomal transfection methods, as well as phage display technology, or in vitro activated ICOS expressing T cells, all of which are known in the art.

"Antibody-dependent cell-mediated cytotoxicity" and "ADCC" refer to a cell-mediated reaction in which non-specific cytotoxic cells (e.g., Natural Killer (NK) cells, neutrophils, and macrophages) recognize bound antibody on a target cell and subsequently cause lysis of the target cell. In one embodiment, such cells are human cells. While not wishing to be limited to any particular mechanism of action, these cytotoxic cells that mediate ADCC generally express Fc receptors (FcRs). The primary cells for mediating ADCC, NK cells, express FcγRIII, whereas monocytes express FcγRI, FcγRII, FcγRIII and/or FcγRIV. FcR expression on hematopoietic cells is summarized in Ravetch and Kinet, Annu. Rev. Immunol., 9:457-92 (1991). To assess ADCC activity of a molecule, an in vitro ADCC assay, such as that described in U.S. Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecules of interest may be assessed in vivo, e.g., in an animal model such as that disclosed in Clynes et al., Proc. Natl. Acad. Sci. (USA), 95:652-656 (1998).

"Complement dependent cytotoxicity" or "CDC" refers to the ability of a molecule to initiate complement activation and lyse a target in the presence of complement. The complement activation pathway is initiated by the binding of the first component of the complement system (C1q) to a molecule (e.g., an antibody) complexed with a cognate antigen. To assess complement activation, a CDC assay, e.g., as described in Gazzano-Santaro et al., J. Immunol. Methods, 202:163 (1996), may be performed.

"Antibody-dependent phagocytosis" or "opsonization" as used herein refers to the cell-mediated reaction wherein nonspecific cytotoxic cells that express FcγRs recognize bound antibody on a target cell and subsequently cause phagocytosis of the target cell.

"Effector cells" are leukocytes which express one or more FcRs and perform effector functions. The cells express at least FcγRI, FCγRII, FcγRIII and/or FcγRIV and carry out ADCC effector function. Examples of human leukocytes which mediate ADCC include peripheral blood mononuclear cells (PBMC), natural killer (NK) cells, monocytes, cytotoxic T cells and neutrophils.

The terms "Fc receptor" or "FcR" are used to describe a receptor that binds to the Fc region of an antibody. In one embodiment, the FcR is a native sequence human FcR. Moreover, in certain embodiments, the FcR is one which binds an IgG antibody (a gamma receptor) and includes receptors of the FcγRI, FcγRII, FcγRIII, and FcγRIV subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (See, Daëron, Annu. Rev. Immunol., 15:203-234 (1997)). FcRs are reviewed in Ravetch and Kinet, Annu. Rev. Immunol., 9:457-92 (1991); Capel et al., Immunomethods, 4:25-34 (1994); and de Haas et al., J. Lab. Clin. Med., 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., Immunol., 117:587 (1976) and Kim et al., J. Immunol., 24:249 (1994)).

"Affinity" of an antibody for an epitope to be used in the treatment(s) described herein is a term well understood in the art and means the extent, or strength, of binding of antibody to epitope. Affinity may be measured and/or expressed in a number of ways known in the art, including, but not limited to, equilibrium dissociation constant (KD or Kd), apparent equilibrium dissociation constant (KD' or Kd'), and IC50 (amount needed to effect 50% inhibition in a competition assay). It is understood that, for purposes of this disclosure, an affinity is an average affinity for a given population of antibodies which bind to an epitope. Values of KD' reported herein in terms of mg IgG per mL or mg/mL indicate mg Ig per mL of serum, although plasma can be used. When antibody affinity is used as a basis for administration of the treatment methods described herein, or selection for the treatment methods described herein, antibody affinity can be measured before and/or during treatment, and the values obtained can be used by a clinician in assessing whether a human patient is an appropriate candidate for treatment.

As used herein, the term "avidity" is a measure of the overall binding strength (i.e., both antibody arms) with which an antibody binds an antigen. Antibody avidity can be determined by measuring the dissociation of the antigen-antibody bond in antigen excess using any means known in the art, such as, but not limited to, by the modification of indirect fluorescent antibody as described by Gray et al., J. Virol. Meth., 44:11-24. (1993).

An "epitope" is a term well understood in the art and means any chemical moiety that exhibits specific binding to an antibody. An "antigen" is a moiety or molecule that contains an epitope, and, as such, also specifically binds to antibody.

The term "antibody half-life" as used herein means a pharmacokinetic property of an antibody that is a measure of the mean survival time of antibody molecules following their administration. Antibody half-life can be expressed as the time required to eliminate 50 percent of a known quantity of immunoglobulin from the patient's body or a specific compartment thereof, for example, as measured in serum or plasma, i.e., circulating half-life, or in other tissues. Half-life may vary from one immunoglobulin or class of immunoglobulin to another. In general, an increase in antibody half-life results in an increase in mean residence time (MRT) in circulation for the antibody administered.

The term "isotype" refers to the classification of an antibody's heavy or light chain constant region. The constant domains of antibodies are not involved in binding to antigen, but exhibit various effector functions. Depending on the amino acid sequence of the heavy chain constant region, a given human antibody or immunoglobulin can be assigned to one of five major classes of immunoglobulins: IgA, IgD, IgE, IgG, and IgM. Several of these classes may be further divided into subclasses (isotypes), e.g., IgG1 (gamma 1), IgG2 (gamma 2), IgG3 (gamma 3), and IgG4 (gamma 4), and IgA1 and IgA2. The heavy chain constant regions that correspond to the different classes of immunoglobulins are called $\alpha, \delta, \varepsilon, \gamma,$ and $\mu,$ respectively. The structures and three-dimensional configurations of different classes of immunoglobulins are well-known. Of the various human immunoglobulin classes, only human IgG1, IgG2, IgG3, IgG4, and IgM are known to activate complement. Human IgG1 and IgG3 are known to mediate ADCC in humans. Human light chain constant regions may be classified into two major classes, kappa and lambda

As used herein, the term "immunogenicity" means that a compound is capable of provoking an immune response (stimulating production of specific antibodies and/or proliferation of specific T cells).

As used herein, the term "antigenicity" means that a compound is recognized by an antibody or may bind to an antibody and induce an immune response.

The term "excipient" as used herein refers to an inert substance which is commonly used as a diluent, vehicle, preservative, binder or stabilizing agent for drugs which imparts a beneficial physical property to a formulation, such as increased protein stability, increased protein solubility, and decreased viscosity. Examples of excipients include, but are not limited to, proteins (for example, but not limited to, serum albumin), amino acids (for example, but not limited to, aspartic acid, glutamic acid, lysine, arginine, glycine), surfactants (for example, but not limited to, SDS, Tween 20, Tween 80, polysorbate and nonionic surfactants), saccharides (for example, but not limited to, glucose, sucrose, maltose and trehalose), polyols (for example, but not limited to, mannitol and sorbitol), fatty acids and phospholipids (for example, but not limited to, alkyl sulfonates and caprylate). For additional information regarding excipients, see Remington's Pharmaceutical Sciences (by Joseph P. Remington, 18th ed., Mack Publishing Co., Easton, PA).

The phrase "pharmaceutically acceptable" as used herein means approved by a regulatory agency of the Federal or a state government, or listed in the U.S. Pharmacopeia, European Pharmacopia or other generally recognized pharmacopeia for use in animals, and more particularly in humans.

The terms "stability" and "stable" as used herein in the context of a liquid formulation comprising an antibody (including antibody fragment thereof) that specifically binds to an antigen of interest (*e.g.,* ICOS) refer to the resistance of the antibody (including antibody fragment thereof) in the formulation to aggregation, degradation or fragmentation under given manufacture, preparation, transportation and storage conditions. The "stable" formulations of the disclosure retain biological activity under given manufacture, preparation, transportation and storage conditions. The stability of said antibody (including antibody fragment thereof) can be assessed by degrees of aggregation, degradation or fragmentation, as measured by HPSEC, reverse phase chromatography, static light scattering (SLS), Dynamic Light Scattering (DLS), Fourier Transform Infrared Spectroscopy (FTIR), circular dichroism (CD), urea unfolding techniques, intrinsic tryptophan fluorescence, differential scanning calorimetry, and/or ANS binding techniques, compared to a reference formulation. For example, a reference formulation may be a reference standard frozen at -70°C consisting of 10 mg/ml of an antibody (including antibody fragment thereof) (for example, but not limited to, an antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain) in 10 mM histidine, pH 6.0-6.5 that contains 80 mM NaCl, 4% trehalose and 0.02% polysorbate 80, which reference formulation regularly gives a single monomer peak (e.g., $\geq$ 97% area) by HPSEC. The overall stability of a formulation comprising an antibody (including antibody fragment thereof) can be assessed by various immunological assays including, for example, ELISA and radioimmunoassay using isolated antigen molecules.

The phrase "low to undetectable levels of aggregation" as used herein refers to samples containing no more than about 5%, no more than about 4%, no more than about 3%, no more than about 2%, no more than about 1% and no more than about 0.5% aggregation by weight of protein as measured by high performance size exclusion chromatography (HPSEC) or static light scattering (SLS) techniques.

The term "low to undetectable levels of fragmentation" as used herein refers to samples containing equal to or more than about 80%, about 85%, about 90%, about 95%, about 98% or about 99% of the total protein, for example, in a single peak as determined by HPSEC or reverse phase chromatography, or in two peaks (e.g., heavy- and light-chains) (or as many peaks as there are subunits) by reduced Capillary Gel Electrophoresis (rCGE), representing the non-degraded antibody or a non-degraded fragment thereof, and containing no other single peaks having more than about 5%, more than about 4%, more than about 3%, more than about 2%, more than about 1%, or more than about 0.5% of the total protein in each. The term "reduced Capillary Gel Electrophoresis" as used herein refers to capillary gel electrophoresis under reducing conditions sufficient to reduce disulfide bonds in an antibody.

As used herein, the terms "disorder" and "disease" are used interchangeably to refer to a condition in a subject in which the subject differs from a healthy, unaffected subject. In particular, the term "autoimmune disease" is used interchangeably with the term "autoimmune disorder" to refer to a condition in a subject characterized by cellular, tissue and/or organ injury caused by an immunologic reaction of the subject to its own cells, tissues and/or organs. The term "inflammatory disease" is used interchangeably with the term "inflammatory disorder" to refer to a condition in a subject characterized by inflammation, for example, but not limited to, chronic inflammation. Autoimmune disorders may or may not be associated with inflammation. Moreover, inflammation may or may not be caused by an autoimmune disorder. Certain conditions may be characterized as more than one disorder. For example, certain conditions may be characterized as both autoimmune and inflammatory disorders.

The terms "therapies" and "therapy" can refer to any protocol(s), method(s), and/or agent(s) that can be used in the prevention, treatment and/or management of a disease or disorder.

By the terms "treat," "treating" or "treatment of' (or grammatically equivalent terms) it is meant that the severity of the subject's condition is reduced or at least partially improved or ameliorated and/or that some alleviation, mitigation or decrease in at least one clinical symptom is achieved and/or there is an inhibition or delay in the progression of the condition and/or prevention or delay of the onset of a disease or illness. Thus, the terms "treat," "treating" or "treatment of' (or grammatically equivalent terms) refer to both prophylactic and therapeutic treatment regimes.

As used herein, the terms "manage," "managing," and "management" refer to the beneficial effects that a subject derives from a therapy (*e.g.*, a prophylactic or therapeutic agent), which does not result in a cure of the disease. In certain embodiments, a subject is administered one or more therapies (*e.g.*, one or more prophylactic or therapeutic agents) to "manage" a disease so as to prevent the progression or worsening of the disease.

As used herein, the terms "prevent," "preventing," and "prevention" refer to the inhibition of the development or onset of disease or disorder, or the prevention of the recurrence, onset, or development of one or more symptoms of a disease or disorder in a subject resulting from the administration of a therapy (*e.g.,* a prophylactic or therapeutic agent), or the administration of a combination of therapies (*e.g.,* a combination of prophylactic or therapeutic agents).

As used herein, the terms "prophylactic agent" and "prophylactic agents" refer to any agent(s) which can be used in the prevention of the onset, recurrence or development of a disease or disorder. In certain embodiments, the term "prophylactic agent" refers to an antibody that specifically binds to human ICOS. In certain other embodiments, the term "prophylactic agent" refers to an agent other than an antibody that specifically binds to human ICOS. In certain embodiments, a prophylactic agent is an agent which is known to be useful to or has been or is currently being used to prevent or impede the onset, development, progression and/or severity of a disease or disorder.

As used herein, the term "immunomodulatory agent" and variations thereof including, but not limited to, immunomodulatory agents, immunomodulants or immunomodulatory drugs, refer to an agent that modulates a host's immune system. In a specific embodiment, an immunomodulatory agent is an agent that shifts one aspect of a subject's immune response. In certain embodiments, an immunomodulatory agent is an agent that inhibits or reduces a subject's immune system (i.e., an immunosuppressant agent). In certain other embodiments, an immunomodulatory agent is an agent that activates or increases a subject's immune system (i.e., an immunostimulatory agent). In accordance with the disclosure, an immunomodulatory agent used in the combination therapies of the disclosure does not include an antibody of the disclosure. Immunomodulatory agents include, but are not limited to, small molecules, peptides, polypeptides, proteins, nucleic acids (for example, but not limited to, DNA and RNA nucleotides including, but not limited to, antisense nucleotide sequences, triple helices, RNAi, and nucleotide sequences encoding biologically active proteins, polypeptides or peptides), antibodies, synthetic or natural inorganic molecules, mimetic agents, and synthetic or natural organic molecules.

As used herein, a "sufficient amount" or "an amount sufficient to" achieve a particular result refers to an amount of an antibody or composition of the disclosure that is effective to produce a desired effect, which is optionally a therapeutic effect (*i.e.,* by administration of a therapeutically effective amount). For example, a "sufficient amount" or "an amount sufficient to" can be an amount that is effective to deplete ICOS expressing T cells.

A "therapeutically effective" amount as used herein is an amount that provides some improvement or benefit to the subject. Stated in another way, a "therapeutically effective" amount is an amount that provides some alleviation, mitigation, and/or decrease in at least one clinical symptom. Clinical symptoms associated with the disorders that can be treated by the methods of the disclosure are well-known to those skilled in the art. Further, those skilled in the art will appreciate that the therapeutic effects need not be complete or curative, as long as some benefit is provided to the subject.

A "therapeutically effective dosage" of an anti-ICOS antibody of the disclosure results in a decrease in severity of at least one disease symptom, an increase in frequency and duration of disease symptom-free periods, or a prevention of impairment or disability due to the disease affliction. For example, in the case of systemic lupus erythematosus (SLE), a therapeutically effective dose prevents further deterioration of at least one physical symptom associated with SLE, such as, for example, pain or fatigue. A therapeutically effective dose also prevents or delays onset of SLE, such as may be desired when early or preliminary signs of the disease are present. Likewise it includes delaying chronic progression associated with SLE. Laboratory tests utilized in the diagnosis of SLE include chemistries, hematology, serology and radiology. Accordingly, any clinical or biochemical assay that monitors any of the foregoing may be used to determine whether a particular treatment is a therapeutically effective dose for treating SLE. One of ordinary skill in the art would be able to determine such amounts based on such factors as the subject's size, the severity of the subject's symptoms, and the particular composition or route of administration selected.

As used herein, the term "subject" includes any human or nonhuman animal. The term "nonhuman animal" includes all vertebrates, for example, but not limited to, mammals and non-mammals, such as nonhuman primates, sheep, dogs, cats, horses, cows, chickens, amphibians, reptiles, etc.

As used herein, the terms "non-responsive" and refractory" describe patients treated with a currently available therapy (e.g., prophylactic or therapeutic agent) for a disease or disorder. Such patients likely suffer from severe, persistently active disease and require additional therapy to ameliorate the symptoms associated with the disorder.

Concentrations, amounts, cell counts, percentages and other numerical values may be presented herein in a range format. It is also to be understood that such range format is used merely for convenience and brevity and should be interpreted flexibly to include not only the numerical values explicitly recited as the limits of the range but also to include all the individual numerical values or sub-ranges encompassed within that range as if each numerical value and sub-range is explicitly recited.

## 4. BRIEF DESCRIPTION OF THE DRAWINGS

For the purpose of illustrating representative embodiments of the disclosure, drawings are provided herein.

**Figure 1.** DSC profile of the 136 anti-ICOS antibody in 25 mM histidine (pH 6.0).

**Figure 2.** Effect of pH on thermal stability of the 136 anti-ICOS antibody. Tryptophan fluorescence intensity profiles (measured at 330 nm) as a function of temperature are shown. Tryptophan fluorescence intensity profile measurements were performed at various pHs.

**Figure 3.** pH dependence of the colloidal stability of anti-ICOS formulations. The 350nm absorption of formulations with various pHs as a function of temperature is shown.

**Figure 4.** Schematics of the use of colloidal stability measurement for excipient screening.

**Figure 5.** Single excipient screening: Effect of polysorbate, trehalose, sucrose and lysine on colloidal stability of 136 formulations.

**Figure 6.** Single excipient screening: Effect of increasing NaCl concentration on colloidal stability of 136 formulations.

**Figure 7.** Single excipient screening: Effect of increasing NaCl or arginine concentration on colloidal stability of 136 formulations.

**Figure 8.** Excipient screening: Effect of the combination of trehalose and arginine on colloidal stability of 136 formulations.

**Figure 9.** Stability of 136 anti-ICOS antibody formulations. The stability of the antibody formulations was ascertained by SEC. Chart displays the percent (%) monomer content of the formulation, as determined by SEC, after storage at 40°C.

**Figure 10.** Stability of 136 anti-ICOS antibody formulations. The stability of the antibody formulations comprising 90 mg/ml 136, 10 mM histidine (pH 6.0), 4% trehalose and either 80mM NaCl (A) or 100 mM arginine HCl (B) was ascertained by SEC. The formulations were stored at 40°C for 21 days prior to performing SEC analysis. SEC protein elution profiles are shown.

**Figure 11.** Effect of polysorbate 80 on the stability of 136 anti-ICOS antibody formulations. The stability of 136 formulations (105 mg/ml 136, 10 mM histidine (pH 6.0), 80 mM NaCl) comprising 0 %, 0.02 % or 0.05 % polysorbate 80 was ascertained following storage at 40°C. Chart displays the percent (%) monomer content of the formulation, as determined by SEC, at various time points.

**Figure 12.** Effect of polysorbate 80 on the stability of 136 anti-ICOS antibody formulations. The stability of 136 formulations (105 mg/ml 136, 10 mM histidine (pH 6.0), 80 mM NaCl) comprising 0 %, 0.02 % or 0.05 % polysorbate 80 was ascertained following storage at 40°C. Chart displays the percent (%) fragment content of the formulation, as determined by SEC, at various time points.

**Figure 13.** Effect of polysorbate 80 on the stability of 136 anti-ICOS antibody formulations. The stability of 136 formulations (105 mg/ml 136, 10 mM histidine (pH 6.0), 80 mM NaCl) comprising 0 %, 0.02 % or 0.05 % polysorbate 80 was ascertained following storage at 40°C. Chart displays the percent (%) dimer content of the formulation, as determined by SEC, at various time points.

**Figure 14.** Stability of a 136 anti-ICOS antibody formulation stored at 2-8, 25 or 40°C. The stability of the 136 formulation comprising 105 mg/ml 136, 10 mM histidine (pH 6.0), 80 mM NaCl and 0.02 % polysorbate 80 was ascertained following storage at 2-8, 25 or 40°C. Chart displays the percent (%) monomer content of the formulation, as determined by SEC, at various time points.

**Figure 15.** A) BIAcore binding affinity of the fucosylated and afucosylated anti-ICOS MAb to mouse FcgRIV. B) Immuno-phenotype characterization in the steady state of ICOS expression on splenic naive and T helper memory cells (central and effector). C) Fucose free anti-ICOS MAb (IgG2a-aFuc) mediates more effective depletion of ICOS bearing T cells. Pharmacodynamic analysis of splenic helper central and effector memory ICOS bearing T cells upon one single intraperitoneal injection of the indicated anti-ICOS MAbs into naive Balb/c mice (250 μg/animal).

**Figure 16.** Anti-ICOS MAb (IgG2a-aFuc) reduces graft versus host scleroderma clinical score. Mean clinical disease score following biweekly treatment (starting time: day 8) with anti-mouse ICOS IG2a-aFuc or isotype control MAb (n=10) is shown. Baseline skin scores measurements were obtained on study day 6. (*p<0.05, **p<0.005)

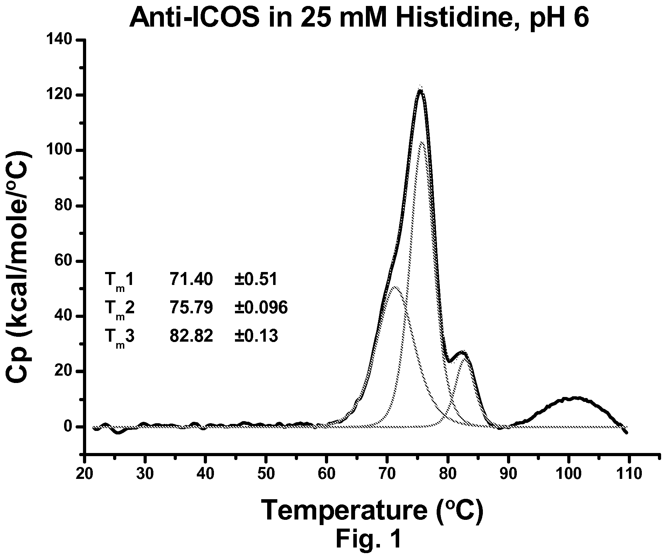

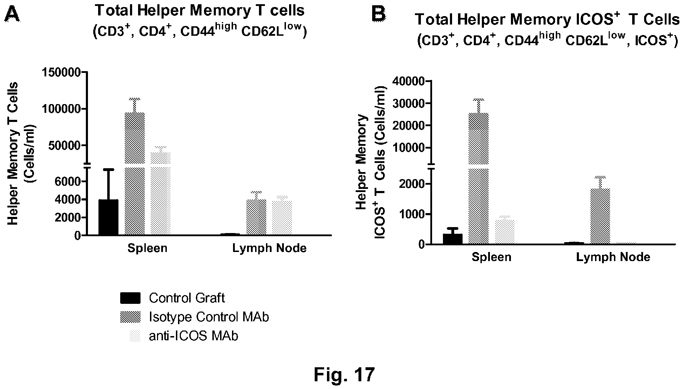

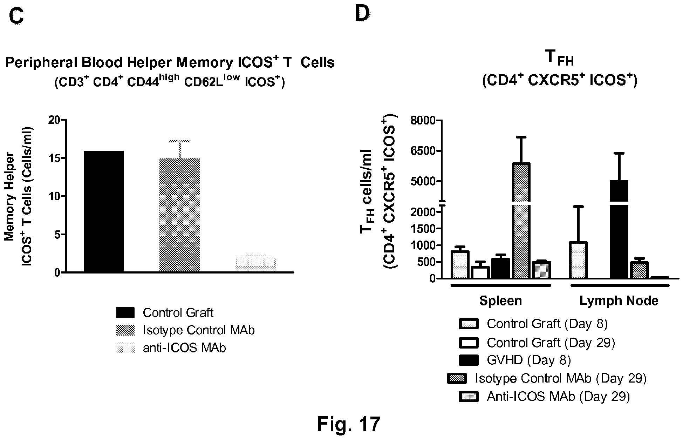

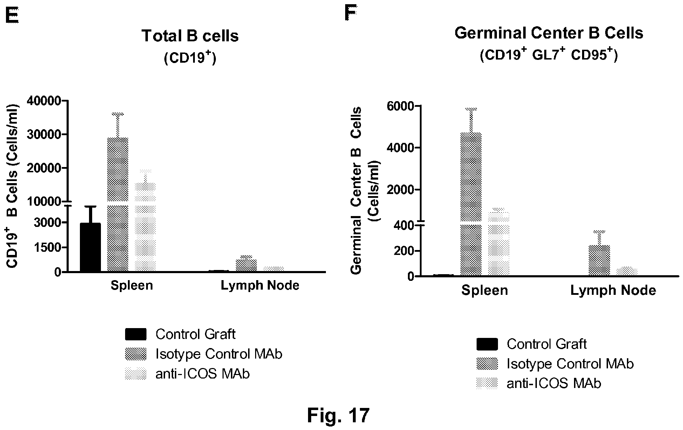

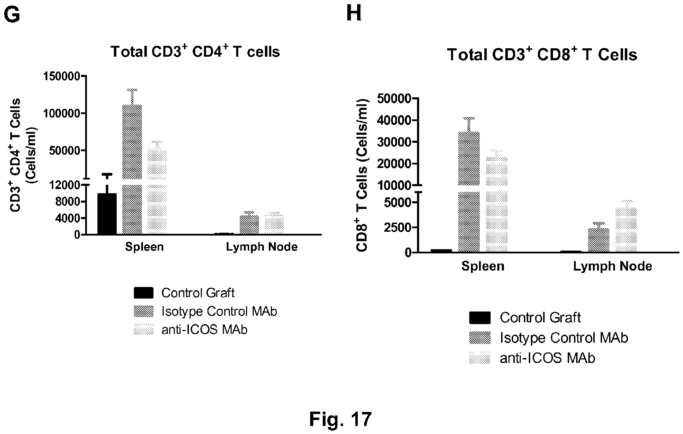

**Figure 17.** Anti-ICOS MAb mediates effective elimination of ICOS bearing TFH and inhibits the expansion of germinal center B cells. Immunophenotype analysis of spleen, lymph node and peripheral blood Th memory (A) and Th memory ICOS+ cells (B, C) (gated as indicated in Fig.1C) isolated from Balb/c control mice and from rag2 deficient mice treated with either anti-ICOS or isotype control MAb. D) Anti-ICOS therapy prevents the expansion of TFH cells. While anti-ICOS MAb does not alter the overall number of total splenic B cells (CD19+) (E), it significantly inhibits the TFH-mediated expansion of germinal center B cells (F). Depletion of ICOS bearing T cells does not perturb the overall CD4+ (G) and CD8+ (H) T cell compartments.

**Figure 18.** Histology of RAG2-/- spleen and kidney from an isotype control MAb treated animal (A, E,) and anti-ICOS MAb treated animal (C). Higher magnification (x200) of the spleen demonstrates lack of germinal center formation in anti-ICOS-treated animals (D) compared to the isotype (B). Original magnification, x100; inset x1000.

**Figure 19.** Treatment with anti-ICOS MAb significantly inhibits the GvHD-SSc skin pathology. Histology of back skin from either Balb/c (A, B), or RAG2-/- mice grafted with splenocytes at 4 weeks from isotype control MAb group (C, D) and anti-ICOS MAb treated group (E, F) is shown. Tissue sections were stained with either hematoxylin and eosin stain (top row) or Masson's Trichrome stain (bottom row). Original magnifications, x200.

**Figure 20.** ICOS MAb treatment impacts T helper- and TFH-associated genes and the autoimmune-gene fingerprint in the skin.

**Figure 21.** Effect of concentration on Hydrodynamic Diameter of the 136 anti-ICOS antibody. In the figure closed triangle represents data obtained with the 136 anti-ICOS antibody and closed circle represents data obtained with a non interacting monoclonal antibody (mAbB).

**Figure 22.** Effect of sodium chloride concentration on the 136 anti-ICOS antibody RSA at 23°C (closed circle) and 37°C (closed triangle).

**Figure 23.** Effect of pH on the 136 anti-ICOS antibody RSA. Data obtained with a control non-interacting antibody (mAbB) is also shown.

**Figure 24.** Effect of temperature on the 136 anti-ICOS antibody RSA. mAbB is a non-interacting control antibody.

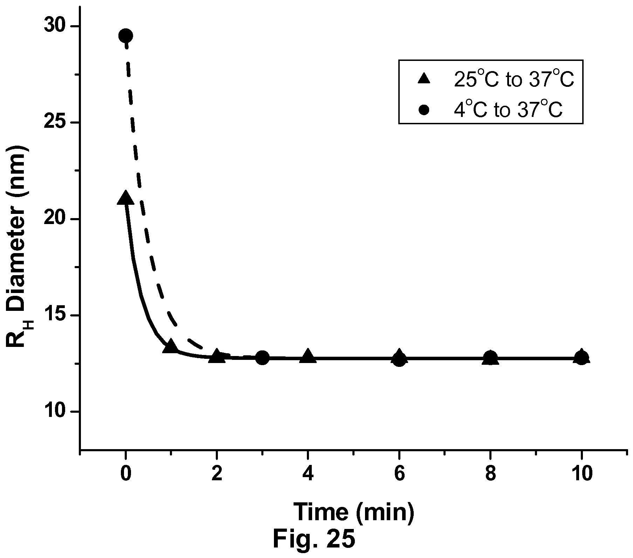

**Figure 25.** Effect of Temperature on the 136 anti-ICOS antibody Dissociation Kinetics.

## 5. DETAILED DESCRIPTION

Characterization of the physico-chemical properties of the 136 anti-ICOS antibody led to the surprising discovery that the antibody undergoes reversible self-association in solution. The observed reversible self-association of the 136 antibody is unique in that it does not lead to aggregate formation. Because of the self-association, a significant fraction of the 136 antibody exists as a trimer in solution. Additional experimental work demonstrated that the equilibrium between the monomer and trimer form of 136 in solution is influenced by antibody concentration, temperature, ionic strength and pH. For example, at least 10 mole percent of the 136 antibody exists as a trimer in PBS at 10 mg/ml antibody concentration at 37°C. Described herein are stable liquid formulations comprising an antibody that specifically binds human ICOS and undergoes reversible self-association in solution.

The present disclosure relates to stable liquid formulations of antibodies or fragments thereof that specifically bind to ICOS, undergo reversible self-association in solution and have an enhanced effector function (e.g., antibody-dependent cellular cytotoxicity (ADCC), complement-dependent cell-mediated cytotoxicity (CDC), and/or antibody-dependent phagocytosis). In certain embodiments, a stable liquid formulation of an anti-human ICOS antibody or a fragment thereof is suitable for parenteral administration to a human subject. In a specific embodiment, a stable liquid formulation of the disclosure is suitable for subcutaneous administration to a human subject.

The present disclosure relates to sterile, stable aqueous formulations comprising an antibody or fragment thereof that specifically binds human ICOS, has enhanced effector functions and undergoes reversible self-association in solution. The present disclosure provides a formulation of an anti-ICOS antibody described in US Patent Application 12/116,512. In a specific embodiment, a formulation of the disclosure comprises an anti-human ICOS antibody comprising an Fc region having complex N-glycoside-linked sugar chains in which fucose is not bound to N-acetylglucosamine in the reducing end in the sugar chain. In another embodiment, a formulation of the disclosure comprises an anti-human ICOS antibody comprising a heavy chain sequence of SEQ ID NO: 6 and a light chain sequence of SEQ ID NO: 1. In a further embodiment, a formulation described herein comprises an anti-human ICOS antibody that undergoes reversible self-association in solution, wherein at least 10 mole percent of the antibody exists as a trimer in PBS at 10 mg/ml antibody concentration at 37°C, and wherein the reversible self-association does not induce aggregate formation. In one embodiment, a formulation of the disclosure is provided in a pre-filled syringe.

The present disclosure provides methods of stabilizing an anti-human ICOS antibody or fragment thereof.

The present disclosure further relates to processes of making a sterile, stable aqueous formulation comprising an antibody or fragment thereof that specifically binds human ICOS.

The present disclosure also encompasses methods of preventing, managing, treating or ameliorating an inflammatory disease or disorder, an autoimmune disease or disorder, a proliferative disease, a T cell proliferative disease, an infection, a disease or disorder associated with or characterized by aberrant expression and/or activity of ICOS, a disease or disorder associated with or characterized by aberrant expression and/or activity of the ICOS receptor, or one or more symptoms thereof, said methods comprising administering to a subject in need thereof a prophylactically or therapeutically effective amount of an anti-human ICOS antibody formulation. The present disclosure also relates to methods of treating or preventing T cell-mediated diseases and disorders, such as, but not limited to, chronic infection, autoimmune disease or disorder, inflammatory disease or disorder, graft-versus-host disease (GVHD), transplant rejection, and T cell proliferative disorder using formulations comprising anti-ICOS antibodies with enhanced effector function.

**5.1.** Antibody Formulations

In specific embodiments, the present disclosure encompasses stable liquid formulations of antibodies that specifically bind to human ICOS, undergo reversible self-association in solution and have an enhanced effector function (e.g., antibody-dependent cellular cytotoxicity (ADCC), complement-dependent cell-mediated cytotoxicity (CDC), and/or antibody-dependent phagocytosis), wherein the formulations exhibit low to undetectable levels of antibody aggregation and/or fragmentation with very little to no loss of the biological activities during manufacture, preparation, transportation, and long periods of storage. The present disclosure also encompasses stable liquid formulations of antibodies that specifically bind to human ICOS, undergo reversible self-association in solution, have an enhanced effector function and have increased *in vivo* half-lives, said formulations exhibiting low to undetectable levels of antibody aggregation and/or fragmentation, and very little to no loss of the biological activities of the antibodies during manufacture, preparation, transportation, and long periods of storage. In specific embodiments, a formulation of the disclosure comprises an anti-human ICOS antibody having increased *in vivo* ADCC activity, said formulation exhibiting low to undetectable levels of antibody aggregation and/or fragmentation, and very little to no loss of the biological activities of the antibodies during manufacture, preparation, transportation, and long periods of storage.

In one embodiment, a liquid formulation of the disclosure is an aqueous formulation. In a specific embodiment, a liquid formulation of the disclosure is an aqueous formulation wherein the aqueous carrier is distilled water.

In one embodiment, a formulation of the disclosure is sterile.

In one embodiment, a formulation of the disclosure is homogeneous.

In one embodiment, a formulation of the disclosure is isotonic.

The present disclosure provides stable high concentration liquid formulations comprising an anti-ICOS antibody having an enhanced effector function. In one embodiment, a formulation of the disclosure comprises an anti-ICOS antibody described in US Patent Application 12/116,512.

In one embodiment, a formulation of the disclosure comprises an anti-ICOS antibody or a fragment thereof, wherein said antibody or a fragment thereof comprises a VH domain having the amino acid sequence of SEQ ID NO:7 and a VL domain having the amino acid sequence of SEQ ID NO:2. In a specific embodiment, a formulation of the disclosure comprises an anti-ICOS antibody comprising a heavy chain having the amino acid sequence of SEQ ID NO:6 and a light chain having the amino acid sequence of SEQ ID NO:1. In a specific embodiment, a formulation of the disclosure comprises an anti-human ICOS antibody comprising an Fc region having complex N-glycoside-linked sugar chains in which fucose is not bound to N-acetylglucosamine in the reducing end in the sugar chain.

The disclosure encompasses stable liquid formulations comprising a single antibody of interest (including antibody fragment thereof), for example, an antibody that specifically binds to an ICOS polypeptide The disclosure also encompasses stable liquid formulations comprising two or more antibodies of interest (including antibody fragments thereof), for example, antibodies that specifically bind to an ICOS polypeptide(s).

In one embodiment, a formulation of the disclosure comprises at least about 1 mg/ml, at least about 5 mg/ml, at least about 10 mg/ml, at least about 20 mg/ml, at least about 30 mg/ml, at least about 40 mg/ml, at least about 50 mg/ml, at least about 60 mg/ml, at least about 70 mg/ml, at least about 80 mg/ml, at least about 90 mg/ml, at least about 100 mg/ml, at least about 110 mg/ml, at least about 120 mg/ml, at least about 130 mg/ml, at least about 140 mg/ml, at least about 150 mg/ml, at least about 160 mg/ml, at least about 170 mg/ml, at least about 180 mg/ml, at least about 190 mg/ml, at least about 200 mg/ml, at least about 250 mg/ml, or at least about 300 mg/ml of an anti-ICOS antibody or a fragment thereof. In a specific embodiment, a formulation of the disclosure comprises at least about 5 mg/ml of an anti-ICOS antibody of a fragment thereof. In a specific embodiment, a formulation of the disclosure comprises at least about 10 mg/ml of an anti-ICOS antibody of a fragment thereof. In a specific embodiment, a formulation of the disclosure comprises at least about 15 mg/ml of an anti-ICOS antibody of a fragment thereof. In a specific embodiment, a formulation of the disclosure comprises at least about 100 mg/ml of an anti-ICOS antibody of a fragment thereof. In a specific embodiment, a formulation of the disclosure comprises at least about 125 mg/ml of an anti-ICOS antibody of a fragment thereof. In a specific embodiment, a formulation of the disclosure comprises at least about 130 mg/ml of an anti-ICOS antibody of a fragment thereof. In a specific embodiment, a formulation of the disclosure comprises at least about 150 mg/ml of an anti-ICOS antibody of a fragment thereof. In a specific embodiment, a formulation of the disclosure comprises at least about 90 mg/ml of an anti-ICOS antibody of a fragment thereof. In another embodiment, a formulation of the disclosure comprises between about 1 mg/ml and about 20 mg/ml, between about 5 mg/ml and about 20 mg/ml, between about 1 mg/ml and about 25 mg/ml, between about 1 mg/ml and about 200 mg/ml, between about 25 mg/ml and about 200 mg/ml, between about 50 mg/ml and about 200 mg/ml, between about 75 mg/ml and about 200 mg/ml, between about 100 mg/ml and about 200 mg/ml, between about 125 mg/ml and about 200 mg/ml, between about 150 mg/ml and about 200 mg/ml, between about 25 mg/ml and about 150 mg/ml, between about 50 mg/ml and about 150 mg/ml, between about 75 mg/ml and about 150 mg/ml, between about 100 mg/ml and about 150 mg/ml, between about 125 mg/ml and about 150 mg/ml, between about 25 mg/ml and about 125 mg/ml, between about 50 mg/ml and about 125 mg/ml, between about 75 mg/ml and about 125 mg/ml, between about 100 mg/ml and about 125 mg/ml, between about 25 mg/ml and about 100 mg/ml, between about 50 mg/ml and about 100 mg/ml, between about 75 mg/ml and about 100 mg/ml, between about 25 mg/ml and about 75 mg/ml, between about 50 mg/ml and about 75 mg/ml, or between about 25 mg/ml and about 50 mg/ml of an anti-ICOS antibody or a fragment thereof. In a specific embodiment, a formulation of the disclosure comprises between about 5 mg/ml and about 20 mg/ml of an anti-ICOS antibody or a fragment thereof. In a specific embodiment, a formulation of the disclosure comprises between about 90 mg/ml and about 110 mg/ml of an anti-ICOS antibody or a fragment thereof. In a specific embodiment, a formulation of the disclosure comprises between about 100 mg/ml and about 210 mg/ml of an anti-ICOS antibody or a fragment thereof. In a further embodiment, a formulation described herein comprises about 1 mg/ml, about 2 mg/ml, about 3 mg/ml, about 4 mg/ml, about 5 mg/ml, about 10 mg/ml, about 15 mg/ml, about 20 mg/ml, about 30 mg/ml, about 40 mg/ml, about 50 mg/ml, about 60 mg/ml, about 70 mg/ml, about 80 mg/ml, about 90 mg/ml, about 100 mg/ml, about 110 mg/ml, about 120 mg/ml, about 130 mg/ml, about 140 mg/ml, about 150 mg/ml, about 160 mg/ml, about 170 mg/ml, about 180 mg/ml, about 190 mg/ml, about 200 mg/ml, about 250 mg/ml, or about 300 mg/ml of an anti-ICOS antibody or a fragment thereof. In a specific embodiment, a formulation of the disclosure comprises about 5 mg/ml of an anti-ICOS antibody or a fragment thereof. In a specific embodiment, a formulation of the disclosure comprises about 10 mg/ml of an anti-ICOS antibody or a fragment thereof. In a specific embodiment, a formulation of the disclosure comprises about 15 mg/ml of an anti-ICOS antibody or a fragment thereof. In a specific embodiment, a formulation of the disclosure comprises about 100 mg/ml of an anti-ICOS antibody or a fragment thereof. In a specific embodiment, a formulation of the disclosure comprises about 125 mg/ml of an anti-ICOS antibody or a fragment thereof. In a specific embodiment, a formulation of the disclosure comprises about 130 mg/ml of an anti-ICOS antibody or a fragment thereof. In a specific embodiment, a formulation of the disclosure comprises about 150 mg/ml of an anti-ICOS antibody or a fragment thereof. In a specific embodiment, a formulation of the disclosure comprises about 200 mg/ml of an anti-ICOS antibody or a fragment thereof. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises at least 1 mg/ml, at least 5 mg/ml, at least 10 mg/ml, at least 20 mg/ml, at least 30 mg/ml, at least 40 mg/ml, at least 50 mg/ml, at least 60 mg/ml, at least 70 mg/ml, at least 80 mg/ml, at least 90 mg/ml, at least 100 mg/ml, at least 110 mg/ml, at least 120 mg/ml, at least 130 mg/ml, at least 140 mg/ml, at least 150 mg/ml, at least 160 mg/ml, at least 170 mg/ml, at least 180 mg/ml, at least 190 mg/ml, at least 200 mg/ml, at least 250 mg/ml, or at least 300 mg/ml of an anti-ICOS antibody or a fragment thereof. In a specific embodiment, a formulation of the disclosure comprises at least 5 mg/ml of an anti-ICOS antibody of a fragment thereof. In a specific embodiment, a formulation of the disclosure comprises at least 10 mg/ml of an anti-ICOS antibody of a fragment thereof. In a specific embodiment, a formulation of the disclosure comprises at least 15 mg/ml of an anti-ICOS antibody of a fragment thereof. In a specific embodiment, a formulation of the disclosure comprises at least 100 mg/ml of an anti-ICOS antibody of a fragment thereof. In a specific embodiment, a formulation of the disclosure comprises at least 125 mg/ml of an anti-ICOS antibody of a fragment thereof. In a specific embodiment, a formulation of the disclosure comprises at least 150 mg/ml of an anti-ICOS antibody of a fragment thereof. In a specific embodiment, a formulation of the disclosure comprises at least 175 mg/ml of an anti-ICOS antibody of a fragment thereof. In a specific embodiment, a formulation of the disclosure comprises at least 200 mg/ml of an anti-ICOS antibody of a fragment thereof. In another embodiment, a formulation of the disclosure comprises between 1 mg/ml and 20 mg/ml, between 5 mg/ml and 20 mg/ml, between 1 mg/ml and 25 mg/ml, between 1 mg/ml and 200 mg/ml, between 25 mg/ml and 200 mg/ml, between 50 mg/ml and 200 mg/ml, between 75 mg/ml and 200 mg/ml, between 100 mg/ml and 200 mg/ml, between 125 mg/ml and 200 mg/ml, between 150 mg/ml and 200 mg/ml, between 25 mg/ml and 150 mg/ml, between 50 mg/ml and 150 mg/ml, between 75 mg/ml and 150 mg/ml, between 100 mg/ml and 150 mg/ml, between 125 mg/ml and 150 mg/ml, between 25 mg/ml and 125 mg/ml, between 50 mg/ml and 125 mg/ml, between 75 mg/ml and 125 mg/ml, between 100 mg/ml and 125 mg/ml, between 25 mg/ml and 100 mg/ml, between 50 mg/ml and 100 mg/ml, between 75 mg/ml and 100 mg/ml, between 25 mg/ml and 75 mg/ml, between 50 mg/ml and 75 mg/ml, or between 25 mg/ml and 50 mg/ml of an anti-ICOS antibody or a fragment thereof. In a specific embodiment, a formulation of the disclosure comprises between 5 mg/ml and 20 mg/ml of an anti-ICOS antibody or a fragment thereof. In a specific embodiment, a formulation of the disclosure comprises between 90 mg/ml and 110 mg/ml of an anti-ICOS antibody or a fragment thereof. In a specific embodiment, a formulation of the disclosure comprises between 100 mg/ml and 210 mg/ml of an anti-ICOS antibody or a fragment thereof. In a further embodiment, a formulation described herein comprises 1 mg/ml, 2 mg/ml, 3 mg/ml, 4 mg/ml, 5 mg/ml, 10 mg/ml, 15 mg/ml, 20 mg/ml, 30 mg/ml, 40 mg/ml, 50 mg/ml, 60 mg/ml, 70 mg/ml, 80 mg/ml, 90 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 250 mg/ml, or 300 mg/ml of an anti-ICOS antibody or a fragment thereof. In a specific embodiment, a formulation of the disclosure comprises 10 mg/ml of an anti-ICOS antibody or a fragment thereof. In a specific embodiment, a formulation of the disclosure comprises 100 mg/ml of an anti-ICOS antibody or a fragment thereof. In a specific embodiment, a formulation of the disclosure comprises 125 mg/ml of an anti-ICOS antibody or a fragment thereof. In a specific embodiment, a formulation of the disclosure comprises 150 mg/ml of an anti-ICOS antibody or a fragment thereof. In a specific embodiment, a formulation of the disclosure comprises 175 mg/ml of an anti-ICOS antibody or a fragment thereof. In a specific embodiment, a formulation of the disclosure comprises 200 mg/ml of an anti-ICOS antibody or a fragment thereof. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

Optionally, the formulations of the disclosure may further comprise common excipients and/or additives such as buffering agents, saccharides, salts and surfactants. Additionally or alternatively, the formulations of the disclosure may further comprise common excipients and/or additives, such as, but not limited to, solubilizers, diluents, binders, stabilizers, salts, lipophilic solvents, amino acids, chelators, preservatives, or the like.

In certain embodiments, the buffering agent is selected from the group consisting of histidine, citrate, phosphate, glycine, and acetate. In other embodiments the saccharide excipient is selected from the group consisting of trehalose, sucrose, mannitol, maltose and raffinose. In still other embodiments the surfactant is selected from the group consisting of polysorbate 20, polysorbate 40, polysorbate 80, and Pluronic F68. In yet other embodiments the salt is selected from the group consisting of NaCl, KCl, $MgCl_2$, and $CaCl_2$

Optionally, the formulations of the disclosure may further comprise other common auxiliary components, such as, but not limited to, suitable excipients, polyols, solubilizers, diluents, binders, stabilizers, lipophilic solvents, chelators, preservatives, or the like.

The formulations of the disclosure include a buffering or pH adjusting agent to provide improved pH control. In one embodiment, a formulation of the disclosure has a pH of between about 3.0 and about 9.0, between about 4.0 and about 8.0, between about 5.0 and about 8.0, between about 5.0 and about 7.0, between about 5.0 and about 6.5, between about 5.5 and about 8.0, between about 5.5 and about 7.0, or between about 5.5 and about 6.5. In a further embodiment, a formulation of the disclosure has a pH of about 3.0, about 3.5, about 4.0, about 4.5, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.5, about 8.0, about 8.5, or about 9.0. In a specific embodiment, a formulation of the disclosure has a pH of about 6.0.

The formulations of the disclosure include a buffering or pH adjusting agent to provide improved pH control. In one embodiment, a formulation of the disclosure has a pH of between 3.0 and 9.0, between 4.0 and 8.0, between 5.0 and 8.0, between 5.0 and 7.0, between 5.0 and 6.5, between 5.5 and 8.0, between 5.5 and 7.0, or between 5.5 and 6.5. In a further embodiment, a formulation of the disclosure has a pH of 3.0, 3.5, 4.0, 4.5, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.5, 8.0, 8.5, or 9.0. In a specific embodiment, a formulation of the disclosure has a pH of 6.0.

The pH of the formulation generally should not be equal to the isoelectric point of the particular antibody (including antibody fragment thereof) to be used in the formulation (for example, but not limited to, the isoelectric point of the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain) and may range from about 4.0 to about 8.0, or may range from about 5.5 to about 6.5.

Typically, the buffering agent is a salt prepared from an organic or inorganic acid or base. Representative buffering agents include, but are not limited to, organic acid salts such as salts of citric acid, ascorbic acid, gluconic acid, carbonic acid, tartaric acid, succinic acid, acetic acid, or phthalic acid; Tris, tromethamine hydrochloride, or phosphate buffers. In addition, amino acid components can also function in a buffering capacity. Representative amino acid components which may be utilized in the formulations of the disclosure as buffering agents include, but are not limited to, glycine and histidine. In certain embodiments, the buffering agent is selected from the group consisting of histidine, citrate, phosphate, glycine, and acetate. In a specific embodiment, the buffering agent is histidine. In another specific embodiment, the buffering agent is citrate. The purity of the buffering agent should be at least 98%, or at least 99%, or at least 99.5%. As used herein, the term "purity" in the context of histidine refers to chemical purity of histidine as understood in the art, e.g., as described in The Merck Index, 13th ed., O'Neil et al. ed. (Merck & Co., 2001).

Buffering agents are typically used at concentrations between about 1 mM and about 200 mM or any range or value therein, depending on the desired ionic strength and the buffering capacity required. The usual concentrations of conventional buffering agents employed in parenteral formulations can be found in: Pharmaceutical Dosage Form: Parenteral Medications, Volume 1, 2nd Edition, Chapter 5, p. 194, De Luca and Boylan, "Formulation of Small Volume Parenterals", Table 5: Commonly used additives in Parenteral Products. In one embodiment, the buffering agent is at a concentration of about 1 mM, or of about 5 mM, or of about 10 mM, or of about 15 mM, or of about 20 mM, or of about 25 mM, or of about 30 mM, or of about 35 mM, or of about 40 mM, or of about 45 mM, or of about 50 mM, or of about 60 mM, or of about 70 mM, or of about 80 mM, or of about 90 mM, or of about 100 mM. In one embodiment, the buffering agent is at a concentration of 1 mM, or of 5 mM, or of 10 mM, or of 15 mM, or of 20 mM, or of 25 mM, or of 30 mM, or of 35 mM, or of 40 mM, or of 45 mM, or of 50 mM, or of 60 mM, or of 70 mM, or of 80 mM, or of 90 mM, or of 100 mM. In a specific embodiment, the buffering agent is at a concentration of between about 5 mM and about 50 mM. In another specific embodiment, the buffering agent is at a concentration of between 5 mM and 20 mM.

Buffering agents are typically used at concentrations between 1 mM and 200 mM or any range or value therein, depending on the desired ionic strength and the buffering capacity required. The usual concentrations of conventional buffering agents employed in parenteral formulations can be found in: Pharmaceutical Dosage Form: Parenteral Medications, Volume 1, 2nd Edition, Chapter 5, p. 194, De Luca and Boylan, "Formulation of Small Volume Parenterals", Table 5: Commonly used additives in Parenteral Products. In one embodiment, the buffering agent is at a concentration of 1 mM, or of 5 mM, or of 10 mM, or of 15 mM, or of 20 mM, or of 25 mM, or of 30 mM, or of 35 mM, or of 40 mM, or of 45 mM, or of 50 mM, or of 60 mM, or of 70 mM, or of 80 mM, or of 90 mM, or of 100 mM. In one embodiment, the buffering agent is at a concentration of 1 mM, or of 5 mM, or of 10 mM, or of 15 mM, or of 20 mM, or of 25 mM, or of 30 mM, or of 35 mM, or of 40 mM, or of 45 mM, or of 50 mM, or of 60 mM, or of 70 mM, or of 80 mM, or of 90 mM, or of 100 mM. In a specific embodiment, the buffering agent is at a concentration of between 5 mM and 50 mM. In another specific embodiment, the buffering agent is at a concentration of between 5 mM and 20 mM.

In certain embodiments, a formulation of the disclosure comprises a buffering agent. In one embodiment, said buffering agent is selected from the group consisting of histidine, citrate, phosphate, glycine, and acetate. In a specific embodiment, a formulation of the disclosure comprises histidine as a buffering agent.

In one embodiment, a formulation of the disclosure comprises at least about 1 mM, at least about 5 mM, at least about 10 mM, at least about 20 mM, at least about 30 mM, at least about 40 mM, at least about 50 mM, at least about 75 mM, at least about 100 mM, at least about 150 mM, or at least about 200 mM histidine. In another embodiment, a formulation of the disclosure comprises between about 1 mM and about 200 mM, between about 1 mM and about 150 mM, between about 1 mM and about 100 mM, between about 1 mM and about 75 mM, between about 10 mM and about 200 mM, between about 10 mM and about 150 mM, between about 10 mM and about 100 mM, between about 10 mM and about 75 mM, between about 10 mM and about 50 mM, between about 10 mM and about 40 mM, between about 10 mM and about 30 mM, between about 20 mM and about 75 mM, between about 20 mM and about 50 mM, between about 20 mM and about 40 mM, or between about 20 mM and about 30 mM histidine. In a further embodiment of the disclosure comprises about 1 mM, about 5 mM, about 10 mM, about 20 mM, about 25 mM, about 30 mM, about 35 mM, about 40 mM, about 45 mM, about 50 mM, about 60 mM, about 70 mM, about 80 mM, about 90 mM, about 100 mM, about 150 mM, or about 200 mM histidine. In a specific embodiment, a formulation of the disclosure comprises about 10 mM histidine.

In one embodiment, a formulation of the disclosure comprises at least 1 mM, at least 5 mM, at least 10 mM, at least 20 mM, at least 30 mM, at least 40 mM, at least 50 mM, at least 75 mM, at least 100 mM, at least 150 mM, or at least 200 mM histidine. In another embodiment, a formulation of the disclosure comprises between 1 mM and 200 mM, between 1 mM and 150 mM, between 1 mM and 100 mM, between 1 mM and 75 mM, between 10 mM and 200 mM, between 10 mM and 150 mM, between 10 mM and 100 mM, between 10 mM and 75 mM, between 10 mM and 50 mM, between 10 mM and 40 mM, between 10 mM and 30 mM, between 20 mM and 75 mM, between 20 mM and 50 mM, between 20 mM and 40 mM, or between 20 mM and 30 mM histidine. In a further embodiment of the disclosure comprises 1 mM, 5 mM, 10 mM, 20 mM, 25 mM, 30 mM, 35 mM, 40 mM, 45 mM, 50 mM, 60 mM, 70 mM, 80 mM, 90 mM, 100 mM, 150 mM, or 200 mM histidine. In a specific embodiment, a formulation of the disclosure comprises 10 mM histidine.

In certain embodiments, the formulations of the disclosure comprise a carbohydrate excipient. Carbohydrate excipients can act, *e.g.,* as viscosity enhancing agents, stabilizers, bulking agents, solubilizing agents, and/or the like. Carbohydrate excipients are generally present at between about 1% and about 99% by weight or volume. In one embodiment, the carbohydrate excipient is present at between about 0.1% and about 20%. In another embodiment, the carbohydrate excipient is present at between about 0.1% and about 15%. In a specific embodiment, the carbohydrate excipient is present at between about 0.1% and about 5%, or between about 1% and about 20%, or between about 5% and about 15%, or between about 8% and about 10%, or between about 10% and about 15%, or between about 15% and about 20%. In another specific embodiment, the carbohydrate excipient is present at between 0.1% and 20%, or between 5% and 15%, or between 8% and 10%, or between 10% and 15%, or between 15% and 20%. In still another specific embodiment, the carbohydrate excipient is present at between about 0.1% and about 5%. In still another specific embodiment, the carbohydrate excipient is present at between about 5% and about 10%. In yet another specific embodiment, the carbohydrate excipient is present at between about 15% and about 20%. In still other specific embodiments, the carbohydrate excipient is present at 1%, or at 1.5%, or at 2%, or at 2.5%, or at 3%, or at 4%, or at 5%, or at 10%, or at 15%, or at 20%.

In certain embodiments, the formulations of the disclosure comprise a carbohydrate excipient. Carbohydrate excipients can act, *e.g.,* as viscosity enhancing agents, stabilizers, bulking agents, solubilizing agents, and/or the like. Carbohydrate excipients are generally present at between 1% and 99% by weight or volume. In one embodiment, the carbohydrate excipient is present at between 0.1% and 20%. In another embodiment, the carbohydrate excipient is present at between 0.1% and 15%. In a specific embodiment, the carbohydrate excipient is present at between 0.1% and 5%, or between 1% and 20%, or between 5% and 15%, or between 8% and 10%, or between 10% and 15%, or between 15% and 20%. In another specific embodiment, the carbohydrate excipient is present at between 0.1% and 20%, or between 5% and 15%, or between 8% and 10%, or between 10% and 15%, or between 15% and 20%. In still another specific embodiment, the carbohydrate excipient is present at between 0.1% and 5%. In still another specific embodiment, the carbohydrate excipient is present at between 5% and 10%. In yet another specific embodiment, the carbohydrate excipient is present at between 15% and 20%. In still other specific embodiments, the carbohydrate excipient is present at 1%, or at 1.5%, or at 2%, or at 2.5%, or at 3%, or at 4%, or at 5%, or at 10%, or at 15%, or at 20%.

Carbohydrate excipients suitable for use in the formulations of the disclosure include, for example, monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and the like. In one embodiment, the carbohydrate excipients for use in the present disclosure are selected from the group consisting of, sucrose, trehalose, lactose, mannitol, and raffinose. In a specific embodiment, the carbohydrate excipient is trehalose. In another specific embodiment, the carbohydrate excipient is mannitol. In yet another specific embodiment, the carbohydrate excipient is sucrose. In still another specific embodiment, the carbohydrate excipient is raffinose. The purity of the carbohydrate excipient should be at least 98%, or at least 99%, or at least 99.5%.

In one embodiment, a formulation of the disclosure comprises at least about 1%, at least about 2%, at least about 4%, at least about 8%, at least about 20%, at least about 30%, or at least about 40% trehalose. In another embodiment, a formulation of the disclosure comprises between about 1% and about 40%, between about 1% and about 30%, between about 1% and about 20%, between about 2% and about 40%, between about 2% and about 30%, between about 2% and about 20%, between about 4% and about 40%, between about 4% and about 30%, or between about 4% and about 20% trehalose. In a further embodiment, a formulation of the disclosure comprises about 1%, about 2%, about 4%, about 8%, about 20%, about 30%, or about 40% trehalose. In a specific embodiment, a formulation of the disclosure comprises about 4% trehalose.

In one embodiment, a formulation of the disclosure comprises at least 1%, at least 2%, at least 4%, at least 8%, at least 20%, at least 30%, or at least 40% trehalose. In another embodiment, a formulation of the disclosure comprises between 1% and 40%, between 1% and 30%, between 1% and 20%, between 2% and 40%, between 2% and 30%, between 2% and 20%, between 4% and 40%, between 4% and 30%, or between 4% and 20% trehalose. In a further embodiment, a formulation of the disclosure comprises 1%, 2%, 4%, 8%, 20%, 30%, or 40% trehalose. In a specific embodiment, a formulation of the disclosure comprises 4 % trehalose.

In one embodiment, a formulation of the disclosure comprises an excipient. In a specific embodiment, a formulation of the disclosure comprises at least one excipient selected from the group consisting of: sugar, salt, surfactant, amino acid, polyol, chelating agent, emulsifier and preservative. In one embodiment, a formulation of the disclosure comprises a salt. In one embodiment, a formulation of the disclosure comprises a salt selected from the group consisting of: NaCl, KCl, CaCl$_2$, and MgCl$_2$. In a specific embodiment, a formulation of the disclosure comprises NaCl.

In one embodiment, a formulation of the disclosure comprises at least about 10 mM, at least about 25 mM, at least about 50 mM, at least about 75 mM, at least about 80 mM, at least about 100 mM, at least about 125 mM, at least about 150 mM, at least about 175 mM, at least about 200 mM, or at least about 300 mM sodium chloride. In a further embodiment, a formulation described herein comprises between about 10 mM and about 300 mM, between about 10 mM and about 200 mM, between about 10 mM and about 175 mM, between about 10 mM and about 150 mM, between about 25 mM and about 300 mM, between about 25 mM and about 200 mM, between about 25 mM and about 175 mM, between about 25 mM and about 150 mM, between about 50 mM and about 300 mM, between about 50 mM and about 200 mM, between about 50 mM and about 175 mM, between about 50 mM and about 150 mM, between about 75 mM and about 300 mM, between about 75 mM and about 200 mM, between about 75 mM and about 175 mM, between about 75 mM and about 150 mM, between about 100 mM and about 300 mM, between about 100 mM and about 200 mM, between about 100 mM and about 175 mM, or between about 100 mM and about 150 mM sodium chloride. In a further embodiment, a formulation of the disclosure comprises about 10 mM. about 25 mM, about 50 mM, about 75 mM, about 80 mM, about 100 mM, about 125 mM, about 150 mM, about 175 mM, about 200 mM, or about 300 mM sodium chloride. In a specific embodiment, a formulation of the disclosure comprises 80 mM sodium chloride.

In one embodiment, a formulation of the disclosure comprises at least 10 mM, at least 25 mM, at least 50 mM, at least 75 mM, at least 80 mM, at least 100 mM, at least 125 mM, at least 150 mM, at least 175 mM, at least 200 mM, or at least 300 mM sodium chloride. In a further embodiment, a formulation described herein comprises between 10 mM and 300 mM, between 10 mM and 200 mM, between 10 mM and 175 mM, between 10 mM and 150 mM, between 25 mM and 300 mM, between 25 mM and 200 mM, between 25 mM and 175 mM, between 25 mM and 150 mM, between 50 mM and 300 mM, between 50 mM and 200 mM, between 50 mM and 175 mM, between 50 mM and 150 mM, between 75 mM and 300 mM, between 75 mM and 200 mM, between 75 mM and 175 mM, between 75 mM and 150 mM, between 100 mM and 300 mM, between 100 mM and 200 mM, between 100 mM and 175 mM, or between 100 mM and 150 mM sodium chloride. In a further embodiment, a formulation of the disclosure comprises 10 mM. 25 mM, 50 mM, 75 mM, 80 mM, 100 mM, 125 mM, 150 mM, 175 mM, 200 mM, or 300 mM sodium chloride. In a specific embodiment, a formulation of the disclosure comprises 80 mM sodium chloride.

The formulations of the disclosure may further comprise a surfactant. The term "surfactant" as used herein refers to organic substances having amphipathic structures; namely, they are composed of groups of opposing solubility tendencies, typically an oil-soluble hydrocarbon chain and a water-soluble ionic group. Surfactants can be classified, depending on the charge of the surface-active moiety, into anionic, cationic, and nonionic surfactants. Surfactants are often used as wetting, emulsifying, solubilizing, and dispersing agents for various pharmaceutical compositions and preparations of biological materials. Pharmaceutically acceptable surfactants like polysorbates (*e.g.* polysorbates 20 or 80); polyoxamers (*e.g.* poloxamer 188); Triton; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (*e.g.* lauroamidopropyl); myristamidopropyl-, palmidopropyl-, or isostearamidopropyl-dimethylamine; sodium methyl cocoyl-, or disodium methyl oleyl-taurate; and the MONAQUA™ series (Mona Industries, Inc., Paterson, N.J.), polyethyl glycol, polypropyl glycol, and copolymers of ethylene and propylene glycol (*e.g.* Pluronics, PF68 etc), can optionally be added to the formulations of the disclosure to reduce aggregation. Surfactants are particularly useful if a pump or plastic container is used to administer the formulation. The presence of a pharmaceutically acceptable surfactant mitigates the propensity for the protein to aggregate. In a specific embodiment, the formulations of the disclosure comprise a polysorbate which is at a concentration ranging from between about 0.001% to about 1%, or about 0.001% to about 0.1%, or about 0.01% to about 0.1%. In other specific embodiments, the formulations of the disclosure comprise a polysorbate which is at a concentration of 0.001%, or 0.002%, or 0.003%, or 0.004%, or 0.005%, or 0.006%, or 0.007%, or 0.008%, or 0.009%, or 0.01%, or 0.015%, or 0.02%. In another specific embodiment, the polysorbate is polysorbate-80. In a specific embodiment, the formulations of the disclosure comprise a polysorbate which is at a concentration ranging from between 0.001% and 1%, or 0.001% and 0.1%, or 0.01% and 0.1%. In other specific embodiments, the formulations of the disclosure comprise a polysorbate which is at a concentration of 0.001%, or 0.002%, or 0.003%, or 0.004%, or 0.005%, or 0.006%, or 0.007%, or 0.008%, or 0.009%, or 0.01%, or 0.015%, or 0.02%. In another specific embodiment, the polysorbate is polysorbate-80.

In one embodiment, a formulation of the disclosure comprises a surfactant. In one embodiment, a formulation of the disclosure comprises Polysorbate 20, Polysorbate 40, Polysorbate 60, or Polysorbate 80. In a specific embodiment, a formulation of the disclosure comprises Polysorbate 80.

In one embodiment, a formulation of the disclosure comprises at least about 0.001%, at least about 0.002%, at least about 0.005%, at least about 0.01%, at least about 0.02%, at least about 0.05%, at least about 0.1%, at least about 0.2%, or at least about 0.5% Polysorbate 80. In another embodiment, a formulation of the disclosure comprises between about 0.001% and about 0.5%, between about 0.001% and about 0.2%, between about 0.001% and about 0.1%, between about 0.001% and about 0.05%, between about 0.002% and about 0.5%, between about 0.002% and about 0.2%, between about 0.002% and about 0.1%, between about 0.002% and about 0.05%, between about 0.005% and about 0.5%, between about 0.005% and about 0.2%, between about 0.005% and about 0.1%, between about 0.005% and about 0.05%, between about 0.01% and about 0.5%, between about 0.01% and about 0.2%, between about 0.01% and about 0.1%, or between about 0.01% and about 0.05% Polysorbate 80. In a further embodiment, a formulation of the disclosure comprises about 0.001%, about 0.002%, about 0.005%, about 0.01%, about 0.02%, about 0.05%, about 0.1%, about 0.2%, and about 0.5% Polysorbate 80. In a specific embodiment, a formulation of the disclosure comprises about 0.02% Polysorbate 80. In a specific embodiment, a formulation of the disclosure comprises about 0.04% Polysorbate 80. In a specific embodiment, a formulation of the disclosure comprises about 0.05% Polysorbate 80.

In one embodiment, a formulation of the disclosure comprises at least 0.001%, at least 0.002%, at least 0.005%, at least 0.01%, at least 0.02%, at least 0.05%, at least 0. 1%, at least 0.2%, or at least 0.5% Polysorbate 80. In another embodiment, a formulation of the disclosure comprises between 0.001% and 0.5%, between 0.001% and 0.2%, between 0.001% and 0.1%, between 0.001% and 0.05%, between 0.002% and 0.5%, between 0.002% and 0.2%, between 0.002% and 0.1%, between 0.002% and 0.05%, between 0.005% and 0.5%, between 0.005% and 0.2%, between 0.005% and 0.1%, between 0.005% and 0.05%, between 0.01% and 0.5%, between 0.01% and 0.2%, between 0.01% and 0.1%, or between 0.01% and 0.05% Polysorbate 80. In a further embodiment, a formulation of the disclosure comprises 0.001%, 0.002%, 0.005%, 0.01%, 0.02%, 0.05%, 0.1%, 0.2%, and 0.5% Polysorbate 80. In a specific embodiment, a formulation of the disclosure comprises 0.02% Polysorbate 80. In a specific embodiment, a formulation of the disclosure comprises 0.04% Polysorbate 80. In a specific embodiment, a formulation of the disclosure comprises 0.05% Polysorbate 80.

Optionally, the formulations of the disclosure may further comprise other common excipients and/or additives including, but not limited to, diluents, binders, stabilizers, lipophilic solvents, preservatives, adjuvants, or the like. Pharmaceutically acceptable excipients and/or additives may be used in the formulations of the disclosure. Commonly used excipients/additives, such as pharmaceutically acceptable chelators (for example, but not limited to, EDTA, DTPA or EGTA) can optionally be added to the formulations of the disclosure to reduce aggregation. These additives are particularly useful if a pump or plastic container is used to administer the formulation.

Preservatives, such as phenol, m-cresol, p-cresol, o-cresol, chlorocresol, benzyl alcohol, phenylmercuric nitrite, phenoxyethanol, formaldehyde, chlorobutanol, magnesium chloride (for example, but not limited to, hexahydrate), alkylparaben (methyl, ethyl, propyl, butyl and the like), benzalkonium chloride, benzethonium chloride, sodium dehydroacetate and thimerosal, or mixtures thereof can optionally be added to the formulations of the disclosure at any suitable concentration such as between about 0.001% to about 5%, or any range or value therein. The concentration of preservative used in the formulations of the disclosure is a concentration sufficient to yield an anti-microbial effect. Such concentrations are dependent on the preservative selected and are readily determined by the skilled artisan.

Other contemplated excipients/additives, which may be utilized in the formulations of the disclosure include, for example, flavoring agents, antimicrobial agents, sweeteners, antioxidants, antistatic agents, lipids such as phospholipids or fatty acids, steroids such as cholesterol, protein excipients such as serum albumin (human serum albumin (HSA), recombinant human albumin (rHA)), gelatin, casein, salt-forming counterions such as sodium and the like. These and additional known pharmaceutical excipients and/or additives suitable for use in the formulations of the disclosure are known in the art, *e.g.,* as listed in "Remington: The Science & Practice of Pharmacy", 21st ed., Lippincott Williams & Wilkins, (2005), and in the "Physician's Desk Reference", 60th ed., Medical Economics, Montvale, N.J. (2005). Pharmaceutically acceptable carriers can be routinely selected that are suitable for the mode of administration, solubility and/or stability of Fc variant protein as well known in the art or as described herein.

It will be understood by one skilled in the art that the formulations of the disclosure may be isotonic with human blood, that is, the formulations of the disclosure have essentially the same osmotic pressure as human blood. Such isotonic formulations will generally have an osmotic pressure from about 250 mOSm to about 350 mOSm. Isotonicity can be measured by, for example, using a vapor pressure or ice-freezing type osmometer. Tonicity of a formulation is adjusted by the use of tonicity modifiers. "Tonicity modifiers" are those pharmaceutically acceptable inert substances that can be added to the formulation to provide an isotonicity of the formulation. Tonicity modifiers suitable for this disclosure include, but are not limited to, saccharides, salts and amino acids.

In certain embodiments, the formulations of the present disclosure have an osmotic pressure from about 100 mOSm to about 1200 mOSm, or from about 200 mOSm to about 1000 mOSm, or from about 200 mOSm to about 800 mOSm, or from about 200 mOSm to about 600 mOSm, or from about 250 mOSm to about 500 mOSm, or from about 250 mOSm to about 400 mOSm, or from about 250 mOSm to about 350 mOSm.

In certain embodiments, the formulations of the present disclosure have an osmotic pressure from 100 mOSm to 1200 mOSm, or from 200 mOSm to 1000 mOSm, or from 200 mOSm to 800 mOSm, or from 200 mOSm to 600 mOSm, or from 250 mOSm to 500 mOSm, or from 250 mOSm to 400 mOSm, or from 250 mOSm to 350 mOSm.

Concentration of any one or any combination of various components of the formulations of the disclosure is adjusted to achieve the desired tonicity of the final formulation. For example, the ratio of the carbohydrate excipient to antibody may be adjusted according to methods known in the art (*e.g.,* U.S. Patent No. 6,685,940). In certain embodiments, the molar ratio of the carbohydrate excipient to antibody may be from about 100 moles to about 1000 moles of carbohydrate excipient to about 1 mole of antibody, or from about 200 moles to about 6000 moles of carbohydrate excipient to about 1 mole of antibody, or from about 100 moles to about 510 moles of carbohydrate excipient to about 1 mole of antibody, or from about 100 moles to about 600 moles of carbohydrate excipient to about 1 mole of antibody.

Concentration of any one or any combination of various components of the formulations of the disclosure is adjusted to achieve the desired tonicity of the final formulation. For example, the ratio of the carbohydrate excipient to antibody may be adjusted according to methods known in the art (*e.g.,* U.S. Patent No. 6,685,940). In certain embodiments, the molar ratio of the carbohydrate excipient to antibody may be from 100 moles to 1000 moles of carbohydrate excipient to 1 mole of antibody, or from 200 moles to 6000 moles of carbohydrate excipient to 1 mole of antibody, or from 100 moles to 510 moles of carbohydrate excipient to 1 mole of antibody, or from 100 moles to 600 moles of carbohydrate excipient to 1 mole of antibody.

The desired isotonicity of the final formulation may also be achieved by adjusting the salt concentration of the formulations. Salts that are pharmaceutically acceptable and suitable for this disclosure as tonicity modifiers include, but are not limited to, sodium chloride, sodium succinate, sodium sulfate, potassium chloride, magnesium chloride, magnesium sulfate, and calcium chloride. In specific embodiments, formulations of the disclosures comprise NaCl, $MgCl_2$, and/or $CaCl_2$. In one embodiment, concentration of NaCl is between about 75 mM and about 150 mM. In another embodiment, concentration of $MgCl_2$ is between about 1 mM and about 100 mM. Amino acids that are pharmaceutically acceptable and suitable for this disclosure as tonicity modifiers include, but are not limited to, proline, alanine, L-arginine, asparagine, L-aspartic acid, glycine, serine, lysine, and histidine.

In one embodiment, a formulation of the disclosure comprises histidine, sodium chloride, trehalose, and Polysorbate 80. In one embodiment, a formulation of the disclosure comprises sodium chloride, trehalose, and Polysorbate 80. In one embodiment, a formulation of the disclosure comprises histidine, trehalose, and Polysorbate 80. In one embodiment, a formulation of the disclosure comprises histidine, sodium chloride, and Polysorbate 80. In one embodiment, a formulation of the disclosure comprises histidine, sodium chloride, and trehalose. In one embodiment, a formulation of the disclosure comprises histidine and sodium chloride. In one embodiment, a formulation of the disclosure comprises histidine and trehalose. In one embodiment, a formulation of the disclosure comprises histidine and Polysorbate 80. In one embodiment, a formulation of the disclosure comprises sodium chloride and trehalose. In one embodiment, a formulation of the disclosure comprises sodium chloride and Polysorbate 80. In one embodiment, a formulation of the disclosure comprises trehalose, and Polysorbate 80.

In one embodiment, a formulation of the disclosure comprises histidine, sodium chloride, trehalose and Polysorbate 80. In one embodiment, a formulation of the disclosure comprises between about 5 mM and about 100 mM histidine, between about 10 mM and about 300 mM sodium chloride, between about 0.3% and about 10% trehalose, and between about 0.005% and about 0.1% Polysorbate 80, wherein said formulation has a pH of between about 5.0 and about 7.0. In another embodiment, a formulation of the disclosure comprises between about 5 mM and about 50 mM histidine, between about 50 mM and about 200 mM sodium chloride, between about 1% and about 8% trehalose, and between about 0.01% and about 0.05% Polysorbate 80, wherein said formulation has a pH of between about 5.5 and about 6.5. In a further embodiment, a formulation of the disclosure comprises about 10 mM histidine, about 80 mM sodium chloride, about 4% trehalose and about 0.02% Polysorbate 80, wherein said formulation has a pH of about 6.0.

In one embodiment, a formulation of the disclosure comprises histidine, sodium chloride, trehalose and Polysorbate 80. In one embodiment, a formulation of the disclosure comprises between 5 mM and 100 mM histidine, between 10 mM and 300 mM sodium chloride, between 1% and 10% trehalose, and between 0.005% and 0.1% Polysorbate 80, wherein said formulation has a pH of between 5.0 and 7.0. In another embodiment, a formulation of the disclosure comprises between 5 mM and 50 mM histidine, between 50 mM and 200 mM sodium chloride, between 1% and 6% trehalose, and between 0.01% and 0.05% Polysorbate 80, wherein said formulation has a pH of between 5.5 and 6.5. In a further embodiment, a formulation of the disclosure comprises 10 mM histidine, 80 mM sodium chloride, 4% trehalose and 0.02% Polysorbate 80, wherein said formulation has a pH of 6.0.

In one embodiment, a formulation of the disclosure consists of between about 20 mg/ml and about 150 mg/ml anti-ICOS antibody, about 10 mM histidine, about 80 mM sodium chloride, about 4% trehalose and about 0.02% Polysorbate 80, wherein said formulation has a pH of about 6.0. In another embodiment, a formulation of the disclosure consists of about 50 mg/ml anti-ICOS antibody, about 10 mM histidine, about 80 mM sodium chloride, about 4% trehalose and about 0.02% Polysorbate 80, wherein said formulation has a pH of about 6.0. In a further embodiment, a formulation of the disclosure consists of about 100 mg/ml anti-ICOS antibody, about 10 mM histidine, about 80 mM sodium chloride, about 4% trehalose and about 0.02% Polysorbate 80, wherein said formulation has a pH of about 6.0. In a further embodiment, a formulation of the disclosure consists of about 110 mg/ml anti-ICOS antibody, about 10 mM histidine, about 80 mM sodium chloride, about 4% trehalose and about 0.02% Polysorbate 80, wherein said formulation has a pH of about 6.0. In a further embodiment, a formulation of the disclosure consists of about 120 mg/ml anti-ICOS antibody, about 10 mM histidine, about 80 mM sodium chloride, about 4% trehalose and about 0.02% Polysorbate 80, wherein said formulation has a pH of about 6.0. In a further embodiment, a formulation of the disclosure consists of about 130 mg/ml anti-ICOS antibody, about 10 mM histidine, about 80 mM sodium chloride, about 4% trehalose and about 0.02% Polysorbate 80, wherein said formulation has a pH of about 6.0. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure consists of between 20 mg/ml and 150 mg/ml anti-ICOS antibody, 10 mM histidine, 80 mM sodium chloride, 4% trehalose and 0.02% Polysorbate 80, wherein said formulation has a pH of 6.0. In another embodiment, a formulation of the disclosure consists of 50 mg/ml anti-ICOS antibody, 10 mM histidine, 80 mM sodium chloride, 4% trehalose and 0.02% Polysorbate 80, wherein said formulation has a pH of 6.0. In a further embodiment, a formulation of the disclosure consists of 100 mg/ml anti-ICOS antibody, 10 mM histidine, 80 mM sodium chloride, 4% trehalose and 0.02% Polysorbate 80, wherein said formulation has a pH of 6.0. In a further embodiment, a formulation of the disclosure consists of 110 mg/ml anti-ICOS antibody, 10 mM histidine, 80 mM sodium chloride, 4% trehalose and 0.02% Polysorbate 80, wherein said formulation has a pH of 6.0. In a further embodiment, a formulation of the disclosure consists of 120 mg/ml anti-ICOS antibody, 10 mM histidine, 80 mM sodium chloride, 4% trehalose and 0.02% Polysorbate 80, wherein said formulation has a pH of 6.0. In a further embodiment, a formulation of the disclosure consists of 130 mg/ml anti-ICOS antibody, 10 mM histidine, 80 mM sodium chloride, 4% trehalose and 0.02% Polysorbate 80, wherein said formulation has a pH of 6.0. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure consists of between about 5 mg/ml and about 20 mg/ml anti-ICOS antibody, about 10 mM histidine, about 80 mM sodium chloride, about 4% trehalose and about 0.02% Polysorbate 80, wherein said formulation has a pH of about 6.0. In another embodiment, a formulation of the disclosure consists of about 5 mg/ml anti-ICOS antibody, about 10 mM histidine, about 80 mM sodium chloride, about 4% trehalose and about 0.02% Polysorbate 80, wherein said formulation has a pH of about 6.0. In a further embodiment, a formulation of the disclosure consists of about 10 mg/ml anti-ICOS antibody, about 10 mM histidine, about 80 mM sodium chloride, about 4% trehalose and about 0.02% Polysorbate 80, wherein said formulation has a pH of about 6.0. In a further embodiment, a formulation of the disclosure consists of about 15 mg/ml anti-ICOS antibody, about 10 mM histidine, about 80 mM sodium chloride, about 4% trehalose and about 0.02% Polysorbate 80, wherein said formulation has a pH of about 6.0. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure consists of between 5 mg/ml and 20 mg/ml anti-ICOS antibody, 10 mM histidine, 80 mM sodium chloride, 4% trehalose and 0.02% Polysorbate 80, wherein said formulation has a pH of 6.0. In another embodiment, a formulation of the disclosure consists of 5 mg/ml anti-ICOS antibody, 10 mM histidine, 80 mM sodium chloride, 4% trehalose and 0.02% Polysorbate 80, wherein said formulation has a pH of 6.0. In a further embodiment, a formulation of the disclosure consists of 10 mg/ml anti-ICOS antibody, 10 mM histidine, 80 mM sodium chloride, 4% trehalose and 0.02% Polysorbate 80, wherein said formulation has a pH of 6.0. In a further embodiment, a formulation of the disclosure consists of 20 mg/ml anti-ICOS antibody, 10 mM histidine, 80 mM sodium chloride, 4% trehalose and 0.02% Polysorbate 80, wherein said formulation has a pH of 6.0. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment the formulations of the disclosure are pyrogen-free formulations which are substantially free of endotoxins and/or related pyrogenic substances. Endotoxins include toxins that are confined inside a microorganism and are released only when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, even low amounts of endotoxins must be removed from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight, as can be the case with antibodies, even trace amounts of harmful and dangerous endotoxin must be removed. In certain specific embodiments, the endotoxin and pyrogen levels in the composition are less then 10 EU/mg, or less then 5 EU/mg, or less then 1 EU/mg, or less then 0.1 EU/mg, or less then 0.01 EU/mg, or less then 0.001 EU/mg.

When used for *in vivo* administration, the formulations of the disclosure should be sterile. The formulations of the disclosure may be sterilized by various sterilization methods, including sterile filtration, radiation, etc. In one embodiment, the antibody formulation is filter-sterilized with a presterilized 0.22-micron filter. Sterile compositions for injection can be formulated according to conventional pharmaceutical practice as described in "Remington: The Science & Practice of Pharmacy", 21st ed., Lippincott Williams & Wilkins, (2005). Formulations comprising antibodies, such as those disclosed herein, ordinarily will be stored in lyophilized form or in solution. It is contemplated that sterile compositions comprising antibodies are placed into a container having a sterile access port, for example, an intravenous solution bag or vial having an adapter that allows retrieval of the formulation, such as a stopper pierceable by a hypodermic injection needle. In one embodiment, a composition of the disclosure is provided as a pre-filled syringe.

**5.2.** Stability of Formulations

In one embodiment, a formulation of the disclosure comprises an antibody or fragment thereof that is susceptible to aggregation, fragmentation and/or deamidation.

In one embodiment, a formulation of the disclosure stabilizes an anti-ICOS antibody. In one embodiment, a formulation of the disclosure prevents aggregation of an anti-ICOS antibody or fragment thereof. In another embodiment, a formulation of the disclosure prevents fragmentation of an anti-ICOS antibody or fragment thereof. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

The present disclosures provide stable liquid formulations comprising anti-ICOS antibodies of the disclosure. The stability of said antibody can be assessed by degrees of aggregation, degradation or fragmentation, as measured by HPSEC, reverse phase chromatography, static light scattering (SLS), Dynamic Light Scattering (DLS), Fourier Transform Infrared Spectroscopy (FTIR), circular dichroism (CD), urea unfolding techniques, intrinsic tryptophan fluorescence, differential scanning calorimetry, and/or ANS binding techniques, compared to a reference formulation comprising a reference antibody. For example, a reference formulation may be a reference standard frozen at -70°C consisting of 10 mg/ml of a reference antibody (including antibody fragment thereof) (for example, but not limited to, the 136 anti-ICOS antibody comprising an Fc region having complex N-glycoside-linked sugar chains in which fucose is not bound to N-acetylglucosamine in the reducing end in the sugar chain) in 10 mM histidine (pH 6.0) that contains 80 mM NaCl, 4% trehalose and 0.02% polysorbate 80, which reference formulation regularly gives a single monomer peak (e.g., ≥ 95% area) by HPSEC. In certain embodiments, a reference formulation is identical to the formulation whose stability is tested; the reference formulation may be stored frozen at -70°C during the stability testing to preserve the reference formulation in its original condition. For example, the reference standard for assessing any loss of ICOS antigen binding activity in a formulation stored at 40°C may be the identical formulation stored at -70°C for 30 days. The overall stability of a formulation comprising an antibody (including antibody fragment thereof) may also be assessed by various immunological assays including, for example, ELISA and radioimmunoassay using isolated antigen molecules. Furthermore, the stability of a formulation comprising an antibody may also be assessed using various assays designed to measure a functional characteristic of the antibody, for example, assays designed to measure antigen binding affinity, in vitro ADCC activity, in vivo depletion activity, in vitro CDC activity.

In one embodiment, a formulation of the disclosure is stable upon storage at about 40°C for at least about 1 week, at least about 2 weeks, at least about 3 weeks, or at least about 4 weeks. In one embodiment, a formulation of the disclosure is stable upon storage at about 40°C for at least about 1 month, at least about 2 months, at least about 3 months, at least about 4 months, at least about 5 months, or at least about 6 months. In a specific embodiment, a formulation of the disclosure is stable upon storage in a pre-filled syringe.

In one embodiment, a formulation of the disclosure is stable upon storage at about 5°C for at least about 1 month, at least about 2 months, at least about 3 months, at least about 4 months, at least about 5 months, at least about 6 months, at least about 7 months, at least about 8 months, at least about 9 months, at least about 10 months, at least about 11 months, or at least about 12 months. In one embodiment, a formulation of the disclosure is stable upon storage at about 5°C for at least about 1 year, at least about 2 years, at least about 3 years, at least about 4 years, at least about 5 years, at least about 6 years, at least about 7 years, at least about 8 years, at least about 9 years, at least about 10 years, at least about 11 years, or at least about 12 years. In a specific embodiment, a formulation of the disclosure is stable upon storage in a pre-filled syringe.

In one embodiment, a formulation of the disclosure is stable upon storage at about 40°C for about 1 week, about 2 weeks, about 3 weeks, or about 4 weeks. In one embodiment, a formulation of the disclosure is stable upon storage at about 40°C for about 1 month, about 2 months, about 3 months, about 4 months, about 5 months, or about 6 months. In a specific embodiment, a formulation of the disclosure is stable upon storage in a pre-filled syringe.

In one embodiment, a formulation of the disclosure is stable upon storage at about 5°C for about 1 month, about 2 months, about 3 months, about 4 months, about 5 months, about 6 months, about 7 months, about 8 months, about 9 months, about 10 months, about 11 months, or about 12 months. In one embodiment, a formulation of the disclosure is stable upon storage at about 5°C for about 1 year, about 2 years, about 3 years, about 4 years, about 5 years, about 6 years, about 7 years, about 8 years, about 9 years, about 10 years, about 11 years, or about 12 years. In a specific embodiment, a formulation of the disclosure is stable upon storage in a pre-filled syringe.

In one embodiment, a formulation of the disclosure comprises an anti-ICOS antibody that has a ICOS binding activity that is at least 50%, at least 60%, at least 70%, at least 80%, at least 90%, at least 95%, or at least 99% of the ICOS binding activity of a reference antibody, wherein said formulation was stored at about 40°C for about 1 week, about 2 weeks, about 3 weeks, or about 4 weeks. In one embodiment, a formulation of the disclosure comprises an anti-ICOS antibody that has a ICOS binding activity that is at least 50%, at least 60%, at least 70%, at least 80%, at least 90%, at least 95%, or at least 99% of the ICOS binding activity of a reference antibody, wherein said formulation was stored at about 40°C for about 1 month, about 2 months, about 3 months, about 4 months, about 5 months, or about 6 months. In a specific embodiment, a formulation of the disclosure comprises an anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody that has a ICOS binding activity that is at least 50%, at least 60%, at least 70%, at least 80%, at least 90%, at least 95%, or at least 99% of the ICOS binding activity of a reference antibody, wherein said formulation was stored at about 25°C for about 1 week, about 2 weeks, about 3 weeks, or about 4 weeks. In one embodiment, a formulation of the disclosure comprises an anti-ICOS antibody that has a ICOS binding activity that is at least 50%, at least 60%, at least 70%, at least 80%, at least 90%, at least 95%, or at least 99% of the ICOS binding activity of a reference antibody, wherein said formulation was stored at about 25°C for about 1 month, about 2 months, about 3 months, about 4 months, about 5 months, or about 6 months. In a specific embodiment, a formulation of the disclosure comprises an anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody that has a ICOS binding activity that is at least 50%, at least 60%, at least 70%, at least 80%, at least 90%, at least 95%, or at least 99% of the ICOS binding activity of a reference antibody, wherein said formulation was stored at about 5°C for about 1 month, about 2 months, about 3 months, about 4 months, about 5 months, about 6 months, about 7 months, about 8 months, about 9 months, about 10 months, about 11 months, or about 12 months. In one embodiment, a formulation of the disclosure comprises an anti-ICOS antibody that has a ICOS binding activity that is at least 50%, at least 60%, at least 70%, at least 80%, at least 90%, at least 95%, or at least 99% of the ICOS binding activity of a reference antibody, wherein said formulation was stored at about 5°C for about 1 year, about 2 years, about 3 years, about 4 years, about 5 years, about 6 years, about 7 years, about 8 years, about 9 years, about 10 years, about 11 years, or about 12 years. In a specific embodiment, a formulation of the disclosure is stored in a pre-filled syringe. In a specific embodiment, a formulation of the disclosure comprises an anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein the antibody loses no more than about 50%, no more than about 40%, no more than about 30%, no more than about 20%, no more than about 10%, no more than about 5%, or no more than about 1% of its ICOS binding activity during storage of the formulation at about 40°C for about 1 week, about 2 weeks, about 3 weeks, or about 4 weeks. In one embodiment, a formulation of the disclosure comprises an anti-ICOS antibody, wherein the antibody loses no more than about 50%, no more than about 40%, no more than about 30%, no more than about 20%, no more than about 10%, no more than about 5%, or no more than about 1% of its ICOS binding activity during storage of the formulation at about 40°C for about 1 month, about 2 months, about 3 months, about 4 months, about 5 months, or about 6 months. In a specific embodiment, a formulation of the disclosure comprises an anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein the antibody loses no more than about 50%, no more than about 40%, no more than about 30%, no more than about 20%, no more than about 10%, no more than about 5%, or no more than about 1% of its ICOS binding activity during storage of the formulation at about 25°C for about 1 week, about 2 weeks, about 3 weeks, or about 4 weeks. In one embodiment, a formulation of the disclosure comprises an anti-ICOS antibody, wherein the antibody loses no more than about 50%, no more than about 40%, no more than about 30%, no more than about 20%, no more than about 10%, no more than about 5%, or no more than about 1% of its ICOS binding activity during storage of the formulation at about 25°C for about 1 month, about 2 months, about 3 months, about 4 months, about 5 months, or about 6 months. In a specific embodiment, a formulation of the disclosure comprises an anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein the antibody loses no more than about 50%, no more than about 40%, no more than about 30%, no more than about 20%, no more than about 10%, no more than about 5%, or no more than about 1% of its ICOS binding activity during storage of the formulation at about 5°C for about 1 month, about 2 months, about 3 months, about 4 months, about 5 months, about 6 months, about 7 months, about 8 months, about 9 months, about 10 months, about 11 months, or about 12 months. In one embodiment, a formulation of the disclosure comprises an anti-ICOS antibody, wherein the antibody loses no more than about 50%, no more than about 40%, no more than about 30%, no more than about 20%, no more than about 10%, no more than about 5%, or no more than about 1% of its ICOS binding activity during storage of the formulation at about 5°C for about 1 year, about 2 years, about 3 years, about 4 years, about 5 years, about 6 years, about 7 years, about 8 years, about 9 years, about 10 years, about 11 years, or about 12 years. In a specific embodiment, a formulation of the disclosure is stored in a pre-filled syringe. In a specific embodiment, a formulation of the disclosure comprises an anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein said antibody retains at least 50%, at least 60%, at least 70%, at least 80%, at least 90%, at least 95%, or at least 99% of binding ability to a human ICOS compared to a reference antibody representing the antibody prior to the storage at about 40°C for at least about 1 week, at least about 2 weeks, at least about 3 weeks, or at least about 4 weeks. In one embodiment, a formulation of the disclosure comprises an anti-ICOS antibody, wherein said antibody retains at least 50%, at least 60%, at least 70%, at least 80%, at least 90%, at least 95%, or at least 99% of binding ability to a human ICOS compared to a reference antibody representing the antibody prior to the storage at about 40°C for at least about 1 month, at least about 2 months, at least about 3 months, at least about 4 months, at least about 5 months, or at least about 6 months. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein said antibody retains at least 50%, at least 60%, at least 70%, at least 80%, at least 90%, at least 95%, or at least 99% of binding ability to a human ICOS compared to a reference antibody representing the antibody prior to the storage at about 5°C for at least about 1 month, at least about 2 months, at least about 3 months, at least about 4 months, at least about 5 months, at least about 6 months, at least about 7 months, at least about 8 months, at least about 9 months, at least about 10 months, at least about 11 months, or at least about 12 months. In one embodiment, a formulation of the disclosure comprises an anti-ICOS antibody, wherein said antibody retains at least 50%, at least 60%, at least 70%, at least 80%, at least 90%, at least 95%, or at least 99% of binding ability to a human ICOS compared to a reference antibody representing the antibody prior to the storage at about 5°C for at least about 1 year, at least about 2 years, at least about 3 years, at least about 4 years, at least about 5 years, at least about 6 years, at least about 7 years, at least about 8 years, at least about 9 years, at least about 10 years, at least about 11 years, or at least about 12 years. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises anti-ICOS antibody, wherein said antibody retains at least 50%, at least 60%, at least 70%, at least 80%, at least 90%, at least 95%, or at least 99% of binding ability to a human ICOS compared to a reference antibody representing the antibody prior to the storage at about 40°C for about 1 week, about 2 weeks, about 3 weeks, or about 4 weeks. In one embodiment, a formulation of the disclosure comprises anti-ICOS antibody, wherein said antibody retains at least 50%, at least 60%, at least 70%, at least 80%, at least 90%, at least 95%, or at least 99% of binding ability to a human ICOS compared to a reference antibody representing the antibody prior to the storage at about 40°C for about 1 month, about 2 months, about 3 months, about 4 months, about 5 months, or about 6 months. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises anti-ICOS antibody, wherein said antibody retains at least 50%, at least 60%, at least 70%, at least 80%, at least 90%, at least 95%, or at least 99% of binding ability to a human ICOS compared to a reference antibody representing the antibody prior to the storage at about 5°C for about 1 month, about 2 months, about 3 months, about 4 months, about 5 months, about 6 months, about 7 months, about 8 months, about 9 months, about 10 months, about 11 months, or about 12 months. In one embodiment, a formulation of the disclosure comprises anti-ICOS antibody, wherein said antibody retains at least 50%, at least 60%, at least 70%, at least 80%, at least 90%, at least 95%, or at least 99% of binding ability to a human ICOS compared to a reference antibody representing the antibody prior to the storage at about 5°C for about 1 year, about 2 years, about 3 years, about 4 years, about 5 years, about 6 years, about 7 years, about 8 years, about 9 years, about 10 years, about 11 years, or about 12 years. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein less than 1%, less than 2%, less than 3%, less than 4%, less than 5%, less than 7% or less than 10% of said antibody forms an aggregate as determined by HPSEC upon storage at about 40°C for at least about 1 week, at least about 2 weeks, at least about 3 weeks, or at least about 4 weeks. In one embodiment, a formulation of the disclosure comprises an anti-ICOS antibody, wherein less than 1%, less than 2%, less than 3%, less than 4%, less than 5%, less than 7% or less than 10% of said antibody forms an aggregate as determined by HPSEC upon storage at about 40°C for at least about 1 month, at least about 2 months, at least about 3 months, at least about 4 months, at least about 5 months, or at least about 6 months. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein less than 1%, less than 2%, less than 3%, less than 4%, less than 5%, less than 7% or less than 10% of said antibody forms an aggregate as determined by HPSEC upon storage at about 5°C for at least about 1 month, at least about 2 months, at least about 3 months, at least about 4 months, at least about 5 months, at least about 6 months, at least about 7 months, at least about 8 months, at least about 9 months, at least about 10 months, at least about 11 months, or at least about 12 months. In one embodiment, a formulation of the disclosure comprises an anti-ICOS antibody, wherein less than 1%, less than 2%, less than 3%, less than 4%, less than 5%, less than 7% or less than 10% of said antibody forms an aggregate as determined by HPSEC upon storage at about 5°C for at least about 1 year, at least about 2 years, at least about 3 years, at least about 4 years, at least about 5 years, at least about 6 years, at least about 7 years, at least about 8 years, at least about 9 years, at least about 10 years, at least about 11 years, or at least about 12 years. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein less than 1%, less than 2%, less than 3%, less than 4%, less than 5%, less than 7% or less than 10% of said antibody forms an aggregate as determined by HPSEC upon storage at about 40°C for about 1 week, about 2 weeks, about 3 weeks, or about 4 weeks. In one embodiment, a formulation of the disclosure comprises an anti-ICOS antibody, wherein less than 1%, less than 2%, less than 3%, less than 4%, less than 5%, less than 7% or less than 10% of said antibody forms an aggregate as determined by HPSEC upon storage at about 40°C for about 1 month, about 2 months, about 3 months, about 4 months, about 5 months, or about 6 months. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein less than 1%, less than 2%, less than 3%, less than 4%, less than 5%, less than 7% or less than 10% of said antibody forms an aggregate as determined by HPSEC upon storage at about 5°C for about 1 month, about 2 months, about 3 months, about 4 months, about 5 months, about 6 months, about 7 months, about 8 months, about 9 months, about 10 months, about 11 months, or about 12 months. In one embodiment, a formulation of the disclosure comprises an anti-ICOS antibody, wherein less than 1%, less than 2%, less than 3%, less than 4%, less than 5%, less than 7% or less than 10% of said antibody forms an aggregate as determined by HPSEC upon storage at about 5°C for about 1 year, about 2 years, about 3 years, about 4 years, about 5 years, about 6 years, about 7 years, about 8 years, about 9 years, about 10 years, about 11 years, or about 12 years. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein less than 1%, less than 2%, less than 3%, less than 4%, less than 5%, less than 7% or less than 10% of said antibody is fragmented as determined by RP-HPLC upon storage at about 40°C for at least about 1 week, at least about 2 weeks, at least about 3 weeks, or at least about 4 weeks. In one embodiment, a formulation of the disclosure comprises an anti-ICOS antibody, wherein less than 1%, less than 2%, less than 3%, less than 4%, less than 5%, less than 7% or less than 10% of said antibody is fragmented as determined by RP-HPLC upon storage at about 40°C for at least about 1 month, at least about 2 months, at least about 3 months, at least about 4 months, at least about 5 months, or at least about 6 months. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein less than 1%, less than 2%, less than 3%, less than 4%, less than 5%, less than 7% or less than 10% of said antibody is fragmented as determined by RP-HPLC upon storage at about 5°C for at least about 1 month, at least about 2 months, at least about 3 months, at least about 4 months, at least about 5 months, at least about 6 months, at least about 7 months, at least about 8 months, at least about 9 months, at least about 10 months, at least about 11 months, or at least about 12 months. In one embodiment, a formulation of the disclosure comprises an anti-ICOS antibody, wherein less than 1%, less than 2%, less than 3%, less than 4%, less than 5%, less than 7% or less than 10% of said antibody is fragmented as determined by RP-HPLC upon storage at about 5°C for at least about 1 year, at least about 2 years, at least about 3 years, at least about 4 years, at least about 5 years, at least about 6 years, at least about 7 years, at least about 8 years, at least about 9 years, at least about 10 years, at least about 11 years, or at least about 12 years. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein less than 1%, less than 2%, less than 3%, less than 4%, less than 5%, less than 7% or less than 10% of said antibody is fragmented as determined by RP-HPLC upon storage at about 40°C for about 1 week, about 2 weeks, about 3 weeks, or about 4 weeks. In one embodiment, a formulation of the disclosure comprises an anti-ICOS antibody, wherein less than 1%, less than 2%, less than 3%, less than 4%, less than 5%, less than 7% or less than 10% of said antibody is fragmented as determined by RP-HPLC upon storage at about 40°C for about 1 month, about 2 months, about 3 months, about 4 months, about 5 months, or about 6 months. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure comprises an anti-ICOS antibody, wherein less than 1%, less than 2%, less than 3%, less than 4%, less than 5%, less than 7% or less than 10% of said antibody is fragmented as determined by RP-HPLC upon storage at about 5°C for about 1 month, about 2 months, about 3 months, about 4 months, about 5 months, about 6 months, about 7 months, about 8 months, about 9 months, about 10 months, about 11 months, or about 12 months. In one embodiment, a formulation of the disclosure comprises an anti-ICOS antibody, wherein less than 1%, less than 2%, less than 3%, less than 4%, less than 5%, less than 7% or less than 10% of said antibody is fragmented as determined by RP-HPLC upon storage at about 5°C for about 1 year, about 2 years, about 3 years, about 4 years, about 5 years, about 6 years, about 7 years, about 8 years, about 9 years, about 10 years, about 11 years, or about 12 years. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure is clear and colorless as determined by visual inspection upon storage at about 40°C for at least about 1 week, at least about 2 weeks, at least about 3 weeks, or at least about 4 weeks. In one embodiment, a formulation of the disclosure is clear and colorless as determined by visual inspection upon storage at about 40°C for at least about 1 month, at least about 2 months, at least about 3 months, at least about 4 months, at least about 5 months, or at least about 6 months. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure is clear and colorless as determined by visual inspection upon storage at about 5°C for at least about 1 month, at least about 2 months, at least about 3 months, at least about 4 months, at least about 5 months, at least about 6 months, at least about 7 months, at least about 8 months, at least about 9 months, at least about 10 months, at least about 11 months, or at least about 12 months. In one embodiment, a formulation of the disclosure is clear and colorless as determined by visual inspection upon storage at about 5°C for at least about 1 year, at least about 2 years, at least about 3 years, at least about 4 years, at least about 5 years, at least about 6 years, at least about 7 years, at least about 8 years, at least about 9 years, at least about 10 years, at least about 11 years, or at least about 12 years. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure is clear and colorless as determined by visual inspection upon storage at about 40°C for about 1 week, about 2 weeks, about 3 weeks, or about 4 weeks. In one embodiment, a formulation of the disclosure is clear and colorless as determined by visual inspection upon storage at about 40°C for about 1 month, about 2 months, about 3 months, about 4 months, about 5 months, or about 6 months. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure is clear and colorless as determined by visual inspection upon storage at about 5°C for about 1 month, about 2 months, about 3 months, about 4 months, about 5 months, about 6 months, about 7 months, about 8 months, about 9 months, about 10 months, about 11 months, or about 12 months. In one embodiment, a formulation of the disclosure is clear and colorless as determined by visual inspection upon storage at about 5°C for about 1 year, about 2 years, about 3 years, about 4 years, about 5 years, about 6 years, about 7 years, about 8 years, about 9 years, about 10 years, about 11 years, or about 12 years. In a specific embodiment, a formulation of the disclosure is stored in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In certain embodiments, the formulations of the disclosure maintain improved aggregation profiles upon storage, for example, for extended periods (for example, but not limited to 1 week, 1 month, 6 months, 1 year, 2 years, 3 years or 5 years) at room temperature or 4°C or for periods (such as, but not limited to 1 week, 2 weeks, 3 weeks, 1 month, 2 months, 3 months, or 6 months) at elevated temperatures such as 38°C-42°C. In certain embodiments, the formulations maintain improved aggregation profiles upon storage while exposed to light or stored in the dark in a variety of humidity conditions including but not limited to a relative humidity of up to 10%, or up to 20%, or up to 30%, or up to 40%, or up to 50%, or up to 60%, or up to 70%, or up to 80%, or up to 90%, or up to 100%. It will be understood in the art that the term "ambient" conditions generally refers to temperatures of about 20 °C at a relative humidity of between 10% and 60% with exposure to light. Similarly, temperatures between about 2 °C and about 8 °C at a relative humidity of less then about 10% are collectively referred to as "4 °C" or "5 °C", temperatures between about 23 °C and about 27 °C at a relative humidity of about 60% are collectively referred to as "25 °C" and temperatures between about 38 °C and about 42 °C at a relative humidity of about 75% are collectively referred to as "40 °C." In a specific embodiment, a formulation of the disclosure is stored in a pre-filled syringe.

In certain embodiments, after storage at 4 °C for at least one month, the formulations of the disclosure comprise (or consists of as the aggregate fraction) a particle profile of less than about 3.4 E +5 particles/ml of diameter 2-4 $\mu$m, less than about 4.0 E +4 particles/ml of diameter 4-10 $\mu$m, less than about 4.2 E +3 particles/ml of diameter 10-20 $\mu$m, less than about 5.0 E +2 particles/ml of diameter 20-30 $\mu$m, less than about 7.5 E +1 particles/ml of diameter 30-40 $\mu$m, and less than about 9.4 particles/ml of diameter 40-60 $\mu$m as determined by a particle multisizer. In certain embodiments, the formulations of the disclosure contain no detectable particles greater than 40 $\mu$m, or greater than 30 $\mu$m. In a specific embodiment, a formulation of the disclosure is stored in a pre-filled syringe.

Numerous methods useful for determining the degree of aggregation, and/or types and/or sizes of aggregates present in a protein formulation (e.g., antibody formulation of the disclosure) are known in the art, including but not limited to, size exclusion chromatography (SEC), high performance size exclusion chromatography (HPSEC), static light scattering (SLS), Fourier Transform Infrared Spectroscopy (FTIR), circular dichroism (CD), urea-induced protein unfolding techniques, intrinsic tryptophan fluorescence, differential scanning calorimetry, and 1-anilino-8-naphthalenesulfonic acid (ANS) protein binding techniques. For example, size exclusion chromatography (SEC) may be performed to separate molecules on the basis of their size, by passing the molecules over a column packed with the appropriate resin, the larger molecules (*e.g.* aggregates) will elute before smaller molecules (*e.g.* monomers). The molecules are generally detected by UV absorbance at 280 nm and may be collected for further characterization. High pressure liquid chromatographic columns are often utilized for SEC analysis (HP-SEC). Specific SEC methods are detailed in the section entitled "Examples" infra. Alternatively, analytical ultracentrifugation (AUC) may be utilized. AUC is an orthogonal technique which determines the sedimentation coefficients (reported in Svedberg, S) of macromolecules in a liquid sample. Like SEC, AUC is capable of separating and detecting antibody fragments/aggregates from monomers and is further able to provide information on molecular mass. Protein aggregation in the formulations may also be characterized by particle counter analysis using a coulter counter or by turbidity measurements using a turbidimeter. Turbidity is a measure of the amount by which the particles in a solution scatter light and, thus, may be used as a general indicator of protein aggregation. In addition, non-reducing polyacrylamide gel electrophoresis (PAGE) or capillary gel electrophoresis (CGE) may be used to characterize the aggregation and/or fragmentation state of antibodies or a fragment thereof in a formulation of the disclosure.

In one embodiment, a formulation of the disclosure is for parenteral administration. In one embodiment, a formulation of the disclosure is an injectable formulation. In one embodiment, a formulation of the disclosure is for intravenous, subcutaneous, or intramuscular administration. In a specific embodiment, a formulation of the disclosure comprises an anti-ICOS antibody wherein said formulation is for subcutaneous injection. In a specific embodiment, a formulation of the disclosure is provided in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure is for intravenous administration wherein said formulation comprises between about 20 mg/ml and about 40 mg/ml of an anti-ICOS antibody or a fragment thereof. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure is for subcutaneous administration wherein said formulation comprises between about 70 mg/ml and about 250 mg/ml of an anti-ICOS antibody or a fragment thereof. In a specific embodiment, a formulation of the disclosure is provided in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure is for aerosol administration.

The present disclosure also provides a pharmaceutical unit dosage form suitable for parenteral administration to a human which comprises an anti-ICOS antibody formulation in a suitable container. In one embodiment, a pharmaceutical unit dosage of the disclosure comprises an intravenously, subcutaneously, or intramuscularly delivered anti-ICOS antibody formulation. In another embodiment, a pharmaceutical unit dosage of the disclosure comprises aerosol delivered anti-ICOS antibody formulation. In a specific embodiment, a pharmaceutical unit dosage of the disclosure comprises a subcutaneously delivered anti-ICOS antibody formulation. In another embodiment, a pharmaceutical unit dosage of the disclosure comprises an aerosol delivered anti-ICOS antibody formulation. In a further embodiment, a pharmaceutical unit dosage of the disclosure comprises an intranasally administered anti-ICOS antibody formulation. In one embodiment, a suitable container is a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

In one embodiment, a formulation of the disclosure is provided in a sealed container. In a specific embodiment, a formulation of the disclosure is provided in a pre-filled syringe. In a specific embodiment, a formulation of the disclosure comprises the anti-ICOS antibody comprising a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

The present disclosure further provided a kit comprising an anti-ICOS antibody formulation of the disclosure.

The present disclosure also relates to methods of treating and preventing T cell-mediated diseases and disorders, such as, but not limited to, chronic infection, autoimmune disease or disorder, inflammatory disease or disorder, graft-versus-host disease (GVHD), transplant rejection, and T cell proliferative disorder in a human, comprising administering to a human in need thereof a formulation comprising an anti-ICOS antibody with enhanced effector function (e.g., antibody-dependent cellular cytotoxicity (ADCC), complement-dependent cell-mediated cytotoxicity (CDC), and/or antibody-dependent phagocytosis) in an amount sufficient to deplete circulating ICOS expressing cells. In a particular aspect, the present disclosure also concerns methods of treating and preventing T cell-mediated diseases and disorders, such as, but not limited to, chronic infection, autoimmune disease or disorder, inflammatory disease or disorder, graft-versus-host disease (GVHD), transplant rejection, and T cell proliferative disorder in a human comprising administration of a therapeutically effective regimen of an anti-ICOS antibody with enhanced effector function, which is of the IgG1 or IgG3 human isotype.

The present disclosure also provides methods of preventing, managing, treating or ameliorating an inflammatory disease or disorder, an autoimmune disease or disorder, a proliferative disease, an infection, a disease or disorder associated with or characterized by aberrant expression and/or activity of ICOS, a disease or disorder associated with or characterized by aberrant expression and/or activity of the ICOS receptor, or one or more symptoms thereof.

In one embodiment, a method of the disclosure comprises administering to a subject in need thereof a prophylactically or therapeutically effective amount of an anti-ICOS antibody formulation. In one embodiment, a method of the disclosure is for the prevention, treatment, management or amelioration of a disease or disorder selected from the group consisting of multiple sclerosis, inflammatory bowel disease, insulin dependent diabetes mellitus, psoriasis, autoimmune thyroiditis, rheumatoid arthritis, glomerulonephritis, systemic lupus erythematosus, idiopathic inflammatory myopathies (IIM), dermatomyositis (DM), polymyositis (PM), and inclusion body myositis (IBM). In a specific embodiment, a method of the disclosure is for the prevention, treatment, management or amelioration of systemic lupus erythematosus. In a specific embodiment, a method of the disclosure is for the prevention, treatment, management or amelioration of psoriasis. In a specific embodiment, a method of the disclosure is for the prevention, treatment, management or amelioration of autoimmune diabetes. In another embodiment, a method of the disclosure is for the prevention, treatment, management or amelioration of transplant rejection or graft versus host disease. In a further embodiment, a method of the disclosure is for the prevention, treatment, management or amelioration of idiopathic inflammatory myopathies (IIM), dermatomyositis (DM), polymyositis (PM), and inclusion body myositis (IBM).

In one embodiment, a method of the disclosure for the prevention, treatment, management or amelioration of a disease or disorder further comprises administering to said subject a prophylactically or therapeutically effective amount of a prophylactic or therapeutic agent other than an antibody or antibody fragment that specifically binds to ICOS.

In one embodiment, a method of the disclosure for the prevention, treatment, management or amelioration of a disease or disorder further comprises administering to said subject a prophylactically or therapeutically effective amount of a prophylactic or therapeutic agent other than an antibody or antibody fragment that specifically binds ICOS, wherein said prophylactic or therapeutic agent is an anti-inflammatory agent, immunomodulatory agent, anti-angiogenic agent, or anti-cancer agent.

5.3. Antibodies Useful in the Formulations of the Disclosure

The present disclosure provides formulations of antibodies that specifically bind to human ICOS and have an enhanced effector function. In one embodiment, a formulation of the disclosure comprises an anti-ICOS antibody with enhanced effector function, such as, but not limited to, enhanced ADCC, enhanced CDC, and enhanced antibody-dependent phagocytosis. In a specific embodiment, a formulation of the disclosure comprises an anti-human ICOS antibody with enhanced ADCC activity. These antibodies can be used for therapeutic, including prophylactic, purposes, for example in situations where the production or expression of ICOS is associated with pathological symptoms. Such antibodies can also be used for the diagnosis of various diseases or for the study of the evolution of such diseases.

The antibodies useful in the present disclosure include, but are not limited to, monoclonal antibodies, synthetic antibodies, multispecific antibodies (including bi-specific antibodies), human antibodies, humanized antibodies, chimeric antibodies, single-chain Fvs (scFv) (including bi-specific scFvs), single chain antibodies, Fab fragments, F(ab') fragments, disulfide-linked Fvs (sdFv), and epitope-binding fragments of any of the above. In particular, antibodies of the present disclosure include immunoglobulin molecules and immunologically active portions of immunoglobulin molecules, i.e., molecules that contain an antigen binding site that specifically binds to an antigen. The immunoglobulin molecules of the disclosure can be of any type (*e.g.,* IgG, IgE, IgM, IgD, IgA and IgY), class (*e.g.,* $IgG_1$, $IgG_2$, $IgG_3$, $IgG_4$, $IgA_1$ and $IgA_2$) or subclass of immunoglobulin molecule.

The antibodies useful in the present disclosure may be from any animal origin including birds and mammals (for example, but not limited to, human, murine, donkey, sheep, rabbit, goat, guinea pig, camel, horse, or chicken). In specific embodiments, the antibodies are human or humanized monoclonal antibodies.

The antibodies useful in the present disclosure may be monospecific, bispecific, trispecific or of greater multispecificity. Multispecific antibodies may specifically bind to different epitopes of a polypeptide or may specifically bind to both a polypeptide as well a heterologous epitope, such as a heterologous polypeptide or solid support material. See, *e.g.*, International Publication Nos. WO 93/17715, WO 92/08802, WO 91/00360, and WO 92/05793; Tutt, et al., 1991, J. Immunol. 147:60-69; U.S. Patent Nos. 4,474,893, 4,714,681, 4,925,648, 5,573,920, and 5,601,819; and Kostelny et al., 1992, J. Immunol. 148:1547-1553.

The antibodies useful in the present disclosure can be single-chain antibodies. The design and construction of a single-chain antibody is described in Marasco et al, 1993, Proc Natl Acad Sci 90:7889-7893.

The present disclosure provides formulations of antibodies that specifically bind to human ICOS and have an enhanced effector function. In one embodiment, a formulation of the disclosure comprises an anti-ICOS antibody with enhanced effector function, such as, but not limited to, enhanced ADCC, enhanced CDC, and enhanced antibody-dependent phagocytosis.

The present disclosure further provides formulations of anti-ICOS antibodies that efficiently deplete ICOS expressing cells in a mouse xenograft model system. In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of ICOS expressing cells in a mouse xenograft model system.

The present disclosure further provides formulations of anti-ICOS antibodies that efficiently deplete ICOS expressing cells in a transgenic mouse model system. In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of ICOS expressing cells in a transgenic mouse model system.

The present disclosure also provides formulations of anti-ICOS antibodies that efficiently deplete ICOS expressing cells in a primate (non-human primate or human). In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of ICOS expressing cells in a primate (non-human primate or human).

The present disclosure also provides formulations of anti-ICOS antibodies that efficiently deplete ICOS expressing T cells in a primate (non-human primate or human). In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of ICOS expressing T cells in a primate (non-human primate or human).

The present disclosure also provides formulations of anti-ICOS antibodies that efficiently deplete ICOS expressing T helper cells in a primate (non-human primate or human). In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of ICOS expressing T helper cells in a primate (non-human primate or human).

The present disclosure also provides formulations of anti-ICOS antibodies that efficiently deplete ICOS expressing Th1 cells in a primate (non-human primate or human). In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of ICOS expressing Th1 cells in a primate (non-human primate or human).

The present disclosure also provides formulations of anti-ICOS antibodies that efficiently deplete ICOS expressing Th2 cells in a primate (non-human primate or human). In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of ICOS expressing Th2 cells in a primate (non-human primate or human).

The present disclosure also provides formulations of anti-ICOS antibodies that efficiently deplete ICOS expressing Th17 cells in a primate (non-human primate or human). In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of ICOS expressing Th17 cells in a primate (non-human primate or human).

The present disclosure also provides formulations of anti-ICOS antibodies that efficiently deplete ICOS expressing memory helper T cells in a primate (non-human primate or human). In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of ICOS expressing memory helper T cells in a primate (non-human primate or human).

Depletion of a particular cell type may lead to the depletion of a secreted product of said cell type. For example, depletion of Th17 cells using an effector function enhanced anti-ICOS antibody of the disclosure may lead to depletion of IL-17. The present disclosure also provides formulations of anti-ICOS antibodies that efficiently deplete IL-17 in a primate (non-human primate or human). In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of IL-17 in a primate (non-human primate or human).

The present disclosure also provides formulations of anti-ICOS antibodies that efficiently deplete IL-2 in a primate (non-human primate or human). In one embodiment, administration of one or more therapeutic doses of an anti-ICOS antibody formulation of the disclosure may achieve at least about 20%, at least about 30%, at least about 40%, at least about 50%, at least about 60%, at least about 70%, at least about 80%, at least about 90%, at least about 95%, at least about 97%, at least about 99%, or at least about 100% depletion of IL-2 in a primate (non-human primate or human).

The present disclosure provides formulations of anti-ICOS antibodies that upon administration efficiently prevent germinal center formation in a secondary lymphoid organ of a primate (non-human primate or human). In one embodiment, the secondary lymphoid organ is a lymph node. In another embodiment, the secondary lymphoid organ is the spleen. In a further embodiment, the secondary lymphoid organ is the tonsil. In one embodiment, the secondary lymphoid organ is a mesenteric lymph node.

The present disclosure also provides formulations of anti-ICOS antibodies that upon administration efficiently disrupt germinal center architecture in a secondary lymphoid organ of a primate (non-human primate or human). In one embodiment, the secondary lymphoid organ is a lymph node. In another embodiment, the secondary lymphoid organ is the spleen. In a further embodiment, the secondary lymphoid organ is the tonsil. In one embodiment, the secondary lymphoid organ is a mesenteric lymph node.

The present disclosure also provides formulations of anti-ICOS antibodies that upon administration efficiently deplete germinal center B cells from a secondary lymphoid organ in a primate (non-human primate or human). In one embodiment, the secondary lymphoid organ is a lymph node. In another embodiment, the secondary lymphoid organ is the spleen. In a further embodiment, the secondary lymphoid organ is the tonsil. In one embodiment, the secondary lymphoid organ is a mesenteric lymph node.

The present disclosure also provides formulations of anti-ICOS antibodies that upon administration efficiently deplete circulating class switched B cells in a primate (non-human primate or human). In one embodiment, the administration of one or more therapeutic doses of an anti-ICOS antibody formulation of the disclosure depletes circulating class switched B cells in a primate (non-human primate or human) for at least 1 day, at least 2 days at least 5 days, at least 1 week, at least 2 weeks, at least 3 weeks, at least 1 month, at least 2 months, at least 3 months, at least 4 months, at least 5 months, at least 6 months, at least 9 months. Depletion of circulating class switched B cells is considered to "substantially persist" during the time period following the administration of one or more doses of anti-ICOS antibody when the number of circulating class switched B cells is at least 10% lower in the antibody treated sample than the number of circulating class switched B cells in the untreated control sample.

In one embodiment, a formulation of the disclosure comprises an anti-ICOS antibody that mediates antibody-dependent cellular cytotoxicity (ADCC), complement-dependent cell-mediated cytotoxicity (CDC), and/or antibody-dependent phagocytosis. In one embodiment, an anti-ICOS antibody of the disclosure mediates antibody-dependent cellular cytotoxicity (ADCC) and/or antibody-dependent phagocytosis. In one embodiment, an anti-ICOS antibody of the disclosure has enhanced antibody-dependent cellular cytotoxicity (ADCC).

In one embodiment, a formulation of the disclosure comprises an anti-ICOS antibody comprising a variant Fc region that mediates enhanced antibody-dependent cellular cytotoxicity (ADCC). In a further embodiment, an anti-ICOS antibody of the disclosure comprises a variant Fc region comprising at least one substitution of an amino acid residue selected from the group consisting of: residue 239, 330, and 332, wherein the amino acid residue positions are determined according to the EU convention. In a specific embodiment, an anti-ICOS antibody of the disclosure comprises a variant Fc region comprising at least on amino acid substitution selected from the group consisting of: S239D, A330L, and I332E; wherein the amino acid residue positions are determined according to the EU convention. In a further embodiment, an anti-ICOS antibody of the disclosure comprises at least one amino acid residue selected from the group consisting of: D at position 239, L at position 330, and E at position 332; wherein the amino acid residue positions are determined according to the EU convention.

In one embodiment, a formulation of the disclosure comprises an anti-ICOS antibody having an engineered Fc region comprising at least one engineered glycoform, wherein said engineered Fc region mediates enhanced antibody-dependent cellular cytotoxicity (ADCC). In one embodiment, an anti-ICOS antibody of the disclosures comprises an engineered Fc region lacking glycosylation. In one embodiment, an anti-ICOS antibody of the disclosure comprises an engineered Fc region having complex N-glycoside-linked sugar chains linked to Asn297 in which fucose is not bound to N-acetylglucosamine in the reducing end.

In certain embodiments, a formulation of the disclosure comprises an anti-ICOS antibody having a variant Fc region that has a higher affinity for an Fc binding protein such as, but not limited to, Fc receptor, C1q than a wild type Fc region. In one embodiment, an anti-ICOS antibody of the disclosure comprises a variant Fc region that has higher affinity for the FcγRIIIA receptor protein than a wild type Fc region.

In certain embodiments, a formulation of the disclosure comprises an anti-ICOS antibody having an engineered Fc region comprising at least one engineered glycoform, wherein said engineered Fc region has a higher affinity for an Fc binding protein such as, but not limited to, Fc receptor, C1q than a wild type Fc region. In one embodiment, an anti-ICOS antibody of the disclosure comprises an engineered Fc region comprising at least one engineered glycoform, wherein said engineered Fc region has higher affinity for the FcγRIIIA receptor protein than a wild type Fc region.

In one embodiment, an anti-ICOS antibody of the disclosure comprises a variant Fc region. In another embodiment, an anti-ICOS antibody of the disclosure comprises a variant Fc region that has an altered affinity for an Fc ligand protein. In a further embodiment, an anti-ICOS antibody of the disclosure comprises a variant Fc region that has an altered affinity for an Fc ligand selected from the group consisting of: FcγRIA, FcγRIIA, FcγRIIB, FcγRIIIA, FcγRIIIB, FcγRIV, and C1q. In a specific embodiment, an anti-ICOS antibody of the disclosure comprises a variant Fc region that has an altered affinity for the FcγRIIIA protein. In a further embodiment, an anti-ICOS antibody of the disclosure comprises a variant Fc region that has an altered affinity for the C1q protein. In a specific embodiment, an Fc ligand protein may be a mouse, human or primate (e.g., cynomolgus) Fc ligand protein.

In one embodiment, an anti-ICOS antibody of the disclosure comprises a variant Fc region that has an increased affinity for an Fc ligand protein. In a further embodiment, an anti-ICOS antibody of the disclosure comprises a variant Fc region that has an increased affinity for an Fc ligand selected from the group consisting of: FcγRIA, FcγRIIA, FcγRIIB, FcγRIIIA, FcγRIIIB, FcγRIV, and C1q. In a specific embodiment, an anti-ICOS antibody of the disclosure comprises a variant Fc region that has an increased affinity for the FcγRIIIA protein. In a further embodiment, an anti-ICOS antibody of the disclosure comprises a variant Fc region that has an increased affinity for the C1q protein. In a specific embodiment, an Fc ligand protein may be a mouse, human or primate (e.g., cynomolgus) Fc ligand protein.

In one embodiment, an anti-ICOS antibody of the disclosure comprises a variant Fc region wherein said variant Fc region comprises at least one amino acid substitution, insertion or deletion. In another embodiment, an anti-ICOS antibody of the disclosure comprises a variant Fc region comprising at least one amino acid substitution, insertion or deletion wherein said at least one amino acid residue substitution, insertion or deletion results in an increased affinity for an Fc ligand selected from the group consisting of: FcγRIA, FcγRIIA, FcγRIIB, FcγRIIIA, FcγRIIIB, FcγRIV, and C1q. In a specific embodiment, an anti-ICOS antibody of the disclosure comprises a variant Fc region comprising at least one amino acid substitution, insertion or deletion wherein said at least one amino acid residue substitution, insertion or deletion results in an increased affinity for the FcγRIIIA protein. In a further embodiment, an anti-ICOS antibody of the disclosure comprises a variant Fc region comprising at least one amino acid substitution, insertion or deletion wherein said at least one amino acid residue substitution, insertion or deletion results in an increased affinity for the C1q protein. In a specific embodiment, an Fc ligand protein may be a mouse, human or primate (e.g., cynomolgus) Fc ligand protein.

In one embodiment, an anti-ICOS antibody of the disclosure comprises a variant Fc region comprising at least one amino acid substitution, insertion or deletion wherein said at least one amino acid residue is selected from the group consisting of: residue 239, 330, and 332, wherein amino acid residues are numbered following the EU index. In another embodiment, an anti-ICOS antibody of the disclosure comprises a variant Fc region comprising at least one amino acid substitution, insertion or deletion wherein said at least one substituted, inserted or deleted amino acid residue is selected from the group consisting of: residue 239, 330, and 332, wherein amino acid residues are numbered following the EU index. In a further embodiment, an anti-ICOS antibody described herein comprises a variant Fc region comprising at least one amino acid substitution wherein said at least one substituted amino acid residue is selected from the group consisting of: residue 239, 330, and 332, wherein amino acid residues are numbered following the EU index. In another embodiment, an anti-ICOS antibody described herein comprises a variant Fc region comprising at least one amino acid substitution wherein said at least one amino acid substitution is selected from the group consisting of: S239D, A330L, A330Y, and I332E, wherein amino acid residues are numbered following the EU index. In a specific embodiment, an anti-ICOS antibody of the disclosure comprises a variant Fc region comprising the S239D, A330L, and I332E amino acid substitutions, wherein amino acid residues are numbered following the EU index.

In one embodiment, an anti-ICOS antibody of the disclosure comprises a variant Fc region comprising at least one of the amino acid residues selected from the group consisting of: D at residue 239, E at residue 239, L at residue 330, Y at residue 330, E at residue 332, and D at residue 332, wherein amino acid residues are numbered following the EU index. In a specific embodiment, an anti-ICOS antibody of the disclosure comprises a variant Fc region comprising D at residue 239, L at residue 330, and E at residue 332, wherein amino acid residues are numbered following the EU index.

In one embodiment, an anti-ICOS antibody of the disclosure comprises an engineered Fc region wherein the engineered Fc region comprises a posttranslational modification that is different from that of the parental anti-ICOS antibody. In a specific embodiment, an anti-ICOS antibody of the disclosure comprises an engineered Fc region wherein said engineered Fc region comprises complex N-glycoside-linked sugar chains in which fucose is not bound to N-acetylglucosamine in the reducing end in the sugar chain.

In one embodiment, an anti-ICOS antibody of the disclosure comprises an engineered Fc region that has an altered affinity for an Fc ligand protein. In a further embodiment, an anti-ICOS antibody of the disclosure comprises an engineered Fc region that has an altered affinity for an Fc ligand selected from the group consisting of: FcγRIA, FcγRIIA, FcγRIIB, FcγRIIIA, FcγRIIIB, FcγRIV, and C1q. In a specific embodiment, an anti-ICOS antibody of the disclosure comprises an engineered Fc region that has an altered affinity for the FcγRIIIA protein. In a further embodiment, an anti-ICOS antibody of the disclosure comprises an engineered Fc region that has an altered affinity for the C1q protein.

In one embodiment, an anti-ICOS antibody of the disclosure comprises an engineered Fc region that has an increased affinity for an Fc ligand protein. In a further embodiment, an anti-ICOS antibody of the disclosure comprises an engineered Fc region that has an increased affinity for an Fc ligand selected from the group consisting of: FcγRIA, FcγRIIA, FcγRIIB, FcγRIIIA, FcγRIIIB, FcγRIV, and C1q. In a specific embodiment, an anti-ICOS antibody of the disclosure comprises an engineered Fc region that has an increased affinity for the FcγRIIIA protein. In a further embodiment, an anti-ICOS antibody of the disclosure comprises an engineered Fc region that has an increased affinity for the C1q protein.

In one embodiment, an anti-ICOS antibody of the disclosure comprises an engineered Fc region wherein said engineered Fc region comprises a reduced level of fucose compared to a native antibody. In another embodiment, an anti-ICOS antibody of the disclosure comprises an engineered Fc region comprising a reduced level of fucose, wherein said reduction in fucose level results in an increased affinity for an Fc ligand selected from the group consisting of: FcγRIA, FcγRIIA, FcγRIIB, FcγRIIIA, FcγRIIIB, FcγRIV, and C1q. In a specific embodiment, an anti-ICOS antibody of the disclosure comprises an engineered Fc region comprising a reduced level of fucose, wherein said reduction in fucose level results in an increased affinity for the FcγRIIIA protein. In a further embodiment, an anti-ICOS antibody of the disclosure comprises an engineered Fc region comprising a reduced level of fucose, wherein said reduction in fucose level results in an increased affinity for the C1q protein.

Anti-ICOS antibodies described herein comprise Fc regions having a high binding affinity for the human FcγRIIIA protein. In one embodiment, an anti-ICOS antibody of the disclosure comprises an Fc region that has an affinity constant or $K_a$ ($k_{on}/k_{off}$) of at least $10^3$ $M^{-1}$, at least 5 X $10^3$ $M^{-1}$, at least $10^4$ $M^{-1}$, at least 5 X $10^4$ $M^{-1}$, at least $10^5$ $M^{-1}$, at least 5 X $10^5$ $M^{-1}$, at least $10^6$ $M^{-1}$, at least 5 X $10^6$ $M^{-1}$, at least $10^7$ $M^{-1}$, at least 5 X $10^7$ $M^{-1}$, at least $10^8$ $M^{-1}$, at least 5 X $10^8$ $M^{-1}$, at least $10^9$ $M^{-1}$, at least 5 X $10^9$ $M^{-1}$, at least $10^{10}$ $M^{-1}$, at least 5 X $10^{10}$ $M^{-1}$, at least $10^{11}$ $M^{-1}$, at least 5 X $10^{11}$ $M^{-1}$, at least $10^{12}$ $M^{-1}$, or at least 5 X $10^{12}$ $M^{-1}$. In another embodiment, an anti-ICOS antibody of the disclosure comprises an Fc region that has a dissociation constant or $K_d$ ($k_{off}/k_{on}$) of less than $5x10^{-3}$ M, less than $10^{-3}$ M, less than $5x10^{-4}$ M, less than $10^{-4}$ M, less than $5x10^{-5}$ M, less than $10^{-5}$ M, less than $5x10^{-6}$ M, less than $10^{-6}$ M, less than $5x10^{-7}$ M, less than $10^{-7}$ M, less than $5x10^{-8}$ M, less than $10^{-8}$ M, less than $5x10^{-9}$ M, less than $10^{-9}$ M, less than $5x10^{-10}$ M, less than $10^{-10}$ M, less than $5x10^{-11}$ M, less than $10^{-11}$ M, less than $5x10^{-12}$ M, or less than $10^{-12}$ M.

An antibody used in accordance with a method described herein may comprise an Fc region that binds to human FcγRIIIA with a dissociation constant ($K_d$) of less than 3000 nM, less than 2500 nM, less than 2000 nM, less than 1500 nM, less than 1000 nM, less than 750 nM, less than 500 nM, less than 250 nM, less than 200 nM, less than 150 nM, less than 100 nM, less than 75 nM, less than 50 nM, less than 25 nM, less than 10 nM, less than 5 nM, less than 1 nM as assessed using a method described herein or known to one of skill in the art (*e.g.,* a BIAcore assay, ELISA) (Biacore International AB, Uppsala, Sweden). In a specific embodiment, an antibody used in accordance with a method described herein may comprise an Fc region that binds to human Fc$\gamma$RIIIA with a dissociation constant (K$_d$) of between 1 to 3000 nM, 1 to 3000 nM, 1 to 2000 nM, 1 to 1500 nM, 1 to 1000 nM, 1 to 750 nM, 1 to 500 nM, 1 to 250 nM, 1 to 100 nM, 1 to 50 nM, 1 to 25 nM, 1 to 10 nM as assessed using a method described herein or known to one of skill in the art (*e.g.,* a BIAcore assay, ELISA). In another embodiment, an anti-ICOS antibody used in accordance with a method described herein may comprise an Fc region that binds to human Fc$\gamma$RIIIA with a dissociation constant (K$_d$) of 500 nM, 250 nM, 100 nM, 75 nM, 50 nM, 25 nM, 10 nM or 1 nM as assessed using a method described herein or known to one of skill in the art (*e.g.,* a BIAcore assay, ELISA).

Anti-ICOS antibodies described herein comprise Fc regions having a high binding affinity for the non-human primate (e.g., cynomolgus) Fc$\gamma$RIIIA protein. In one embodiment, an anti-ICOS antibody of the disclosure comprises an Fc region that has an affinity constant or K$_a$ (k$_{on}$/k$_{off}$) of at least $10^3$ M$^{-1}$, at least $5 \times 10^3$ M$^{-1}$, at least $10^4$ M$^{-1}$, at least $5 \times 10^4$ M$^{-1}$, at least $10^5$ M$^{-1}$, at least $5 \times 10^5$ M$^{-1}$, at least $10^6$ M$^{-1}$, at least $5 \times 10^6$ M$^{-1}$, at least $10^7$ M$^{-1}$, at least $5 \times 10^7$ M$^{-1}$, at least $10^8$ M$^{-1}$, at least $5 \times 10^8$ M$^{-1}$, at least $10^9$ M$^{-1}$, at least $5 \times 10^9$ M$^{-1}$, at least $10^{10}$ M$^{-1}$, at least $5 \times 10^{10}$ M$^{-1}$, at least $10^{11}$ M$^{-1}$, at least $5 \times 10^{11}$ M$^{-1}$, at least $10^{12}$ M$^{-1}$, or at least $5 \times 10^{12}$ M$^{-1}$. In another embodiment, an anti-ICOS antibody of the disclosure comprises an Fc region that has a dissociation constant or K$_d$ (k$_{off}$/k$_{on}$) of less than $5 \times 10^{-3}$ M, less than $10^{-3}$ M, less than $5 \times 10^{-4}$ M, less than $10^{-4}$ M, less than $5 \times 10^{-5}$ M, less than $10^{-5}$ M, less than $5 \times 10^{-6}$ M, less than $10^{-6}$ M, less than $5 \times 10^{-7}$ M, less than $10^{-7}$ M, less than $5 \times 10^{-8}$ M, less than $10^{-8}$ M, less than $5 \times 10^{-9}$ M, less than $10^{-9}$ M, less than $5 \times 10^{-10}$ M, less than $10^{-10}$ M, less than $5 \times 10^{-11}$ M, less than $10^{-11}$ M, less than $5 \times 10^{-12}$ M, or less than $10^{-12}$ M.

An antibody used in accordance with a method described herein may comprise an Fc region that binds to non-human primate (e.g., cynomolgus) Fc$\gamma$RIIIA with a dissociation constant (K$_d$) of less than 3000 nM, less than 2500 nM, less than 2000 nM, less than 1500 nM, less than 1000 nM, less than 750 nM, less than 500 nM, less than 250 nM, less than 200 nM, less than 150 nM, less than 100 nM, less than 75 nM, less than 50 nM, less than 25 nM, less than 10 nM, less than 5 nM, less than 1 nM as assessed using a method described herein or known to one of skill in the art (*e.g.,* a BIAcore assay, ELISA) (Biacore International AB, Uppsala, Sweden). In a specific embodiment, an antibody used in accordance with a method described herein may comprise an Fc region that binds to non-human primate (e.g., cynomolgus) Fc$\gamma$RIIIA with a dissociation constant (K$_d$) of between 1 to 3000 nM, 1 to 3000 nM, 1 to 2000 nM, 1 to 1500 nM, 1 to 1000 nM, 1 to 750 nM, 1 to 500 nM, 1 to 250 nM, 1 to 100 nM, 1 to 50 nM, 1 to 25 nM, 1 to 10 nM as assessed using a method described herein or known to one of skill in the art (*e.g.,* a BIAcore assay, ELISA). In another embodiment, an anti-ICOS antibody used in accordance with a method described herein may comprise an Fc region that binds to non-human primate (e.g., cynomolgus) Fc$\gamma$RIIIA with a dissociation constant (K$_d$) of 500 nM, 250 nM, 100 nM, 75 nM, 50 nM, 25 nM, 10 nM or 1 nM as assessed using a method described herein or known to one of skill in the art (*e.g.,* a BIAcore assay, ELISA).

Anti-ICOS antibodies described herein comprise Fc regions having a high binding affinity for the mouse Fc$\gamma$RIIIA protein. In one embodiment, an anti-ICOS antibody of the disclosure comprises an Fc region that has an affinity constant or K$_a$ (k$_{on}$/k$_{off}$) of at least $10^3$ M$^{-1}$, at least $5 \times 10^3$ M$^{-1}$, at least $10^4$ M$^{-1}$, at least $5 \times 10^4$ M$^{-1}$, at least $10^5$ M$^{-1}$, at least $5 \times 10^5$ M$^{-1}$, at least $10^6$ M$^{-1}$, at least $5 \times 10^6$ M$^{-1}$, at least $10^7$ M$^{-1}$, at least $5 \times 10^7$ M$^{-1}$, at least $10^8$ M$^{-1}$, at least $5 \times 10^8$ M$^{-1}$, at least $10^9$ M$^{-1}$, at least $5 \times 10^9$ M$^{-1}$, at least $10^{10}$ M$^{-1}$, at least $5 \times 10^{10}$ M$^{-1}$, at least $10^{11}$ M$^{-1}$, at least $5 \times 10^{11}$ M$^{-1}$, at least $10^{12}$ M$^{-1}$, or at least $5 \times 10^{12}$ M$^{-1}$. In another embodiment, an anti-ICOS antibody of the disclosure comprises an Fc region that has a dissociation constant or K$_d$ (k$_{off}$/k$_{on}$) of less than $5 \times 10^{-3}$ M, less than $10^{-3}$ M, less than $5 \times 10^{-4}$ M, less than $10^{-4}$ M, less than $5 \times 10^{-5}$ M, less than $10^{-5}$ M, less than $5 \times 10^{-6}$ M, less than $10^{-6}$ M, less than $5 \times 10^{-7}$ M, less than $10^{-7}$ M, less than $5 \times 10^{-8}$ M, less than $10^{-8}$ M, less than $5 \times 10^{-9}$ M, less than $10^{-9}$ M, less than $5 \times 10^{-10}$ M, less than $10^{-10}$ M, less than $5 \times 10^{-11}$ M, less than $10^{-11}$ M, less than $5 \times 10^{-12}$ M, or less than $10^{-12}$ M.

An antibody used in accordance with a method described herein may comprise an Fc region that binds to mouse Fc$\gamma$RIIIA with a dissociation constant (K$_d$) of less than 3000 nM, less than 2500 nM, less than 2000 nM, less than 1500 nM, less than 1000 nM, less than 750 nM, less than 500 nM, less than 250 nM, less than 200 nM, less than 150 nM, less than 100 nM, less than 75 nM, less than 50 nM, less than 25 nM, less than 10 nM, less than 5 nM, less than 1 nM as assessed using a method described herein or known to one of skill in the art (*e.g.,* a BIAcore assay, ELISA) (Biacore International AB, Uppsala, Sweden). In a specific embodiment, an antibody used in accordance with a method described herein may comprise an Fc region that binds to mouse Fc$\gamma$RIIIA with a dissociation constant (Kd) of between 1 to 3000 nM, 1 to 3000 nM, 1 to 2000 nM, 1 to 1500 nM, 1 to 1000 nM, 1 to 750 nM, 1 to 500 nM, 1 to 250 nM, 1 to 100 nM, 1 to 50 nM, 1 to 25 nM, 1 to 10 nM as assessed using a method described herein or known to one of skill in the art (*e.g.,* a BIAcore assay, ELISA). In another embodiment, an anti-ICOS antibody used in accordance with a method described herein may comprise an Fc region that binds to mouse Fc$\gamma$RIIIA with a dissociation constant (K$_d$) of 500 nM, 250 nM, 100 nM, 75 nM, 50 nM, 25 nM, 10 nM or 1 nM as assessed using a method described herein or known to one of skill in the art (*e.g.,* a BIAcore assay, ELISA).

The present disclosure provides formulations of antibodies that specifically bind to human ICOS and have an enhanced effector function. In one embodiment, a formulation of the disclosure comprises an anti-ICOS antibody with enhanced effector function, such as, but not limited to, enhanced ADCC, enhanced CDC, and enhanced antibody-dependent phagocytosis. In one embodiment, a formulation of the disclosure comprises an anti-ICOS antibody disclosed in US Patent Application 12/116,512, filed on May 7, 2008. In one embodiment, anti-ICOS antibodies of the disclosure comprise one, two, three, four, five, or all six of the CDRs of JMAb-136 (*see,* US Patent 6,803,039).

The amino acid sequences for CDR1, CDR2, and CDR3 of the heavy chain variable region of JMAb-136 defined according to Kabat are identified as SEQ ID NO: 8, SEQ ID NO: 9, and SEQ ID NO: 10, respectively. The amino acid sequences for CDR1, CDR2 and CDR3 of the light chain variable region of JMAb-136 defined according to Kabat are identified as SEQ ID NO:3, SEQ ID NO:4, and SEQ ID NO:5, respectively.

Kabat numbering is based on the seminal work of Kabat et al. (1991) Sequences of Proteins of Immunological Interest, Publication No. 91-3242, published as a three volume set by the National Institutes of Health, National Technical Information Service (hereinafter "Kabat"). Kabat provides multiple sequence alignments of immunoglobulin chains from numerous species antibody isotypes. The aligned sequences are numbered according to a single numbering system, the Kabat numbering system. The Kabat sequences have been updated since the 1991 publication and are available as an electronic sequence database (latest downloadable version 1997). Any immunoglobulin sequence can be numbered according to Kabat by performing an alignment with the Kabat reference sequence. Accordingly, the Kabat numbering system provides a uniform system for numbering immunoglobulin chains. Unless indicated otherwise, all immunoglobulin amino acid sequences described herein are numbered according to the Kabat numbering system. Similarly, all single amino acid positions referred to herein are numbered according to the Kabat numbering system.

In certain embodiments, an anti-ICOS antibody of the disclosure may comprise a heavy chain variable region, VH, comprising at least one CDR having the amino acid sequence selected from the group consisting of SEQ ID NO:8, SEQ ID NO:9, and SEQ ID NO:10. In certain embodiments, an anti-ICOS antibody of the disclosure may comprise a VH domain having the amino acid sequence of SEQ ID NO: 7.

In certain embodiments, an anti-ICOS antibody described herein may comprise a light chain variable region, VK, comprising at least one CDR having an amino acid sequence selected from the group consisting of SEQ ID NO:3, SEQ ID NO:4, and SEQ ID NO:5. In certain embodiments, an anti-ICOS antibody of the disclosure may comprise a VK domain having the amino acid sequence of SEQ ID NO: 2.

In one embodiment, an anti-ICOS antibody of the disclosure comprises a VK domain having the amino acid sequence of SEQ ID NO: 2 and further comprises a VH domain having the amino acid sequence of SEQ ID NO: 7.

The present disclosure encompasses antibodies that bind to human ICOS, comprising derivatives of the VH domain, VH CDR1, VH CDR2, VH CDR3, VK domain, VK CDR1, VK CDR2, or VK CDR3 described herein that may bind to human ICOS. Standard techniques known to those of skill in the art can be used to introduce mutations (*e.g.*, additions, deletions, and/or substitutions) in the nucleotide sequence encoding an antibody, including, for example, site-directed mutagenesis and PCR-mediated mutagenesis that are routinely used to generate amino acid substitutions. In one embodiment, the VH and/or VK CDR derivatives may include less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, less than 2 amino acid substitutions, or 1 amino acid substitution relative to the original VH and/or VK CDRs of the JMab-136 anti-ICOS antibody. In another embodiment, the VH and/or VK CDR derivatives may have conservative amino acid substitutions (*e.g.* supra) made at one or more predicted non-essential amino acid residues (i.e., amino acid residues which are not critical for the antibody to specifically bind to human ICOS). Mutations can also be introduced randomly along all or part of the VH and/or VK CDR coding sequences, such as by saturation mutagenesis, and the resultant mutants can be screened for biological activity to identify mutants that retain activity. Following mutagenesis, the encoded antibody can be expressed and the activity of the antibody can be determined.

The present disclosure further encompasses antibodies that bind to human ICOS, said antibodies or antibody fragments comprising one or more CDRs wherein said CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of one or more CDRs of the JMab-136 anti-ICOS antibody. The percent identity of two amino acid sequences can be determined by any method known to one skilled in the art, including, but not limited to, BLAST protein searches.

The present disclosure further encompasses antibodies that bind to human ICOS, said antibodies or antibody fragments comprising a VH and/or a VK domain wherein said VH and/or VK domains comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the VH and VK domain of the JMab-136 anti-ICOS antibody. The percent identity of two amino acid sequences can be determined by any method known to one skilled in the art, including, but not limited to, BLAST protein searches.

In one embodiment, an anti-ICOS antibody of the disclosure may bind to human ICOS with an affinity comparable to that of the JMab-136 anti-ICOS antibody.

In one embodiment, an anti-ICOS antibody of the disclosure specifically binds the same epitope of ICOS as the JMab-136 anti-ICOS antibody.

In one embodiment, an anti-ICOS antibody specifically competes the JMab-136 anti-ICOS antibody for ICOS binding. The competition assay may be performed using any binding assay known in the art, for example, but not limited to ELISA assay, radioimmunoassay, and flow cytometry.

The disclosure further provides polynucleotides comprising a nucleotide sequence encoding an anti-ICOS antibody with enhanced effector function. The disclosure also encompasses polynucleotides that hybridize under stringent or lower stringency hybridization conditions, as defined herein, to polynucleotides that encode an anti-ICOS antibody with enhanced effector function.

Another embodiment of the disclosure is a vector comprising one or more nucleotide sequences encoding an anti-ICOS antibody with enhanced effector function.

The present disclosure further relates to an isolated cell comprising a vector wherein said vector comprises one or more nucleotide sequences encoding an anti-ICOS antibody with enhanced effector function.

Anti-ICOS antibodies of the disclosure include those of the IgG1, IgG2, IgG3, or IgG4 human isotype.

The present disclosure relates to anti-ICOS antibodies with enhanced effector function, as well as to compositions comprising those antibodies. In certain embodiments, an anti-ICOS antibody of the disclosure may mediate antigen-dependent-cell-mediated- cytotoxicity (ADCC). In other embodiments, the present disclosure is directed toward compositions comprising an anti-ICOS antibody of the IgG1 and/or IgG3 human isotype, as well as to an anti-ICOS antibody of the IgG2 and/or IgG4 human isotype, that may mediate human ADCC, CDC, and/or antibody-dependent phagocytosis.

Anti-ICOS antibodies described herein may have a high binding affinity for the human ICOS antigen. For example, an antibody described herein may have an association rate constant or $k_{on}$ rate (antibody (Ab) + antigen $(Ag)^{k\text{-}on} \rightarrow$ Ab-Ag) of at least $2 \times 10^5$ M$^{-1}$s$^{-1}$, at least $5 \times 10^5$ M$^{-1}$s$^{-1}$, at least $10^6$ M$^{-1}$s$^{-1}$, at least $5 \times 10^6$ M$^{-1}$s$^{-1}$, at least $10^7$ M$^{-1}$s$^{-1}$, at least $5 \times 10^7$ M$^{-1}$s$^{-1}$, or at least $10^8$ M$^{-1}$s$^{-1}$.

In another embodiment, an anti-ICOS antibody may have a $k_{off}$ rate ((Ab-Ag)$^{k\text{-}off} \rightarrow$ antibody (Ab) + antigen (Ag)) of less than $5 \times 10^{-1}$ s$^{-1}$, less than $10^{-1}$ s$^{-1}$, less than $5 \times 10^{-2}$ s$^{-1}$, less than $10^{-2}$ s$^{-1}$, less than $5 \times 10^{-3}$ s$^{-1}$, less than $10^{-3}$ s$^{-1}$, less than $5 \times 10^{-4}$ s$^{-1}$, or less than $10^{-4}$ s$^{-1}$. In a another embodiment, an antibody of the disclosure has a $k_{off}$ of less than $5 \times 10^{-5}$ s$^{-1}$, less than $10^{-5}$ s$^{-1}$, less than $5 \times 10^{-6}$ s$^{-1}$, less than $10^{-6}$ s$^{-1}$, less than $5 \times 10^{-7}$ s$^{-1}$, less than $10^{-7}$ s$^{-1}$, less than $5 \times 10^{-8}$ s$^{-1}$, less than $10^{-8}$ s$^{-1}$, less than $5 \times 10^{-9}$ s$^{-1}$, less than $10^{-9}$ s$^{-1}$, or less than $10^{-10}$ s$^{-1}$.

In another embodiment, an anti-ICOS antibody may have an affinity constant or $K_a$ ($k_{on}/k_{off}$) of at least $10^2$ M$^{-1}$, at least $5 \times 10^2$ M$^{-1}$, at least $10^3$ M$^{-1}$, at least $5 \times 10^3$ M$^{-1}$, at least $10^4$ M$^{-1}$, at least $5 \times 10^4$ M$^{-1}$, at least $10^5$ M$^{-1}$, at least $5 \times 10^5$ M$^{-1}$, at least $10^6$ M$^{-1}$, at least $5 \times 10^6$ M$^{-1}$, at least $10^7$ M$^{-1}$, at least $5 \times 10^7$ M$^{-1}$, at least $10^8$ M$^{-1}$, at least $5 \times 10^8$ M$^{-1}$, at least $10^9$ M$^{-1}$, at least $5 \times 10^9$ M$^{-1}$, at least $10^{10}$ M$^{-1}$, at least $5 \times 10^{10}$ M$^{-1}$, at least $10^{11}$ M$^{-1}$, at least $5 \times 10^{11}$ M$^{-1}$, at least $10^{12}$ M$^{-1}$, at least $5 \times 10^{12}$ M$^{-1}$, at least $10^{13}$ M$^{-1}$, at least $5 \times 10^{13}$ M$^{-1}$, at least $10^{14}$ M$^{-1}$, at least $5 \times 10^{14}$ M$^{-1}$, at least $10^{15}$ M$^{-1}$, or at least $5 \times 10^{15}$ M$^{-1}$. In yet another embodiment, an anti-ICOS antibody may have a dissociation constant or $K_d$ ($k_{off}/k_{on}$) of less than $5 \times 10^{-2}$ M, less than $10^{-2}$ M, less than $5 \times 10^{-3}$ M, less than $10^{-3}$ M, less than $5 \times 10^{-4}$ M, less than $10^{-4}$ M, less than $5 \times 10^{-5}$ M, less than $10^{-5}$ M, less than $5 \times 10^{-6}$ M, less than $10^{-6}$ M, less than $5 \times 10^{-7}$ M, less than $10^{-7}$ M, less than $5 \times 10^{-8}$ M, less than $10^{-8}$ M, less than $5 \times 10^{-9}$ M, less than $10^{-9}$ M, less than $5 \times 10^{-10}$ M, less than $10^{-10}$ M, less than $5 \times 10^{-11}$ M, less than $10^{-11}$ M, less than $5 \times 10^{-12}$ M, less than $10^{-12}$ M, less than $5 \times 10^{-13}$ M, less than $10^{-13}$ M, less than $5 \times 10^{-14}$ M, less than $10^{-14}$ M, less than $5 \times 10^{-15}$ M, or less than $10^{-15}$ M.

An antibody used in accordance with a method described herein may immunospecifically bind to ICOS and may have a dissociation constant ($K_d$) of less than 3000 pM, less than 2500 pM, less than 2000 pM, less than 1500 pM, less than 1000 pM, less than 750 pM, less than 500 pM, less than 250 pM, less than 200 pM, less than 150 pM, less than 100 pM, less than 75 pM as assessed using a method described herein or known to one of skill in the art (*e.g.,* a BIAcore assay, ELISA) (Biacore International AB, Uppsala, Sweden). In a specific embodiment, an antibody used in accordance with a method described herein may immunospecifically bind to a human ICOS antigen and may have a dissociation constant ($K_d$) of between 25 to 3400 pM, 25 to 3000 pM, 25 to 2500 pM, 25 to 2000 pM, 25 to 1500 pM, 25 to 1000 pM, 25 to 750 pM, 25 to 500 pM, 25 to 250 pM, 25 to 100 pM, 25 to 75 pM, 25 to 50 pM as assessed using a method described herein or known to one of skill in the art (*e.g.,* a BIAcore assay, ELISA). In another embodiment, an anti-ICOS antibody used in accordance with a method described herein may immunospecifically bind to ICOS and may have a dissociation constant ($K_d$) of 500 pM, 100 pM, 75 pM or 50 pM as assessed using a method described herein or known to one of skill in the art (*e.g.,* a BIAcore assay, ELISA).

The antibodies that specifically bind to ICOS include derivatives that are modified, i.e., by the covalent attachment of any type of molecule to the antibody such that covalent attachment does not eliminate binding to human ICOS. For example, but not by way of limitation, the antibody derivatives include antibodies that have been modified, for example, but not limited to, by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

The formulations of antibodies of the present disclosure that specifically bind to human ICOS may be monospecific, bispecific, trispecific or of greater multispecificity. Multispecific antibodies may be specific for different epitopes of human ICOS or may be specific for both human ICOS as well as for a heterologous epitope, such as a heterologous polypeptide or solid support material.

**5.4.** Monoclonal Anti-ICOS Antibodies

A monoclonal anti-ICOS antibody exhibits binding specificity to human ICOS antigen and may mediate human ADCC, CDC and/or antibody-dependent phagocytosis. Such an antibody can be generated using a wide variety of techniques known in the art including the use of hybridoma, recombinant, and phage display technologies, or a combination thereof. Antibodies are highly specific, being directed against a single antigenic site. An engineered anti-ICOS antibody can be produced by any means known in the art, including, but not limited to, those techniques described below and improvements to those techniques. Large-scale high-yield production typically involves culturing a host cell that produces the engineered anti-ICOS antibody and recovering the anti-ICOS antibody from the host cell culture.

5.4.1. Hybridoma Technique

Monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling et al., in Monoclonal Antibodies and T Cell Hybridomas, 563-681 (Elsevier, N.Y., 1981). For example, in the hybridoma method, a mouse or other appropriate host animal, such as a hamster or macaque monkey, is immunized to elicit lymphocytes that produce or are capable of producing antibodies that will specifically bind to the protein used for immunization. Lymphocytes may also be immunized *in vitro.* Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium that contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Specific embodiments employ myeloma cells that fuse efficiently, support stable high-level production of antibody by the selected antibody-producing cells, and are sensitive to a medium such as HAT medium. Among these myeloma cell lines are murine myeloma lines, such as those derived from MOPC-21 and MPC-11 mouse tumors available from the Salk Institute Cell Distribution Center, San Diego, CA, USA, and SP-2 or X63-Ag8.653 cells available from the American Type Culture Collection, Rockville, MD, USA. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the human ICOS antigen. The binding specificity of monoclonal antibodies produced by hybridoma cells can be determined by immunoprecipitation or by an *in vitro* binding assay, such as radioimmunoassay (RIA) or enzyme-linked immunoabsorbent assay (ELISA).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI 1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

5.4.2. Recombinant DNA Techniques

DNA encoding an anti-ICOS antibody described herein is readily isolated and sequenced using conventional procedures (*e.g.*, by using oligonucleotide probes that are capable of binding specifically to genes encoding the heavy and light chains of anti-ICOS antibodies). The hybridoma cells serve as a source of such DNA. Once isolated, the DNA may be placed into expression vectors, which are then transfected into host cells such as *E. coli* cells, simian COS cells, Chinese hamster ovary (CHO) cells, or myeloma cells that do not otherwise produce immunoglobulin protein, to obtain the synthesis of anti-ICOS antibodies in the recombinant host cells.

In phage display methods, functional antibody domains are displayed on the surface of phage particles which carry the polynucleotide sequences encoding them. In particular, DNA sequences encoding $V_H$ and $V_L$ domains are amplified from animal cDNA libraries (*e.g.*, human or murine cDNA libraries of affected tissues). The DNA encoding the $V_H$ and $V_L$ domains are recombined together with a scFv linker by PCR and cloned into a phagemid vector. The vector is electroporated into *E. coli* and the *E. coli* is infected with helper phage. Phage used in these methods is typically filamentous phage including fd and M13 and the $V_H$ and $V_L$ domains are usually recombinantly fused to either the phage gene III or gene VIII. Phage expressing an antigen-binding domain that binds to a particular antigen can be selected or identified with antigen, *e.g.*, using labeled antigen or antigen bound or captured to a solid surface or bead. Examples of phage display methods that can be used to make the antibodies of the present disclosure include those disclosed in Brinkman et al., 1995, J. Immunol. Methods, 182:41-50; Ames et al., 1995, J. Immunol. Methods, 184:177-186; Kettleborough et al., 1994, Eur. J. Immunol., 24:952-958; Persic et al., 1997, Gene, 187:9-18; Burton et al., 1994, Advances in Immunology, 57:191-280; International Application No. PCT/GB91/O1 134; International Publication Nos. WO 90/02809, WO 91/10737, WO 92/01047, WO 92/18619, WO 93/11236, WO 95/15982, WO 95/20401, and WO97/13844; and U.S. Patent Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743, and 5,969,108.

As described in the above references, after phage selection, the antibody coding regions from the phage can be isolated and used to generate whole antibodies, including human antibodies, or any other desired antigen-binding fragment, and expressed in any desired host, including mammalian cells, insect cells, plant cells, yeast, and bacteria, *e.g.*, as described below. Techniques to recombinantly produce Fab, Fab' and F(ab')$_2$ fragments can also be employed using methods known in the art such as those disclosed in PCT Publication No. WO 92/22324; Mullinax et al., 1992, BioTechniques, 12(6):864-869; Sawai et al., 1995, AJRI, 34:26-34; and Better et al., 1988, Science, 240:1041-1043.

Antibodies may be isolated from antibody phage libraries generated using the techniques described in McCafferty et al., Nature, 348:552-554 (1990). Clackson et al., Nature, 352:624-628 (1991). Marks et al., J. Mol. Biol., 222:581-597 (1991) describe the isolation of murine and human antibodies, respectively, using phage libraries. Chain shuffling can be used in the production of high affinity (nM range) human antibodies (Marks et al., Bio/Technology, 10:779-783 (1992)), as well as combinatorial infection and *in vivo* recombination as a strategy for constructing very large phage libraries (Waterhouse et al., Nucl. Acids. Res., 21:2265-2266 (1993)). Thus, these techniques are viable alternatives to traditional monoclonal antibody hybridoma techniques for isolation of anti-ICOS antibodies.

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a heavy chain constant region, *e.g.,* the human gamma 4 constant region, and the PCR amplified VL domains can be cloned into vectors expressing a light chain constant region, *e.g.*, human kappa or lambda constant regions. The vectors for expressing the VH or VL domains may comprise an EF-1$\alpha$ promoter, a secretion signal, a cloning site for the variable domain, constant domains, and a selection marker such as neomycin. The VH and VL domains may also be cloned into one vector expressing the necessary constant regions. The heavy chain conversion vectors and light chain conversion vectors are then co-transfected into cell lines to generate stable or transient cell lines that express full-length antibodies, *e.g.*, IgG, using techniques known to those of skill in the art.

The DNA also may be modified, for example, by substituting the coding sequence for human heavy and light chain constant domains in place of the homologous murine sequences (U.S. Patent No. 4,816,567; Morrison et al., Proc. Natl. Acad. Sci. USA, 81:6851 (1984)), or by covalently joining to the immunoglobulin coding sequence all or part of the coding sequence for a non-immunoglobulin polypeptide.

**5.5.** Chimeric Antibodies

The anti-ICOS antibodies herein specifically include chimeric antibodies (immunoglobulins) in which a portion of the heavy and/or light chain is identical with or homologous to corresponding sequences in antibodies derived from a particular species or belonging to a particular antibody class or subclass, while another portion of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Chimeric antibodies of interest herein include "primatized" antibodies comprising variable domain antigen-binding sequences derived from a nonhuman primate (*e.g.,* Old World Monkey, such as baboon, rhesus or cynomolgus monkey) and human constant region sequences (U.S. Patent No. 5,693,780).

**5.6.** Altered/Mutant Antibodies

Anti-ICOS antibodies of compositions and methods described herein can be mutant antibodies. As used herein, "antibody mutant" or "altered antibody" refers to an amino acid sequence variant of an anti-ICOS antibody wherein one or more of the amino acid residues of an anti-ICOS antibody have been modified. The modifications to the amino acid sequence of an anti-ICOS antibody include modifications to the sequence that may improve affinity or avidity of the antibody for its antigen, and/or modifications to the Fc portion of the antibody that may improve effector function.

The present disclosure therefore relates to anti-ICOS antibodies with enhanced effector function disclosed herein as well as altered/mutant derivatives thereof including, but not limited to ones exhibiting altered human ICOS binding characteristics; e.g. altered association constants $k_{ON}$, dissociation constants $k_{OFF}$, and/or equilibrium constant or binding affinity, $K_D$. In certain embodiments the $K_D$ of an anti-ICOS antibody described herein, or an altered/mutant derivative thereof, for human ICOS may be no more than about $10^{-6}$M, $10^{-7}$M, $10^{-8}$M, or $10^{-9}$M. Methods and reagents suitable for determination of such binding characteristics of an antibody of the present disclosure, or an altered/mutant derivative thereof, are known in the art and/or are commercially available (*se above* and, *e.g.,* U.S. Patent No. 6,849,425, U.S. Patent No. 6,632,926, U.S. Patent No. 6,294,391, and U.S. Patent No. 6,143,574). Moreover, equipment and software designed for such kinetic analyses are commercially available (*e.g.* Biacore® A100, and Biacore® 2000 instruments; Biacore International AB, Uppsala, Sweden).

The modifications may be made to any known anti-ICOS antibodies or anti-ICOS antibodies identified as described herein. Such altered antibodies necessarily have less than 100% sequence identity or similarity with a known anti-ICOS antibody. By way of example, an altered antibody may have an amino acid sequence that is within the range of from about 25% to about 95% identical or similar to the amino acid sequence of either the heavy or light chain variable domain of an anti-ICOS antibody as described herein. An altered antibody may have an amino acid sequence having at least 25%, 35%, 45%, 55%, 65%, 75%, 80%, 85%, 90%, or 95% amino acid sequence identity or similarity with the amino acid sequence of either the heavy or light chain variable domain of an anti-ICOS antibody as described herein. In another embodiment, an altered antibody may have an amino acid sequence having at least 25%, 35%, 45%, 55%, 65%, 75%, 80%, 85%, 90%, or 95% amino acid sequence identity or similarity with the amino acid sequence of the heavy chain CDR1, CDR2, or CDR3 of an anti-ICOS antibody as described herein. In one embodiment, an altered antibody may maintain human ICOS binding capability. In certain embodiments, an anti-ICOS antibody as described herein may comprise a VH that is at least or about 10%, 15%, 20%, 25%, 30%, 35%, 40%, 45%, 50%, 55%, 60%, 65%, 70%, 75%, 80%, 85%, 90%, 95% or more identical to the amino acid sequence of SEQ ID NO:7.

In another embodiment, an altered antibody may have an amino acid sequence having at least 25%, 35%, 45%, 55%, 65%, 75%, 80%, 85%, 90%, or 95% amino acid sequence identity or similarity with the amino acid sequence of the light chain CDR1, CDR2, or CDR3 of an anti-ICOS antibody as described herein. In certain embodiments, an anti-ICOS antibody of the disclosure may comprise a VL that is at least or about 10%, 15%, 20%, 25%, 30%, 35%, 40%, 45%, 50%, 55%, 60%, 65%, 70%, 75%, 80%, 85%, 90%, 95% or more identical to an amino acid sequence of SEQ ID NO: 2.

Identity or similarity with respect to a sequence is defined herein as the percentage of amino acid residues in the candidate sequence that are identical (*i.e.,* same residue) or similar (*i.e.,* amino acid residue from the same group based on common side-chain properties, *see* below) with anti-ICOS antibody residues, after aligning the sequences and introducing gaps, if necessary, to achieve the maximum percent sequence identity. None of N-terminal, C-terminal, or internal extensions, deletions, or insertions into the antibody sequence outside of the variable domain shall be construed as affecting sequence identity or similarity.

"% identity," as known in the art, is a measure of the relationship between two polynucleotides or two polypeptides, as determined by comparing their sequences. In general, the two sequences to be compared are aligned to give a maximum correlation between the sequences. The alignment of the two sequences is examined and the number of positions giving an exact amino acid or nucleotide correspondence between the two sequences determined, divided by the total length of the alignment and multiplied by 100 to give a % identity figure. This % identity figure may be determined over the whole length of the sequences to be compared, which is particularly suitable for sequences of the same or very similar length and which are highly homologous, or over shorter defined lengths, which is more suitable for sequences of unequal length or which have a lower level of homology.

For example, sequences can be aligned with the software clustalw under Unix which generates a file with an ".aln" extension, this file can then be imported into the Bioedit program (Hall, T.A. 1999, BioEdit: a user-friendly biological sequence alignment editor and analysis program for Windows 95/98/NT. Nucl. Acids. Symp. Ser. 41:95-98) which opens the .aln file. In the Bioedit window, one can choose individual sequences (two at a time) and alignment them. This method allows for comparison of the entire sequence.

Methods for comparing the identity of two or more sequences are well known in the art. Thus for instance, programs are available in the Wisconsin Sequence Analysis Package, version 9.1 (Devereux J. et al., Nucleic Acids Res., 12:387-395, 1984, available from Genetics Computer Group, Madison, WI, USA). The determination of percent identity between two sequences can be accomplished using a mathematical algorithm. For example, the programs BESTFIT and GAP, may be used to determine the % identity between two polynucleotides and the % identity between two polypeptide sequences. BESTFIT uses the "local homology" algorithm of Smith and Waterman (Advances in Applied Mathematics, 2:482-489, 1981) and finds the best single region of similarity between two sequences. BESTFIT is more suited to comparing two polynucleotide or two polypeptide sequences which are dissimilar in length, the program assuming that the shorter sequence represents a portion of the longer. In comparison, GAP aligns two sequences finding a "maximum similarity" according to the algorithm of Neddleman and Wunsch (J. Mol. Biol., 48:443-354, 1970). GAP is more suited to comparing sequences which are approximately the same length and an alignment is expected over the entire length. Preferably the parameters "Gap Weight " and "Length Weight" used in each program are 50 and 3 for polynucleotides and 12 and 4 for polypeptides, respectively. Preferably % identities and similarities are determined when the two sequences being compared are optimally aligned.

Other programs for determining identity and/or similarity between sequences are also known in the art, for instance the BLAST family of programs (Karlin & Altschul, 1990, Proc. Natl. Acad. Sci. USA, 87:2264-2268, modified as in Karlin & Altschul, 1993, Proc. Natl. Acad. Sci. USA, 90:5873-5877, available from the National Center for Biotechnology Information (NCB), Bethesda, MD, USA and accessible through the home page of the NCBI at www.ncbi.nlm.nih.gov). These programs are non-limiting examples of a mathematical algorithm utilized for the comparison of two sequences. Such an algorithm is incorporated into the NBLAST and XBLAST programs of Altschul et al., 1990, J. Mol. Biol., 215:403-410. BLAST nucleotide searches can be performed with the NBLAST program, score = 100, wordlength = 12 to obtain nucleotide sequences homologous to a nucleic acid molecule encoding all or a portion if an anti-ICOS antibody of the disclosure. BLAST protein searches can be performed with the XBLAST program, score = 50, wordlength = 3 to obtain amino acid sequences homologous to a protein molecule of the disclosure. To obtain gapped alignments for comparison purposes, Gapped BLAST can be utilized as described in Altschul et al., 1997, Nucleic Acids Res., 25:3389-3402. PSI-Blast can also be used to perform an iterated search which detects distant relationships between molecules (*Id.*). When utilizing BLAST, Gapped BLAST, and PSI-Blast programs, the default parameters of the respective programs (*e.g.*, XBLAST and NBLAST) can be used. *See,* http://www.ncbi.nlm.nih.gov.

Another non-limiting example of a program for determining identity and/or similarity between sequences known in the art is FASTA (Pearson W.R. and Lipman D.J., Proc. Natl. Acad. Sci. USA, 85:2444-2448, 1988, available as part of the Wisconsin Sequence Analysis Package). Preferably the BLOSUM62 amino acid substitution matrix (Henikoff S. and Henikoff J.G., Proc. Natl. Acad. Sci. USA, 89:10915-10919, 1992) is used in polypeptide sequence comparisons including where nucleotide sequences are first translated into amino acid sequences before comparison.

Yet another non-limiting example of a program known in the art for determining identity and/or similarity between amino acid sequences is SeqWeb Software (a web-based interface to the GCG Wisconsin Package: Gap program) which is utilized with the default algorithm and parameter settings of the program: blosum62, gap weight 8, length weight 2.

The percent identity between two sequences can be determined using techniques similar to those described above, with or without allowing gaps. In calculating percent identity, typically exact matches are counted.

Preferably the program BESTFIT is used to determine the % identity of a query polynucleotide or a polypeptide sequence with respect to a polynucleotide or a polypeptide sequence of the present disclosure, the query and the reference sequence being optimally aligned and the parameters of the program set at the default value.

To generate an altered antibody, one or more amino acid alterations (*e.g.*, substitutions) are introduced in one or more of the hypervariable regions of the species-dependent antibody. One or more alterations (*e.g.*, substitutions) of framework region residues may also be introduced in an anti-ICOS antibody where these result in an improvement in the binding affinity of the antibody mutant for the antigen from the second mammalian species. Examples of framework region residues to modify include those which non-covalently bind antigen directly (Amit et al., Science, 233:747-753 (1986)); interact with/effect the conformation of a CDR (Chothia et al., J. Mol. Biol., 196:901-917 (1987)); and/or participate in the $V_L$-$V_H$ interface (EP 239 400B1). In certain embodiments, modification of one or more of such framework region residues results in an enhancement of the binding affinity of the antibody for the antigen from the second mammalian species. For example, from about one to about five framework residues may be altered in this embodiment of the disclosure. Sometimes, this may be sufficient to yield an antibody mutant suitable for use in preclinical trials, even where none of the hypervariable region residues have been altered. Normally, however, an altered antibody will comprise additional hypervariable region alteration(s).

The hypervariable region residues which are altered may be changed randomly, especially where the starting binding affinity of an anti-ICOS antibody for the antigen from the second mammalian species is such that such randomly produced altered antibody can be readily screened.

One useful procedure for generating such an altered antibody is called "alanine scanning mutagenesis" (Cunningham and Wells, Science, 244:1081-1085 (1989)). Here, one or more of the hypervariable region residue(s) are replaced by alanine or polyalanine residue(s) to affect the interaction of the amino acids with the antigen from the second mammalian species. Those hypervariable region residue(s) demonstrating functional sensitivity to the substitutions then are refined by introducing additional or other mutations at or for the sites of substitution. Thus, while the site for introducing an amino acid sequence variation is predetermined, the nature of the mutation *per se* need not be predetermined. The Ala-mutants produced this way are screened for their biological activity as described herein.

Another procedure for generating such an altered antibody involves affinity maturation using phage display (Hawkins et al., J. Mol. Biol., 254:889-896 (1992) and Lowman et al., Biochemistry, 30(45):10832-10837 (1991)). Briefly, several hypervariable region sites (*e.g.,* 6-7 sites) are mutated to generate all possible amino acid substitutions at each site. The antibody mutants thus generated are displayed in a monovalent fashion from filamentous phage particles as fusions to the gene III product of M13 packaged within each particle. The phage-displayed mutants are then screened for their biological activity (*e.g.*, binding affinity) as herein disclosed.

Mutations in antibody sequences may include substitutions, deletions, including internal deletions, additions, including additions yielding fusion proteins, or conservative substitutions of amino acid residues within and/or adjacent to the amino acid sequence, but that result in a "silent" change, in that the change produces a functionally equivalent anti-ICOS antibody. Conservative amino acid substitutions may be made on the basis of similarity in polarity, charge, solubility, hydrophobicity, hydrophilicity, and/or the amphipathic nature of the residues involved. For example, non-polar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid. In addition, glycine and proline are residues that can influence chain orientation. Non-conservative substitutions will entail exchanging a member of one of these classes for a member of another class. Furthermore, if desired, non-classical amino acids or chemical amino acid analogs can be introduced as a substitution or addition into the antibody sequence. Non-classical amino acids include, but are not limited to, the D-isomers of the common amino acids, $\alpha$-amino isobutyric acid, 4-aminobutyric acid, Abu, 2-amino butyric acid, $\gamma$-Abu, $\epsilon$-Ahx, 6-amino hexanoic acid, Aib, 2-amino isobutyric acid, 3-amino propionic acid, ornithine, norleucine, norvaline, hydroxyproline, sarcosine, citrulline, cysteic acid, t-butylglycine, t-butylalanine, phenylglycine, cyclohexylalanine, $\beta$-alanine, fluoro-amino acids, designer amino acids such as $\beta$-methyl amino acids, $C\alpha$-methyl amino acids, $N\alpha$-methyl amino acids, and amino acid analogs in general.

In another embodiment, the sites selected for modification are affinity matured using phage display (*see* above).

Any technique for mutagenesis known in the art can be used to modify individual nucleotides in a DNA sequence, for purposes of making amino acid substitution(s) in the antibody sequence, or for creating/deleting restriction sites to facilitate further manipulations. Such techniques include, but are not limited to, chemical mutagenesis, *in vitro* site-directed mutagenesis (Kunkel, Proc. Natl. Acad. Sci. USA, 82:488 (1985); Hutchinson, C. et al., J. Biol. Chem., 253:6551 (1978)), oligonucleotide-directed mutagenesis (Smith, Ann. Rev. Genet., 19:423-463 (1985); Hill et al., Methods Enzymol., 155:558-568 (1987)), PCR-based overlap extension (Ho et al., Gene, 77:51-59 (1989)), PCR-based megaprimer mutagenesis (Sarkar et al., Biotechniques, 8:404-407 (1990)), etc. Modifications can be confirmed by double-stranded dideoxy DNA sequencing.

In certain embodiments of the disclosure, an anti-ICOS antibody can be modified to produce fusion proteins; *i.e.,* the antibody, or a fragment thereof, fused to a heterologous protein, polypeptide or peptide. In certain embodiments, the protein fused to the portion of an anti-ICOS antibody is an enzyme component of Antibody-Directed Enzyme Prodrug Therapy (ADEPT). Examples of other proteins or polypeptides that can be engineered as a fusion protein with an anti-ICOS antibody include, but are not limited to toxins such as ricin, abrin, ribonuclease, DNase I, Staphylococcal enterotoxin-A, pokeweed anti-viral protein, gelonin, diphtheria toxin, Pseudomonas exotoxin, and Pseudomonas endotoxin. *See,* for example, Pastan et al., Cell, 47:641 (1986), and Goldenberg et al., Cancer Journal for Clinicians, 44:43 (1994). Enzymatically active toxins and fragments thereof which can be used include diphtheria A chain, non-binding active fragments of diphtheria toxin, exotoxin A chain (from Pseudomonas aeruginosa), ricin A chain, abrin A chain, modeccin A chain, alpha-sarcin, *Aleurites fordii* proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. *See,* for example, WO 93/21232 published October 28, 1993.

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of the anti-ICOS antibody or fragments thereof (*e.g.,* an antibody or a fragment thereof with higher affinities and lower dissociation rates). *See,* generally, U.S. Patent Nos. 5,605,793; 5,811,238; 5,830,721; 5,834,252; and 5,837,458, and Patten et al., 1997, Curr. Opinion Biotechnol., 8:724-33; Harayama, 1998, Trends Biotechnol. 16(2):76-82; Hansson et al., 1999, J. Mol. Biol., 287:265-76; and Lorenzo and Blasco, 1998, Biotechniques 24(2):308- 313. The antibody can further be a binding-domain immunoglobulin fusion protein as described in U.S. Publication 20030118592, U.S. Publication 200330133939, and PCT Publication WO 02/056910, all to Ledbetter *et al.*

5.7. Domain Antibodies

Anti-ICOS antibodies of compositions and methods of the disclosure can be domain antibodies, *e.g*., antibodies containing the small functional binding units of antibodies, corresponding to the variable regions of the heavy ($V_H$) or light ($V_L$) chains of human antibodies. Examples of domain antibodies include, but are not limited to, those available from Domantis Limited (Cambridge, UK) and Domantis Inc. (Cambridge, MA, USA) that are specific to therapeutic targets (*see*, for example, WO04/058821; WO04/003019; U.S. Patent Nos. 6,291,158; 6,582,915; 6,696,245; and 6,593,081). Commercially available libraries of domain antibodies can be used to identify anti-ICOS domain antibodies. In certain embodiments, anti-ICOS antibodies comprise an ICOS functional binding unit and an Fc gamma receptor functional binding unit.

In one embodiment, an anti-ICOS domain antibody may comprise any one of, or any combination of the CDRs of the heavy or light chains of the JMab-136 monoclonal antibody.

In another embodiment, an anti-ICOS domain antibody may comprise VH CDR3 of JMab-136 together with any combination of the CDRs comprised by the heavy or light chains variable regions of the JMab-136 monoclonal antibody. An anti-ICOS domain antibody may also comprise VK CDR3 of JMab-136 together with any combination of the CDRs comprised by the heavy or light chains variable regions of the JMab-136 monoclonal antibody.

In yet another embodiment, an anti-ICOS domain antibody may comprise VH CDR3 of JMab-136. An anti-ICOS domain antibody may also comprise VK CDR3 of JMab-136.

**5.8.** Diabodies

In certain embodiments of the disclosure, anti-ICOS antibodies are "diabodies". The term "diabodies" refers to small antibody fragments with two antigen-binding sites, which fragments comprise a heavy chain variable domain ($V_H$) connected to a light chain variable domain ($V_L$) in the same polypeptide chain ($V_H$-$V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

**5.9.** Vaccibodies

In certain embodiments of the disclosure, anti-ICOS antibodies are Vaccibodies. Vaccibodies are dimeric polypeptides. Each monomer of a vaccibody consists of a scFv with specificity for a surface molecule on APC connected through a hinge region and a Cy3 domain to a second scFv. In other embodiments of the disclosure, vaccibodies containing as one of the scFv's an anti-ICOS antibody fragment may be used to juxtapose those ICOS expressing cells to be destroyed and an effector cell that mediates ADCC. For example, *see,* Bogen et al., U.S. Patent Application Publication No. 20040253238.

**5.10.** Linear Antibodies

In certain embodiments of the disclosure, anti-ICOS antibodies are linear antibodies. Linear antibodies comprise a pair of tandem Fd segments ($V_H$-$C_{H1}$-$V_H$-$C_{H1}$) which form a pair of antigen-binding regions. Linear antibodies can be bispecific or monospecific. *See,* Zapata et al., Protein Eng., 8(10):1057-1062 (1995).

**5.11.** Parent Antibody

In certain embodiments of the disclosure, an anti-ICOS antibody is a parent antibody. A "parent antibody" is an antibody comprising an amino acid sequence which may lack, or may be deficient in, one or more amino acid residues in or adjacent to one or more hypervariable regions thereof compared to an altered/mutant antibody as herein disclosed. Thus, the parent antibody may have a shorter hypervariable region than the corresponding hypervariable region of an antibody mutant as herein disclosed. The parent polypeptide may comprise a native antibody sequence (*i.e.,* a naturally occurring, including a naturally occurring allelic variant) or an antibody sequence with pre-existing amino acid sequence modifications (such as other insertions, deletions and/or substitutions) of a naturally occurring sequence. The parent antibody may be a humanized antibody or a human antibody.

**5.12.** Antibody Fragments

"Antibody fragments" comprise a portion of a full-length antibody, generally the antigen binding or variable region thereof. Examples of antibody fragments include Fab, Fab', F(ab')$_2$, and Fv fragments; diabodies; linear antibodies; single-chain antibody molecules; and multispecific antibodies formed from antibody fragments.

Traditionally, these fragments were derived via proteolytic digestion of intact antibodies (*see, e.g.,* Morimoto et al., Journal of Biochemical and Biophysical Methods, 24:107-117 (1992) and Brennan et al., Science, 229:81 (1985)).

However, these fragments can now be produced directly by recombinant host cells. For example, the antibody fragments can be isolated from the antibody phage libraries discussed above. Fab'-SH fragments can also be directly recovered from *E. coli* and chemically coupled to form F(ab')$_2$ fragments (Carter et al., Bio/Technology, 10:163-167 (1992)). According to another approach, F(ab')$_2$ fragments can be isolated directly from recombinant host cell culture. Other techniques for the production of antibody fragments will be apparent to the skilled practitioner. In other embodiments, the antibody of choice is a single-chain Fv fragment (scFv). *See,* for example, WO 93/16185. In certain embodiments, the antibody is not a Fab fragment.

**5.13.** Bispecific Antibodies

Bispecific antibodies are antibodies that have binding specificities for at least two different epitopes. Exemplary bispecific antibodies may bind to two different epitopes of the ICOS expressing T cell surface marker. Other such antibodies may bind a first ICOS expressing T cell marker and further bind a second ICOS expressing T cell surface marker. An anti-ICOS expressing T cell marker binding arm may also be combined with an arm which binds to a triggering molecule on a leukocyte such as a T cell receptor molecule (*e.g.,* CD2 or CD3), or Fc receptors for IgG (FcγR), so as to focus cellular defense mechanisms to the ICOS expressing T cell. Bispecific antibodies may also be used to localize cytotoxic agents to the ICOS expressing T cell. These antibodies possess an ICOS expressing T cell marker-binding arm and an arm which binds the cytotoxic agent (*e.g.*, saporin, anti-interferon-a, vinca alkaloid, ricin A chain, metholaexate or radioactive isotope hapten). Bispecific antibodies can be prepared as full-length antibodies or antibody fragments (*e.g.*, F(ab'): bispecific antibodies).

Methods for making bispecific antibodies are known in the art. (*See,* for example, Millstein et al., Nature, 305:537-539 (1983); Traunecker et al., EMBO J., 10:3655-3659 (1991); Suresh et al., Methods in Enzymology, 121:210 (1986); Kostelny et al., J. Immunol., 148(5):1547-1553 (1992); Hollinger et al., Proc. Natl Acad. Sci. USA, 90:6444-6448 (1993); Gruber et al., J. Immunol., 152:5368 (1994); U.S. Patent Nos. 4,474,893; 4,714,681; 4,925,648; 5,573,920; 5,601,81; 95,731,168; 4,676,980; and 4,676,980, WO 94/04690; WO 91/00360; WO 92/200373; WO 93/17715; WO 92/08802; and EP 03089.)

In one embodiment, where an anti-ICOS antibody of compositions and methods of the disclosure is bispecific, the anti-ICOS antibody may be human or humanized and may have specificity for human ICOS and an epitope on a T cell or may be capable of binding to a human effector cell such as, for example, a monocyte/macrophage and/or a natural killer cell to effect cell death.

**5.14.** Variant Fc Regions

The present disclosure provides formulation of proteins comprising a variant Fc region. That is, a non-naturally occurring Fc region, for example an Fc region comprising one or more non-naturally occurring amino acid residues. Also encompassed by the variant Fc regions of present disclosure are Fc regions which comprise amino acid deletions, additions and/or modifications.

It will be understood that Fc region as used herein includes the polypeptides comprising the constant region of an antibody excluding the first constant region immunoglobulin domain. Thus Fc refers to the last two constant region immunoglobulin domains of IgA, IgD, and IgG, and the last three constant region immunoglobulin domains of IgE and IgM, and the flexible hinge N-terminal to these domains. For IgA and IgM Fc may include the J chain. For IgG, Fc comprises immunoglobulin domains Cgamma2 and Cgamma3 (Cγ2 and Cγ3) and the hinge between Cgamma1 (Cγ1) and Cgamma2 (Cγ2). Although the boundaries of the Fc region may vary, the human IgG heavy chain Fc region is usually defined to comprise residues C226 or P230 to its carboxyl-terminus, wherein the numbering is according to the EU index as in Kabat et al. (1991, NIH Publication 91-3242, National Technical Information Service, Springfield, VA). The "EU index as set forth in Kabat" refers to the residue numbering of the human IgG1 EU antibody as described in Kabat et al. *supra.* Fc may refer to this region in isolation, or this region in the context of an antibody, antibody fragment, or Fc fusion protein. An Fc variant protein may be an antibody, Fc fusion, or any protein or protein domain that comprises an Fc region including, but not limited to, proteins comprising variant Fc regions, which are non-naturally occurring variants of an Fc. Note: Polymorphisms have been observed at a number of Fc positions, including but not limited to Kabat 270, 272, 312, 315, 356, and 358, and thus slight differences between the presented sequence and sequences in the prior art may exist.

The present disclosure encompasses Fc variant proteins which have altered binding properties for an Fc ligand (e.g., an Fc receptor, C1q) relative to a comparable molecule (e.g., a protein having the same amino acid sequence except having a wild type Fc region). Examples of binding properties include but are not limited to, binding specificity, equilibrium dissociation constant ($K_D$), dissociation and association rates ($k_{off}$ and $k_{on}$ respectively), binding affinity and/or avidity. It is generally understood that a binding molecule (e.g., an Fc variant protein such as an antibody) with a low $K_D$ may be preferable to a binding molecule with a high $K_D$. However, in some instances the value of the $k_{on}$ or $k_{off}$ may be more relevant than the value of the $K_D$. One skilled in the art can determine which kinetic parameter is most important for a given antibody application.

The affinities and binding properties of an Fc domain for its ligand may be determined by a variety of in vitro assay methods (biochemical or immunological based assays) known in the art for determining Fc-FcγR interactions, i.e., specific binding of an Fc region to an FcγR including but not limited to, equilibrium methods (e.g., enzyme-linked immunoabsorbent assay (ELISA), or radioimmunoassay (RIA)), or kinetics (e.g., BIACORE® analysis), and other methods such as indirect binding assays, competitive inhibition assays, fluorescence resonance energy transfer (FRET), gel electrophoresis and chromatography (e.g., gel filtration). These and other methods may utilize a label on one or more of the components being examined and/or employ a variety of detection methods including but not limited to chromogenic, fluorescent, luminescent, or isotopic labels. A detailed description of binding affinities and kinetics can be found in Paul, W.E., ed., Fundamental Immunology, 4th Ed., Lippincott-Raven, Philadelphia (1999), which focuses on antibody-immunogen interactions.

In one embodiment, the Fc variant protein has enhanced binding to one or more Fc ligand relative to a comparable molecule. In another embodiment, the Fc variant protein has an affinity for an Fc ligand that is at least 2 fold, or at least 3 fold, or at least 5 fold, or at least 7 fold, or a least 10 fold, or at least 20 fold, or at least 30 fold, or at least 40 fold, or at least 50 fold, or at least 60 fold, or at least 70 fold, or at least 80 fold, or at least 90 fold, or at least 100 fold, or at least 200 fold greater than that of a comparable molecule. In a specific embodiment, the Fc variant protein has enhanced binding to an Fc receptor. In another specific embodiment, the Fc variant protein has enhanced binding to the Fc receptor FcγRIIIA. In still another specific embodiment, the Fc variant protein has enhanced binding to the Fc receptor FcRn. In yet another specific embodiment, the Fc variant protein has enhanced binding to C1q relative to a comparable molecule.

The serum half-life of proteins comprising Fc regions may be increased by increasing the binding affinity of the Fc region for FcRn. In one embodiment, the Fc variant protein has enhanced serum half life relative to comparable molecule.

"Antibody-dependent cell-mediated cytotoxicity" or "ADCC" refers to a form of cytotoxicity in which secreted Ig bound onto Fc receptors (FcRs) present on certain cytotoxic cells (e.g., Natural Killer (NK) cells, neutrophils, and macrophages) enables these cytotoxic effector cells to bind specifically to an antigen-bearing target cell and subsequently kill the target cell with cytotoxins. Specific high-affinity IgG antibodies directed to the surface of target cells "arm" the cytotoxic cells and are absolutely required for such killing. Lysis of the target cell is extracellular, requires direct cell-to-cell contact, and does not involve complement. It is contemplated that, in addition to antibodies, other proteins comprising Fc regions, specifically Fc fusion proteins, having the capacity to bind specifically to an antigen-bearing target cell will be able to effect cell-mediated cytotoxicity. For simplicity, the cell-mediated cytotoxicity resulting from the activity of an Fc fusion protein is also referred to herein as ADCC activity.

The ability of any particular Fc variant protein to mediate lysis of the target cell by ADCC can be assayed. To assess ADCC activity an Fc variant protein of interest is added to target cells in combination with immune effector cells, which may be activated by the antigen antibody complexes resulting in cytolysis of the target cell. Cytolysis is generally detected by the release of label (e.g. radioactive substrates, fluorescent dyes or natural intracellular proteins) from the lysed cells. Useful effector cells for such assays include peripheral blood mononuclear cells (PBMC) and Natural Killer (NK) cells. Specific examples of in vitro ADCC assays are described in Wisecarver et al., 1985 79:277-282; Bruggemann et al., 1987, J Exp Med 166:1351-1361; Wilkinson et al., 2001, J Immunol Methods 258:183-191; Patel et al., 1995 J Immunol Methods 184:29-38. ADCC activity of the Fc variant protein of interest may also be assessed in vivo, e.g., in an animal model such as that disclosed in Clynes et al., 1998, Proc. Natl. Acad. Sci. USA 95:652-656.

In one embodiment, an Fc variant protein has enhanced ADCC activity relative to a comparable molecule. In a specific embodiment, an Fc variant protein has ADCC activity that is at least 2 fold, or at least 3 fold, or at least 5 fold or at least 10 fold or at least 50 fold or at least 100 fold greater than that of a comparable molecule. In another specific embodiment, an Fc variant protein has enhanced binding to the Fc receptor FcγRIIIA and has enhanced ADCC activity relative to a comparable molecule. In other embodiments, the Fc variant protein has both enhanced ADCC activity and enhanced serum half life relative to a comparable molecule.

"Complement dependent cytotoxicity" and "CDC" refer to the lysing of a target cell in the presence of complement. The complement activation pathway is initiated by the binding of the first component of the complement system (C1q) to a molecule, an antibody for example, complexed with a cognate antigen. To assess complement activation, a CDC assay, e.g. as described in Gazzano-Santoro et al., 1996, J. Immunol. Methods, 202:163, may be performed. In one embodiment, an Fc variant protein has enhanced CDC activity relative to a comparable molecule. In a specific embodiment, an Fc variant protein has CDC activity that is at least 2 fold, or at least 3 fold, or at least 5 fold or at least 10 fold or at least 50 fold or at least 100 fold greater than that of a comparable molecule. In other embodiments, the Fc variant protein has both enhanced CDC activity and enhanced serum half life relative to a comparable molecule.

In one embodiment, the present disclosure provides compositions, wherein the Fc region comprises a non naturally occurring amino acid residue at one or more positions selected from the group consisting of 234, 235, 236, 237, 238, 239, 240, 241, 243, 244, 245, 247, 251, 252, 254, 255, 256, 262, 263, 264, 265, 266, 267, 268, 269, 279, 280, 284, 292, 296, 297, 298, 299, 305, 313, 316, 325, 326, 327, 328, 329, 330, 332, 333, 334, 339, 341, 343, 370, 373, 378, 392, 416, 419, 421, 440 and 443 as numbered by the EU index as set forth in Kabat. Optionally, the Fc region may comprise a non naturally occurring amino acid residue at additional and/or alternative positions known to one skilled in the art (see, e.g., U.S. Patents 5,624,821; 6,277,375; 6,737,056; PCT Patent Publications WO 01/58957; WO 02/06919; WO 04/016750; WO 04/029207; WO 04/035752; WO 04/074455; WO 04/099249; WO 04/063351; WO 05/070963; WO 05/040217, WO 05/092925 and WO 06/020114).

In a specific embodiment, the present disclosure provides an Fc variant protein composition, wherein the Fc region comprises at least one non naturally occurring amino acid residue selected from the group consisting of 234D, 234E, 234N, 234Q, 234T, 234H, 234Y, 2341, 234V, 234F, 235A, 235D, 235R, 235W, 235P, 235S, 235N, 235Q, 235T, 235H, 235Y, 2351, 235V, 235F, 236E, 239D, 239E, 239N, 239Q, 239F, 239T, 239H, 239Y, 2401, 240A, 240T, 240M, 241W, 241 L, 241Y, 241E, 241 R. 243W, 243L 243Y, 243R, 243Q, 244H, 245A, 247L, 247V, 247G, 251F, 252Y, 254T, 255L, 256E, 256M, 2621, 262A, 262T, 262E, 2631, 263A, 263T, 263M, 264L, 2641, 264W, 264T, 264R, 264F, 264M, 264Y, 264E, 265G, 265N, 265Q, 265Y, 265F, 265V, 2651, 265L, 265H, 265T, 2661, 266A, 266T, 266M, 267Q, 267L, 268E, 269H, 269Y, 269F, 269R, 270E, 280A, 284M, 292P, 292L, 296E, 296Q, 296D, 296N, 296S, 296T, 296L, 2961, 296H, 269G, 297S, 297D, 297E, 298H, 2981, 298T, 298F, 2991, 299L, 299A, 299S, 299V, 299H, 299F, 299E, 3051, 313F, 316D, 325Q, 325L, 3251, 325D, 325E, 325A, 325T, 325V, 325H, 327G, 327W, 327N, 327L, 328S, 328M, 328D, 328E, 328N, 328Q, 328F, 3281, 328V, 328T, 328H, 328A, 329F, 329H, 329Q, 330K, 330G, 330T, 330C, 330L, 330Y, 330V, 3301, 330F, 330R, 330H, 332D, 332S, 332W, 332F, 332E, 332N, 332Q, 332T, 332H, 332Y, 332A, 339T, 370E, 370N, 378D, 392T, 396L, 416G, 419H, 421K, 440Yand 434W as numbered by the EU index as set forth in Kabat. Optionally, the Fc region may comprise additional and/or alternative non naturally occurring amino acid residues known to one skilled in the art (see, e.g., U.S. Patents 5,624,821; 6,277,375; 6,737,056; PCT Patent Publications WO 01/58957; WO 02/06919; WO 04/016750; WO 04/029207; WO 04/035752 and WO 05/040217).

In another embodiment, the present disclosure provides an Fc variant protein composition, wherein the Fc region comprises at least a non naturally occurring amino acid at one or more positions selected from the group consisting of 239, 330 and 332, as numbered by the EU index as set forth in Kabat. In a specific embodiment, the present disclosure provides an Fc variant protein formulation, wherein the Fc region comprises at least one non naturally occurring amino acid selected from the group consisting of 239D, 330L and 332E, as numbered by the EU index as set forth in Kabat. Optionally, the Fc region may further comprise additional non naturally occurring amino acid at one or more positions selected from the group consisting of 252, 254, and 256, as numbered by the EU index as set forth in Kabat. In a specific embodiment, the present disclosure provides an Fc variant protein formulation, wherein the Fc region comprises at least one non naturally occurring amino acid selected from the group consisting of 239D, 330L and 332E, as numbered by the EU index as set forth in Kabat and at least one non naturally occurring amino acid at one or more positions are selected from the group consisting of 252Y, 254T and 256E, as numbered by the EU index as set forth in Kabat.

In one embodiment, the Fc variants of the present disclosure may be combined with other known Fc variants such as those disclosed in Ghetie et al., 1997, Nat Biotech. 15:637-40; Duncan et al, 1988, Nature 332:563-564; Lund et al., 1991, J. Immunol 147:2657-2662; Lund et al, 1992, Mol Immunol 29:53-59; Alegre et al, 1994, Transplantation 57:1537-1543; Hutchins et al., 1995, Proc Natl. Acad Sci U S A 92:11980-11984; Jefferis et al, 1995, Immunol Lett. 44:111-117; Lund et al., 1995, Faseb J 9:115-119; Jefferis et al, 1996, Immunol Lett 54:101-104; Lund et al, 1996, J Immunol 157:4963-4969; Armour et al., 1999, Eur J Immunol 29:2613-2624; Idusogie et al, 2000, J Immunol 164:4178-4184; Reddy et al, 2000, J Immunol 164:1925-1933; Xu et al., 2000, Cell Immunol 200:16-26; Idusogie et al, 2001, J Immunol 166:2571-2575; Shields et al., 2001, J Biol Chem 276:6591-6604; Jefferis et al, 2002, Immunol Lett 82:57-65; Presta et al., 2002, Biochem Soc Trans 30:487-490); U.S. Patent Nos. 5,624,821; 5,885,573; 5,677,425; 6,165,745; 6,277,375; 5,869,046; 6,121,022; 5,624,821; 5,648,260; 6,528,624; 6,194,551; 6,737,056; 6,821,505; 6,277,375; U.S. Patent Publication Nos. 2004/0002587 and PCT Publications WO 94/29351; WO 99/58572; WO 00/42072; WO 02/060919; WO 04/029207; WO 04/099249; WO 04/063351. Also encompassed by the present disclosure are Fc regions which comprise deletions, additions and/or modifications. Still other modifications/substitutions/additions/deletions of the Fc domain will be readily apparent to one skilled in the art.

Methods for generating non naturally occurring Fc regions are known in the art. For example, amino acid substitutions and/or deletions can be generated by mutagenesis methods, including, but not limited to, site- directed mutagenesis (Kunkel, Proc. Natl. Acad. Sci. USA 82:488-492 (1985)), PCR mutagenesis (Higuchi, in "PCR Protocols: A Guide to Methods and Applications", Academic Press, San Diego, pp. 177-183 (1990)), and cassette mutagenesis (Wells et al., Gene 34:315-323 (1985)). Preferably, site-directed mutagenesis is performed by the overlap-extension PCR method (Higuchi, in "PCR Technology: Principles and Applications for DNA Amplification", Stockton Press, New York, pp. 61-70 (1989)). The technique of overlap-extension PCR (Higuchi, ibid.) can also be used to introduce any desired mutation(s) into a target sequence (the starting DNA). For example, the first round of PCR in the overlap-extension method involves amplifying the target sequence with an outside primer (primer 1) and an internal mutagenesis primer (primer 3), and separately with a second outside primer (primer 4) and an internal primer (primer 2), yielding two PCR segments (segments A and B). The internal mutagenesis primer (primer 3) is designed to contain mismatches to the target sequence specifying the desired mutation(s). In the second round of PCR, the products of the first round of PCR (segments A and B) are amplified by PCR using the two outside primers (primers 1 and 4). The resulting full-length PCR segment (segment C) is digested with restriction enzymes and the resulting restriction fragment is cloned into an appropriate vector. As the first step of mutagenesis, the starting DNA (e.g., encoding an Fc fusion protein, an antibody or simply an Fc region), is operably cloned into a mutagenesis vector. The primers are designed to reflect the desired amino acid substitution. Other methods useful for the generation of variant Fc regions are known in the art (see, e.g., U.S. Patent Nos. 5,624,821; 5,885,573; 5,677,425; 6,165,745; 6,277,375; 5,869,046; 6,121,022; 5,624,821; 5,648,260; 6,528,624; 6,194,551; 6,737,056; 6,821,505; 6,277,375; U.S. Patent Publication Nos. 2004/0002587 and PCT Publications WO 94/29351; WO 99/58572; WO 00/42072; WO 02/060919; WO 04/029207; WO 04/099249; WO 04/063351).

In some embodiments, an Fc variant protein comprises one or more engineered glycoforms, i.e., a carbohydrate composition that is covalently attached to the molecule comprising an Fc region. Engineered glycoforms may be useful for a variety of purposes, including but not limited to enhancing or reducing effector function. Engineered glycoforms may be generated by any method known to one skilled in the art, for example by using engineered or variant expression strains, by co-expression with one or more enzymes, for example DI N-acetylglucosaminyltransferase III (GnTI11), by expressing a molecule comprising an Fc region in various organisms or cell lines from various organisms, or by modifying carbohydrate(s) after the molecule comprising Fc region has been expressed. Methods for generating engineered glycoforms are known in the art, and include but are not limited to those described in Umana et al, 1999, Nat. Biotechnol 17:176-180; Davies et al., 20017 Biotechnol Bioeng 74:288-294; Shields et al, 2002, J Biol Chem 277:26733-26740; Shinkawa et al., 2003, J Biol Chem 278:3466-3473) U.S. Pat. No. 6,602,684; U.S. Ser. No. 10/277,370; U.S. Ser. No. 10/113,929; PCT WO 00/61739A1; PCT WO 01/292246A1; PCT WO 02/311140A1; PCT WO 02/30954A1; Potelligent™ technology (Biowa, Inc. Princeton, N.J.); GlycoMAb™ glycosylation engineering technology (GLYCART biotechnology AG, Zurich, Switzerland). See, e.g., WO 00061739; EA01229125; US 20030115614; Okazaki et al., 2004, JMB, 336: 1239-49.

### 5.15. Glycosylation of Antibodies

In still another embodiment, the glycosylation of antibodies utilized in accordance with the disclosure is modified. For example, an aglycosylated antibody can be made (i.e., the antibody lacks glycosylation). Glycosylation can be altered to, for example, increase the affinity of the antibody for a target antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Patent Nos. 5,714,350 and 6,350,861. One or more amino acid substitutions can also be made that result in elimination of a glycosylation site present in the Fc region (e.g., Asparagine 297 of IgG). Furthermore, aglycosylated antibodies may be produced in bacterial cells which lack the necessary glycosylation machinery.

An antibody can also be made that has an altered type of glycosylation, such as a hypofucosylated antibody having reduced amounts of fucosyl residues or an antibody having increased bisecting GlcNAc structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the disclosure to thereby produce an antibody with altered glycosylation. See, for example, Shields, R.L. et al. (2002) J. Biol. Chem. 277:26733-26740; Umana et al. (1999) Nat. Biotech. 17:176-1, as well as, U.S. Patent No: US 6,946,292; European Patent No: EP 1,176,195; PCT Publications WO 03/035835; WO 99/54342.

Antibodies with altered glycosylation pattern may also be generated using lower eukaryotic host cells comprising modified glycosylation machinery as described in U.S. Patent No. US7029872, US Patent Publication US20060148035A1.

### 5.16. Engineering Effector Function

It may be desirable to modify an anti-ICOS antibody of the disclosure with respect to effector function, so as to enhance the effectiveness of the antibody in treating T cell-mediated diseases, for example. For example, cysteine residue(s) may be introduced in the Fc region, thereby allowing interchain disulfide bond formation in this region. The homodimeric antibody thus generated may have improved internalization capability and/or increased complement-mediated cell killing and/or antibody-dependent cellular cytotoxicity (ADCC) and/or antibody dependent phagocytosis. *See,* Caron et al., J. Exp Med., 176:1191-1195 (1992) and Shopes, B., J. Immunol., 148:2918-2922 (1992). Homodimeric antibodies with enhanced anti-tumor activity may also be prepared using heterobifunctional cross-linkers as described in Wolff et al., Cancer Research, 53:2560-2565 (1993). An antibody can also be engineered which has dual Fc regions and may thereby have enhanced complement lysis, antibody-dependent phagocytosis and/or ADCC capabilities. *See,* Stevenson et al., Anti-Cancer Drug Design, 3:219-230 (1989).

Other methods of engineering Fc regions of antibodies so as to alter effector functions are known in the art (*e.g.,* U.S. Patent Publication No. 20040185045 and PCT Publication No. WO 2004/016750, both to Koenig et al., which describe altering the Fc region to enhance the binding affinity for FcγRIIB as compared with the binding affinity for FCγRIIA; *see, also,* PCT Publication Nos. WO 99/58572 to Armour et al., WO 99/51642 to Idusogie et al., and U.S. 6,395,272 to Deo et al.). Methods of modifying the Fc region to decrease binding affinity to FcγRIIB are also known in the art (*e.g.,* U.S. Patent Publication No. 20010036459 and PCT Publication No. WO 01/79299, both to Ravetch et al.). Modified antibodies having variant Fc regions with enhanced binding affinity for FcγRIIIA and/or FcγRIIA as compared with a wildtype Fc region have also been described (*e.g.,* PCT Publication Nos. WO 2004/063351, to Stavenhagen et al.).

*In vitro* assays known in the art can be used to determine whether anti-ICOS antibodies used in compositions and methods of the disclosure are capable of mediating ADCC, CDC, and/or antibody-depenedent phagocytosis, such as those described herein.

**5.17.** Manufacture/Production of Anti-ICOS Antibodies

Once a desired anti-ICOS antibody is engineered, the anti-ICOS antibody can be produced on a commercial scale using methods that are well-known in the art for large scale manufacturing of antibodies. For example, this can be accomplished using recombinant expressing systems such as, but not limited to, those described below. The antibodies (including antibody fragments thereof) that specifically bind to an antigen can be produced by any method known in the art for the synthesis of antibodies, in particular, by chemical synthesis or by recombinant expression techniques (*see,* US Patent Application 12/116,512).

**5.18.** Recombinant Expression Systems

Recombinant expression of an antibody or variant thereof, generally requires construction of an expression vector containing a polynucleotide that encodes the antibody. Once a polynucleotide encoding an antibody molecule or a heavy or light chain of an antibody, or portion thereof, has been obtained, the vector for the production of the antibody molecule may be produced by recombinant DNA technology using techniques well-known in the art. *See, e.g.,* U.S. Patent No. 6,331,415. Thus, methods for preparing a protein by expressing a polynucleotide containing an antibody encoding nucleotide sequence are described herein. Methods which are well-known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The disclosure, thus, provides replicable vectors comprising a nucleotide sequence encoding an antibody molecule, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule (*see, e.g.,* International Publication Nos. WO 86/05807 and WO 89/01036; and U.S. Patent No. 5,122,464) and the variable domain of the antibody may be cloned into such a vector for expression of the entire heavy, the entire light chain, or both the entire heavy and light chains.

In another embodiment, anti-ICOS antibodies can be made using targeted homologous recombination to produce all or portions of the anti-ICOS antibodies (*see,* U.S. Patent Nos. 6,063,630, 6,187,305, and 6,692,737). In certain embodiments, anti-ICOS antibodies can be made using random recombination techniques to produce all or portions of the anti-ICOS antibodies (*see,* U.S. Patent Nos. 6,361,972, 6,524,818, 6,541,221, and 6,623,958). Anti-ICOS antibodies can also be produced in cells expressing an antibody from a genomic sequence of the cell comprising a modified immunoglobulin locus using Cre-mediated site-specific homologous recombination (*see,* U.S. Patent No. 6,091,001). The host cell line may be derived from human or nonhuman species including but not limited to mouse, and Chinese hamster. Where human or humanized antibody production is desired, the host cell line should be a human cell line. These methods may advantageously be used to engineer stable cell lines which permanently express the antibody molecule.

Once the expression vector is transferred to a host cell by conventional techniques, the transfected cells are then cultured by conventional techniques to produce an antibody. Thus, the disclosure includes host cells containing a polynucleotide encoding an antibody of the disclosure or fragments thereof, or a heavy or light chain thereof, or portion thereof, or a single-chain antibody of the disclosure, operably linked to a heterologous promoter. In certain embodiments for the expression of double-chained antibodies, vectors encoding both the heavy and light chains may be co-expressed in the host cell for expression of the entire immunoglobulin molecule, as detailed below.

A variety of host-expression vector systems may be utilized to express an anti-ICOS antibody or portions thereof that can be used in the engineering and generation of anti-ICOS antibodies (*see, e.g.,* U.S. Patent No. 5,807,715). For example, mammalian cells such as Chinese hamster ovary cells (CHO), in conjunction with a vector such as the major intermediate early gene promoter element from human cytomegalovirus is an effective expression system for antibodies (Foecking et al., Gene, 45:101 (1986); and Cockett et al., Bio/Technology, 8:2 (1990)). In addition, a host cell strain may be chosen which modulates the expression of inserted antibody sequences, or modifies and processes the antibody gene product in the specific fashion desired. Such modifications (*e.g.,* glycosylation) and processing (*e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the antibody or portion thereof expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W138, BT483, Hs578T, HTB2, BT2O and T47D, NS0 (a murine myeloma cell line that does not endogenously produce any functional immunoglobulin chains), CRL7O3O and HsS78Bst cells.

In one embodiment, human cell lines developed by immortalizing human lymphocytes can be used to recombinantly produce monoclonal human anti-ICOS antibodies. In one embodiment, the human cell line PER.C6. (Crucell, Netherlands) can be used to recombinantly produce monoclonal human anti-ICOS antibodies.

In bacterial systems, a number of expression vectors may be advantageously selected depending upon the use intended for the antibody molecule being expressed. For example, when a large quantity of such an antibody is to be produced, for the generation of pharmaceutical compositions comprising an anti-ICOS antibody, vectors which direct the expression of high levels of fusion protein products that are readily purified may be desirable. Such vectors include, but are not limited to, the *E. coli* expression vector pUR278 (Ruther et al., EMBO, 12:1791 (1983)), in which the antibody coding sequence may be ligated individually into the vector in frame with the lac Z coding region so that a fusion protein is produced; pIN vectors (Inouye & Inouye, 1985, Nucleic Acids Res. 13:3101-3109 (1985); Van Heeke & Schuster, 1989, J. Biol. Chem., 24:5503-5509 (1989)); and the like. pGEX vectors may also be used to express foreign polypeptides as fusion proteins with glutathione-S-transferase (GST). In general, such fusion proteins are soluble and can easily be purified from lysed cells by adsorption and binding to glutathione-agarose affinity matrix followed by elution in the presence of free glutathione. The pGEX vectors are designed to introduce a thrombin and/or factor Xa protease cleavage sites into the expressed polypeptide so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example, the polyhedrin gene) of the virus and placed under control of an AcNPV promoter (for example, the polyhedrin promoter).

In mammalian host cells, a number of virus based expression systems may be utilized. In cases where an adenovirus is used as an expression vector, the antibody coding sequence of interest may be ligated to an adenovirus transcription/translation control complex, *e.g.,* the late promoter and tripartite leader sequence. This chimeric gene may then be inserted in the adenovirus genome by *in vitro* or *in vivo* recombination. Insertion into a non-essential region of the viral genome (e.g., region EI or E3) will result in a recombinant virus that is viable and capable of expressing the antibody molecule in infected hosts (*e.g., see,* Logan & Shenk, Proc. Natl. Acad. Sci. USA, 81:355-359 (1984)). Specific initiation signals may also be required for efficient translation of inserted antibody coding sequences. These signals include the ATG initiation codon and adjacent sequences. Furthermore, the initiation codon should generally be in frame with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, *etc.* (*see, e.g.,* Bittner et al., Methods in Enzymol., 153:51-544(1987)).

Stable expression can be used for long-term, high-yield production of recombinant proteins. For example, cell lines which stably express the antibody molecule may be generated. Host cells can be transformed with an appropriately engineered vector comprising expression control elements (e.g., promoter, enhancer, transcription terminators, polyadenylation sites, etc.), and a selectable marker gene. Following the introduction of the foreign DNA, cells may be allowed to grow for 1-2 days in an enriched media, and then are switched to a selective media. The selectable marker in the recombinant plasmid confers resistance to the selection and allows cells that stably integrated the plasmid into their chromosomes to grow and form foci which in turn can be cloned and expanded into cell lines. Plasmids that encode an anti-ICOS antibody can be used to introduce the gene/cDNA into any cell line suitable for production in culture.

A number of selection systems may be used, including, but not limited to, the herpes simplex virus thymidine kinase (Wigler et al., Cell, 11:223 (1977)), hypoxanthineguanine phosphoribosyltransferase (Szybalska & Szybalski, Proc. Natl. Acad. Sci. USA, 48:202 (1992)), and adenine phosphoribosyltransferase (Lowy et al., Cell, 22:8-17 (1980)) genes can be employed in tk⁻, hgprt⁻ or aprT⁻cells, respectively. Also, antimetabolite resistance can be used as the basis of selection for the following genes: *dhfr,* which confers resistance to methotrexate (Wigler et al., Natl. Acad. Sci. USA, 77:357 (1980); O'Hare et al., Proc. Natl. Acad. Sci. USA, 78:1527 (1981)); *gpt,* which confers resistance to mycophenolic acid (Mulligan & Berg, Proc. Natl. Acad. Sci. USA, 78:2072 (1981)); *neo,* which confers resistance to the aminoglycoside G-418 (Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); May, TIB TECH 11(5):155-2 15 (1993)); and *hygro,* which confers resistance to hygromycin (Santerre et al., Gene, 30:147 (1984)). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds.), Current Protocols in Human Genetics, John Wiley & Sons, NY (1994); Colberre-Garapin et al., 1981, J. Mol. Biol., 150:1.

The expression levels of an antibody molecule can be increased by vector amplification (for a review, *see,* Bebbington and Hentschel, The use of vectors based on gene amplification for the expression of cloned genes in mammalian cells in DNA cloning, Vol. 3. Academic Press, New York (1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., Mol. Cell. Biol., 3:257 (1983)). Antibody expression levels may be amplified through the use recombinant methods and tools known to those skilled in the art of recombinant protein production, including technologies that remodel surrounding chromatin and enhance transgene expression in the form of an active artificial transcriptional domain.

The host cell may be co-transfected with two expression vectors, the first vector encoding a heavy chain derived polypeptide and the second vector encoding a light chain derived polypeptide. The two vectors may contain identical or different selectable markers. A single vector which encodes, and is capable of expressing, both heavy and light chain polypeptides may also be used. In such situations, the light chain should be placed 5' to the heavy chain to avoid an excess of toxic free heavy chain (Proudfoot, Nature 322:562-65 (1986); and Kohler, 1980, Proc. Natl. Acad. Sci. USA, 77:2197 (1980)). The coding sequences for the heavy and light chains may comprise cDNA or genomic DNA.

Once an antibody molecule has been produced by recombinant expression, it may be purified by any method known in the art for purification of an immunoglobulin molecule, for example, by chromatography (e.g., ion exchange, affinity, particularly by affinity for the specific antigens Protein A or Protein G, and sizing column chromatography), centrifugation, differential solubility, or by any other standard technique for the purification of proteins. Further, the antibodies of the present disclosure or fragments thereof may be fused to heterologous polypeptide sequences described herein or otherwise known in the art to facilitate purification.

**5.19.** Antibody Purification and Isolation

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology, 10:163-167 (1992) describe a procedure for isolating antibodies which are secreted into the periplasmic space *of E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody mutant is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, hydrophobic interaction chromatography, ion exchange chromatography, gel electrophoresis, dialysis, and/or affinity chromatography either alone or in combination with other purification steps. The suitability of protein A as an affinity ligand depends on the species and isotype of any immunoglobulin Fc domain that is present in the antibody mutant. Protein A can be used to purify antibodies that are based on human $\gamma 1$, $\gamma 2$, or $\gamma 4$ heavy chains (Lindmark et al., J. Immunol. Methods, 62:1-13 (1983)). Protein G is recommended for all mouse isotypes and for human $\gamma 3$ (Guss et al., EMBO J., 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a $CH_3$ domain, the Bakerbond ABX resin (J.T. Baker, Phillipsburg, NJ) is useful for purification. Other techniques for protein purification such as fractionation on an ion-exchange column, ethanol precipitation, Reverse Phase HPLC, chromatography on silica, chromatography on heparin, SEPHAROSE chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, and performed at low salt concentrations (e.g., from about 0-0.25 M salt).

**5.20.** Therapeutic Anti-ICOS Antibodies

An anti-ICOS antibody used in compositions and methods of the disclosure may be a human antibody or a humanized antibody that may mediate T cell lineage ADCC, antibody-dependent phagocytosis and/or CDC, or can be selected from known anti-ICOS antibodies that may mediate T lineage cell ADCC, antibody-dependent phagocytosis and/or CDC. In certain embodiments, anti-ICOS antibodies can be chimeric antibodies. In certain embodiments, an anti-ICOS antibody can be a monoclonal human, humanized, or chimeric anti-ICOS antibody. An anti-ICOS antibody used in compositions and methods of the disclosure may be a human antibody or a humanized antibody of the IgG1 or IgG3 human isotype or any IgG1 or IgG3 allele found in the human population. In other embodiments, an anti-ICOS antibody used in compositions and methods of the disclosure can be a human antibody or a humanized antibody of the IgG2 or IgG4 human isotype or any IgG2 or IgG4 allele found in the human population.

While such antibodies can be generated using the techniques described above, in other embodiments of the disclosure, the human JMab-136 anti-ICOS antibody (see, US Patent 6,803,039) can be modified to generate an anti-ICOS antibody with enhanced effector function such as, but not limited to, ADCC, antibody-dependent phagocytosis and/or CDC. For example, known anti-ICOS antibodies that can be used include, but are not limited to, anti-human ICOS monoclonal antibodies disclosed in US Patent 6,803,039, and clone ISA-3 (eBioscience, US).

In certain embodiments, the antibody is an isotype switched variant of a known antibody (e.g., to an IgG1 or IgG3 human isotype) such as those described above.

An anti-ICOS antibodies used in compositions and methods of the disclosure can be naked antibodies, immunoconjugates or fusion proteins. Anti-ICOS antibodies described above for use in compositions and methods of the disclosure may be able to reduce or deplete ICOS expressing T cells and circulating immunoglobulin in a human treated therewith. Depletion of T cells can be in circulating T cells, or in particular tissues such as, but not limited to, bone marrow, spleen, gut-associated lymphoid tissues, and/or lymph nodes. Such depletion may be achieved via various mechanisms such as antibody-dependent cell-mediated cytotoxicity (ADCC), and/or antibody dependent phagocytosis, and/or by blocking of ICOS interaction with its intended ligand, and/or complement dependent cytotoxicity (CDC). By "depletion" of T cells it is meant a reduction in circulating ICOS expressing T cells and/or ICOS expressing T cells in particular tissue(s) by at least about 25%, 40%, 50%, 65%, 75%, 80%, 85%, 90%, 95% or more. In particular embodiments, virtually all detectable ICOS expressing T cells are depleted from the circulation and/or particular tissue(s). By "depletion" of circulating immunoglobulin (Ig) it is meant a reduction by at least about 25%, 40%, 50%, 65%, 75%, 80%, 85%, 90%, 95% or more. In particular embodiments, virtually all detectable Ig is depleted from the circulation.

**5.21.** Screening of Antibodies for Human ICOS Binding

Binding assays can be used to identify antibodies that bind the human ICOS antigen. Binding assays may be performed either as direct binding assays or as competition-binding assays. Binding can be detected using standard ELISA or standard Flow Cytometry assays. In a direct binding assay, a candidate antibody is tested for binding to human ICOS antigen. In certain embodiments, the screening assays comprise, in a second step, determining the ability to of an antibody to induce downstream signaling events in T cells expressing human ICOS. Competition-binding assays, on the other hand, assess the ability of a candidate antibody to compete with a known anti-ICOS antibody or other compound that binds human ICOS.

In a direct binding assay, the human ICOS antigen is contacted with a candidate antibody under conditions that allow binding of the candidate antibody to the human ICOS antigen. The binding may take place in solution or on a solid surface. The candidate antibody may have been previously labeled for detection. Any detectable compound can be used for labeling, such as, but not limited to, a luminescent, fluorescent, or radioactive isotope or group containing same, or a nonisotopic label, such as an enzyme or dye. After a period of incubation sufficient for binding to take place, the reaction is exposed to conditions and manipulations that remove excess or non-specifically bound antibody. Typically, it involves washing with an appropriate buffer. Finally, the presence of an ICOS-antibody complex is detected.

In a competition-binding assay, a candidate antibody is evaluated for its ability to inhibit or displace the binding of a known anti-ICOS antibody (or other compound) to the human ICOS antigen. A labeled known binder of ICOS may be mixed with the candidate antibody, and placed under conditions in which the interaction between them would normally occur, with and without the addition of the candidate antibody. The amount of labeled known binder of ICOS that binds the human ICOS may be compared to the amount bound in the presence or absence of the candidate antibody.

In one embodiment, the binding assay is carried out with one or more components immobilized on a solid surface to facilitate antibody antigen complex formation and detection. In various embodiments, the solid support could be, but is not restricted to, polyvinylidene fluoride, polycarbonate, polystyrene, polypropylene, polyethylene, glass, nitrocellulose, dextran, nylon, polyacrylamide and agarose. The support configuration can include beads, membranes, microparticles, the interior surface of a reaction vessel such as a microtiter plate, test tube or other reaction vessel. The immobilization of human ICOS, or other component, can be achieved through covalent or non-covalent attachments. In one embodiment, the attachment may be indirect, *i.e.,* through an attached antibody. In another embodiment, the human ICOS antigen and negative controls are tagged with an epitope, such as glutathione S-transferase (GST) so that the attachment to the solid surface can be mediated by a commercially available antibody such as anti-GST (Santa Cruz Biotechnology).

For example, such an affinity binding assay may be performed using the human ICOS antigen which is immobilized to a solid support. Typically, the non-mobilized component of the binding reaction, in this case the candidate anti-ICOS antibody, is labeled to enable detection. A variety of labeling methods are available and may be used, such as luminescent, chromophore, fluorescent, or radioactive isotope or group containing same, and nonisotopic labels, such as enzymes or dyes. In one embodiment, the candidate anti-ICOS antibody is labeled with a fluorophore such as fluorescein isothiocyanate (FITC, available from Sigma Chemicals, St. Louis). Such an affinity binding assay may be performed using the human ICOS antigen immobilized on a solid surface. Anti-ICOS antibodies are then incubated with the antigen and the specific binding of antibodies is detected by methods known in the art including, but not limited to, BiaCore Analyses, ELISA, FMET and RIA methods.

Finally, the label remaining on the solid surface may be detected by any detection method known in the art. For example, if the candidate anti-ICOS antibody is labeled with a fluorophore, a fluorimeter may be used to detect complexes.

The human ICOS antigen can be added to binding assays in the form of intact cells that express human ICOS antigen, or isolated membranes containing human ICOS antigen. Thus, direct binding to human ICOS antigen may be assayed in intact cells in culture or in animal models in the presence and absence of the candidate anti-ICOS antibody. A labeled candidate anti-ICOS antibody may be mixed with cells that express human ICOS antigen, or with crude extracts obtained from such cells, and the candidate anti-ICOS antibody may be added. Isolated membranes may be used to identify candidate anti-ICOS antibodies that interact with human ICOS. For example, in a typical experiment using isolated membranes, cells may be genetically engineered to express human ICOS antigen. Membranes can be harvested by standard techniques and used in an *in vitro* binding assay. Labeled candidate anti-ICOS antibody (e.g., fluorescent labeled antibody) is bound to the membranes and assayed for specific activity; specific binding is determined by comparison with binding assays performed in the presence of excess unlabeled (cold) candidate anti-ICOS antibody. Soluble human ICOS antigen may also be recombinantly expressed and utilized in non-cell based assays to identify antibodies that bind to human ICOS antigen. The recombinantly expressed human ICOS polypeptides can be used in the non-cell based screening assays. Peptides corresponding to one or more of the binding portions of human ICOS antigen, or fusion proteins containing one or more of the binding portions of human ICOS antigen can also be used in non-cell based assay systems to identify antibodies that bind to portions of human ICOS antigen. In non-cell based assays the recombinantly expressed human ICOS is attached to a solid substrate such as a test tube, microtiter well or a column, by means well-known to those in the art (*see,* Ausubel *et al., supra*). The test antibodies are then assayed for their ability to bind to human ICOS antigen.

The binding reaction may also be carried out in solution. In this assay, the labeled component is allowed to interact with its binding partner(s) in solution. If the size differences between the labeled component and its binding partner(s) permit such a separation, the separation can be achieved by passing the products of the binding reaction through an ultrafilter whose pores allow passage of unbound labeled component but not of its binding partner(s) or of labeled component bound to its partner(s). Separation can also be achieved using any reagent capable of capturing a binding partner of the labeled component from solution, such as an antibody against the binding partner and so on.

In another specific embodiment, the solid support is membrane containing human ICOS antigen attached to a microtiter dish. Candidate antibodies, for example, can bind cells that express library antibodies cultivated under conditions that allow expression of the library members in the microtiter dish. Library members that bind to the human ICOS are harvested. Such methods, are generally described by way of example in Parmley and Smith, 1988, Gene, 73:305-318; Fowlkes et al., 1992, BioTechniques, 13:422-427; PCT Publication No. WO94/18318; and in references cited hereinabove. Antibodies identified as binding to human ICOS antigen can be of any of the types or modifications of antibodies described above.

5.21.1. Screening Of Antibodies for Human ADCC Effector Function

Antibodies of the human IgG class, which have functional characteristics such a long half-life in serum and the ability to mediate various effector functions are used in certain embodiments of the disclosure (Monoclonal Antibodies: Principles and Applications, Wiley-Liss, Inc., Chapter 1 (1995)). The human IgG class antibody is further classified into the following 4 subclasses: IgG1, IgG2, IgG3 and IgG4. A large number of studies have so far been conducted for ADCC and CDC as effector functions of the IgG class antibody, and it has been reported that among antibodies of the human IgG class, the IgG1 subclass has the highest ADCC activity and CDC activity in humans (Chemical Immunology, 65, 88 (1997)).

Expression of ADCC activity and CDC activity of the human IgG1 subclass antibodies generally involves binding of the Fc region of the antibody to a receptor for an antibody (hereinafter referred to as "FcγR") existing on the surface of effector cells such as killer cells, natural killer cells or activated macrophages. Various complement components can be bound. Regarding the binding, it has been suggested that several amino acid residues in the hinge region and the second domain of C region (hereinafter referred to as "Cγ2 domain") of the antibody are important (Eur. J. Immunol., 23, 1098 (1993), Immunology, 86, 319 (1995), Chemical Immunology, 65, 88 (1997)) and that a sugar chain in the Cγ2 domain (Chemical Immunology, 65, 88 (1997)) is also important.

Anti-ICOS antibodies can be modified with respect to effector function, *e.g.*, so as to enhance ADCC and/or complement dependent cytotoxicity (CDC) of the antibody. This may be achieved by introducing one or more amino acid substitutions in the Fc region of an antibody. Cysteine residue(s) may also be introduced in the Fc region, allowing for interchain disulfide bond formation in this region. In this way a homodimeric antibody can be generated that may have improved internalization capability and or increased complement-mediated cell killing and ADCC (Caron et al., J. Exp. Med., 176:1191-1195 (1992) and Shopes, J. Immunol., 148:2918-2922 (1992)). Heterobifunctional cross-linkers can also be used to generate homodimeric antibodies with enhanced anti-tumor activity (Wolff et al., Cancer Research, 53:2560-2565 (1993)). Antibodies can also be engineered to have two or more Fc regions resulting in enhanced complement lysis and ADCC capabilities (Stevenson et al., Anti-Cancer Drug Design, (3)219-230 (1989)).

Other methods of engineering Fc regions of antibodies so as to alter effector functions are known in the art (*e.g.,* U.S. Patent Publication No. 20040185045 and PCT Publication No. WO 2004/016750, both to Koenig et al.*,* which describe altering the Fc region to enhance the binding affinity for FcγRIIB as compared with the binding affinity for FCγRIIA; *see also* PCT Publication Nos. WO 99/58572 to Armour et al.*,* WO 99/51642 to Idusogie et al.*,* and U.S. 6,395,272 to Deo et al.). Methods of modifying the Fc region to decrease binding affinity to FcγRIIB are also known in the art (*e.g.,* U.S. Patent Publication No. 20010036459 and PCT Publication No. WO 01/79299, both to Ravetch et al.). Modified antibodies having variant Fc regions with enhanced binding affinity for FcγRIIIA and/or FcγRIIA as compared with a wildtype Fc region have also been described (*e.g.,* PCT Publication No. WO 2004/063351, to Stavenhagen et al.).

At least four different types of FcγR have been found, which are respectively called FcγRI (CD64), FcγRII (CD32), FcγRIII (CD16), and FcγRIV. In human, FcγRII and FcγRIII are further classified into FcγRIIa and FcγRIIb, and FcγRIIIa and FcγRIIIb, respectively. FcγR is a membrane protein belonging to the immunoglobulin superfamily, FcγRII, FcγRIII, and FcγRIV have an α chain having an extracellular region containing two immunoglobulin-like domains, FcγRI has an α chain having an extracellular region containing three immunoglobulin-like domains, as a constituting component, and the α chain is involved in the IgG binding activity. In addition, FcγRI and FcγRIII have a γ chain or ζ chain as a constituting component which has a signal transduction function in association with the α chain (Annu. Rev. Immunol., 18, 709 (2000), Annu. Rev. Immunol., 19, 275 (2001)). FcγRIV has been described by Bruhns et al., Clin. Invest. Med., (Canada) 27:3D (2004).

To assess ADCC activity of an anti-ICOS antibody of interest, an *in vitro* ADCC assay can be used, such as that described in U.S. Patent No. 5,500,362 or 5,821,337. The assay may also be performed using a commercially available kit, e.g. CytoTox 96 ® (Promega). Useful effector cells for such assays include, but are not limited to peripheral blood mononuclear cells (PBMC), Natural Killer (NK) cells, and NK cell lines. NK cell lines expressing a transgenic Fc receptor (e.g. CD16) and associated signaling polypeptide (e.g. FCεRI-γ) may also serve as effector cells (*see, e.g.* WO 2006/023148 A2 to Campbell). For example, the ability of any particular antibody to mediate lysis of the target cell by complement activation and/or ADCC can be assayed. The cells of interest are grown and labeled *in vitro*; the antibody is added to the cell culture in combination with immune cells which may be activated by the antigen antibody complexes; *i.e.,* effector cells involved in the ADCC response. The antibody can also be tested for complement activation. In either case, cytolysis of the target cells is detected by the release of label from the lysed cells. The extent of target cell lysis may also be determined by detecting the release of cytoplasmic proteins (e.g. LDH) into the supernatant. In fact, antibodies can be screened using the patient's own serum as a source of complement and/or immune cells. The antibodies that are capable of mediating human ADCC in the *in vitro* test can then be used therapeutically in that particular patient. ADCC activity of the molecule of interest may also be assessed *in vivo, e.g.,* in an animal model such as that disclosed in Clynes et al., Proc. Natl. Acad. Sci. (USA) 95:652-656 (1998). Moreover, techniques for modulating *(i.e.,* increasing or decreasing) the level of ADCC, and optionally CDC activity, of an antibody are well-known in the art. *See, e.g.,* U.S. Patent No. 6,194,551. Antibodies of the present disclosure may be capable or may have been modified to have the ability of inducing ADCC and/or CDC. Assays to determine ADCC function can be practiced using human effector cells to assess human ADCC function. Such assays may also include those intended to screen for antibodies that induce, mediate, enhance, block cell death by necrotic and/or apoptotic mechanisms. Such methods including assays utilizing viable dyes, methods of detecting and analyzing caspases, and assays measuring DNA breaks can be used to assess the apoptotic activity of cells cultured *in vitro* with an anti-ICOS antibody of interest.

For example, Annexin V or TdT-mediated dUTP nick-end labeling (TUNEL) assays can be carried out as described in Decker et al., Blood (USA) 103:2718-2725 (2004) to detect apoptotic activity. The TUNEL assay involves culturing the cell of interest with fluorescein-labeled dUTP for incorporation into DNA strand breaks. The cells are then processed for analysis by flow cytometry. The Annexin V assay detects the appearance of phosphatidylserine (PS) on the outside of the plasma membrane of apoptotic cells using a fluorescein-conjugated Annexin V that specifically recognizes the exposed PS molecules. Concurrently, a viable dye such as propidium iodide can be used to exclude late apoptotic cells. The cells are stained with the labeled Annexin V and are analyzed by flow cytometry.

**5.22. Immunoconjugates and Fusion Proteins**

According to certain aspects of the disclosure, therapeutic agents or toxins can be conjugated to anti-ICOS antibodies for use in compositions and methods of the disclosure. In certain embodiments, these conjugates can be generated as fusion proteins. Examples of therapeutic agents and toxins include, but are not limited to, members of the enediyne family of molecules, such as calicheamicin and esperamicin. Chemical toxins can also be taken from the group consisting of duocarmycin (*see, e.g.,* U.S. Patent No. 5,703,080 and U.S. Patent No. 4,923,990), methotrexate, doxorubicin, melphalan, chlorambucil, ARA-C, vindesine, mitomycin C, cis-platinum, etoposide, bleomycin and 5-fluorouracil. Examples of chemotherapeutic agents also include Adriamycin, Doxorubicin, 5-Fluorouracil, Cytosine arabinoside (Ara-C), Cyclophosphamide, Thiotepa, Taxotere (docetaxel), Busulfan, Cytoxin, Taxol, Methotrexate, Cisplatin, Melphalan, Vinblastine, Bleomycin, Etoposide, Ifosfamide, Mitomycin C, Mitoxantrone, Vincreistine, Vinorelbine, Carboplatin, Teniposide, Daunomycin, Carminomycin, Aminopterin, Dactinomycin, Mitomycins, Esperamicins *(see,* U.S. Patent No. 4,675,187), Melphalan, and other related nitrogen mustards.

In certain embodiments, anti-ICOS antibodies are conjugated to a cytostatic, cytotoxic or immunosuppressive agent wherein the cytotoxic agent is selected from the group consisting of an enediyne, a lexitropsin, a duocarmycin, a taxane, a puromycin, a dolastatin, a maytansinoid, and a vinca alkaloid. In certain, more specific embodiments, the cytotoxic agent is paclitaxel, docetaxel, CC-1065, SN-38, topotecan, morpholino-doxorubicin, rhizoxin, cyanomorpholino-doxorubicin, dolastatin-10, echinomycin, combretastatin, calicheamicin, maytansine, DM-1, auristatin E, AEB, AEVB, AEFP, MMAE (*see,* US Patent Application No. 10/983,340), or netropsin.

In certain embodiments, the cytotoxic agent of an anti-ICOS antibody-cytotoxic agent conjugate of the disclosure is an anti-tubulin agent. In specific embodiments, the cytotoxic agent is selected from the group consisting of a vinca alkaloid, a podophyllotoxin, a taxane, a baccatin derivative, a cryptophysin, a maytansinoid, a combretastatin, and a dolastatin. In other embodiments, the cytotoxic agent is vincristine, vinblastine, vindesine, vinorelbine, VP-16, camptothecin, paclitaxel, docetaxel, epithilone A, epithilone B, nocodazole, coichicine, colcimid, estramustine, cemadotin, discodermolide, maytansine, DM-1, AEFP, auristatin E, AEB, AEVB, AEFP, MMAE or eleutherobin.

In specific embodiments, an anti-ICOS antibody is conjugated to the cytotoxic agent via a linker, wherein the linker is peptide linker. In other embodiments, an anti-ICOS antibody is conjugated to the cytotoxic agent via a linker, wherein the linker is a val-cit linker, a phe-lys linker, a hydrazone linker, or a disulfide linker.

In certain embodiments, the anti-ICOS antibody of an anti-ICOS antibody-cytotoxic agent conjugate is conjugated to the cytotoxic agent via a linker, wherein the linker is hydrolysable at a pH of less than 5.5. In a specific embodiment the linker is hydrolyzable at a pH of less than 5.0.

In certain embodiments, the anti-ICOS antibody of an anti-ICOS antibody-cytotoxic agent conjugate is conjugated to the cytotoxic agent via a linker, wherein the linker is cleavable by a protease. In a specific embodiment, the protease is a lysosomal protease. In other embodiments, the protease is, inter alia, a membrane-associated protease, an intracellular protease, or an endosomal protease.

Other toxins that can be used in immunoconjugates of the disclosure include poisonous lectins, plant toxins such as ricin, abrin, modeccin, botulina, and diphtheria toxins. Of course, combinations of the various toxins could also be coupled to one antibody molecule thereby accommodating variable cytotoxicity. Illustrative of toxins which are suitably employed in combination therapies of the disclosure are ricin, abrin, ribonuclease, DNase I, Staphylococcal enterotoxin-A, pokeweed anti-viral protein, gelonin, diphtheria toxin, Pseudomonas exotoxin, and Pseudomonas endotoxin. *See,* for example, Pastan et al., Cell, 47:641 (1986), and Goldenberg et al., Cancer Journal for Clinicians, 44:43 (1994). Enzymatically active toxins and fragments thereof which can be used include diphtheria A chain, non-binding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), *Momordica charantia* inhibitor, curcin, crotin, *Sapaonaria officinalis* inhibitor, gelonin, mitogellin, restrictocin, phenomycin, enomycin and the tricothecenes. *See,* for example, WO 93/21232 published October 28, 1993.

Suitable toxins and chemotherapeutic agents are described in Remington's Pharmaceutical Sciences, 19th Ed. (Mack Publishing Co. 1995), and in Goodman and Gilman's The Pharmacological Basis of Therapeutics, 7th Ed. (MacMillan Publishing Co. 1985). Other suitable toxins and/or chemotherapeutic agents are known to those of skill in the art.

The present disclosure further encompasses antibodies (including antibody fragments or variants thereof) comprising or conjugated to a radioactive agent suitable for diagnostic purposes. Examples of suitable radioactive materials include, but are not limited to, iodine ($^{121}$I, $^{123}$I, $^{125}$I, $^{131}$I), carbon ($^{14}$C), sulfur ($^{35}$S), tritium ($^{3}$H), indium ($^{111}$In, $^{112}$In, $^{113m}$In, $^{115m}$In), technetium ($^{99}$Tc, $^{99m}$Tc), thallium ($^{201}$Ti), gallium ($^{68}$Ga, $^{67}$Ga), palladium ($^{103}$Pd), molybdenum ($^{99}$Mo), xenon ($^{135}$Xe), fluorine ($^{18}$F), $^{153}$Sm, $^{177}$Lu, $^{159}$Gd, $^{149}$Pm, $^{140}$La, $^{175}$Yb, $^{166}$Ho, $^{90}$Y, $^{47}$Sc, $^{186}$Re, $^{188}$Re, $^{142}$Pr, $^{105}$Rh, and $^{97}$Ru.

Further, an anti-ICOS antibody of the disclosure (including a scFv or other molecule comprising, or alternatively consisting of, antibody fragments or variants thereof), may be coupled or conjugated to a radioactive metal ion utilized for therapeutic purposes. Examples of suitable radioactive ions include, but are not limited to, alpha-emitters such as $^{213}$Bi, or other radioisotopes such as $^{103}$Pd, $^{135}$Xe, $^{131}$I, $^{68}$Ge, $^{57}$Co, $^{65}$Zn, $^{85}$Sr, $^{32}$P, $^{35}$S, $^{90}$Y, $^{153}$sm, $^{153}$Gd, $^{169}$Yb, $^{51}$Cr, $^{54}$Mn, $^{75}$Se, $^{113}$Sn, $^{90}$Y, $^{117}$Tin, $^{186}$Re, $^{188}$Re and $^{166}$Ho. In specific embodiments, an antibody or fragment thereof is attached to macrocyclic chelators that chelate radiometal ions, including but not limited to, $^{177}$Lu, $^{90}$Y, $^{166}$Ho, and $^{153}$Sm, to polypeptides. In specific embodiments, the macrocyclic chelator is 1,4,7,10-tetraazacyclod- odecane-N,N',N'',N'''-tetraacetic acid (DOTA). In other specific embodiments, the DOTA is attached to an antibody of the disclosure or fragment thereof via a linker molecule. Examples of linker molecules useful for conjugating DOTA to a polypeptide are commonly known in the art, see, for example, DeNardo et al., Clin Cancer Res 4(10):2483-90, 1998; Peterson et al., Bioconjug Chem 10(4):553-7, 1999; and Zimmerman et al., Nucl Med Biol 26(8):943-50, 1999.

An anti-ICOS antibody of the present disclosure may also be used in ADEPT by conjugating the antibody to a prodrug-activating enzyme which converts a prodrug (e.g., a peptidyl chemotherapeutic agent, see, WO81/01145) to an active anti-cancer drug. See, for example, WO 88/07378 and U.S. Patent No. 4,975,278. The enzyme component of the immunoconjugate useful for ADEPT includes any enzyme capable of acting on a prodrug in such a way so as to covert it into its more active, cytotoxic form.

Enzymes that are useful in the method of this disclosure include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with α-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Antibodies with enzymatic activity, also known in the art as "abzymes," can be used as well to convert the prodrugs into free active drugs (see, e.g., Massey, Nature 328:457-458 (1987)). Antibody-abzyme conjugates can be prepared as described herein for delivery of the abzyme as desired to portions of a human affected by an ICOS expressing T cell malignancy.

Antibodies of this disclosure may be covalently bound to the enzymes by techniques well-known in the art such as the use of the heterobifunctional crosslinking reagents discussed above. Fusion proteins comprising at least the antigen-binding region of an anti-ICOS antibody linked to at least a functionally active portion of an enzyme may also be constructed using recombinant DNA techniques well-known in the art (see, e.g., Neuberger et al., Nature, 312:604-608 (1984)).

Covalent modifications of an anti-ICOS antibody are included within the scope of this disclosure. They may be made by chemical synthesis or by enzymatic or chemical cleavage of the antibody, if applicable. Other types of covalent modifications of an anti-ICOS antibody are introduced into the molecule by reacting targeted amino acid residues of the antibody with an organic derivatizing agent that is capable of reacting with selected side chains or the N- or C-terminal residues.

Cysteinyl residues most commonly are reacted with α-haloacetates (and corresponding amines), such as chloroacetic acid or chloroacetamide, to give carboxymethyl or carboxyamidomethyl derivatives. Similarly, iodo-reagents may also be used. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction can be performed in 0.1 M sodium cacodylate at pH 6.0.

Lysyl and amino-terminal residues are reacted with succinic or other carboxylic acid anhydrides. Derivatization with these agents has the effect of reversing the charge of the lysinyl residues. Other suitable reagents for derivatizing α-amino-containing residues and/or ε-amino-containing residues include imidoesters such as methyl picolinimidate, pyridoxal phosphate, pyridoxal, chloroborohydride, trinitrobenzenesulfonic acid, 0-methylisourea, 2,4-pentanedione, and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginyl residues generally requires that the reaction be performed in alkaline conditions because of the high pKa of the guanidine functional group. Furthermore, these reagents may react with the ε-amino groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using $^{125}$I or $^{131}$I to prepare labeled proteins for use in radioimmunoassay.

Carboxyl side groups (aspartyl or glutamyl) are selectively modified by reaction with carbodiimides (R--N=C=N--R'), where R and R' are different alkyl groups, such as 1-cyclohexyl-3-(2-morpholinyl-- 4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues, respectively. These residues are deamidated under neutral or basic conditions. The deamidated form of these residues falls within the scope of this disclosure.

Other modifications include hydroxylation of proline and lysine, phosphorylation of hydroxyl groups of seryl or threonyl residues, methylation of the α-amino groups of lysine, arginine, and histidine side chains (T.E. Creighton, Proteins: Structure and Molecular Properties, W.H. Freeman & Co., San Francisco, pp. 79-86 (1983)), acetylation of the N-terminal amine, and amidation of any C-terminal carboxyl group.

Another type of covalent modification involves chemically or enzymatically coupling glycosides to the antibody. These procedures are advantageous in that they do not require production of the antibody in a host cell that has glycosylation capabilities for N- or O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published 11 Sep. 1987, and in Aplin and Wriston, CRC Crit. Rev. Biochem., pp. 259-306 (1981).

**5.23.** Chemotherapeutic Combinations

According to the disclosure, cancer or one or more symptoms thereof may be prevented, treated, managed or ameliorated by the administration of an anti-ICOS antibody formulation in combination with the administration of one or more therapies such as, but not limited to, chemotherapies, radiation therapies, hormonal therapies, and/or biological therapies/immunotherapies.

In a specific embodiment, methods of the disclosure encompass the administration of one or more angiogenesis antagonists such as but not limited to: Angiostatin (plasminogen fragment); antiangiogenic antithrombin III; Angiozyme; ABT-627; Bay 12-9566; Benefin; Bevacizumab; BMS-275291; cartilage-derived inhibitor (CDI); CAI; CD59 complement fragment; CEP-7055; Col 3; Combretastatin A-4; Endostatin (collagen XVIII fragment); Fibronectin fragment; Gro-beta; Halofuginone; Heparinases; Heparin hexasaccharide fragment; HMV833; Human chorionic gonadotropin (hCG); IM-862; Interferon alpha/beta/gamma; Interferon inducible protein (IP-10); Interleukin-12; Kringle 5 (plasminogen fragment); Marimastat; Metalloproteinase inhibitors (TIMPs); 2-Methoxyestradiol; MMI 270 (CGS 27023A); MoAb IMC-1C11; Neovastat; NM-3; Panzem; PI-88; Placental ribonuclease inhibitor; Plasminogen activator inhibitor; Platelet factor-4 (PF4); Prinomastat; Prolactin 16kD fragment; Proliferin-related protein (PRP); PTK 787/ZK 222594; Retinoids; Solimastat; Squalamine; SS 3304; SU 5416; SU6668; SU11248; Tetrahydrocortisol-S; tetrathiomolybdate; thalidomide; Thrombospondin-1 (TSP-1); TNP-470; Transforming growth factor-beta (TGF-b); Vasculostatin; Vasostatin (calreticulin fragment); ZD6126; ZD 6474; farnesyl transferase inhibitors (FTI); and bisphosphonates (such as but are not limited to, alendronate, clodronate, etidronate, ibandronate, pamidronate, risedronate, tiludronate, and zoledronate).

In a specific embodiment, methods of the disclosure encompass the administration of one or more immunomodulatory agents, such as but not limited to, chemotherapeutic agents and non-chemotherapeutic immunomodulatory agents. Non-limiting examples of chemotherapeutic agents include methotrexate, cyclosporin A, leflunomide, cisplatin, ifosfamide, taxanes such as taxol and paclitaxol, topoisomerase I inhibitors (e.g., CPT-11, topotecan, 9-AC, and GG-211), gemcitabine, vinorelbine, oxaliplatin, 5-fluorouracil (5-FU), leucovorin, vinorelbine, temodal, cytochalasin B, gramicidin D, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin homologues, and cytoxan. Examples of non-chemotherapeutic immunomodulatory agents include, but are not limited to, anti-T cell receptor antibodies (*e.g.,* anti-CD4 antibodies (*e.g.,* cM-T412 (Boeringer), IDEC-CE9.1® (IDEC and SKB), mAB 4162W94, Orthoclone and OKTcdr4a (Janssen-Cilag)), anti-CD3 antibodies (*e.g.,* Nuvion (Product Design Labs), OKT3 (Johnson & Johnson), or Rituxan (IDEC)), anti-CD5 antibodies (*e.g.,* an anti-CD5 ricin-linked immunoconjugate), anti-CD7 antibodies (*e.g.,* CHH-380 (Novartis)), anti-CD8 antibodies, anti-CD40 ligand monoclonal antibodies (*e.g.,* IDEC-131 (IDEC)), anti-CD52 antibodies (*e.g.,* CAMPATH 1H (Ilex)), anti-CD2 antibodies (*e.g.,* MEDI-507 (MedImmune, Inc., International Publication Nos. WO 02/098370 and WO 02/069904), anti-CD11a antibodies (*e.g.,* Xanelim (Genentech)), and anti-B7 antibodies *(e.g.,* IDEC-114) (IDEC)); anti-cytokine receptor antibodies *(e.g.,* anti-IFN receptor antibodies, anti-IL-2 receptor antibodies (*e.g.,* Zenapax (Protein Design Labs)), anti-IL-4 receptor antibodies, anti-IL-6 receptor antibodies, anti-IL-10 receptor antibodies, and anti-IL-12 receptor antibodies), anti-cytokine antibodies (*e.g.,* anti-IFN antibodies, anti-TNF-a antibodies, anti-IL-1$\beta$ antibodies, anti-IL-6 antibodies, anti-IL-8 antibodies (*e.g.,* ABX-IL-8 (Abgenix), anti-IL-12 antibodies and anti-IL-23 antibodies)); CTLA4-immunoglobulin; LFA-3TIP (Biogen, International Publication No. WO 93/08656 and U.S. Patent No. 6,162,432); soluble cytokine receptors (*e.g.,* the extracellular domain of a TNF-$\alpha$ receptor or a fragment thereof, the extracellular domain of an IL-1$\beta$ receptor or a fragment thereof, and the extracellular domain of an IL-6 receptor or a fragment thereof); cytokines or fragments thereof (*e.g.,* interleukin (IL)-2, IL-3, IL-4, IL-5, IL-6, IL-7, IL-8, IL-9, IL-10, IL-11, IL-12, IL-15, IL-23, TNF-$\alpha$, TNF-$\beta$, interferon (IFN)-$\alpha$, IFN-$\beta$, IFN-$\gamma$, and GM-CSF); and anti-cytokine antibodies (e.g., anti-IL-2 antibodies, anti-IL-4 antibodies, anti-IL-6 antibodies, anti-IL-10 antibodies, anti-IL-12 antibodies, anti-IL-15 antibodies, anti-TNF-a antibodies, and anti-IFN-y antibodies), and antibodies that immunospecifically bind to tumor-associated antigens (*e.g.,* Herceptin®). In certain embodiments, an immunomodulatory agent is an immunomodulatory agent other than a chemotherapeutic agent. In other embodiments an immunomodulatory agent is an immunomodulatory agent other than a cytokine or hemapoietic such as IL-1, IL-2, IL-4, IL-12, IL-15, TNF, IFN-$\alpha$, IFN-$\beta$, IFN-$\gamma$, M-CSF, G-CSF, IL-3 or erythropoietin. In yet other embodiments, an immunomodulatory agent is an agent other than a chemotherapeutic agent and a cytokine or hemapoietic factor.

In a specific embodiment, methods of the disclosure encompass the administration of one or more anti-inflammatory agents, such as but not limited to, non-steroidal anti-inflammatory drugs (NSAIDs), steroidal anti-inflammatory drugs, beta-agonists, anticholinergic agents, and methyl xanthines. Examples of NSAIDs include, but are not limited to, aspirin, ibuprofen, celecoxib (CELEBREX™), diclofenac (VOLTAREN™), etodolac (LODINE™), fenoprofen (NALFON™), indomethacin (INDOCIN™), ketoralac (TORADOL™), oxaprozin (DAYPRO™), nabumentone (RELAFEN™), sulindac (CLINORIL™), tolmentin (TOLECTIN™), rofecoxib (VIOXX™), naproxen (ALEVE™, NAPROSYN™), ketoprofen (ACTRON™) and nabumetone (RELAFEN™). Such NSAIDs function by inhibiting a cyclooxygenase enzyme (*e.g.,* COX-1 and/or COX-2). Examples of steroidal anti-inflammatory drugs include, but are not limited to, glucocorticoids, dexamethasone (DECADRON™), cortisone, hydrocortisone, prednisone (DELTASONE™), prednisolone, triamcinolone, azulfidine, and eicosanoids such as prostaglandins, thromboxanes, and leukotrienes.

In another specific embodiment, methods of the disclosure encompass the administration of one or more antiviral agents (*e.g.,* amantadine, ribavirin, rimantadine, acyclovir, famciclovir, foscarnet, ganciclovir, trifluridine, vidarabine, didanosine, stavudine, zalcitabine, zidovudine, interferon), antibiotics (*e.g.,* dactinomycin (formerly actinomycin), bleomycin, mithramycin, and anthramycin (AMC)), anti-emetics (*e.g.,* alprazolam, dexamethoasone, domperidone, dronabinol, droperidol, granisetron, haloperidol, haloperidol, iorazepam, methylprednisolone, metoclopramide, nabilone, ondansetron, prochlorperazine), antifungal agents (*e.g.,* amphotericin, clotrimazole, econazole, fluconazole, flucytosine, griseofulvin, itraconazole, ketoconazole, miconazole and nystatin), anti-parasite agents (*e.g.,* dehydroemetine, diloxanide furoate, emetine, mefloquine, melarsoprol, metronidazole, nifurtimox, paromomycin, pentabidine, pentamidine isethionate, primaquine, quinacrine, quinidine) or a combination thereof.

Specific examples of anti-cancer agents that can be used in various embodiments of the disclosure, including pharmaceutical compositions and dosage forms and kits, include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alpha-2a; interferon alpha-2b; interferon alpha-n1 ; interferon alpha-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin;

plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride. Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; HMG-CoA reductase inhibitor (such as but not limited to, Lovastatin, Pravastatin, Fluvastatin, Statin, Simvastatin, and Atorvastatin); loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; Vitaxin®; vorozole; zanoterone; zeniplatin; zilascorb; and zinostatin stimalamer. Additional anti-cancer drugs are 5-fluorouracil and leucovorin. These two agents may be useful when used in methods employing thalidomide and a topoisomerase inhibitor. In specific embodiments, an anti-cancer agent is not a chemotherapeutic agent.

In more particular embodiments, the present disclosure also comprises the administration of an anti-ICOS antibody formulation in combination with the administration of one or more therapies such as, but not limited to, anti-cancer agents such as those disclosed in Table 1, for the treatment of breast, ovary, melanoma, prostate, colon and lung cancers as described above. When used in a combination therapy, the dosages and/or the frequency of administration listed in Table 1 may be decreased.

**Table 1. Anti-cancer agents**

| Therapeutic Agent | Dose/Administration/Formulation | | |
|---|---|---|---|
| doxorubicin hydrochloride (Adriamycin RDF® and Adriamycin PFS® | Intravenous | 2 60-75 mg/m$^2$ on Day 1 | 21 day intervals |
| epirubicin hydrochloride (Ellence™) | Intravenous | 100-120 mg/m$^2$ on Day 1 of each cycle or divided equally and given on Days 1-8 of the cycle | 3-4 week cycles |
| fluorousacil | Intravenous | How supplied: 5 mL and 10 mL vials (containing 250 and 500 mg flourouracil respectively) | |
| docetaxel (Taxotere®) | Intravenous | 60- 100 mg/m$^2$ over 1 hour | Once every 3 weeks |
| paclitaxel (Taxol®) | Intravenous | 175 mg/m$^2$ over 3 hours | Every 3 weeks for 4 courses (administered sequentially to doxorubicin-containing combination chemotherapy) |
| tamoxifen citrate (Nolvadex®) | Oral (tablet) | 20-40 mg Dosages greater than 20 mg should be given in divided doses (morning and evening) | Daily |
| leucovorin calcium for injection | intravenous or intramuscular injection | How supplied: 350 mg vial | Dosage is unclear from text. PDR 3610 |
| luprolide acetate Lupron®) | single subcutaneous injection | 1 mg (0.2 mL or 20 unit mark) | Once a day |

(continued)

| Therapeutic Agent | Dose/Administration/Formulation | | |
|---|---|---|---|
| flutamide (Eulexin®) | Oral (capsule) | 50 mg (capsules contain 125 mg flutamide each) | 3 times a day at 8 hour intervals (total daily dosage 750 mg) |
| nilutamide (Nilandron®) | Oral (tablet) | 300 mg or 150 mg (tablets contain 50 or 150 mg nilutamide each) | 300 mg once a day for 30 days followed by 150 mg once a day |
| bicalutamide (Casodex®) | Oral (tablet) | 50 mg (tablets contain 50 mg bicalutamide each) | Once a day |
| progesterone | Injection | USP in sesame oil 50 mg/mL | |
| ketoconazole (Nizoral®) | Cream | 2% cream applied once or twice daily depending on symptoms | |
| prednisone | Oral (tablet) | Initial dosage may vary from 5 mg to 60 mg per day depending on the specific disease entity being treated. | |
| estramustine phosphate sodium (Emcyt®) | Oral (capsule) | 14 mg/kg of body weight (i.e. one 140 mg capsule for each 10 kg or 22 lb of body weight) | Daily given in 3 or 4 divided doses |
| etoposide or VP-16 | Intravenous | 5 mL of 20 mg/mL solution (100 mg) | |
| dacarbazine (DTIC-Dome®) | Intravenous | 2-4.5 mg/kg | Once a day for 10 days. May be repeated at 4 week intervals |
| polifeprosan 20 with carmustine implant (BCNU) (nitrosourea) (Gliadel®) | wafer placed in resection cavity | 8 wafers, each containing 7.7 mg of carmustine, for a total of 61.6 mg, if size and shape of resection cavity allows | |
| cisplatin | Injection | [n/a in PDR 861] How supplied: solution of 1 mg/mL in multi-dose vials of 50mL and 100mL | |
| mitomycin | Injection | supplied in 5 mg and 20 mg vials (containing 5 mg and 20 mg mitomycin) | |
| gemcitabine HCI (Gemzar®) | Intravenous | For NSCLC- 2 schedules have been investigated and the optimum schedule has not been determined 4 week schedule-administration intravenously at 1000 mg/m$^2$ over 30 minutes on 3 week schedule-Gemzar administered intravenously at 1250 mg/m$^2$ over 30 minutes | 4 week schedule-Days 1, 8 and 15 of each 28-day cycle. Cisplatin intravenously at 100 mg/m$^2$ on day 1 after the infusion of Gemzar. 3 week scheduleDays 1 and 8 of each 21 day cycle. Cisplatin at dosage of 100 mg/m$^2$ administered intravenously after administration of Gemzar on day 1. |
| carboplatin (Paraplatin®) | Intravenous | Single agent therapy: 360 mg/m$^2$ I.V. on day 1 (infusion lasting 15 minutes or longer) Other dosage calculations: Combination therapy with cyclophosphamide, Dose adjustment recommendations, Formula dosing, etc. | Every 4 weeks |

(continued)

| Therapeutic Agent | Dose/Administration/Formulation | | |
|---|---|---|---|
| ifosamide (Ifex®) | Intravenous | 1.2 g/m$^2$ daily | 5 consecutive days Repeat every 3 weeks or after recovery from hematologic toxicity |
| topotecan hydrochloride (Hycamtin®) | Intravenous | 1.5 mg/m by intravenous infusion over 30 minutes daily | 5 consecutive days, starting on day 1 of 21 day course |
| Bisphosphonates Pamidronate Alendronate Risedronate | Intravenous or Oral take with 6-8 oz water. | 60 mg or 90 mg single infusion over 4 - 24 hours to correct hypercalcemia in cancer patients 5 mg/d daily for 2 years and then 10 mg/d for 9 month to prevent or control bone resorption. 5.0 mg to prevent or control bone resorption. | |
| Lovastatin (Mevacor™) | Oral | 10 - 80 mg/day in single or two divided dose. | |

The disclosure also encompasses administration of an anti-ICOS antibody formulation of the disclosure in combination with radiation therapy comprising the use of x-rays, gamma rays and other sources of radiation to destroy the cancer cells. In particular embodiments, the radiation treatment is administered as external beam radiation or teletherapy wherein the radiation is directed from a remote source. In other embodiments, the radiation treatment is administered as internal therapy or brachytherapy wherein a radiaoactive source is placed inside the body close to cancer cells or a tumor mass.

Cancer therapies and their dosages, routes of administration and recommended usage are known in the art and have been described in such literature as the Physician's Desk Reference (56th ed., 2002).

**5.24.** Antibodies Having Increased Half-Lives

The present disclosure provides for formulations of antibodies and antibody fragments that specifically bind to an antigen of interest (*e.g.,* ICOS) which have an extended half-life *in vivo.* In particular, the present disclosure provides formulations of antibodies and antibody fragments that specifically bind to an antigen of interest (*e.g.,* ICOS) which have a half-life in a mammal (for example, but not limited to, a human), of greater than 3 days, greater than 7 days, greater than 10 days, greater than 15 days, greater than 25 days, greater than 30 days, greater than 35 days, greater than 40 days, greater than 45 days, greater than 2 months, greater than 3 months, greater than 4 months, or greater than 5 months.

To prolong the serum circulation of antibodies (for example, but not limited to, monoclonal antibodies and single chain antibodies) or antibody fragments (for example, but not limited to, Fab fragments) *in vivo,* for example, inert polymer molecules such as high molecular weight polyethyleneglycol (PEG) can be attached to the antibodies (including antibody fragments thereof) with or without a multifunctional linker either through site-specific conjugation of the PEG to the N- or C-terminus of the antibodies or via epsilon-amino groups present on lysine residues. Linear or branched polymer derivatization that results in minimal loss of biological activity will be used. The degree of conjugation can be closely monitored by SDS-PAGE and mass spectrometry to ensure proper conjugation of PEG molecules to the antibodies. Unreacted PEG can be separated from antibody-PEG conjugates by size-exclusion or by ion-exchange chromatography. PEG-derivatized antibodies (including antibody fragments thereof) can be tested for binding activity as well as for *in vivo* efficacy using methods known to those of skill in the art, for example, by immunoassays described herein.

Antibodies having an increased half-life *in vivo* can also be generated introducing one or more amino acid modifications (i.e., substitutions, insertions or deletions) into an IgG constant domain, or FcRn binding fragment thereof *(e.g.,* Fc or hinge-Fc domain fragment). See, e.g., International Publication No. WO 98/23289; International Publication No. WO 97/34631; and U.S. Patent No. 6,277,375.

Further, antibodies (including antibody fragments thereof) can be conjugated to albumin in order to make the antibody (including antibody fragment thereof) more stable *in vivo* or have a longer half life *in vivo.* The techniques are well known in the art, see *e.g.,* International Publication Nos. WO 93/15199, WO 93/15200, and WO 01/77137; and European Patent No. EP 413, 622.

**5.25.** Methods of Preparing the Antibody Formulations

The present disclosure provides methods for preparing liquid formulations of antibodies or derivatives, analogues, or fragments thereof that specifically bind to an antigen of interest (e.g., human ICOS polypeptide). The methods for preparing liquid formulations of the present disclosure may comprise: purifying the antibody (including antibody fragment thereof) from conditioned medium (either single lots or pooled lots of medium) and concentrating a fraction of the purified antibody (including antibody fragment thereof) to a final concentration of about 15 mg/ml, about 20 mg/ml, about 30 mg/ml, about 40 mg/ml, about 50 mg/ml, about 60 mg/ml, about 70 mg/ml, about 80 mg/ml, about 90 mg/ml, about 100 mg/ml, about 150 mg/ml, about 175 mg/ml, about 200 mg/ml, about 250 mg/ml, or about 300 mg/ml. Conditioned medium containing the antibody (including antibody fragment thereof), for example, an antibody that specifically binds to ICOS may be subjected to CUNO filtration and the filtered antibody is subjected to HS50 cation exchange chromatography. The fraction from the HS50 cation exchange chromatography is then subjected to low pH treatment followed by MEP Hypercel chromatography. The fraction from the MEP Hypercel chromatography is subject to nanofiltration. The purified antibody or a fragment thereof obtained after nanofiltration is then subjected to diafiltration and ultrafiltration to buffer exchange and concentrate into the formulation buffer using the same membrane.

The liquid formulations of the present disclosure can be prepared as unit dosage forms by preparing a vial containing an aliquot of the liquid formulation for a one-time use. For example, a unit dosage per vial may contain 1 ml, 2 ml, 3 ml, 4 ml, 5 ml, 6 ml, 7 ml, 8 ml, 9 ml, 10 ml, 15 ml, or 20 ml of different concentrations of an antibody (including antibody fragment thereof) that specifically binds to ICOS ranging from about 10 mg/ml to about 300 mg/ml. If necessary, these preparations can be adjusted to a desired concentration by adding a sterile diluent to each vial. In a specific embodiment, the liquid formulations of the present disclosure are formulated into single dose vials as a sterile liquid that contains 10 mM histidine buffer at pH 6.0, 80 mM NaCl, 4% trehalose and 0.02% polysorbate 80. Each 1.0 mL of solution contains 100 mg of the antibody (including antibody fragment thereof). In one embodiment, the antibody (including antibody fragment thereof) of the disclosure is supplied at 100 mg/ml in 3 cc USP Type I borosilicate amber vials (West Pharmaceutical Services - Part No. 6800-0675). The target fill volume is 1.2 mL.

The liquid formulations of the present disclosure can be prepared as unit dosage forms by preparing a pre-filled syringe containing an aliquot of the liquid formulation for a one-time use. For example, a unit dosage per pre-filled syringe may contain 0.1ml, 0.2 ml, 0.3 ml, 0.4 ml, 0.5 ml, 0.6 ml, 0.7 ml, 0.8 ml, 0.9 ml, 1 ml, 2 ml, 3 ml, 4 ml, 5 ml, 6 ml, 7 ml, 8 ml, 9 ml, 10 ml, 15 ml, or 20 ml of different concentrations of an antibody (including antibody fragment thereof) that specifically binds to ICOS ranging from about 10 mg/ml to about 300 mg/ml. In a specific embodiment, the liquid formulations of the present disclosure are formulated into single dose pre-filled syringes as a sterile liquid that contains 10 mM histidine buffer at pH 6.0, 80 mM NaCl, 4% trehalose and 0.02% polysorbate 80. Each 1.0 mL of solution contains 100 mg of the antibody (including antibody fragment thereof).

The liquid formulations of the present disclosure may be sterilized by various sterilization methods, including sterile filtration, radiation, etc. In a specific embodiment, the diafiltrated antibody formulation is filter-sterilized with a presterilized 0.2 micron filter. Sterilized liquid formulations of the present disclosure may be administered to a subject to prevent, treat and/or manage a disease or disorder associated with or characterized by aberrant expression and/or activity of ICOS, a disease or disorder associated with or characterized by aberrant expression and/or activity of ICOS receptor, an autoimmune disease or disorder, an inflammatory disease or disorder, a T cell proliferative disease or disorder, a malignancy, a T cell malignancy, transplant rejection, graft versus host disease, or one or more symptoms thereof.

Although the disclosure is directed to liquid non-lyophilized formulations, it should be noted for the purpose of equivalents that the formulations of the disclosure may be lyophilized if desired. Thus, the disclosure encompasses lyophilized forms of the formulations of the disclosure.

**5.26.** Methods of Monitoring the Stability and Aggregation of Antibody Formulations

There are various methods available for assessing the stability of protein formulations, including antibody formulations, based on the physical and chemical structures of the proteins as well as on their biological activities. For example, to study denaturation of proteins, methods such as charge-transfer absorption, thermal analysis, fluorescence spectroscopy, circular dichroism (CD), NMR, reducing capillary gel electrophoresis (rCGE) and high performance size exclusion chromatography (HPSEC), tangential flow filtration (TFF), static light scattering (SLS), Fourier Transform Infrared Spectroscopy (FTIR), urea-induced protein unfolding techniques, intrinsic tryptophan fluorescence, differential scanning calorimetry, and 1-anilino-8-naphthalenesulfonic acid (ANS) protein binding techniques are available. See, for example, Wang et al., 1988, J. of Parenteral Science & Technology 42(Suppl):S4-S26.

rCGE and HPSEC are the most common and simplest methods to assess the formation of protein aggregates, protein degradation, and protein fragmentation. Accordingly, the stability of the liquid formulations of the present disclosure may be assessed by these methods.

For example, the stability of the liquid formulations of the present disclosure may be evaluated by HPSEC, wherein the percent area of the peaks represents the non-degraded antibody or non-degraded antibody fragments. In particular, approximately 250 $\mu$g of the antibody (including antibody fragment thereof) (approximately 25 $\mu$l of a liquid formulation comprising 10 mg/ml said antibody or antibody fragment) is injected onto a TosoH Biosep TSK G3000SW$_{XL}$ column (7.8 mm x 30 cm) fitted with a TSK SW x1 guard column (6.0 mm CX 4.0 cm). The antibody (including antibody fragment thereof) is eluted isocratically with 0.1 M disodium phosphate containing 0.1 M sodium sulfate and 0.05% sodium azide, at a flow rate of 0.8 to 1.0 ml/min. Eluted protein is detected using UV absorbance at 280 nm. Reference standards are run in the assay as controls, and the results are reported as the area percent of the product monomer peak compared to all other peaks excluding the included volume peak observed at approximately 12 to 14 minutes. Peaks eluting earlier than the monomer peak are recorded as percent aggregate.

The liquid formulations of the present disclosure exhibit low to undetectable levels of aggregation as measured by any of the methods described above, that is, no more than 5%, no more than 4%, no more than 3%, no more than 2%, no more than 1%, and no more than 0.5% aggregate by weight protein, and low to undetectable levels of fragmentation, that is, 80% or higher, 85% or higher, 90% or higher, 95% or higher, 98% or higher, or 99% or higher, or 99.5% or higher of the total peak area in the peak(s) representing intact antibodies (including antibody fragments thereof). When SDS-PAGE is used to measure antibody fragmentation, the density or the radioactivity of each band stained or labeled with radioisotope can be measured and the % density or % radioactivity of the band representing non-degraded antibodies (including antibody fragments thereof) can be obtained.

The stability of the liquid formulations of the present disclosure can be also assessed by any assays which measure the biological activity of the antibody in the formulation. The biological activities of antibodies include, but are not limited to, antigen-binding activity, blocking of ligand-receptor interaction, and so forth (see *infra*). Antigen-binding activity of the antibodies (including antibody fragments thereof) can be measured by any method known to those skilled in the art, including but not limited to ELISA, radioimmunoassay, Western blot, and the like. Also see Harlow et al., Antibodies: A Laboratory Manual, (Cold Spring Harbor Laboratory Press, 2nd ed. 1988). An ELISA based assay, *e.g.,* may be used to compare the ability of an antibody (including antibody fragments thereof) to specifically bind to an ICOS polypeptide to that of a reference standards antibody.

The purity of the liquid antibody formulations of the disclosure may be measured by any method well-known to one of skill in the art such as, for example, but not limited to, HPSEC. The sterility of the liquid antibody formulations may be assessed by any method well-known to one of skill in the art such as, e.g.: sterile soybean-casein digest medium and fluid thioglycollate medium are inoculated with a test liquid antibody formulation by filtering the liquid antibody formulation through a sterile filter having a nominal porosity of 0.45 $\mu$m. When using the Sterisure™ or Steritest™ method, each filter device is aseptically filled with approximately 100 ml of sterile soybean-casein digest medium or fluid thioglycollate medium. When using the conventional method, the challenged filter is aseptically transferred to 100 ml of sterile soybean-casein digest medium or fluid thioglycollate medium. The media are incubated at appropriate temperatures and observed three times over a 14 day period for evidence of bacterial or fungal growth.

**5.27.** Methods of Administering the Antibody Formulations

The disclosure provides methods of prevention, treatment and/or management of a disorder, for example, a disease or disorder associated with or characterized by aberrant expression and/or activity of ICOS, a disease or disorder associated with or characterized by aberrant expression and/or activity of ICOS receptor, an autoimmune disease or disorder, an inflammatory disease or disorder, a T cell proliferative disease or disorder, a malignancy, a T cell malignancy, transplant rejection, graft versus host disease, or one or more symptoms thereof by administrating to a subject of an effective amount of liquid formulations of the disclosure. Various delivery systems are known and can be used to administer a liquid formulation of the present disclosure or a prophylactic or therapeutic agent. Methods of administering antibody liquid formulations of the present disclosure or a therapy *(e.g.,* a prophylactic or therapeutic agent) include, but are not limited to, parenteral administration (e.g., intradermal, intramuscular, intraperitoneal, intravenous and, and subcutaneous), epidural administration, topical administration, and mucosal administration (for example, but not limited to, intranasal and oral routes). In a specific embodiment, liquid formulations of the present disclosure are administered intramuscularly, intravenously, or subcutaneously. In one embodiment, the liquid formulations of the disclosure are administered subcutaneously. The formulations may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local.

The disclosure also provides that a liquid formulation of the present disclosure is packaged in a hermetically sealed container such as an ampoule or sachette indicating the quantity of antibody (including antibody fragment thereof). In one embodiment, a liquid formulation of the present disclosure is in a hermetically sealed container indicating the quantity and concentration of the antibody (including antibody fragment thereof). In one embodiment, a liquid formulation of the present disclosure is supplied in a hermetically sealed container and comprises about 10 mg/ml, about 15 mg/ml, about 20 mg/ml, about 30 mg/ml, about 40 mg/ml, about 50 mg/ml, about 60 mg/ml, about 70 mg/ml, about 80 mg/ml, about 90 mg/ml, about 100 mg/ml, about 110 mg/ml, about 120 mg/ml, about 130 mg/ml, about 150 mg/ml, about 175 mg/ml, about 200 mg/ml, about 250 mg/ml, or about 300 mg/ml of an antibody (including antibody fragment thereof) that specifically binds to human ICOS, in a quantity of about 1 ml, about 2 ml, about 3 ml, about 4 ml, about 5 ml, 6 about ml, about 7 ml, about 8 ml, about 9 ml, about 10 ml, about 15 ml, or about 20 ml. In a specific embodiment of the disclosure, a liquid formulation of the disclosure is supplied in a hermetically sealed container and comprises at least about 15 mg/ml, at least about 20 mg/ml, at least about 25 mg/ml, at least about 50 mg/ml, at least about 100 mg/ml, at least about 110 mg/ml, at least about 120 mg/ml, at least about 130 mg/ml, at least about 150 mg/ml, at least about 175 mg/ml, at least about 200 mg/ml, at least about 250 mg/ml or at least about 300 mg/ml of an antibody (including antibody fragment thereof) that specifically binds to human ICOS (for example, but not limited to, or an antigen-binding fragment thereof) for intravenous injections, and at least about 15 mg/ml, at least about 20 mg/ml, at least about 50 mg/ml, at least about 80 mg/ml, at least about 100 mg/ml, at least about 110 mg/ml, at least about 120 mg/ml, at least about 130 mg/ml, at least about 150 mg/ml, at least about 175 mg/ml, at least about 200 mg/ml, at least about 250 mg/ml or at least about 300 mg/ml of an antibody (including antibody fragment thereof) that specifically binds to human ICOS (for example, but not limited to, or a fragment thereof) for repeated subcutaneous administration.

The amount of a liquid formulation of the present disclosure which will be effective in the prevention, treatment and/or management of a disease or disorder associated with or characterized by aberrant expression and/or activity of ICOS, a disease or disorder associated with or characterized by aberrant expression and/or activity of ICOS receptor, an autoimmune disease or disorder, an inflammatory disease or disorder, a T cell proliferative disease or disorder, a malignancy, a T cell malignancy, transplant rejection, graft versus host disease, or one or more symptoms thereof can be determined by standard clinical techniques well-known in the art or described herein. The precise dose to be employed in the formulation will also depend on the route of administration, and the seriousness of the inflammatory disorder, or autoimmune disorder, and should be decided according to the judgment of the practitioner and each patient's circumstances. Effective doses may be extrapolated from dose-response curves derived from *in vitro* or animal model test systems.

## 5.28. PHARMACEUTICAL FORMULATIONS

The disclosure also relates to immunotherapeutic formulations and methods for the treatment of T cell-mediated diseases and disorders in human subjects, such as, but not limited to, chronic infection, autoimmune disease or disorder, inflammatory disease or disorder, graft-versus-host disease (GVHD), transplant rejection, and T cell proliferative disorder in human subjects, using therapeutic antibodies that bind to the ICOS antigen and mediate human ADCC.

The present disclosure relates to pharmaceutical formulations comprising effector function enhanced anti-ICOS antibodies of the IgG1 or IgG3 human isotype. The present disclosure also relates to pharmaceutical formulations comprising human or humanized anti-ICOS antibodies of the IgG2 or IgG4 human isotype that mediate human ADCC. In certain embodiments, the present disclosure also relates to pharmaceutical formulations comprising monoclonal anti-ICOS antibodies with enhanced effector.

Therapeutic formulations and regimens are described for treating human subjects diagnosed with autoimmune diseases, such as, but not limited to, systemic lupus erythematosus, rheumatoid arthritis, immune thrombocytopenic purpura (ITP), diabetes, psoriasis, and hypersensitivity reactions (e.g., allergies, hay fever, asthma, and acute edema cause type I hypersensitivity reactions). The present disclosure also relates to formulations and regimens for the treatment of human subjects diagnosed with chronic inflammatory diseases, such as, but not limited to, inflammatory bowel disease (Crohn's disease and ulcerative colitis), Grave's disease, Hashimoto's thyroiditis, and diabetes mellitus.

Therapeutic formulations and regimens are described for treating human subjects diagnosed with T cell malignancies that derive from ICOS expressing T cells and their precursors.

In particular embodiments, a formulation of the disclosure comprises an anti-ICOS antibody that may mediate ADCC, complement-dependent cellular cytotoxicity, or antibody-dependent phagocytosis. Formulations and methods of the present disclosure also have the advantage of targeting a narrower population of T cells than other T cell directed immunotherapies. For example, formulations of the present disclosure may be effective to specifically target activated T cells, for example, but not limited to, activated T cells. Accordingly, methods and formulations of the disclosure may be effective to reduce or deplete circulating activated CD4+ T cells as well as activated CD8+ T cells.

Accordingly, in one aspect, the disclosure provides anti-ICOS antibody formulations for the treatment and prevention of GVHD and graft rejection, which are associated with fewer and/or less severe complications than less-targeted therapeutic agents and regimens. In one embodiment, formulations and methods of the disclosure are used with lower doses of traditional therapeutic agents than would be possible in the absence of the methods and formulations of the disclosure. In another embodiment, formulations and methods of the disclosure obviate the need for a more severe form of therapy, such as radiation therapy, high-dose chemotherapy, or splenectomy.

In certain embodiments, anti-ICOS antibody formulations may be administered to a transplant recipient patient prior to or following transplantation, alone or in combination with other therapeutic agents or regimens for the treatment or prevention of GVHD and graft rejection. For example, anti-ICOS antibody formulations may be used to deplete activated T cells from a transplant recipient prior to or following transplantation of an allogeneic graft. Anti-ICOS antibody formulations may also be used to deplete activated T cells from the graft *ex vivo,* prior to transplantation, or in the donor, or as prophylaxis against GVHD and graft rejection.

## 5.29. PHARMACEUTICAL FORMULATIONS, ADMINISTRATION AND DOSING

Pharmaceutical formulations of the disclosure contain as the active ingredient anti-ICOS antibodies with enhanced effector function. The formulations contain naked antibody, immunoconjugate, or fusion protein in an amount effective for producing the desired response in a unit of weight or volume suitable for administration to a human patient, and are preferably sterile. The response can, for example, be measured by determining the physiological effects of the anti-ICOS antibody formulation, such as, but not limited to, T cell depletion, IL-17 depletion, regression of a T cell malignancy, or decrease of disease symptoms. Other assays will be known to one of ordinary skill in the art and can be employed for measuring the level of the response (for example, but not limited to SLEDAI, BILAG, PRO). Additional assays that may be used to monitor response include, but are not limited to, immunohistochemistry of tissue biopsy (e.g., skin biopsy), ICOS mRNA expression in tissue sample (e.g., skin biopsy, tonsil biopsy, blood), flow cytometry of blood cells, microarray analysis of tissue sample (e.g., skin biopsy, blood), proteomics analysis of tissue sample (e.g., skin biopsy, blood), antibody array analysis, SNP analysis.

### 5.29.1. ADMINISTRATION AND DOSING

Administration of formulations of the disclosure to a human patient can be by any route, including but not limited to intravenous, intradermal, transdermal, subcutaneous, intramuscular, inhalation (*e.g., via* an aerosol), buccal (*e.g.,* sub-lingual), topical *(i.e.,* both skin and mucosal surfaces, including airway surfaces), intrathecal, intraarticular, intraplural, intracerebral, intra-arterial, intraperitoneal, oral, intralymphatic, intranasal, rectal or vaginal administration, by perfusion through a regional catheter, or by direct intralesional injection. In one embodiment, formulations of the disclosure are administered by intravenous push or intravenous infusion given over defined period *(e.g.,* 0.5 to 2 hours). Formulations of the disclosure can be delivered by peristaltic means or in the form of a depot, although the most suitable route in any given case will depend, as is well known in the art, on such factors as the species, age, gender and overall condition of the subject, the nature and severity of the condition being treated and/or on the nature of the particular formulation *(i.e.,* dosage, formulation) that is being administered. In particular embodiments, the route of administration is *via* bolus or continuous infusion over a period of time, once or twice a week. In other particular embodiments, the route of administration is by subcutaneous injection, optionally once, twice, three times or four times monthly. In one embodiment, formulations, and/or methods of the disclosure are administered on an outpatient basis.

In certain embodiments, the dose of a formulation comprising anti-ICOS antibody is measured in units of mg/kg of patient body weight. In other embodiments, the dose of a formulation comprising anti-ICOS antibody is measured in units of mg/kg of patient lean body weight *(i.e.,* body weight minus body fat content). In yet other embodiments, the dose of a formulation comprising anti-ICOS antibody is measured in units of $mg/m^2$ of patient body surface area. In yet other embodiments, the dose of a formulation comprising anti-ICOS antibody is measured in units of mg per dose administered to a patient. Any measurement of dose can be used in conjunction with formulations and methods of the disclosure and dosage units can be converted by means standard in the art.

Those skilled in the art will appreciate that dosages can be selected based on a number of factors including the age, sex, species and condition of the subject *(e.g.,* stage of disease), the desired degree of cellular depletion, the disease to be treated and/or the particular antibody or antigen-binding fragment being used and can be determined by one of skill in the art. For example, effective amounts of formulations of the disclosure may be extrapolated from dose-response curves derived *in vitro* test systems or from animal model *(e.g.,* the cotton rat or monkey) test systems. Models and methods for evaluation of the effects of antibodies are known in the art (Wooldridge et al., Blood, 89(8): 2994-2998 (1997))). In certain embodiments, for particular ICOS expressing T cell malignancies, therapeutic regimens standard in the art for antibody therapy can be used with formulations and methods of the disclosure.

Examples of dosing regimens that can be used in methods of the disclosure include, but are not limited to, daily, three times weekly (intermittent), weekly, bi-weekly, monthly, bi-monthly, or quarterly (once every three month). In certain embodiments, dosing regimens include, but are not limited to, monthly dosing or dosing every 6-8 weeks.

Those skilled in the art will appreciate that dosages are generally higher and/or frequency of administration greater for initial treatment as compared with maintenance regimens.

In some embodiments of the disclosure, anti-ICOS antibodies bind to ICOS expressing T cells and may result in efficient *(i.e.,* at low dosage) depletion of ICOS expressing T cells (as described herein). In certain embodiments, dosages of the antibody (optionally in a pharmaceutically acceptable carrier as part of a pharmaceutical formulation) are at least about 0.0005, 0.001, 0.05, 0.075, 0.1, 0.25, 0.375, 0.5, 1, 2.5, 5, 10, 20, 37.5, or 50 mg/m$^2$ and/or less than about 500, 475, 450, 425, 400, 375, 350, 325, 300, 275, 250, 225, 200, 175, 150, 125, 100, 75, 60, 50, 37.5, 20, 15, 10, 5, 2.5, 1, 0.5, 0.375, 0.1, 0.075 or 0.01 mg/m$^2$. In certain embodiments, the dosage is between about 0.0005 to about 200 mg/m$^2$, between about 0.001 and 150 mg/m$^2$, between about 0.075 and 125 mg/m$^2$, between about 0.375 and 100 mg/m$^2$, between about 2.5 and 75 mg/m$^2$, between about 10 and 75 mg/m$^2$, and between about 20 and 50 mg/m$^2$. In related embodiments, the dosage of anti-ICOS antibody used is at least about 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5 mg/kg of body weight of a patient. In certain embodiments, the dose of naked anti-ICOS antibody used is at least about 1 to 10, 5 to 15, 10 to 20, or 15 to 25 mg/kg of body weight of a patient. In certain embodiments, the dose of anti-ICOS antibody used is at least about 1 to 20, 3 to 15, or 5 to 10 mg/kg of body weight of a patient. In other embodiments, the dose of anti-ICOS antibody used is at least about 5, 6, 7, 8, 9, or 10 mg/kg of body weight of a patient. In certain embodiments, a single dosage unit of the antibody (optionally in a pharmaceutically acceptable carrier as part of a pharmaceutical formulation) can be at least about 0.5, 1, 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 204, 206, 208, 210, 212, 214, 216, 218, 220, 222, 224, 226, 228, 230, 232, 234, 236, 238, 240, 242, 244, 246, 248, or 250 micrograms/m$^2$. In other embodiments, dose is up to 100 mg per single dosage unit.

In some embodiments of methods of this disclosure, antibodies and/or formulations of this disclosure can be administered at a dose lower than about 375 mg/m$^2$; at a dose lower than about 37.5 mg/m$^2$; at a dose lower than about 0.375 mg/m$^2$; and/or at a dose between about 0.075 mg/m$^2$ and about 125 mg/m$^2$. In certain embodiments of methods of the disclosure, dosage regimens comprise low doses, administered at repeated intervals. For example, in one embodiment, formulations of the disclosure can be administered at a dose lower than about 375 mg/m$^2$ at intervals of approximately every 1, 2, 3, 4, 5, 6, 7, 8, 9, 10, 14, 15, 20, 21, 25, 30, 35, 40, 45, 50, 60, 70, 80, 90, 100, 120, 125, 150, 175, or 200 days.

The specified dosage can result in ICOS expressing T cell depletion in the human treated using formulations and methods of the disclosure for a period of at least about 1, 2, 3, 5, 7, 10, 14, 20, 30, 45, 60, 75, 90, 120, 150 or 180 days or longer. In certain embodiments of methods of the disclosure, ICOS expressing T cells are depleted by at least 30%, 40%, 50%, 60%, 70%, 80%, 90%, or 100% in comparison to ICOS expressing T cell levels in the patient being treated before use of formulations and methods of the disclosure. In other embodiments of methods of the disclosure, ICOS expressing T cells are depleted by at least 30%, 40%, 50%, 60%, 70%, 80%, 90%, or 100% in comparison to typical standard ICOS expressing T cell levels for humans. In related embodiments, the typical standard ICOS expressing T cell levels for humans are determined using patients comparable to the patient being treated with respect to age, sex, weight, and other factors.

In certain embodiments of the disclosure, a dosage of about 125 mg/m$^2$ or less of an antibody or antigen-binding fragment results in ICOS expressing T cell depletion for a period of at least about 7, 14, 21, 30, 45, 60, 90, 120, 150, or 200 days. In another representative embodiment, a dosage of about 37.5 mg/m$^2$ or less depletes ICOS expressing T cells for a period of at least about 7, 14, 21, 30, 45, 60, 90, 120, 150, or 200 days. In still other embodiments, a dosage of about 0.375 mg/m$^2$ or less results in depletion of ICOS expressing T cells for at least about 7, 14, 21, 30, 45 or 60 days. In another embodiment, a dosage of about 0.075 mg/m$^2$ or less results in depletion of ICOS expressing T cells for a period of at least about 7, 14, 21, 30, 45, 60, 90, 120, 150, or 200 days. In yet other embodiments, a dosage of about 0.01 mg/m$^2$, 0.005 mg/m$^2$ or even 0.001 mg/m$^2$ or less results in depletion of ICOS expressing T cells for at least about 3, 5, 7, 10, 14, 21, 30, 45, 60, 90, 120, 150, or 200 days. According to these embodiments, the dosage can be administered by any suitable route, but is optionally administered by a subcutaneous route.

As another aspect, the disclosure provides the discovery that ICOS expressing T cell depletion and/or treatment of T cell-mediated disorders can be achieved at lower dosages of antibody or antibody fragments than employed in currently available methods. Thus, in another embodiment, the disclosure provides a method of depleting ICOS expressing T cells and/or treating a T cell-mediated disorder, comprising administering to a human, an effective amount of an antibody that specifically binds to ICOS, wherein a dosage of about 500, 475, 450, 425, 400, 375, 350, 325, 300, 275, 250, 225, 200, 175, 150, 125, 100, 75, 60, 50, 37.5, 20, 10, 5, 2.5, 1, 0.5, 0.375, 0.25, 0.1, 0.075, 0.05, 0.001, 0.0005 mg/m$^2$ or less results in a depletion of ICOS expressing T cells (circulating and/or tissue ICOS expressing T cells) of 25%, 35%, 50%, 60%, 75%, 80%, 85%, 90%, 95%, 98% or more for a period at least about 3, 5, 7, 10, 14, 21, 30, 45, 60, 75, 90, 120, 150, 180, or 200 days or longer. In representative embodiments, a dosage of about 125 mg/m$^2$ or 75 mg/m$^2$ or less results in at least about 50%, 75%, 85% or 90% depletion of ICOS expressing T cells for at least about 7, 14, 21, 30, 60, 75, 90, 120, 150 or 180 days. In other embodiments, a dosage of about 50, 37.5 or 10 mg/m results in at least about a 50%, 75%, 85% or 90% depletion of ICOS expressing T cells for at least about 7, 14, 21, 30, 60, 75, 90, 120 or 180 days. In still other embodiments, a dosage of about 0.375 or 0.1 mg/m results in at least about a 50%, 75%, 85% or 90% depletion of ICOS expressing T cells for at least about 7, 14, 21, 30, 60, 75 or 90 days. In further embodiments, a dosage of about 0.075, 0.01, 0.001, or 0.0005 mg/m$^2$ results in at least about a 50%, 75%, 85% or 90% depletion of ICOS expressing T cells for at least about 7, 14, 21, 30 or 60 days.

In certain embodiments of the disclosure, the dose can be escalated or reduced to maintain a constant dose in the blood or in a tissue, such as, but not limited to, bone marrow. In related embodiments, the dose is escalated or reduced by about 2%, 5%, 8%, 10%, 15%, 20%, 30%, 40%, 50%, 60%, 70%, 80%, 90%, and 95% in order to maintain a desired level of an antibody of formulations and methods of the disclosure.

In certain embodiments, the dosage can be adjusted and/or the infusion rate can be reduced based on patient's immunogenic response to formulations and methods of the disclosure.

For formulations of the antibodies, proteins, polypeptides, peptides and fusion proteins encompassed by the disclosure, the dosage administered to a patient may be calculated using the patient's weight in kilograms (kg) multiplied by the dose to be administered in mg/kg. The required volume (in mL) to be given is then determined by taking the mg dose required divided by the concentration of the antibody formulation. The final calculated required volume will be obtained by pooling the contents of as many vials as are necessary into syringe(s) to administer the antibody formulation of the disclosure. The final calculated required volume will be obtained by pooling the contents of as many vials as are necessary into syringe(s) to administer the drug. A maximum volume of 2.0 mL of the antibody formulation can be injected per site. The dose (in mL) can be calculated using the following formula: Dose (mL) = [volunteer weight] (kg) x [dose] mg/kg ÷ 100 mg/mL of the antibody formulation. Generally, human antibodies have a longer half-life within the human body than antibodies from other species due to the immune response to the foreign polypeptides. Thus, lower dosages of human antibodies and less frequent administration is often possible. Further, the dosage, volume and frequency of administration of liquid formulations of the present disclosure may be reduced by increasing the concentration of an antibody (including antibody fragment thereof) in the formulations, increasing affinity and/or avidity of the antibody (including antibody fragment thereof), and/or increasing the half-life of the antibody (including antibody fragment thereof).

In a specific embodiment, the dosage administered to a patient will be calculated using the patient's weight in kilograms (kg) multiplied by the dose to be administered in mg/kg. The required volume (in mL) to be given is then determined by taking the mg dose required divided by the concentration of the antibody (including antibody fragment thereof) in the formulations (100 mg/mL). The final calculated required volume may be obtained by pooling the contents of as many vials as are necessary into syringe(s) to administer the drug. A maximum volume of 2.0 mL of antibody (including antibody fragment thereof) in the formulations can be injected per site.

In one embodiment, 0.01 to 20 mg/kg/week, 0.01 to 10 mg/kg/week, 0.01 to 5 mg/week, 0.01 to 2 mg/week, 0.01 to 1 mg/week, 0.01 to 0.5 mg/week, 0.01 to 0.2 mg/week, 0.01 to 0.1 mg/week of an antibody (including antibody fragment thereof) that specifically binds to human ICOS (for example, but not limited to, or a fragment thereof) in a liquid formulation of the disclosure is administered to a subject with an inflammatory disorder, an autoimmune disorder or a malignancy. In another embodiment, 0.01 to 20 mg/kg/month, 0.01 to 10 mg/kg/month, 0.01 to 5 mg/month, 0.01 to 2 mg/month, 0.01 to 1 mg/month, 0.01 to 0.5 mg/month, 0.01 to 0.2 mg/month, 0.01 to 0.1 mg/month of an antibody (including antibody fragment thereof) that specifically binds to human ICOS (for example, but not limited to, or a fragment thereof) in a liquid formulation of the disclosure is administered to a subject with an inflammatory disorder, an autoimmune disorder or a malignancy. In a further embodiment, 0.01 to 20 mg/kg/2 month, 0.01 to 10 mg/kg/2 month, 0.01 to 5 mg/2 month, 0.01 to 2 mg/2 month, 0.01 to 1 mg/2 month, 0.01 to 0.5 mg/2 month, 0.01 to 0.2 mg/2 month, 0.01 to 0.1 mg/2 month of an antibody (including antibody fragment thereof) that specifically binds to human ICOS (for example, but not limited to, or a fragment thereof) in a liquid formulation of the disclosure is administered to a subject with an inflammatory disorder, an autoimmune disorder or a malignancy. In another embodiment, a subject is administered one or more doses of a prophylactically or therapeutically effective amount of a liquid formulation of the disclosure, wherein the prophylactically or therapeutically effective amount is not the same for each dose.

In one embodiment, a liquid formulation of the disclosure is administered in a dosing regimen that maintains the plasma concentration of the antibody specific for human ICOS at a desirable level (e.g., from about 0.001 to about 100 μg/ml), which continuously depletes ICOS expressing cells. In a specific embodiment, the plasma concentration of the antibody is maintained at about 0.001 μg/ml, about 0.01 μg/ml, about 0.1 μg/ml, about 0.2 μg/ml, about 0.5 μg/ml, about 1 μg/ml, about 2 μg/ml, about 3 μg/ml, about 4 μg/ml, about 5 μg/ml, about 6 μg/ml, about 7 μg/ml, about 8 μg/ml, about 9 μg/ml, about 10 μg/ml, about 15 μg/ml, about 20 μg/ml, about 25 μg/ml, about 30 μg/ml, about 35 μg/ml, about 40 μg/ml, about 45 μg/ml or about 50 μg/ml. The plasma concentration that is desirable in a subject will vary depending on several factors, including but not limited to, the nature of the disease or disorder, the severity of the disease or disorder and the condition of the subject. Such dosing regimens are especially beneficial in prevention, treatment and/or management of a chronic disease or disorder.

In another embodiment, a human subject is administered one or more doses of a prophylactically or therapeutically effective amount of an antibody that specifically binds to human ICOS in a liquid formulation of the disclosure, wherein the dose of a prophylactically or therapeutically effective amount of the antibody in the liquid formulation of the disclosure administered to said subject is increased by, e.g., about 0.01 μg/kg, about 0.02 μg/kg, about 0.04 μg/kg,

about 0.05 μg/kg, about 0.06 μg/kg, about 0.08 μg/kg, about 0.1 μg/kg, about 0.2 μg/kg, about 0.25 μg/kg, about 0.5 μg/kg, about 0.75 μg/kg, about 1 μg/kg, about 1.5 μg/kg, about 2 μg/kg, about 4 μg/kg, about 5 μg/kg, about 10 μg/kg, about 15 μg/kg, about 20 μg/kg, about 25 μg/kg, about 30 μg/kg, about 35 μg/kg, about 40 μg/kg, about 45 μg/kg, about 50 μg/kg, about 55 μg/kg, about 60 μg/kg, about 65 μg/kg, about 70 μg/kg, about 75 μg/kg, about 80 μg/kg, about 85 μg/kg, about 90 μg/kg, about 95 μg/kg, about 100 μg/kg, or about 125 μg/kg, as treatment progresses.

In another embodiment, a subject (*e.g.,* a human) is administered one or more doses of a prophylactically or therapeutically effective amount of an antibody that specifically binds to human ICOS in a liquid formulation of the disclosure, wherein the dose of a prophylactically or therapeutically effective amount of the antibody in the liquid formulation of the disclosure administered to said subject is decreased by, *e.g.,* about 0.01 μg/kg, about 0.02 μg/kg, about 0.04 μg/kg, about 0.05 μg/kg, about 0.06 μg/kg, about 0.08 μg/kg, about 0.1 μg/kg, about 0.2 μg/kg, about 0.25 μg/kg, about 0.5 μg/kg, about 0.75 μg/kg, about 1 μg/kg, about 1.5 μg/kg, about 2 μg/kg, about 4 μg/kg, about 5 μg/kg, about 10 μg/kg, about 15 μg/kg, about 20 μg/kg, about 25 μg/kg, about 30 μg/kg, about 35 μg/kg, about 40 μg/kg, about 45 μg/kg, about 50 μg/kg, about 55 μg/kg, about 60 μg/kg, about 65 μg/kg, about 70 μg/kg, about 75 μg/kg, about 80 μg/kg, about 85 μg/kg, about 90 μg/kg, about 95 μg/kg, about 100 μg/kg, or about 125 μg/kg, as treatment progresses.

The dosages of prophylactic or therapeutic agents are described in the Physicians' Desk Reference (60th ed., 2006).

### 5.29.2. Toxicity Testing

The tolerance, toxicity and/or efficacy of the formulations and/or treatment regimens of the present disclosure can be determined by standard pharmaceutical procedures in cell cultures or experimental animals, *e.g.,* for determining the LD50 (the dose lethal to 50% of the population), the ED50 (the dose therapeutically effective in 50% of the population), and IC50 (the dose effective to achieve a 50% inhibition). In one embodiment, the dose is a dose effective to achieve at least a 60%, 70%, 80%, 90%, 95%, or 99% depletion of circulating ICOS expressing T cells. The dose ratio between toxic and therapeutic effects is the therapeutic index and it can be expressed as the ratio LD50/ED50. Therapies that exhibit large therapeutic indices may be preferred. While therapies that exhibit toxic side effects may be used, care should be taken to design a delivery system that targets such agents to ICOS-expressing cells in order to minimize potential damage to ICOS negative cells and, thereby, reduce side effects.

Data obtained from the cell culture assays and animal studies can be used in formulating a range of dosages of the formulations and/or treatment regimens for use in humans. The dosage of such agents may lie within a range of circulating concentrations that include the ED50 with little or no toxicity. The dosage may vary within this range depending upon the dosage form employed and the route of administration utilized. For any therapy used in methods of the disclosure, a therapeutically effective dose can be estimated by appropriate animal models. Depending on the species of the animal model, the dose can be scaled for human use according to art-accepted formulas, for example, as provided by Freireich et al., Quantitative comparison of toxicity of anticancer agents in mouse, rat, monkey, dog, and human, Cancer Chemotherapy Reports, NCI 1966 40:219-244. Data obtained from cell culture assays can be useful for predicting potential toxicity. Animal studies can be used to formulate a specific dose to achieve a circulating plasma concentration range that includes the IC50 (*i.e.,* the concentration of the test compound that achieves a half-maximal inhibition of symptoms) as determined in cell culture. Such information can be used to more accurately determine useful doses in humans. Plasma drug levels may be measured, for example, by high performance liquid chromatography, ELISA, or by cell based assays.

### 5.30. THERAPEUTIC USES

Formulations comprising an anti-ICOS antibody with enhanced effector function may be used for the treatment of autoimmune diseases, such as systemic lupus erythematosus, rheumatoid arthritis, multiple sclerosis, diabetes, immune thrombocytopenic purpura (ITP), and psoriasis; chronic inflammatory diseases, such as inflammatory bowel disease (Crohn's disease and ulcerative colitis), Grave's disease, Hashimoto's thyroiditis, and diabetes mellitus. Anti-ICOS formulations described herein may also be used to alleviate toxic shock syndrome, inflammatory bowel disease, allosensitization due to blood transfusions, T-cell dependent B-cell-mediated diseases, and the treatment of graft vs. host disease. In addition, formulations and methods of the disclosure may be useful in therapeutic indications that call for the inhibition or enhancement of antibody production.

Formulations comprising an anti-ICOS antibody with enhanced effector function may also be used as immunosuppressive agents for bone marrow and organ transplantation and may be used to prolong graft survival. Such formulations may provide significant advantages over existing treatment. Bone marrow and organ transplantation therapy must contend with T-cell-mediated rejection of the foreign cells or tissue by the host. Present therapeutic regimens for inhibiting T-cell-mediated rejection involve treatment with the drugs cyclosporine or FK506. While drugs are effective, patients suffer from serious side effects, including hepatotoxicity, nephrotoxicity, and neurotoxicity. The target for the cyclosporin/FK506 class of therapeutics is calcineurin, a phosphatase with ubiquitous expression. Since ICOS expression is restricted to T-cells, depletion of ICOS expressing T cells may lack the severe side effects observed with the use of the present immunotherapeutic agents.

Hypersensitivity is a normally beneficial immune response that is exaggerated or inappropriate, and leads to inflammatory reactions and tissue damage. Hypersensitivity reactions which are antibody-mediated may be particularly susceptible to antagonism by depletion of ICOS expressing cells. Allergies, hay fever, asthma, and acute edema cause type I hypersensitivity reactions, and these reactions may be suppressed by depletion of ICOS expressing cells.

Diseases that cause antibody-mediated hypersensitivity reactions, including systemic lupus erythematosus, arthritis (rheumatoid arthritis, reactive arthritis, psoriatic arthritis), nephropathies (glomerulonephritis, membranous, mesangiocapillary, focal segmental, focal necrotizing, crescentic, proliferative--tubulopathies), skin disorders (pemphigus and pemphigoid, erythema nodosum), endocrinopathies (thyroiditis--Grave's, Hashimoto's--insulin dependent diabetes mellitus), various pneumopathies (especially extrinsic alveolitis), various vasculopathies, coeliac disease, with aberrant production of IgA, many anemias and thrombocytopenias, Guillain-Barre Syndrome, and myasthenia gravis, may be treated using formulations comprising an anti-ICOS antibody with enhanced effector function.

In addition, lymphoproliferative disorders, such as multiple myeloma, Waldenstrom's macroglobulinemia, and crioglobulinemias may be inhibited by administering a formulation comprising an anti-ICOS antibody with enhanced effector function. Additionally, graft versus host disease, an "artificial" immune disorder, may benefit from the depletion of ICOS expressing cells.

The ICOS dependent co-stimulatory pathway is involved in regulating IgE production. IgE is an immunoglobulin isotype specifically involved in mediating allergic responses such as asthma, food allergies, hay fever, type 1 hypersensitivity and sinus inflammation. Upon exposure to an allergen, a process involving T-cell and B cell collaboration results in B cell production of IgE specific for the allergen. Allergen-specific IgE released into the circulation by B cells bind to mast cells and basophils through the high affinity IgE receptor (FceRI). Mast cells and basophils to which IgE is bound become sensitized and subsequent exposure to the allergen results in cross-linking of the surface receptors and release of histamines.

The disclosure provides for the use of an anti-ICOS antibody to regulate IgE production and to prevent or treat IgE-mediated disorders. By way of example, such disorders include allergic responses such as asthma, food allergies, hay fever, hypersensitivity, and sinus inflammation. In one embodiment, an anti-ICOS antibody of the disclosure is used to partially or completely inhibit IgE production. An anti-ICOS antibody of the disclosure may be used separately, or in combination, in a treatment regimen for decreasing IgE levels.

The disclosure also provides for the use of an anti-ICOS antibody in combination with an IgE antagonist to partially or completely inhibit IgE production and to prevent and/or treat disorders characterized by excessive or inappropriate IgE production. As used herein the term "IgE antagonist" refers to a compound capable of disrupting or blocking the interaction of IgE with its high affinity receptor FceRI on cells such that the response to allergen stimulus is attenuated or eliminated. Antagonists include an anti-IgE antibody and fragments thereof, soluble FceRI receptor and fragments thereof, anti- FceRI antibody and fragments thereof, IgE variants and fragments thereof, IgE binding peptides, FceRI receptor binding peptides, and small molecules capable of binding to IgE or competing with IgE for binding to FceRI receptor. An anti-ICOS antibody of the disclosure may also be used with in combination with antihistamines, allergen desensitization, reduction in exposure to allergen and the like for treatment of allergic disorders.

The disclosure also provides for the prevention and/or treatment of asthma comprising administering an anti-ICOS antibody of the disclosure alone or in conjunction with one or more agents for treating asthma. Examples of such agents include bronchodilators (anticholinergic agents, .beta-2 adrenergic receptor agonists, lenkotriene D-4 antagonists, neurokinin antagonists, potassium channel openers, substance P antagonists, thromboxane A-2 antagonists, and xanthines), anti-inflammatories (5-lipoxygenase inhibitors, 5-lipoxygenase activating protein inhibitors, phosphodiesterase IV inhibitors, platelet activating factor antagonists, respiratory NSAIDS, steroids, and tyrosine kinase inhibitors), cytokine inhibitors (CD4, IL-4 and IL-5 inhibitors) and IgE antagonists as set forth above.

Formulations and methods according to this disclosure are able to control (suppress or stimulate) proliferation of ICOS expressing cells or production of cytokine (for example, IL-17) by ICOS expressing cells, thereby enabling suppression of various pathological conditions and treatment or prevention of various disorders caused by diverse physiological phenomena related to signal transduction mediated by ICOS.

Formulations comprising an anti-ICOS antibody of this disclosure enables suppression, prevention and/or treatment of, for example, but not limited to, rheumatoid arthritis, multiple sclerosis, autoimmune thyroiditis, allergic contact-type dermatitis, chronic inflammatory dermatosis (e.g., lichen planus), systemic lupus erythematosus, insulin-dependent diabetes mellitus, psoriasis, autoimmune or allergic disorders, autoimmune disease and delayed allergy caused by cellular immunity; arthropathia (for example, but not limited to, rheumatoid arthritis (RA) and osteoarthritis (OA)), inflammation (e.g., hepatitis), graft versus host reaction (GVH reaction), graft versus host disease (GVHD), immune rejection accompanying transplantation of a tissue (e.g., skin, cornea, bone) or organ (e.g., liver, heart, lung, kidney, pancreas), immune response triggered by a foreign antigen or autoantigen (for example, production of antibodies against said antigen, cell proliferation, production of cytokines), and disorders caused by the abnormal intestinal immunity (e.g., inflammatory intestinal disorders, Crohn's disease, ulcerative colitis, alimentary allergy).

Furthermore, formulations and methods described herein may be utilized for the suppression/treatment of transplant rejection or GVHD in combination with known immunosuppressive agents such as inhibitors of cytokine transcription (*e.g.,* cyclosporin A, tacrolimus), nucleotide synthesis (*e.g.,* azathiopurine, mycophenolate mofetil), growth factor signal transduction (e.*g.,* sirolimus, rapamycin), and the T cell interleukin 2 receptor (*e.g.,* daclizumab, basiliximab). In a particular embodiment, an immunosuppressant agent used in combination with formulations and methods of the disclosure includes one or more of the following: adriamycin, azathiopurine, busulfan, cyclophosphamide, cyclosporin A ("CyA"), cytoxin, fludarabine, 5-fluorouracil, methotrexate, mycophenolate mofetil (MOFETIL), nonsteroidal anti-inflammatories (NSAIDs), rapamycin, and tacrolimus (FK506).

The formulations and methods of the present disclosure can be applied to inflammatory disease for example, inflammation accompanying various arthritis (for example, rheumatoid arthritis, osteoarthritis), pneumonia, hepatitis (including viral hepatitis), inflammation accompanying infectious diseases, inflammatory bowel diseases, intestinal enteritis, nephritis (e.g., glomerular nephritis, nephrofibrosis), gastritis, angiitis, pancreatitis, peritonitis, bronchitis, myocarditis, cerebritis, inflammation in postischemic reperfusion injury (myocardial ischemic reperfusion injury), inflammation attributed to immune rejection after transplantation of tissue and organ, burn, various skin inflammation (psoriasis, allergic contact-type dermatitis, lichen planus), inflammation in multiple organ failure, inflammation after operation of PTCA or PTCR, and inflammation accompanying arteriosclerosis, and autoimmune thyroiditis.

Formulations of the disclosure comprising an anti-ICOS antibody with enhanced effector function as an active ingredient may be used to inhibit, treat and/or prevent a variety of diseases, for example, but not limited to rheumatoid arthritis, multiple sclerosis, autoimmune thyroiditis, allergic contact dermatitis, lichen planus, systemic lupus erythematosus, insulin dependent diabetes mellitus, psoriasis, autoimmune diseases or allergic diseases, delayed allergies mediated by cellular immunity; arthropathies (e.g., rheumatoid arthritis (RA), osteoarthritis (OA)), inflammation (e.g., hepatitis), graft versus host reaction (GVH reaction), graft versus host disease (GVHD), immunorejection associated with transplantation of tissues (e.g., skin, cornea and bone) or organs (e.g., liver, heart, lung, kidney, pancreas), inflammatory bowel disease, Crohn's disease, ulcerative colitis, and alimentary allergy.

The formulations in accordance with the present disclosure make it possible to treat or prevent some inflammations for which various steroidal drugs are used as anti-inflammatory drugs, for example, inflammation associated with various arthritides (e.g., rheumatoid arthritis, osteoarthritis), pneumonia, hepatitis (including viral hepatitis), inflammation associated with infectious diseases, inflammatory bowel disease, enteritis, nephritis, glomerular nephritis, inflammation associated with kidney fibrosis, gastritis, vasculitis, pancreatitis, peritonitis, bronchitis, myocarditis, encephalitis, inflammation associated with ischemia-reperfusion injury, myocardial ischemia-reperfusion injury, inflammation associated with immunorejection after transplantation of tissues or organs, psoriasis, allergic contact dermatitis, lichen planus, inflammation associated with multiple organ failure, inflammation after operation of PTCA or PTCR, inflammation associated with atherosclerosis, and autoimmune thyroiditis.

## 5.31. TRANSPLANTATION

According to certain aspects of the disclosure, the treatment regimen and dose used with formulations and methods of the disclosure is chosen based on a number of factors including, for example, clinical manifestation that place a patient at risk for developing transplant rejection, or clinical evidence that such a rejection is developing.

The present disclosure provides formulations, methods and regimens effective to reduce the incidence, severity, or duration of GVHD, a rejection episode, or post-transplant lymphoproliferative disorder. In certain embodiments, formulations and methods of the disclosure are effective to attenuate the host response to ischemic reperfusion injury of a solid tissue or organ graft. In one embodiment, formulations and methods of the disclosure are effective to prolong survival of a graft in a transplant recipient.

The present disclosure encompasses grafts that are autologous, allogeneic, or xenogeneic to the recipient. The types of grafts encompassed by the disclosure include tissue and organ grafts, including but not limited to, bone marrow grafts, peripheral stem cell grafts, skin grafts, arterial and venous grafts, pancreatic islet cell grafts, and transplants of the kidney, liver, pancreas, thyroid, and heart. The terms "graft" and "transplant" are used interchangeably herein. In one embodiment, the autologous graft is a bone marrow graft, an arterial graft, a venous graft or a skin graft. In one embodiment, the allograft is a bone marrow graft, a corneal graft, a kidney transplant, a pancreatic islet cell transplant, or a combined transplant of a kidney and pancreas. In one embodiment, the graft is a xenograft, wherein the possible animal donors include, but are not limited to pigs. The formulations and methods of the present disclosure may also be used to suppress a deleterious immune response to a non-biological graft or implant, including but not limited to an artificial joint, a stent, or a pacemaker device.

Anti-ICOS antibodies, formulations, and methods of the disclosure may be used to treat or prevent GVHD, rejection, or post-transplant lymphoproliferative disorder without regard to the particular indications initially giving rise to the need for the transplant or the particular type of tissue transplanted.

Therapeutic formulations and regimens of the present disclosure are described for treating human subjects diagnosed with autoimmune diseases or disorders, including but not limited to, rheumatoid arthritis, SLE, ITP, pemphigus-related disorders, diabetes, and scleroderma.

Appropriate treatment regimens can be determined by one of skill in the art for the particular patient or patient population. In particular embodiments, the treatment regimen is a pre-transplant conditioning regimen, a post-transplant maintenance regimen, or post-transplant treatment regimen for an acute or a chronic rejection. In certain embodiments, the particular regimen is varied for a patient who is assessed as being at a high or intermediate risk of developing a rejection response, compared with the regimen for a patient who is assessed as being at a low risk of rejection.

In certain embodiments, the particular regimen is varied according to the stage of rejection, with more aggressive therapy being indicated for patients at later stages of rejection. The stages of humoral rejection may be classified according to the knowledge and skill in the art. For example, the stages of humoral rejection may be classified as one of stages I to IV according to the following criteria: Stage I Latent Response, characterized by circulating anti-donor alloantibodies, especially anti-HLA antibodies; Stage II Silent Reaction, characterized by circulating anti-donor alloantibodies, especially anti-HLA antibodies, and C4d deposition, but without histologic changes or graft dysfunction; Stage III Subclinical Rejection: characterized by circulating anti-donor alloantibodies, especially anti-HLA antibodies, C4d deposition, and tissue pathology, but without graft dysfunction; Stage IV Humoral Rejection: characterized by circulating anti-donor alloantibodies, especially anti-HLA antibodies, C4d deposition, tissue pathology, and graft dysfunction.

Anti-ICOS antibodies, formulations and methods of the disclosure may be practiced to treat or prevent GVHD, rejection, or post-transplantation lymphoproliferative disorders, either alone or in combination with other therapeutic agents or treatment regimens. Other therapeutic regimens for the treatment or prevention of GVHD, rejection, or post-transplantation lymphoproliferative disorders may comprise, for example, one or more of anti-lymphocyte therapy, steroid therapy, antibody depletion therapy, immunosuppression therapy, and plasmapheresis.

Anti-lymphocyte therapy may comprise the administration to the transplant recipient of anti-thymocyte globulins, also referred to as thymoglobulin. Anti-lymphocyte therapy may also comprise the administration of one or more monoclonal antibodies directed against T cell surface antigens. Examples of such antibodies include, without limitation, OKT3™ (muromonab-CD3), CAMPATH™-1H (alemtuzumab), CAMPATH™-1G, CAMPATH™-1M, SIMULECT™ (basiliximab), and ZENAPAX™ (daclizumab). In a specific embodiment, the anti-lymphocyte therapy comprises one or more antibodies directed against B cells, including, without limitation, RITUXAN™ (rituximab).

Steroid therapy may comprise administration to the transplant recipient of one or more steroids selected from the group consisting of cortisol, prednisone, methyl prednisolone, dexamethazone, and indomethacin. One or more of the steroids may be corticosteroids, including without limitation, cortisol, prednisone, and methylprednisolone.

Antibody depletion therapy may include, for example, administration to the transplant recipient of intravenous immunoglobulin. Antibody depletion therapy may also comprise immunoadsorption therapy applied to the graft *ex vivo,* prior to transplantation. Immunoadsorption may be accomplished using any suitable technique, for example, protein A affinity, or antibody based affinity techniques using antibodies directed against T cell or B cell surface markers such as anti-CD3 antibodies, anti-CD19 antibodies, anti-CD20 antibodies, and anti-CD22 antibodies.

Immunosuppression therapy may comprise the administration of one or more immunosuppressive agents such as inhibitors of cytokine transcription (*e.g.,* cyclosporin A, tacrolimus), nucleotide synthesis (*e.g.,* azathioprine, mycophenolate mofetil), growth factor signal transduction (*e.g.,* sirolimus, rapamycin), and the T cell interleukin 2 receptor (*e.g.,* daclizumab, basiliximab). In a particular embodiment, an immunosuppressant agent used in combination with formulations and methods of the disclosure includes one or more of the following: adriamycin, azathioprine, busulfan, cyclophosphamide, cyclosporin A ("CyA"), cytoxin, fludarabine, 5-fluorouracil, methotrexate, mycophenolate mofetil (MOFETIL), nonsteroidal anti-inflammatories (NSAIDs), rapamycin, and tacrolimus (FK506). Immunosuppressive agents may also comprise inhibitors of complement, for example, soluble complement receptor-1, anti-C5 antibody, or a small molecule inhibitor of CIs, for example as described in Buerke et al. (J. Immunol., 167:5375-80 (2001).

In one embodiment, formulations and methods of the disclosure are used in combination with one or more therapeutic regimens for suppressing rejection, including, without limitation, tacrolimus and mycophenolate mofetil therapy, immunoadsorption, intravenous immunoglobulin therapy, and plasmapheresis.

## 5.32. INFLAMMATORY DISORDER

Anti-ICOS antibodies of the disclosure may be administered to a subject in need thereof to prevent, manage, treat or ameliorate an inflammatory disorder (e.g., asthma) or one or more symptoms thereof. Formulations of the disclosure may also be administered in combination with one or more other therapies, preferably therapies useful for the prevention, management, treatment or amelioration of an inflammatory disorder (including, but not limited to the prophylactic or therapeutic agents listed herein) to a subject in need thereof to prevent, manage, treat or ameliorate an inflammatory disorder or one or more symptoms thereof. In a specific embodiment, the disclosure provides a method of preventing, managing, treating or ameliorating an inflammatory disorder or one or more symptoms thereof, said method comprising administering to a subject in need thereof a dose of a prophylactically or therapeutically effective amount of an anti-ICOS antibody of the disclosure. In another embodiment, the disclosure provides a method of preventing, managing, treating or ameliorating an inflammatory disorder or one or more symptoms thereof, said method comprising administering to a subject in need thereof a dose of a prophylactically or therapeutically effective amount of an effector function enhanced anti-ICOS antibody of the disclosure and a dose of a prophylactically or therapeutically effective amount of one or more therapies (e.g., prophylactic or therapeutic agents) other than antibodies (including antibody fragments thereof) that immunospecifically bind to an ICOS polypeptide.

The disclosure provides methods for managing, treating or ameliorating one or more symptoms of an inflammatory disorder in a subject refractory to conventional therapies (e.g., methotrexate and a TNF-alpha antagonist (e.g., REMICADE™ or ENBREL™)) for such an inflammatory disorder, said methods comprising administering to said subject a dose of a prophylactically or therapeutically effective amount of an effector function enhanced anti-ICOS antibody of the disclosure. The disclosure also provides methods for managing, treating or ameliorating one or more symptoms of an inflammatory disorder in a subject refractory to existing single agent therapies for such an inflammatory disorder, said methods comprising administering to said subject a dose of a prophylactically or therapeutically effective amount of an effector function enhanced anti-ICOS antibody of the disclosure and a dose of a prophylactically or therapeutically effective amount of one or more therapies (e.g., prophylactic or therapeutic agents) other than antibodies (including antibody fragments thereof) that immunospecifically bind to an ICOS polypeptide. The disclosure also provides methods for managing or treating an inflammatory disorder by administering an effector function enhanced anti-ICOS antibody of the disclosure in combination with any other treatment to patients who have proven refractory to other treatments but are no longer on these treatments. The disclosure also provides alternative methods for the treatment of an inflammatory disorder where another therapy has proven or may prove too toxic, i.e., results in unacceptable or unbearable side effects, for the subject being treated. For example, a formulation of the disclosure may be administered to a subject, wherein the subject is refractory to a TNF antagonist or methotrexate. Further, the disclosure provides methods for preventing the recurrence of an inflammatory disorder in patients that have been treated and have no disease activity by administering an effector function enhanced anti-ICOS antibody of the disclosure.

Inflammatory disorders that can be treated by the methods encompassed by the disclosure include, but are not limited to, asthma, encephalitis, inflammatory bowel disease, chronic obstructive pulmonary disease (COPD), allergic disorders, septic shock, pulmonary fibrosis, undifferentiated spondyloarthropathy, undifferentiated arthropathy, arthritis, osteoarthritis, spondyloarthropathies (e.g., psoriatic arthritis, ankylosing spondylitis, Reiter's Syndrome (reactive arthritis), inflammatory osteolysis, Wilson's disease and chronic inflammation resulting from chronic viral or bacteria infections. As described herein, some autoimmune disorders are associated with an inflammatory condition.

Anti-inflammatory therapies and their dosages, routes of administration and recommended usage are known in the art and have been described in such literature as the Physician's Desk Reference (61th ed., 2007).

5.32.1.Anti-Inflammatory Therapies

The present disclosure provides methods of preventing, managing, treating or ameliorating an inflammatory disorder or one or more symptoms thereof, said methods comprising administering to a subject in need thereof an effector function enhanced anti-ICOS antibody of the disclosure and one or more therapies (e.g., prophylactic or therapeutic agents other than antibodies (including antibody fragments thereof) that immunospecifically bind to an ICOS polypeptide. Any agent or therapy which is known to be useful, or which has been used or is currently being used for the prevention, management, treatment or amelioration of an inflammatory disorder or one or more symptoms thereof can be used in combination with an effector function enhanced anti-ICOS antibody of the disclosure in accordance with the disclosure described herein.

Any anti-inflammatory agent, including agents useful in therapies for inflammatory disorders, well-known to one of skill in the art can be used in the formulations and methods of the disclosure. Non-limiting examples of anti-inflammatory agents include non-steroidal anti-inflammatory drugs (NSAIDs), steroidal anti-inflammatory drugs, anti-cholinergics (e.g., atropine sulfate, atropine methylnitrate, and ipratropium bromide (ATROVENT™)), beta2-agonists (e.g., aburterol (VENTOLIN™ and PROVENTIL™), bitolterol (TORNALATE™), levalbuterol (XOPONEX™), metaproterenol (ALUPENT™), pirbuterol (MAXAIR™), terbutlaine (BRETHAIRE™ and BRETHINE™), albuterol (PROVENTIL™, REPETABS™, and VOLMAX™), formoterol (FORADIL AEROLIZER™), and salmeterol (SEREVEN™ and SEREVENT DISKUS™)), and methylxanthines (e.g., theophylline (UNIPHYL™, THEO-DUR™, SLO-BID™, AND TEHO-42™)). Examples of NSAIDs include, but are not limited to, aspirin, ibuprofen, celecoxib (CELEBREX™), diclofenac (VOLTAREN™), etodolac (LODINE™), fenoprofen (NALFON™), indomethacin (INDOCIN™), ketoralac (TORADOL™), oxaprozin (DAYPRO™), nabumentone (RELAFEN™), sulindac (CLINORIL™), tolmentin (TOLECTIN™), rofecoxib (VIOXX™), naproxen (ALEVE™, NAPROSYN™), ketoprofen (ACTRON™) and nabumetone (RELAFEN™). Such NSAIDs function by inhibiting a cyclooxgenase enzyme (e.g., COX-1 and/or COX-2). Examples of steroidal anti-inflammatory drugs include, but are not limited to, glucocorticoids, dexamethasone (DECADRON™), corticosteroids (e.g., methylprednisolone (MEDROL™)), cortisone, hydrocortisone, prednisone (PREDNISONE™ and DELTASONE™), prednisolone (PRELONE™ and PEDIAPRED™), triamcinolone, azulfidine, and inhibitors of eicosanoids (e.g., prostaglandins, thromboxanes, and leukotrienes).

In one embodiment, an effective amount of one or more formulations of the disclosure is administered in combination with a mast cell protease inhibitor to a subject at risk of or with an inflammatory disorder. In another embodiment, the mast cell protease inhibitor is a tryptase kinase inhibitor, such as, but not limited to GW-45, GW-58, and genisteine. In a specific embodiment, the mast cell protease inhibitor is phosphatidylinositide-3' (PI3)-kinase inhibitors, such as, but not limited to calphostin C. In another embodiment, the mast cell protease inhibitor is a protein kinase inhibitor such as, but not limited to staurosporine. In one embodiment, the mast cell protease inhibitor is administered locally to the affected area.

Specific examples of immunomodulatory agents which can be administered in combination with an effector function enhanced anti-ICOS antibody of the disclosure to a subject with an inflammatory disorder include, but are not limited to, methothrexate, leflunomide, cyclophosphamide, cytoxan, Immuran, cyclosporine A, minocycline, azathioprine, antibiotics (e.g., FK506 (tacrolimus)), methylprednisolone (MP), corticosteroids, steroids, mycophenolate mofetil, rapamycin (sirolimus), mizoribine, deoxyspergualin, brequinar, malononitriloamindes (e.g., leflunamide), anti-T cell receptor antibodies (e.g., anti-CD4 antibodies (e.g., cM-T412 (Boeringer), IDEC-CE9.1.RTM. (IDEC and SKB), mAB 4162W94, Orthoclone and OKTcdr4a (Janssen-Cilag)), anti-CD3 antibodies (e.g., Nuvion (Product Design Labs), OKT3 (Johnson & Johnson), or Rituxan (IDEC)), anti-CD5 antibodies (e.g., an anti-CD5 ricin-linked immunoconjugate), anti-CD7 antibodies (e.g., CHH-380 (Novartis)), anti-CD8 antibodies, anti-CD40 ligand monoclonal antibodies (e.g., IDEC-131 (IDEC)), anti-CD52 antibodies (e.g., CAMPATH 1H (Ilex)), anti-CD2 antibodies (e.g., MEDI-507 (MedImmune, Inc., International Publication Nos. WO 02/098370 and WO 02/069904), anti-CD11a antibodies (e.g., Xanelim (Genentech)), and anti-B7 antibodies (e.g., IDEC-114) (IDEC)); anti-cytokine receptor antibodies (e.g., anti-IFN receptor antibodies, anti-IL-2 receptor antibodies (e.g., Zenapax (Protein Design Labs)), anti-IL-4 receptor antibodies, anti-IL-6 receptor antibodies, anti-IL-10 receptor antibodies, and anti-IL-12 receptor antibodies), anti-cytokine antibodies (e.g., anti-IFN antibodies, anti-TNF-alpha antibodies, anti-IL-lbeta antibodies, anti-IL-6 antibodies, anti-IL-8 antibodies (e.g., ABX-IL-8 (Abgenix)), and anti-IL-12 antibodies)); CTLA4-immunoglobulin; LFA-3TIP (Biogen, International Publication No. WO 93/08656 and U.S. Pat. No. 6,162,432); soluble cytokine receptors (e.g., the extracellular domain of a TNF-alpha receptor or a fragment thereof, the extracellular domain of an IL-1beta receptor or a fragment thereof, and the extracellular domain of an IL-6 receptor or a fragment thereof); cytokines or fragments thereof (e.g., interleukin (IL)-2, IL-3, IL-4, IL-5, IL-6, IL-7, IL-8, IL-9, IL-10, IL-11, IL-112, IL-15, TNF-alpha, TNF-beta, interferon (IFN)-alpha, IFN-beta, IFN-gamma, and GM-CSF); and anti-cytokine antibodies (e.g., anti-IL-2 antibodies, anti-IL-4 antibodies, anti-IL-6 antibodies, anti-IL-9 antibodies, anti-IL-10 antibodies, anti-IL-12 antibodies, anti-IL-15 antibodies, anti-IL17 antibodies, anti-TNF-alpha antibodies, and anti-IFN-gamma antibodies).

Any TNF-alpha antagonist well-known to one of skill in the art can be used in the formulations and methods of the disclosure. Non-limiting examples of TNF-alpha antagonists which can be administered in combination with an effector function enhanced anti-ICOS antibody of the disclosure to a subject with an inflammatory disorder include proteins, polypeptides, peptides, fusion proteins, antibodies (e.g., human, humanized, chimeric, monoclonal, polyclonal, Fvs, ScFvs, Fab fragments, F(ab)$_2$ fragments, and antigen-binding fragments thereof) such as antibodies that immunospecifically bind to TNF-alpha, nucleic acid molecules (e.g., antisense molecules or triple helices), organic molecules, inorganic molecules, and small molecules that blocks, reduces, inhibits or neutralizes the function, activity and/or expression of TNF-alpha. In various embodiments, a TNF-alpha antagonist reduces the function, activity and/or expression of TNF-alpha by at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or at least 99% relative to a control such as phosphate buffered saline (PBS). Examples of antibodies that immunospecifically bind to TNF-alpha include, but are not limited to, infliximab (REMICADE™; Centacor), D2E7 (Abbott Laboratories/Knoll Pharmaceuticals Co., Mt. Olive, N.J.), CDP571 which is also known as HUMICADE™ and CDP-870 (both of Celltech/Pharmacia, Slough, U.K.), and TN3-19.12 (Williams et al., 1994, Proc. Natl. Acad. Sci. USA 91: 2762-2766; Thorbecke et al., 1992, Proc. Natl. Acad. Sci. USA 89:7375-7379). The present disclosure also encompasses the use of antibodies that immunospecifically bind to TNF-alpha disclosed in the following U.S. Patents in the formulations and methods of the disclosure: 5,136,021; 5,147,638; 5,223,395; 5,231,024; 5,334,380; 5,360,716; 5,426,181; 5,436,154; 5,610,279; 5,644,034; 5,656,272; 5,658,746; 5,698,195; 5,736,138; 5,741,488; 5,808,029; 5,919,452; 5,958,412; 5,959,087; 5,968,741; 5,994,510; 6,036,978; 6,114,517; and 6,171,787. Examples of soluble TNF-alpha receptors include, but are not limited to, sTNF-R1 (Amgen), etanercept (ENBREL™; Immunex) and its rat homolog RENBREL™, soluble inhibitors of TNF-alpha derived from TNFrI, TNFrII (Kohno et al., 1990, Proc. Natl. Acad. Sci. USA 87:8331-8335), and TNF-alpha Inh (Seckinger et al, 1990, Proc. Natl. Acad. Sci. USA 87:5188-5192).

Other TNF-alpha antagonists encompassed by the disclosure include, but are not limited to, IL-10, which is known to block TNF-alpha production via interferon gamma-activated macrophages (Oswald et al. 1992, Proc. Natl.

Acad. Sci. USA 89:8676-8680), TNFR-IgG (Ashkenazi et al., 1991, Proc. Natl. Acad. Sci. USA 88:10535-10539), the murine product TBP-1 (Serono/Yeda), the vaccine CytoTAb (Protherics), antisense molecule 104838 (ISIS), the peptide RDP-58 (SangStat), thalidomide (Celgene), CDC-801 (Celgene), DPC-333 (Dupont), VX-745 (Vertex), AGIX-4207 (AtheroGenics), ITF-2357 (Italfarmaco), NPI-13021-31 (Nereus), SCIO-469 (Scios), TACE targeter (Immunix/AHP), CLX-120500 (Calyx), Thiazolopyrim (Dynavax), auranofin (Ridaura) (SmithKline Beecham Pharmaceuticals), quinacrine (mepacrine dichlorohydrate), tenidap (Enablex), Melanin (Large Scale Biological), and anti-p38 MAPK agents by Uriach.

Non-limiting examples of anti-inflammatory agents which can be administered in combination with an effector function enhanced anti-ICOS antibody of the disclosure to a subject with an inflammatory disorder include non-steroidal anti-inflammatory drugs (NSAIDs), steroidal anti-inflammatory drugs, beta-agonists, anticholingeric agents, and methyl xanthines. Examples of NSAIDs include, but are not limited to, aspirin, ibuprofen, celecoxib (CELEBREX™), diclofenac (VOLTAREN™), etodolac (LODINE™), fenoprofen (NALFON™), indomethacin (INDOCIN™), ketoralac (TORADOL™), oxaprozin (DAYPRO™), nabumentone (RELAFEN™), sulindac (CLINORIL™), tolmentin (TOLECTIN™), rofecoxib (VIOXX™), naproxen (ALEVE™, NAPROSYN™), ketoprofen (ACTRON™) and nabumetone (RELAFEN™). Such NSAIDs function by inhibiting a cyclooxgenase enzyme (e.g., COX-1 and/or COX-2). Examples of steroidal anti-inflammatory drugs include, but are not limited to, glucocorticoids, dexamethasone (DECADRON™), cortisone, hydrocortisone, prednisone (DELTASONE™), prednisolone, triamcinolone, azulfidine, and eicosanoids such as prostaglandins, thromboxanes, and leukotrienes.

In specific embodiments, patients with osteoarthritis are administered a prophylactically or therapeutically effective amount of an effector function enhanced anti-ICOS antibody of the disclosure in combination with other agents or therapies useful for osteoarthritis prevention, treatment, management or amelioration including but not limited to: analgesics (non-limiting examples are acetaminophen, in a dose up to 4000 mg/d; phenacetin; and tramadol, in a daily dose in the range of 200 to 300 mg); NSAIDs (non-limiting examples include but not limited to, aspirin, diflunisal, diclofenac, etodolac, fenamates, fenoprofen, flurbiprofen, ibuprofen, indomethacin, ketoprofen, methylsalicylate, nebumetone, naproxin, oxaprazin, phenylbutazone, piroxicam, sulindac, and tolmetin. Low dose NSAIDs are preferred, e.g., ibuprofen at 1200 mg/d, naproxen at 500 mg/d. A gastroprotective agent, e.g., misoprostol, famotidine or omeprazole, is preferred to use concurrently with a NSAID); nonacetylated salicylates including but not limited to salsalate; cyclooxygenase (Cox)-2-specific inhibitors (CSIs), including but not limited to, celecoxib and rofecoxib; intra- or periarticular injection of a depot glucocorticoid preparation; intra-articular injection of hyaluronic acid; capsaicin cream; copious irrigation of the osteoarthritis knee to flush out fibrin, cartilage shards and other debris; and joint replacement surgery. Formulations and methods of the disclosure can also be used in combination with other nonpharmacologic measures in prevention, treatment, management and amelioration of osteoarthritis including but not limited to: reduction of joint loading (non-limiting examples are correction of poor posture, support for excessive lumbar lordosis, avoid excessive loading of the involved joint, avoid prolonged standing, kneeling and squatting); application of heat to the affected joint; aerobic exercise and other physical therapies.

In specific embodiments, patients with rheumatoid arthritis are administered a prophylactically or therapeutically effective amount of an effector function enhanced anti-ICOS antibody of the disclosure in combination with other agents or therapies useful in prevention, treatment, management and amelioration of rheumatoid arthritis including but not limited to: NSAIDs (non-limiting examples include but not limited to, aspirin, diflunisal, diclofenac, etodolac, fenamates, fenoprofen, flurbiprofen, ibuprofen, indomethacin, ketoprofen, methylsalicylate, nebumetone, naproxin, oxaprazin, phenylbutazone, piroxicam, sulindac, and tolmetin.); analgesics (non-limiting examples are acetaminophen, phenacetin and tramadol); CSIs including but not limited to, celecoxib and rofecoxib; glucocorticoids (preferably low-dose oral glucocorticoids, e.g., <7.5 mg/d prednisone, or monthly pulses with high-dose glucocorticoids, or intra-articular glucocorticoids); disease-modifying anti-rheumatic drugs (DMARDs) including but not limited to, methotrexate (preferably given intermittent low dose, e.g., 7.5-30 mg once weekly), gold compounds (e.g., gold salts), D-penicillamine, the antimalarials (e.g., chloroquine), and sulfasalazine; TNF-alpha neutralizing agents including but not limited to, etanercept and infliximab; immunosuppressive and cytotoxic agents (examples include but not limited to, azathioprine, leflunomide, cyclosporine, and cyclophosphamide), and surgery (examples include but not limited to, arthroplasties, total joint replacement, reconstructive hand surgery, open or arthroscopic synovectomy, and early tenosynovectomy of the wrist). The formulations and methods of the disclosure may also be used in combination with other measures in prevention, treatment, management and amelioration of the rheumatoid arthritis including but not limited to: rest, splinting to reduce unwanted motion of inflamed joint, exercise, used of a variety of orthotic and assistive devices, and other physical therapies. The formulations and methods of the disclosure may also be used in combination with some nontraditional approaches in prevention, treatment, management and amelioration of rheumatoid arthritis including but not limited to, diets (e.g., substituting omega-3 fatty acids such as eicosapentaenoic acid found in certain fish oils for dietary omega-6 essential fatty acids found in meat), vaccines, hormones and topical preparations.

In specific embodiments, patients with chronic obstructive pulmonary disease (COPD) are administered a prophylactically or therapeutically effective amount of an effector function enhanced anti-ICOS antibody of the disclosure in combination with other agents or therapies useful in prevention, treatment, management and amelioration of COPD including but not limited to: bronchodilators including but not limited to, short- and long-acting beta2 - adrenergic agonists (examples of short-acting beta2 agonist include but not limited to, albuterol, pirbuterol, terbutaline, and metaproterenol; examples of long-acting beta2 agonist include but not limited to, oral sustained-release albuterol and inhaled salmeterol), anticholinergics (examples include but not limited to ipratropium bromide), and theophylline and its derivatives (therapeutic range for theophylline is preferably 10-20 .mu.g/mL); glucocorticoids; exogenous alphalAT (e.g., alphalAT derived from pooled human plasma administered intravenously in a weekly dose of 60 mg/kg); oxygen; lung transplantation; lung volume reduction surgery; endotracheal intubation, ventilation support; yearly influenza vaccine and pneumococcal vaccination with 23-valent polysaccharide; exercise; and smoking cessation.

In specific embodiments, patients with asthma are administered a prophylactically or therapeutically effective amount of an effector function enhanced anti-ICOS antibody of the disclosure in combination with an effective amount of one or more other agents useful for asthma therapy. Non-limiting examples of such agents include adrenergic stimulants (e.g., catecholamines (e.g., epinephrine, isoproterenol, and isoetharine), resorcinols (e.g., metaproterenol, terbutaline, and fenoterol), and saligenins (e.g., salbutamol)), adrenocorticoids, blucocorticoids, corticosteroids (e.g., beclomethadonse, budesonide, flunisolide, fluticasone, triamcinolone, methylprednisolone, prednisolone, and prednisone), other steroids, beta2-agonists (e.g., albtuerol, bitolterol, fenoterol, isoetharine, metaproterenol, pirbuterol, salbutamol, terbutaline, formoterol, salmeterol, and albutamol terbutaline), anti-cholinergics (e.g., ipratropium bromide and oxitropium bromide), IL-4 antagonists (including antibodies), IL-5 antagonists (including antibodies), IL-9 antagonists (including antibodies), IL-13 antagonists (including antibodies), IL_17 antagonists (including antibodies), PDE4-inhibitor, NF-Kappa-beta inhibitor, VLA-4 inhibitor, CpG, anti-CD23, selectin antagonists (TBC 1269), mast cell protease inhibitors (e.g., tryptase kinase inhibitors (e.g., GW-45, GW-58, and genisteine), phosphatidylinositide-3' (PI3)-kinase inhibitors (e.g., calphostin C), and other kinase inhibitors (e.g., staurosporine) (see Temkin et al., 2002 J Immunol 169(5):2662-2669; Vosseller et al., 1997 Mol. Biol. Cell 8(5):909-922; and Nagai et al., 1995 Biochem Biophys Res Commun 208(2):576-581)), a C3 receptor antagonists (including antibodies), immunosuppressant agents (e.g., methotrexate and gold salts), mast cell modulators (e.g., cromolyn sodium (INTAL™) and nedocromil sodium (TILADE™)), and mucolytic agents (e.g., acetylcysteine)). In a specific embodiment, the anti-inflammatory agent is a leukotriene inhibitor (e.g., montelukast (SINGULAIR™), zafirlukast (ACCOLATE™), pranlukast (ONON™), or zileuton (ZYFLO™)).

In specific embodiments, patients with allergy are administered a prophylactically or therapeutically effective amount of an effector function enhanced anti-ICOS antibody of the disclosure in combination with an effective amount of one or more other agents useful for allergy therapy. Non-limiting examples of such agents include antimediator drugs (e.g., antihistamine), corticosteroids, decongestants, sympathomimetic drugs (e.g., alpha-adrenergic and .beta-adrenergic drugs), TNX901 (Leung et al., N Engl J Med 348(11):986-993 (2003)), IgE antagonists (e.g., antibodies rhuMAb-E25 omalizumab (see Finn et al., 2003 J Allergy Clin Immuno 111(2):278-284; Corren et al., 2003 J Allergy Clin Immuno 111(1):87-90; Busse and Neaville, 2001 Curr Opin Allergy Clin Immuno 1(1):105-108; and Tang and Powell, 2001, Eur J Pediatr 160(12): 696-704), HMK-12 and 6HD5 (see Miyajima et al., 2202 Int Arch Allergy Immuno 128(1):24-32), and mAB Hu-901 (see van Neerven et al., 2001 Int Arch Allergy Immuno 124(1-3):400), theophylline and its derivatives, glucocorticoids, and immunotherapies (e.g., repeated long-term injection of allergen, short course desensitization, and venom immunotherapy).

### 5.33. AUTOIMMUNE DISEASE

According to certain aspects of the disclosure, the treatment regimen and dose used with formulations and methods of the disclosure is chosen based on a number of factors including, but not limited to, the stage of the autoimmune disease or disorder being treated. Appropriate treatment regimens can be determined by one of skill in the art for particular stages of an autoimmune disease or disorder in a patient or patient population. Dose response curves can be generated using standard protocols in the art in order to determine the effective amount of formulations of the disclosure for treating patients having different stages of an autoimmune disease or disorder. In general, patients having more activity of a autoimmune disease or disorder will require higher doses and/or more frequent doses which may be administered over longer periods of time in comparison to patients having less activity of an autoimmune disease or disorder.

Anti-ICOS antibodies, formulations and methods may be practiced to treat an autoimmune disease or disorder. The term "autoimmune disease or disorder" refers to a condition in a subject characterized by cellular, tissue and/or organ injury caused by an immunologic reaction of the subject to its own cells, tissues and/or organs. The term "inflammatory disease" is used interchangeably with the term "inflammatory disorder" to refer to a condition in a subject characterized by inflammation, including, but not limited to chronic inflammation. Autoimmune disorders may or may not be associated with inflammation. Moreover, inflammation may or may not be caused by an autoimmune disorder. Thus, certain disorders may be characterized as both autoimmune and inflammatory disorders. Exemplary autoimmune diseases or disorders include, but are not limited to: alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, diabetes, eosinophilic fascites, fibromyalgia-fibromyositis, glomerulonephritis, Graves' disease, Guillain-Barre, Hashimoto's thyroiditis, Henoch-Schonlein purpura, idiopathic pulmonary fibrosis, idiopathic/autoimmune thrombocytopenia purpura (ITP), IgA neuropathy, juvenile arthritis, lichen planus, lupus erthematosus, Ménière's disease, mixed connective tissue disease, multiple sclerosis, type 1 or immune-mediated diabetes mellitus, myasthenia gravis, pemphigus-related disorders (e.g., pemphigus vulgaris), pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, Sjogren's syndrome, stiff-man syndrome, systemic lupus erythematosus (SLE), Sweet's syndrome, Still's disease, lupus erythematosus, takayasu arteritis, temporal arteritis/ giant cell arteritis, ulcerative colitis, uveitis, vasculitides such as dermatitis herpetiformis vasculitis, vitiligo, and Wegener's granulomatosis. Examples of inflammatory disorders include, but are not limited to, asthma, encephalitis, inflammatory bowel disease, chronic obstructive pulmonary disease (COPD), allergic disorders, septic shock, pulmonary fibrosis, undifferentiated spondyloarthropathy, undifferentiated arthropathy, arthritis, inflammatory osteolysis, graft versus host disease, urticaria, Vogt-Koyanagi-Hareda syndrome and chronic inflammation resulting from chronic viral or bacteria infections.

5.33.1. Autoimmune Disorder Treatment

An effector function enhanced anti-ICOS antibody of the disclosure may be administered to a subject in need thereof to prevent, manage, treat or ameliorate an autoimmune disorder or one or more symptoms thereof. Formulations of the disclosure may also be administered in combination with one or more other therapies, preferably therapies useful for the prevention, management or treatment of an autoimmune disorder (including, but not limited to the prophylactic or therapeutic agents) to a subject in need thereof to prevent, manage, treat or ameliorate an autoimmune disorder or one or more symptoms thereof. In a specific embodiment, the disclosure provides a method of preventing, managing, treating or ameliorating an autoimmune disorder or one or more symptoms thereof, said method comprising administering to a subject in need thereof a dose of a prophylactically or therapeutically effective amount of an effector function enhanced anti-ICOS antibody of the disclosure. In another embodiment, the disclosure provides a method of preventing, managing, treating or ameliorating an autoimmune disorder or one or more symptoms thereof, said method comprising administering to a subject in need thereof a dose of a prophylactically or therapeutically effective amount of an effector function enhanced anti-ICOS antibody of the disclosure and a dose of a prophylactically or therapeutically effective amount of one or more therapies (e.g., prophylactic or therapeutic agents) other than antibodies (including antibody fragments thereof) that immunospecifically bind to an ICOS polypeptide.

The disclosure provides methods for managing, treating or ameliorating an autoimmune disorder or one or more symptoms thereof in a subject refractory to conventional therapies for such an autoimmune disorder, said methods comprising administering to said subject a dose of a prophylactically or therapeutically effective amount of an effector function enhanced anti-ICOS antibody of the disclosure. The disclosure also provides methods for managing, treating or ameliorating an autoimmune disorder or one or more symptoms thereof in a subject refractory to existing single agent therapies for such an autoimmune disorder, said methods comprising administering to said subject a dose of a prophylactically or therapeutically effective amount of an effector function enhanced anti-ICOS antibody of the disclosure and a dose of a prophylactically or therapeutically effective amount of one or more therapies (e.g., prophylactic or therapeutic agents) other than antibodies (including antibody fragments thereof) that immunospecifically bind to an ICOS polypeptide. The disclosure also provides methods for managing, treating or ameliorating an autoimmune disorder or one or more symptoms thereof by administering an effector function enhanced anti-ICOS antibody of the disclosure in combination with any other treatment to patients who have proven refractory to other treatments but are no longer on these treatments. The disclosure also provides alternative methods for the management or treatment of an autoimmune disorder where another therapy has proven or may prove too toxic, i.e., results in unacceptable or unbearable side effects, for the subject being treated. Particularly, the disclosure provides alternative methods for the management or treatment of an autoimmune disorder where the patient is refractory to other therapies. Further, the disclosure provides methods for preventing the recurrence of an autoimmune disorder in patients that have been treated and have no disease activity by administering an effector function enhanced anti-ICOS antibody of the disclosure.

Examples of autoimmune disorders that can be treated by the methods of the disclosure include, but are not limited to, alopecia greata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac sprue-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erythematosus, Mnire's disease, mixed connective tissue disease, multiple sclerosis, type 1 or immune-mediated diabetes mellitus, myasthenia gravis, pemphigus vulgaris, pernicious anemia, polyarteritis nodosa, polychrondritis, polyglandular syndromes, polymyalgia rheumatica, polymyositis and dermatomyositis, primary agammaglobulinemia, primary biliary cirrhosis, psoriasis, psoriatic arthritis, Raynauld's phenomenon, Reiter's syndrome, Rheumatoid arthritis, sarcoidosis, scleroderma, Sjogren's syndrome, stiff-man syndrome, systemic lupus erythematosus, lupus erythematosus, takayasu arteritis, temporal arterists/giant cell arteritis, ulcerative colitis, uveitis, vasculitides such as dermatitis herpetiformis vasculitis, vitiligo, and Wegener's granulomatosis.

Autoimmune therapies and their dosages, routes of administration and recommended usage are known in the art and have been described in such literature as the Physician's Desk Reference (61th ed., 2007).

5.33.2.Autoimmune Disorder Therapies

The present disclosure provides methods of preventing, managing, treating or ameliorating an autoimmune disorder or one or more symptoms thereof, said methods comprising administering to a subject in need thereof an effector function enhanced anti-ICOS antibody of the disclosure and one or more therapies (e.g., prophylactic or therapeutic agents) other than antibodies (including antibody fragments thereof) that immunospecifically bind to an ICOS polypeptide. Any agent or therapy which is known to be useful, or which has been used or is currently being used for the prevention, management, treatment or amelioration of an autoimmune disorder or one or more symptoms thereof can be used in combination with an effector function enhanced anti-ICOS antibody of the disclosure in accordance with the disclosure described herein. Examples of such agents include, but are not limited to, immunomodulatory agents, anti-inflammatory agents and TNF-alpha antagonists. Specific examples of immunomodulatory agents, anti-inflammatory agents and TNF-alpha antagonists which can be used in combination with an effector function enhanced anti-ICOS antibody of the disclosure for the prevention, management, treatment or amelioration of an autoimmune disorder are disclosed herein.

In specific embodiments, patients with multiple sclerosis (MS) are administered a prophylactically or therapeutically effective amount of an effector function enhanced anti-ICOS antibody of the disclosure in combination with other agents or therapies useful in prevention, treatment, management and amelioration of MS including but not limited to: IFN-betalb (Betaseron) (e.g., 8.0 million international unites (MIU) is administered by subcutaneous injection every other day); IFN-betala (Avonex) (e.g., 6.0 MIU is administered by intramuscular injection once every week); glatiramer acetate (Copaxone) (e.g., 20 mg is administered by subcutaneous injection every day); mitoxantrone (e.g., 12 mg/m$^2$ is administered by intravenous infusion every third month); azathioprine (e.g., 2-3 mg/kg body weight is administered orally each day); methotrexate (e.g., 7.5 mg is administered orally once each week); cyclophosphamide; intravenous immunoglobulin (e.g., 0.15-0.2 g/kg body weight administered monthly for up to 2 years); glucocorticoids; methylprednisolone (e.g., administered in bimonthly cycles at high doses); 2-chlorodeoxyadenosine (cladribine); baclofen (e.g., 15 to 80 mg/d in divided doses, or orally in higher doses up to 240 mg/d, or intrathecally via an indwelling catheter); cycloenzaprine hydrochloride (e.g., 5-10 mg bid or tid); clonazepam (e.g., 0.5 to 1.0 mg tid, including bedtime dose); clonidine hydrochloride (e.g., 0.1 to 0.2 mg tid, including a bedtime dose); carbamazepine (e.g., 100-1200 mg/d in divided, escalating doses); gabapentin (e.g., 300-3600 mg/d); dilantin (e.g., 300-400 mg/d); amitriptyline (e.g., 25-150 mg/d); baclofen (e.g., 10-80 mg/d); primidone (e.g., 125-250 mg bid or tid); ondansetron (e.g., 4 to 8 mg bid or tid); isoniazid (e.g., up to 1200 mg in divided doses); oxybutynin (e.g., 5 mg bid or tid); tolterodine (e.g., 1-2 mg bid); propantheline (e.g., 7.5 to 15 mg qid); bethanecol (e.g., 10-50 mg tid or qid); terazosin hydrochloride (e.g., 1-5 mg at bedtime); sildenafil citrate (e.g., 50-100 mg po prn); amantading (e.g., 100 mg bid); pemoline (e.g., 37.5 mg bid); high dose vitamins; calcium orotate; gancyclovir; antibiotic; and plasma exchange.

In specific embodiments, patients with psoriasis are administered a prophylactically or therapeutically effective amount of an effector function enhanced anti-ICOS antibody of the disclosure in combination with other agents or therapies useful in prevention, treatment, management and amelioration of psoriasis including but not limited to: topical steroid cream or ointment; tar (examples including but not limited to, Estar, Psorigel, Fototar cream, and LCD 10% in Nutraderm lotion or mixed directly with triamcinolone 0.1% cream); occlusion; topical vitamin D analogue (a non-limiting example is calcipotriene ointment); ultraviolet light; PUVA (psoralen plus ultraviolet A); methotrexate (e.g., up to 25 mg once weekly or in divided doses every 12 hours for three doses once a week); synthetic retinoid (a non-limiting examples is etretinate, e.g., in dosage of 0.5-1 mg/kg/d); immunomodulatory therapy (a non-limiting example is cyclosporine); sulfasalazine (e.g., in dosages of 1 g three times daily).

In specific embodiments, patients with Crohn's disease are administered a prophylactically or therapeutically effective amount of an effector function enhanced anti-ICOS antibody of the disclosure in combination with other agents or therapies useful in prevention, treatment, management and amelioration of Crohn's disease including but not limited to: antidiarrheals (e.g., loperamide 2-4 mg up to 4 times a day, diphenoxylate with atropine 1 tablet up to 4 times a day, tincture of opium 8-15 drops up to 4 times a day, cholestyramine 2-4 g or colestipol 5 g once or twice daily), antispasmodics (e.g., propantheline 15 mg, dicyclomine 10-20 mg, or hyoscyamine 0.125 mg given before meals), 5-aminosalicylic acid agents (e.g., sulfasalazine 1.5-2 g twice daily, mesalamine (ASACOL™) and its slow release form (PENTASA™), especially at high dosages, e.g., PENTASA™ 1 g four times daily and ASACOL™ 0.8-1.2 g four times daily), corticosteroids, immunomodulatory drugs (e.g., azathioprine (1-2 mg/kg), mercaptopurine (50-100 mg), cyclosporine, and methotrexate), antibiotics, TNF inhibitors (e.g., inflixmab (REMICADE™)), immunosuppressive agents (e.g., tacrolimus, mycophenolate mofetil, and thalidomide), anti-inflammatory cytokines (e.g., IL-10 and IL-11), nutritional therapies, enteral therapy with elemental diets (e.g., Vivonex for 4 weeks), and total parenteral nutrition.

In specific embodiments, patients with lupus erythematosus are administered a prophylactically or therapeutically effective amount of an effector function enhanced anti-ICOS antibody of the disclosure in combination with other agents or therapies useful in prevention, treatment, management and amelioration of lupus erythematosus including but not limited to: antimalarials (including but not limited to, hydroxychloroquine); glucocorticoids (e.g., low dose, high dose, or high-dose intravenous pulse therapy can be used); immunosuppressive agents (including but not limited to, cyclophosphamide, chlorambucil, and azanthioprine); cytotoxic agents (including but not limited to methotrexate and mycophenolate mofetil); androgenic steroids (including but not limited to danazol); anticoagulants (including but not limited to warfarin); and B-lymphocyte stimulator inhibitor (e.g. belimumab). In specific embodiments, patients with lupus erythematosus are administered a prophylactically or therapeutically effective amount of a formulation described herein in combination with belimumab.

The antibody formulations of the disclosure or combination therapies of the disclosure may be used as the first, second, third, fourth, or fifth therapy to prevent, manage, treat, and/or ameliorate an autoimmune disorder or one or more symptom thereof. The disclosure also includes methods of preventing, treating, managing, and/or ameliorating an autoimmune disorder or one or more symptoms thereof in a patient undergoing therapy for other disease or disorder. The disclosure encompasses methods of preventing, managing, treating, and/or ameliorating an autoimmune disorder or one or more symptoms thereof in a patient before any adverse effects or intolerance to therapies other than antibodies of the disclosure develops. The disclosure also encompasses methods of preventing, treating, managing, and/or ameliorating an autoimmune disorder or a symptom thereof in refractory patients. The disclosure encompasses methods for preventing, treating, managing, and/or ameliorating a proliferative disorder or a symptom thereof in a patient who has proven refractory to therapies other than antibodies, formulations, or combination therapies of the disclosure. The determination of whether a patient is refractory can be made either in vivo or in vitro by any method known in the art for assaying the effectiveness of a treatment of autoimmune disorders, using art-accepted meanings of "refractory" such a context. In certain embodiments, a patent with an autoimmune disorder is refractory to a therapy when one or more symptoms of an autoimmune disorder is not prevented, managed, and/or alleviated. The disclosure also encompasses methods of preventing, managing, treating, and/or ameliorating an autoimmune disorder or a symptom thereof in patients who are susceptible to adverse reactions to conventional therapies.

The present disclosure encompasses methods for preventing, treating, managing, and/or ameliorating an autoimmune disorder or one or more symptoms thereof as an alternative to other conventional therapies. In specific embodiments, the patient being managed or treated in accordance with the methods of the disclosure is refractory to other therapies or is susceptible to adverse reactions from such therapies. The patient may be a person with a suppressed immune system (e.g., post-operative patients, chemotherapy patients, and patients with immunodeficiency disease, patients with broncho-pulmonary dysplasia, patients with congenital heart disease, patients with cystic fibrosis, patients with acquired or congenital heart disease, and patients suffering from an infection), a person with impaired renal or liver function, the elderly, children, infants, infants born prematurely, persons with neuropsychiatric disorders or those who take psychotropic drugs, persons with histories of seizures, or persons on medication that would negatively interact with conventional agents used to prevent, manage, treat, or ameliorate an autoimmune disease or disorder.

Autoimmune therapies and their dosages, routes of administration and recommended usage are known in the art and have been described in such literature as the Physician's Desk Reference (61th ed., 2007).

5.33.3. Diagnosis of Autoimmune Diseases or Disorders

The diagnosis of an autoimmune disease or disorder is complicated in that each type of autoimmune disease or disorder manifests differently among patients. This heterogeneity of symptoms means that multiple factors are typically used to arrive at a clinical diagnosis. Generally, clinicians use factors, such as, but not limited to, the presence of autoantibodies, elevated cytokine levels, specific organ dysfunction, skin rashes, joint swelling, pain, bone remodeling, and/or loss of movement as primarily indicators of an autoimmune disease or disorder. For certain autoimmune diseases or disorders, such as RA and SLE, standards for diagnosis are known in the art. For certain autoimmune diseases or disorders, stages of disease have been characterized and are well known in the art. These art recognized methods for diagnosing autoimmune diseases and disorders as well as stages of disease and scales of activity and/or severity of disease that are well known in the art can be used to identify patients and patient populations in need of treatment for an autoimmune disease or disorder using formulations and methods of the disclosure.

5.33.4. Clinical criteria for diagnosing autoimmune diseases or disorders

Diagnostic criteria for different autoimmune diseases or disorders are known in the art. Historically, diagnosis is typically based on a combination of physical symptoms. More recently, molecular techniques such as gene-expression profiling have been applied to develop molecular definitions of autoimmune diseases or disorders. Exemplary methods for clinical diagnosis of particular autoimmune diseases or disorders are provided below. Other suitable methods will be apparent to those skilled in the art.

In certain embodiments, patients with low levels of autoimmune disease activity or patients with an early stage of an autoimmune disease (for diseases where stages are recognized) can be identified for treatment using anti-ICOS antibody formulations and methods. The early diagnosis of autoimmune disease is difficult due to the general symptoms and overlap of symptoms among diseases. In such embodiments, a patient treated at an early stage or with low levels of an autoimmune disease activity has symptoms comprising at least one symptom of an autoimmune disease or disorder. In related embodiments, a patient treated at an early stage or with low levels of an autoimmune disease has symptoms comprising at least 1, 2, 3, 4, 5, 6, 7, 8, 9, 10, 11, 12, 13, 14, or 15 symptoms of an autoimmune disease or disorder. The symptoms may be of any autoimmune diseases and disorders or a combination thereof. Examples of autoimmune disease and disorder symptoms are described below.

**5.34. IMMUNOTHERAPEUTIC PROTOCOLS**

Anti-ICOS antibody formulations used in the therapeutic regimen/protocols, referred to herein as "anti-ICOS immunotherapy" can be naked antibodies, immunoconjugates and/or fusion proteins. Formulations of the disclosure can be used as a single agent therapy or in combination with other therapeutic agents or regimens. Anti-ICOS antibodies or immunoconjugates can be administered prior to, concurrently with, or following the administration of one or more therapeutic agents. Therapeutic agents that can be used in combination therapeutic regimens with formulations of the disclosure include any substance that inhibits or prevents the function of cells and/or causes destruction of cells. Examples include, but are not limited to, radioactive isotopes, chemotherapeutic agents, and toxins such as enzymatically active toxins of bacterial, fungal, plant or animal origin, or fragments thereof.

The therapeutic regimens described herein, or any desired treatment regimen can be tested for efficacy using a transgenic animal model which expresses human ICOS antigen in place of native ICOS antigen. Thus, an anti-ICOS antibody treatment regimen can be tested in an animal model to determine efficacy before administration to a human.

**5.35. ANTI-ICOS IMMUNOTHERAPY**

In accordance with the present disclosure "anti-ICOS immunotherapy" encompasses the administration of any of the anti-ICOS antibodies of the disclosure in accordance with any therapeutic regimen described herein. Anti-ICOS antibodies can be administered as naked antibodies, or immunoconjugates or fusion proteins. In one embodiment, a human subject having a T cell-mediated disease or disorder can be treated by administering an anti-ICOS antibody capable to mediate human ADCC.

Antibodies of IgG1 or IgG3 human isotypes are in some cases preferred for therapy. However, the IgG2 or IgG4 human isotypes can be used as well, provided they have the relevant effector function, for example human ADCC. Such effector function can be assessed by measuring the ability of the antibody in question to mediate target cell lysis by effector cells *in vitro* or *in vivo.*

In one embodiment, the dose of antibody used should be sufficient to deplete circulating ICOS expressing T cells. Progress of the therapy can be monitored in the patient by analyzing blood samples. Other signs of clinical improvement can be used to monitor therapy.

Methods for measuring depletion of ICOS expressing T cells that can be used in connection with formulations and methods of the disclosure are well known in the art and include, but are not limited to the following embodiments. In one embodiment, circulating ICOS expressing T cells depletion can be measured with flow cytometry using a reagent other than an anti-ICOS antibody that binds to ICOS expressing T cells to define the amount of ICOS expressing T cells. In another embodiment, ICOS expressing T cell depletion can be measured by immunochemical staining to identify ICOS expressing T cells. In such embodiments, ICOS expressing T cells or tissues or serum comprising ICOS expressing T cells extracted from a patient can be placed on microscope slides, labeled and examined for presence or absence. In related embodiments, a comparison is made between ICOS expressing T cells extracted prior to therapy and after therapy to determine differences in the presence of ICOS expressing T cells.

In embodiments of the disclosure where an anti-ICOS antibody is administered as a single agent therapy, the disclosure contemplates use of different treatment regimens.

According to certain aspects of the disclosure, an anti-ICOS antibody used in formulations and methods of the disclosure, is a naked antibody. In related embodiments, the dose of naked anti-ICOS antibody used is at least about 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5 mg/kg of body weight of a patient. In certain embodiments, the dose of naked anti-ICOS antibody used is at least about 1 to 10, 5 to 15, 10 to 20, or 15 to 25 mg/kg of body weight of a patient. In certain embodiments, the dose of naked anti-ICOS antibody used is at least about 1 to 20, 3 to 15, or 5 to 10 mg/kg of body weight of a patient. In other embodiments, the dose of naked anti-ICOS antibody used is at least about 5, 6, 7, 8, 9, or 10 mg/kg of body weight of a patient.

In certain embodiments, the dose comprises about 375 mg/m$^2$ of anti-ICOS antibody administered weekly for about 1, 2, 3, 4, 5, 6, 7 or 8 consecutive weeks. In certain embodiments, the dose is at least about 1, 2, 3, 4, 5, 6, 7, 8, 9, 10, 11, 12, 13, 14, or 15 mg/kg of body weight of the patient administered weekly for about 1, 2, 3, 4, 5, 6, 7 or 8 consecutive weeks.

The exemplary doses of anti-ICOS antibody described above can be administered as described herein. In one embodiment, the above doses are single dose injections. In other embodiments, the doses are administered over a period of time. In other embodiments, the doses are administered multiple times over a period of time. The period of time may be measured in days, weeks, or months. Multiple doses of an anti-ICOS antibody can be administered at intervals suitable to achieve a therapeutic benefit while balancing toxic side effects. For example, where multiple doses are used, it may be preferred to time the intervals to allow for recovery of the patient's monocyte count prior to the repeat treatment with antibody. This dosing regimen will optimize the efficiency of treatment, since the monocyte population reflects ADCC function in the patient.

In certain embodiments, formulations of the disclosure are administered to a human patient as long as the patient is responsive to therapy. In other embodiments, formulations of the disclosure are administered to a human patient as long as the patient's disease does not progress. In related embodiments, formulations of the disclosure are administered to a human patient until a patient's disease does not progress or has not progressed for a period of time, then the patient is not administered formulations of the disclosure unless the disease reoccurs or begins to progress again. If disease progression stops or reverses, then he patient will not be administered formulations of the disclosure until that patient relapses, *i.e.,* the disease being treated reoccurs or progresses. Upon this reoccurrence or progression, the patient can be treated again with the same dosing regimen initially used or using other doses described above.

In certain embodiments, formulations of the disclosure can be administered as a loading dose followed by multiple lower doses (maintenance doses) over a period of time. In such embodiments, the doses may be timed and the amount adjusted to maintain effective ICOS expressing T cell depletion. In certain embodiments, the loading dose is about 10, 11, 12, 13, 14, 15, 16, 17, or 18 mg/kg of patient body weight and the maintenance dose is at least about 5 to 10 mg/kg of patient body weight. In other embodiments, the maintenance dose is administered at intervals of every 7, 10, 14 or 21 days.

The antibody compositions of the disclosure can be used in the treatment of autoimmune diseases, such as systemic lupus erythematosus (SLE), multiple sclerosis (MS), inflammatory bowel disease (IBD; including Crohn's Disease, Ulcerative Colitis and Celiac's Disease), insulin dependent diabetes mellitus (IDDM), psoriasis, autoimmune thyroiditis, rheumatoid arthritis (RA) and glomerulonephritis. Furthermore, the antibody compositions of the disclosure can be used for inhibiting or preventing transplant rejection or in the treatment of graft versus host disease (GVHD).

The liquid formulations of the present disclosure may be used locally or systemically in the body as a therapeutic. The formulations of the present disclosure may also be utilized in combination with one or more other therapies (e.g., one or more other prophylactic or therapeutic agents). When one or more other therapies (e.g., prophylactic or therapeutic agents) are used, they can be administered separately, in any appropriate form and by any suitable route. Therapeutic or prophylactic agents include, but are not limited to, small molecules, synthetic drugs, peptides, polypeptides, proteins, nucleic acids (for example, but not limited to, DNA and RNA nucleotides including, but not limited to, antisense nucleotide sequences, triple helices, RNAi, and nucleotide sequences encoding biologically active proteins, polypeptides or peptides) antibodies, synthetic or natural inorganic molecules, mimetic agents, and synthetic or natural organic molecules.

Any therapy (e.g., prophylactic or therapeutic agents) which is known to be useful, or which has been used or is currently being used for the prevention, treatment and/or management of one or more symptoms associated with a disease or disorder associated with or characterized by aberrant expression and/or activity of ICOS, a disease or disorder associated with or characterized by aberrant expression and/or activity of the ICOS receptor or one or more subunits thereof, an autoimmune disease, transplant rejection, graft versus host disease can be used in combination with the liquid antibody formulations of the present disclosure in accordance with the disclosure described herein. See, *e.g.,* Gilman et al., Goodman and Gilman's: The Pharmacological Basis of Therapeutics, Tenth Ed., McGraw-Hill, New York, 2001; The Merck Manual of Diagnosis and Therapy, Berkow, M.D. et al. (eds.), 17th Ed., Merck Sharp & Dohme Research Laboratories, Rahway, NJ, 1999; and Cecil Textbook of Medicine, 20th Ed., Bennett and Plum (eds.), W.B. Saunders, Philadelphia, 1996 for information regarding therapies, in particular prophylactic or therapeutic agents, which have been or are currently being used for preventing, treating and/or managing diseases or disorders associated with or characterized by aberrant expression and/or activity of ICOS, diseases or disorders associated with or characterized by aberrant expression and/or activity of the ICOS receptor or one or more subunits thereof, autoimmune diseases, inflammatory diseases, or one or more symptoms thereof. Examples of prophylactic and therapeutic agents include, but are not limited to, immunomodulatory agents, anti-inflammatory agents (for example, but not limited to, adrenocorticoids, corticosteroids (for example, but not limited to, beclomethasone, budesonide, flunisolide, fluticasone, triamcinolone, methlyprednisolone, prednisolone, prednisone, hydrocortisone), glucocorticoids, steroids, non-steroidal anti-inflammatory drugs (for example, but not limited to, aspirin, ibuprofen, diclofenac, and COX-2 inhibitors), and leukotreine antagonists (for example, but not limited to, montelukast, methyl xanthines, zafirlukast, and zileuton), beta2-agonists (for example, but not limited to, albuterol, biterol, fenoterol, isoetharie, metaproterenol, pirbuterol, salbutamol, terbutalin formoterol, salmeterol, and salbutamol terbutaline), anticholinergic agents (for example, but not limited to, ipratropium bromide and oxitropium bromide), sulphasalazine, penicillamine, dapsone, antihistamines, anti-malarial agents (for example, but not limited to, hydroxychloroquine), anti-viral agents, and antibiotics (for example, but not limited to, dactinomycin (formerly actinomycin), bleomycin, erythomycin, penicillin, mithramycin, and anthramycin (AMC)).

A liquid formulation of the disclosure may be administered to a human concurrently with one or more other therapies (*e.g.,* one or more other prophylactic or therapeutic agents). The term "concurrently" is not limited to the administration of prophylactic or therapeutic agents/therapies at exactly the same time, but rather it is meant that a liquid formulation of the disclosure and the other agent/therapy are administered to a mammal in a sequence and within a time interval such that the antibody (including antibody fragment thereof) that specifically binds to ICOS contained in the liquid formulation can act together with the other agent/therapy to provide an increased benefit than if they were administered otherwise.

In various embodiments, a liquid formulation of the disclosure and one or more other therapies (e.g., one or more other prophylactic or therapeutic agents), are administered less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, no more than 24 hours apart or no more than 48 hours apart. In specific embodiments, a liquid formulation of the disclosure and one or more other therapies are administered within the same patient visit. In other embodiments, a liquid formulation of the disclosure and one or more other therapies are administered at about 2 to 4 days apart, at about 4 to 6 days apart, at about 1 week part, at about 1 to 2 weeks apart, or more than 2 weeks apart. In specific embodiments, a liquid formulation of the disclosure and one or more other therapies are administered in a time frame where both agents are still active. One skilled in the art would be able to determine such a time frame by determining the half-life of the administered agents.

In certain embodiments, a liquid formulation of the disclosure and one or more other therapies (e.g., one or more other prophylactic or therapeutic agents), are cyclically administered to a subject. Cycling therapy involves the administration of a first agent for a period of time, followed by the administration of a second agent and/or third agent for a period of time and repeating this sequential administration. Cycling therapy can reduce the development of resistance to one or more of the therapies, avoid or reduce the side effects of one of the therapies, and/or improves the efficacy of the treatment.

In other embodiments, liquid formulation of the disclosure and one or more other therapies (e.g., prophylactic or therapeutic agents) are administered in metronomic dosing regimens, either by continuous infusion or frequent administration without extended rest periods. Such metronomic administration can involve dosing at constant intervals without rest periods. Typically the prophylactic or therapeutic agents, in particular cytotoxic agents, are used at lower doses. Such dosing regimens encompass the chronic daily administration of relatively low doses for extended periods of time. In specific embodiments, the use of lower doses can minimize toxic side effects and eliminate rest periods. In certain embodiments, the prophylactic and therapeutic agents are delivered by chronic low-dose or continuous infusion ranging from about 24 hours to about 2 days, to about 1 week, to about 2 weeks, to about 3 weeks to about 1 month to about 2 months, to about 3 months, to about 4 months, to about 5 months, to about 6 months.

In one embodiment, a liquid formulation of the disclosure is administered in a dosing regimen that maintains the plasma concentration of the antibody (including antibody fragment thereof) specific for ICOS at a desirable level (*e.g.,* about 0.1 to about 100 μg/ml), which maintains depletion of ICOS expressing cells. In a specific embodiment, the plasma concentration of the antibody (including antibody fragment thereof) is maintained at 0.001 μg/ml, 0.005 μg/ml, 0.01 μg/ml, 0.05 μg/ml, 0.1 μg/ml, 0.2 μg/ml, 0.5 μg/ml, 1 μg/ml, 2 μg/ml, 3 μg/ml, 4 μg/ml, 5 μg/ml, 6 μg/ml, 7 μg/ml, 8 μg/ml, 9 μg/ml, 10 μg/ml, 15 μg/ml, 20 μg/ml, 25 μg/ml, 30 μg/ml, 35 μg/ml, 40 μg/ml, 45 μg/ml or 50 μg/ml. The plasma concentration that is desirable in a subject will vary depending on several factors, including but not limited to, the nature of the disease or disorder, the severity of the disease or disorder and the condition of the subject. Such dosing regimens are especially beneficial in prevention, treatment and/or management of a chronic disease or disorder.

In one embodiment, a liquid formulation of the disclosure is administered to a subject with a disease or disorder associated with or characterized by aberrant expression and/or activity of ICOS, a disease or disorder associated with or characterized by aberrant expression and/or activity of the ICOS receptor or one or more subunits thereof, an autoimmune disease, a malignant disease, transplant rejection, graft versus host disease, or one or more symptoms thereof using a dosing regimen that maintains the plasma concentration of the an antibody (including antibody fragment thereof) that specifically binds to ICOS at a level that maintains at least 40%, at least 50%, at least 55%, at least 60%, at least 65%, at least 70%, at least 75%, at least 80%, at least 85%, at least 90% or at least 95% depletion of ICOS expressing cells. In a specific embodiment, the plasma concentration of the an antibody (including antibody fragment thereof) that specifically binds to ICOS is maintained at about 0.001 μg/ml to about 100 μg/ml in a subject with a disease or disorder associated with or characterized by aberrant expression and/or activity of ICOS, a disease or disorder associated with or characterized by aberrant expression and/or activity of the ICOS receptor or one or more subunits thereof, an autoimmune disease, a malignancy, transplant rejection, graft versus host disease, or one or more symptoms thereof.

In some embodiments, a liquid formulation of the disclosure is administered intermittently to a subject, wherein the liquid formulation comprises an antibody (including antibody fragment thereof) conjugated to a moiety.

When used in combination with other therapies (*e.g.,* prophylactic and/or therapeutic agents) the liquid formulations of the disclosure and the other therapy can act additively or synergistically. The disclosure contemplates administration of a liquid formulation of the disclosure in combination with other therapies (*e.g.,* prophylactic or therapeutic agents) by the same or different routes of administration, for example, but not limited to, oral and parenteral. In certain embodiments, when a liquid formulation of the disclosure is administered concurrently with one or more therapies (*e.g.,* prophylactic or therapeutic agents) that potentially produce adverse side effects (including, but not limited to, toxicity), the therapies (*e.g.,* prophylactic or therapeutic agents) can advantageously be administered at a dose that falls below the threshold that the adverse side effect is elicited.

## 5.36. COMBINATION WITH CHEMOTHERAPEUTIC AGENTS

Anti-ICOS immunotherapy (using naked antibody, immunoconjugates, or fusion proteins) can be used in conjunction with other therapies including but not limited to, chemotherapy, radioimmunotherapy (RIT), chemotherapy and external beam radiation (combined modality therapy, CMT), or combined modality radioimmunotherapy (CMRIT) alone or in combination, *etc.* In certain embodiments, an anti-ICOS antibody therapy of the present disclosure can be administered in conjunction with CHOP (Cyclophosphamide-Hydroxydoxorubicin-Oncovin (vincristine)-Prednisolone). As used herein, the term "administered in conjunction with" means that an anti-ICOS immunotherapy can be administered before, during, or subsequent to the other therapy employed.

In certain embodiments, an anti-ICOS immunotherapy is in conjunction with a cytotoxic radionuclide or radiotherapeutic isotope. For example, an alpha-emitting isotope such as $^{225}$Ac, $^{224}$Ac, $^{211}$At, $^{212}$Bi, $^{213}$Bi, $^{212}$Pb, $^{224}$Ra, or $^{223}$Ra. The cytotoxic radionuclide may also be a beta-emitting isotope such as $^{186}$Re, $^{188}$Re, $^{90}$Y, $^{131}$I, $^{67}$Cu, $^{177}$Lu, $^{153}$Sm, $^{166}$Ho, or $^{64}$Cu. Further, the cytotoxic radionuclide may emit Auger and low energy electrons and include the isotopes $^{125}$I, $^{123}$I or $^{77}$Br. In other embodiments the isotope may be $^{198}$Au, $^{32}$P, and the like. In certain embodiments, the amount of the radionuclide administered to the subject is between about 0.001 mCi/kg and about 10 mCi/kg.

In some embodiments, the amount of the radionuclide administered to the subject is between about 0.1 mCi/kg and about 1.0 mCi/kg. In other embodiments, the amount of the radionuclide administered to the subject is between about 0.005 mCi/kg and 0.1 mCi/kg.

In certain embodiments, an anti-ICOS immunotherapy is in conjunction with a chemical toxin or chemotherapeutic agent. The chemical toxin or chemotherapeutic agent may be selected from the group consisting of an enediyne such as calicheamicin and esperamicin; duocarmycin, methotrexate, doxorubicin, melphalan, chlorambucil, ARA-C, vindesine, mitomycin C, cis-platinum, etoposide, bleomycin and 5-fluorouracil.

Suitable chemical toxins or chemotherapeutic agents that can be used in combination therapies with an anti-ICOS immunotherapy include members of the enediyne family of molecules, such as calicheamicin and esperamicin. Chemical toxins can also be taken from the group consisting of duocarmycin (*see, e.g.,* U.S. Pat. No. 5,703,080 and U.S. Pat. No. 4,923,990), methotrexate, doxorubicin, melphalan, chlorambucil, ARA-C, vindesine, mitomycin C, cis-platinum, etoposide, bleomycin and 5-fluorouracil. Examples of chemotherapeutic agents also include Adriamycin, Doxorubicin, 5-Fluorouracil, Cytosine arabinoside ("Ara-C"), Cyclophosphamide, Thiotepa, Taxotere (docetaxel), Busulfan, Cytoxin, Taxol, Methotrexate, Cisplatin, Melphalan, Vinblastine, Bleomycin, Etoposide, Ifosfamide, Mitomycin C, Mitoxantrone, Vincreistine, Vinorelbine, Carboplatin, Teniposide, Daunomycin, Carminomycin, Aminopterin, Dactinomycin, Mitomycins, Esperamicins (*see,* U.S. Pat. No. 4,675,187), Melphalan and other related nitrogen mustards.

In other embodiments, for example, "CVB" (1.5 g/m² cyclophosphamide, 200-400 mg/m² etoposide, and 150-200 mg/m² carmustine) can be used in combination therapies of the disclosure. CVB is a regimen used to treat non-Hodgkin's lymphoma. Patti et al., Eur. J. Haematol. 51:18 (1993). Other suitable combination chemotherapeutic regimens are well-known to those of skill in the art. *See,* for example, Freedman et al., "Non-Hodgkin's Lymphomas," in CANCER MEDICINE, VOLUME 2, 3rd Edition, Holland et al. (eds.), pp. 2028-2068 (Lea & Febiger 1993). As an illustration, first generation chemotherapeutic regimens for treatment of intermediate-grade non-Hodgkin's lymphoma include C-MOPP (cyclophosphamide, vincristine, procarbazine and prednisone) and CHOP (cyclophosphamide, doxorubicin, vincristine, and prednisone). A useful second generation chemotherapeutic regimen is m-BACOD (methotrexate, bleomycin, doxorubicin, cyclophosphamide, vincristine, dexamethasone and leucovorin), while a suitable third generation regimen is MACOP-B (methotrexate, doxorubicin, cyclophosphamide, vincristine, prednisone, bleomycin and leucovorin). Additional useful drugs include phenyl butyrate and brostatin-1. In a multimodal therapy, both chemotherapeutic drugs and cytokines are co-administered with an antibody, immunoconjugate or fusion protein according to the present disclosure. The cytokines, chemotherapeutic drugs and antibody, immunoconjugate or fusion protein can be administered in any order, or together.

Other toxins that may be used in formulations and methods of the disclosure include poisonous lectins, plant toxins such as ricin, abrin, modeccin, botulina and diphtheria toxins. Of course, combinations of the various toxins could also be coupled to one antibody molecule thereby accommodating variable cytotoxicity. Illustrative of toxins which are suitably employed in combination therapies of the disclosure are ricin, abrin, ribonuclease, DNase I, Staphylococcal enterotoxin-A, pokeweed antiviral protein, gelonin, diphtherin toxin, *Pseudomonas* exotoxin, and *Pseudomonas* endotoxin. *See,* for example, Pastan et al., Cell 47:641 (1986), and Goldenberg et al., Cancer Journal for Clinicians 44:43 (1994). Enzymatically active toxins and fragments thereof which can be used include diphtheria A chain, nonbinding active fragments of diphtheria toxin, exotoxin A chain (from *Pseudomonas aeruginosa*), ricin A chain, abrin A chain, modeccin A chain, alpha-sarcin, Aleuritesfordii proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. *See,* for example, WO 93/21232 published October 28, 1993.

Suitable toxins and chemotherapeutic agents are described in REMINGTON'S PHARMACEUTICAL SCIENCES, 19th Ed. (Mack Publishing Co. 1995), and in GOODMAN AND GILMAN'S THE PHARMACOLOGICAL BASIS OF THERAPEUTICS, 7th Ed. (MacMillan Publishing Co. 1985). Other suitable toxins and/or chemotherapeutic agents are known to those of skill in the art.

An anti-ICOS immunotherapy of the present disclosure may also be in conjunction with a prodrug-activating enzyme which converts a prodrug (*e.g.,* a peptidyl chemotherapeutic agent, *see,* WO81/01145) to an active anti-cancer drug. *See,* for example, WO 88/07378 and U.S. Patent No. 4,975,278. The enzyme component of such combinations includes any enzyme capable of acting on a prodrug in such a way so as to covert it into its more active, cytotoxic form. The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al. (ed.), pp. 247-267, Humana Press (1985). Prodrugs that can be used in combination with anti-ICOS antibodies include, but are not limited to, phosphate-containing prodrugs, thiophosphate-containing prodrugs, sulfate-containing prodrugs, peptide-containing prodrugs, D-amino acid-modified prodrugs, glycosylated prodrugs, $\alpha$-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this disclosure include, but are not limited to, those chemotherapeutic agents described above.

In certain embodiments, administration of formulations and methods of the disclosure may enable the postponement of toxic therapy and may help avoid unnecessary side effects and the risks of complications associated with chemotherapy and delay development of resistance to chemotherapy. In certain embodiments, toxic therapies and/or resistance to toxic therapies is delayed in patients administered formulations and methods of the disclosure delay for up to about 6 months, 1, 2, 3, 4, 5, 6, 7, 8, 9, or 10 years.

**5.37. COMBINATION WITH THERAPEUTIC ANTIBODIES**

An anti-ICOS immunotherapy described herein may be administered in combination with other antibodies, including, but not limited to, anti-CD 19 mAb, anti-CD52 mAb, anti-CD22 antibody, and anti-CD20 antibodies, such as RITUXAN™ (C2B8; RITUXIMAB™; IDEC Pharmaceuticals). Other examples of therapeutic antibodies that can be used in combination with antibodies of the disclosure or used in formulations of the disclosure include, but are not limited to, HERCEPTIN™ (Trastuzumab; Genentech), MYLOTARG™ (Gemtuzumab ozogamicin; Wyeth Pharmaceuticals), CAMPATH™ (Alemtuzumab; Berlex), ZEVALIN™ (Ipritumomab tiuxetan; Biogen Idec), BEXXAR™ (Tositumomab; GlaxoSmithKline Corixa), ERBITUX™ (Cetuximab; Imclone), and AVASTIN™ (Bevacizumab; Genentech).

**5.38. COMBINATION COMPOUNDS THAT ENHANCE MONOCYTE OR MACROPHAGE FUNCTION**

In certain embodiments of methods of the disclosure, a compound that enhances monocyte or macrophage function (*e.g.,* at least about 25%, 50%, 75%, 85%, 90%, 95% or more) can be used in conjunction with an anti-ICOS immunotherapy. Such compounds are known in the art and include, without limitation, cytokines such as interleukins (*e.g.,* IL-12), and interferons (*e.g.,* alpha or gamma interferon).

The compound that enhances monocyte or macrophage function or enhancement can be formulated in the same pharmaceutical formulation as the antibody, immunoconjugate or antigen-binding fragment. When administered separately, the antibody/fragment and the compound can be administered concurrently (within a period of hours of each other), can be administered during the same course of therapy, or can be administered sequentially (*i.e.,* the patient first receives a course of the antibody/fragment treatment and then a course of the compound that enhances macrophage/monocyte function or vice versa). In such embodiments, the compound that enhances monocyte or macrophage function is administered to the human subject prior to, concurrently with, or following treatment with other therapeutic regimens and/or formulations of the disclosure. In one embodiment, the human subject has a blood leukocyte, monocyte, neutrophil, lymphocyte, and/or basophil count that is within the normal range for humans. Normal ranges for human blood leukocytes (total) is about 3.5- about 10.5 ($10^9$/L). Normal ranges for human blood neutrophils is about 1.7- about 7.0 ($10^9$/L), monocytes is about 0.3- about 0.9 ($10^9$/L), lymphocytes is about 0.9- about 2.9 ($10^9$/L), basophils is about 0- about 0.3 ($10^9$/L), and eosinophils is about 0.05- about 0.5 ($10^9$/L). In other embodiments, the human subject has a blood leukocyte count that is less than the normal range for humans, for example at least about 0.01, 0.05, 0.1, 0.2, 0.3, 0.4, 0.5, 0.6, 0.7, or 0.8 ($10^9$/L) leukocytes.

## 5.39. COMBINATION WITH IMMUNOREGULATORY AGENTS

The anti-ICOS immunotherapy of the present disclosure may also be in conjunction with an immunoregulatory agent. The term "immunoregulatory agent" as used herein for combination therapy refers to substances that act to suppress, mask, or enhance the immune system of the host.

Examples of immunomodulatory agents include, but are not limited to, proteinaceous agents such as cytokines, peptide mimetics, and antibodies (e.g., human, humanized, chimeric, monoclonal, polyclonal, Fvs, ScFvs, Fab or F(ab)$_2$ fragments or epitope binding fragments), nucleic acid molecules (e.g., antisense nucleic acid molecules, RNAi and triple helices), small molecules, organic compounds, and inorganic compounds. In particular, immunomodulatory agents include, but are not limited to, methothrexate, leflunomide, cyclophosphamide, cytoxan, Immuran, cyclosporine A, minocycline, azathioprine, antibiotics (e.g., FK506 (tacrolimus)), methylprednisolone (MP), corticosteroids, steriods, mycophenolate mofetil, rapamycin (sirolimus), mizoribine, deoxyspergualin, brequinar, malononitriloamindes (e.g., leflunamide), T cell receptor modulators, and cytokine receptor modulators. Examples of immunosupressant, include, but are not limited to, mycophenolate mofetil (CELLCEPT™), D-penicillamine (CUPRIMINE™, DEPEN™), methotrexate (RHEUMATREX™, TREXALL™), and hydroxychloroquine sulfate (PLAQUENIL™).

Immunomodulatory agents would also include substances that suppress cytokine production, downregulate or suppress self-antigen expression, or mask the MHC antigens. Examples of such agents include 2-amino-6-aryl-5-substituted pyrimidines (see, U.S. Pat. No. 4,665,077), azathioprine (or cyclophosphamide, if there is an adverse reaction to azathioprine); bromocryptine; glutaraldehyde (which masks the MHC antigens, as described in U.S. Pat. No. 4,120,649); anti-idiotypic antibodies for MHC antigens and MHC fragments; cyclosporin A; steroids such as glucocorticosteroids, e.g., prednisone, methylprednisolone, and dexamethasone; cytokine or cytokine receptor antagonists including anti-interferon-gamma, -beta, or -alpha antibodies; anti-tumor necrosis factor-alpha antibodies; anti-tumor necrosis factor-beta antibodies; anti-interleukin-2 antibodies and anti-IL-2 receptor antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 90/08187 published Jul. 26, 1990); streptokinase; TGF-.beta.; streptodomase; RNA or DNA from the host; FK506; RS-61443; deoxyspergualin; rapamycin; T-cell receptor (U.S. Pat. No. 5,114,721); T-cell receptor fragments (Offner et al., Science 251:430-432 (1991); WO 90/11294; and WO 91/01133); and T-Cell receptor antibodies (EP 340,109) such as T10B9.

Examples of cytokines include, but are not limited to lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha; mullerian-inhibiting substance; mouse gonadotropin-associated peptide; inhibin; activin; vascular endothelial growth factor; integrin; thrombopoietin (TPO); nerve growth factors such as NGF-alpha; platelet-growth factor; transforming growth factors (TGFs) such as TGF-alpha and TGF-alpha; insulin-like growth factor-I and -II; erythropoietin (EPO); osteoinductive factors; interferons; colony stimulating factors (CSFs) such as macrophage-CSF (M-CSF); granulocyte-macrophage-CgP (GM-CSP); and granulocyte-CSF (G-CSF); interleukins (ILs) such as IL-1, IL-1a, IL-2, 1L-3, IL-4, IL-5, IL-6, IL-7, IL-8, IL-9, IL-11, IL-12, IL-15; a tumor necrosis factor such as TNF-alpha or TNF-beta; and other polypeptide factors including LIF and kit ligand (KL). As used herein, the term cytokine includes proteins from natural sources or from recombinant cell culture and biologically active equivalents of the native sequence cytokines. In certain embodiments, the methods further include administering to the subject one or more immunomodulatory agents, preferably a cytokine. Preferred cytokines are selected from the group consisting of interleukin-1 (IL-1), IL-2, IL-3, IL-12, IL-15, IL-18, G-CSF, GM-CSF, thrombopoietin, and gamma interferon.

In certain embodiments, the immunomodulatory agent is a cytokine receptor modulator. Examples of cytokine receptor modulators include, but are not limited to, soluble cytokine receptors (e.g., the extracellular domain of a TNF-alpha receptor or a fragment thereof, the extracellular domain of an IL-1beta receptor or a fragment thereof, and the extracellular domain of an IL-6 receptor or a fragment thereof), cytokines or fragments thereof (e.g., interleukin (IL)-2, IL-3, IL-4, IL-5, IL-6, IL-7, IL-8, IL-9, IL-10, IL-11, IL-12, IL-15, TNF-alpha, TNF-beta, interferon (IFN)-alpha, IFN-beta, IFN-gamma, and GM-CSF), anti-cytokine receptor antibodies (e.g., anti-IL-2 receptor antibodies, anti-IL-4 receptor antibodies, anti-IL-6 receptor antibodies, anti-IL-10 receptor antibodies, and anti-IL-12 receptor antibodies), anti-cytokine antibodies (e.g., anti-IFN receptor antibodies, anti-TNF-alpha antibodies, anti-IL-lbeta antibodies, anti-IL-6 antibodies, anti-IL-9, anti-IL-17 antibodies, antibodies, and anti-IL-12 antibodies). In a specific embodiment, a cytokine receptor modulator is IL-4, IL-10, or a fragment thereof. In another embodiment, a cytokine receptor modulator is an anti-IL-lbeta antibody, anti-IL-6 antibody, anti-IL-12 receptor antibody, anti-TNF-alpha antibody. In another embodiment, a cytokine receptor modulator is the extracellular domain of a TNF-alpha receptor or a fragment thereof. In certain embodiments, a cytokine receptor modulator is not a TNF-alpha antagonist.

In certain embodiments, the immunomodulatory agent is a T cell receptor modulator. Examples of T cell receptor modulators include, but are not limited to, anti-T cell receptor antibodies (e.g., anti-CD4 antibodies (e.g., cM-T412 (Boeringer), IDEC-CE9.1 (IDEC and SKB), mAB 4162W94, Orthoclone and OKTcdr4a (Janssen-Cilag)), anti-CD3 antibodies, anti-CD5 antibodies (e.g., an anti-CD5 ricin-linked immunoconjugate), anti-CD7 antibodies (e.g., CHH-380 (Novartis)), anti-CD8 antibodies, anti-CD40 ligand monoclonal antibodies, anti-CD52 antibodies (e.g., CAMPATH 1H (Ilex)), anti-CD2 monoclonal antibodies) and CTLA4-immunoglobulin.

In certain embodiments, the immunomodulatory agent is a TNF-alpha antagonist. Examples of TNF-alpha antagonists include, but are not limited to, antibodies (e.g., infliximab (REMICADE™; Centocor), D2E7 (Abbott Laboratories/Knoll Pharmaceuticals Co., Mt. Olive, N.J.), CDP571 which is also known as HUMIRA™ and CDP-870 (both of Celltech/Pharmacia, Slough, U.K.), and TN3-19.12 (Williams et al., 1994, Proc. Natl. Acad. Sci. USA 91: 2762-2766; Thorbecke et al., 1992, Proc. Natl. Acad. Sci. USA 89:7375-7379)) soluble TNF-alpha receptors (e.g., sTNF-R1 (Amgen), etanercept (ENBREL™; Immunex) and its rat homolog RENBREL™, soluble inhibitors of TNF-alpha derived from TNFrI, TNFrII (Kohno et al., 1990, Proc. Natl. Acad. Sci. USA, 87:8331-8335), and TNF-alpha Inh (Seckinger et al, 1990, Proc. Natl. Acad. Sci. USA, 87:5188-5192)), IL-10, TNFR-IgG (Ashkenazi et al., 1991, Proc. Natl. Acad. Sci. USA, 88:10535-10539), the murine product TBP-1 (Serono/Yeda), the vaccine CytoTAb (Protherics), antisense molecule 104838 (ISIS), the peptide RDP-58 (SangStat), thalidomide (Celgene), CDC-801 (Celgene), DPC-333 (Dupont), VX-745 (Vertex), AGIX-4207 (AtheroGenics), ITF-2357 (Italfarmaco), NPI-13021-31 (Nereus), SCIO-469 (Scios), TACE targeter (Immunix/AHP), CLX-120500 (Calyx), Thiazolopyrim (Dynavax), auranofin (Ridaura) (SmithKline Beecham Pharmaceuticals), quinacrine (mepacrine dichlorohydrate), tenidap (Enablex), Melanin (Large Scale Biological), and anti-p38 MAPK agents by Uriach.

An anti-ICOS immunotherapy may also be in conjunction with an immunoregulatory agent. In this approach, a chimeric, human or humanized anti-ICOS antibody can be used. The term "immunoregulatory agent" as used herein for combination therapy refers to substances that act to suppress, mask, or enhance the immune system of the host. This would include substances that suppress cytokine production, downregulate or suppress self-antigen expression, or mask the MHC antigens. Examples of such agents include 2-amino-6-aryl-5-substituted pyrimidines (*see,* U.S. Pat. No. 4,665,077), azathioprine (or cyclophosphamide, if there is an adverse reaction to azathioprine); bromocryptine; glutaraldehyde (which masks the MHC antigens, as described in U.S. Pat. No. 4,120,649); anti-idiotypic antibodies for MHC antigens and MHC fragments; cyclosporin A; steroids such as glucocorticosteroids, *e.g.,* prednisone, methylprednisolone, and dexamethasone; cytokine or cytokine receptor antagonists including anti-interferon-γ, -β, or -α antibodies; anti-tumor necrosis factor-a antibodies; anti-tumor necrosis factor-β antibodies; anti-interleukin-2 antibodies and anti-IL-2 receptor antibodies; anti-L3T4 antibodies; heterologous anti-lymphocyte globulin; pan-T antibodies, for example anti-CD3 or anti-CD4/CD4a antibodies; soluble peptide containing a LFA-3 binding domain (WO 90/08187 published Jul. 26, 1990); streptokinase; TGF-β; streptodornase; RNA or DNA from the host; FK506; RS-61443; deoxyspergualin; rapamycin; T-cell receptor (U.S. Pat. No. 5,114,721); T-cell receptor fragments (Offner et al., Science 251:430-432 (1991); WO 90/11294; and WO 91/01133); and T-cell receptor antibodies (EP 340,109) such as T10B9. Examples of cytokines include, but are not limited to lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor -α; mullerian-inhibiting substance; mouse gonadotropin-associated peptide; inhibin; activin; vascular endothelial growth factor; integrin; thrombopoiotin (TPO);

nerve growth factors such as NGF-α; platelet-growth factor; transforming growth factors (TGFs) such as TGF-α and TGF- α; insulin-like growth factor-I and -II; erythropoietin (EPO); osteoinductive factors; interferons; colony stimulating factors (CSFs) such as macrophage-CSF (M-CSF); granulocyte-macrophage-CgP (GM-CSP); and granulocyte-CSF (G-CSF); interleukins (ILs) such as IL-1, IL-Ia, IL-2, 1L-3, IL-4, IL-5, IL-6, IL-7, IL-8, IL-9, IL-1 I, IL-12, IL-15; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines. In certain embodiments, the methods further include administering to the subject one or more immunomodulatory agents, for example a cytokine. Suitable cytokines may be selected from the group consisting of interleukin-1 (IL-1), IL-2, IL-3, IL-12, IL-15, IL-18, G-CSF, GM-CSF, thrombopoietin, and γ interferon.

These immunoregulatory agents are administered at the same time or at separate times from anti-ICOS antibodies. The preferred immunoregulatory agent will depend on many factors, including the type of disorder being treated, as well as the patient's history, but the agent frequently may be selected from cyclosporin A, a glucocorticosteroid (for example prednisone or methylprednisolone), azathioprine, bromocryptine, heterologous anti-lymphocyte globulin, or a mixture thereof.

**5.40.** COMBINATION WITH OTHER THERAPEUTIC AGENTS

Agents that act on the tumor neovasculature can also be used in conjunction with anti-ICOS immunotherapy and include tubulin-binding agents such as combrestatin A4 (Griggs et al., Lancet Oncol. 2:82, (2001)) and angiostatin and endostatin (reviewed in Rosen, Oncologist 5:20 (2000)). Immunomodulators suitable for use in combination with anti-ICOS antibodies include, but are not limited to, of α-interferon, γ-interferon, and tumor necrosis factor alpha (TNFα). In certain embodiments, the therapeutic agents used in combination therapies using formulations and methods of the disclosure are peptides.

In certain embodiments, an anti-ICOS immunotherapy is in conjunction with one or more calicheamicin molecules. The calicheamicin family of antibiotics are capable of producing double-stranded DNA breaks at sub-picomolar concentrations. Structural analogues of calicheamicin which may be used include, but are not limited to, $\gamma1^I$, $\gamma2^I$, $\gamma3^I$, N-acetyl-$\gamma1^I$, PSAG and 011 Hinman et al., Cancer Research 53:3336-3342 (1993) and Lode et al., Cancer Research 58: 2925-2928 (1998)).

In certain embodiments, a treatment regimen includes compounds that mitigate the cytotoxic effects of an anti-ICOS antibody formulation. Such compounds include analgesics (*e.g.,* acetaminophen), bisphosphonates, antihistamines (*e.g.,* chlorpheniramine maleate), and steroids (*e.g.,* dexamethasone, retinoids, deltoids, betamethasone, cortisol, cortisone, prednisone, dehydrotestosterone, glucocorticoids, mineralocorticoids, estrogen, testosterone, progestins).

In certain embodiments, the therapeutic agent used in combination with an anti-ICOS immunotherapy is a small molecule (*i.e.,* inorganic or organic compounds having a molecular weight of less than about 2500 daltons). For example, libraries of small molecules may be commercially obtained from Specs and BioSpecs B.V. (Rijswijk, The Netherlands), Chembridge Corporation (San Diego, CA), Comgenex USA Inc. (Princeton, NJ), and Maybridge Chemicals Ltd. (Cornwall PL34 OHW, United Kingdom).

In certain embodiments an anti-ICOS immunotherapy can be administered in combination with an anti-bacterial agent. Non-limiting examples of anti-bacterial agents include proteins, polypeptides, peptides, fusion proteins, antibodies, nucleic acid molecules, organic molecules, inorganic molecules, and small molecules that inhibit and/or reduce a bacterial infection, inhibit and/or reduce the replication of bacteria, or inhibit and/or reduce the spread of bacteria to other cells or subjects. Specific examples of anti-bacterial agents include, but are not limited to, antibiotics such as penicillin, cephalosporin, imipenem, axtreonam, vancomycin, cycloserine, bacitracin, chloramphenicol, erythromycin, clindamycin, tetracycline, streptomycin, tobramycin, gentamicin, amikacin, kanamycin, neomycin, spectinomycin, trimethoprim, norfloxacin, rifampin, polymyxin, amphotericin B, nystatin, ketoconazole, isoniazid, metronidazole, and pentamidine.

In certain embodiments an anti-ICOS immunotherapy can be administered in combination with an anti-fungal agent. Specific examples of anti-fungal agents include, but are not limited to, azole drugs (*e.g.,* miconazole, ketoconazole (NIZORAL®), caspofungin acetate (CANCIDAS®), imidazole, triazoles (*e.g.,* fluconazole (DIFLUCAN®)), and itraconazole (SPORANOX®)), polyene (*e.g.,* nystatin, amphotericin B (FUNGIZONE®), amphotericin B lipid complex ("ABLC") (ABELCET®), amphotericin B colloidal dispersion ("ABCD") (AMPHOTEC®), liposomal amphotericin B (AMBISONE®)), potassium iodide (KI), pyrimidine (*e.g.,* flucytosine (ANCOBON®), and voriconazole (VFEND®)). Administration of anti bacterial and anti-fungal agents can mitigate the effects or escalation of infectious disease that may occur in methods of the disclosure where a patient's ICOS expressing T cells are significantly depleted.

In certain embodiments of the disclosure, an anti-ICOS immunotherapy can be administered in combination with one or more of the agents described above to mitigate the toxic side effects that may accompany administration of formulations of the disclosure. In other embodiments, an anti-ICOS immunotherapy can be administered in combination with one or more agents that are well known in the art for use in mitigating the side effects of antibody administration, chemotherapy, toxins, or drugs.

In embodiments of the disclosure where an anti-ICOS immunotherapy is administered in combination with another antibody or antibodies and/or agent, the additional antibody or antibodies and/or agents can be administered in any sequence relative to the administration of the antibody of this disclosure. For example, the additional antibody or antibodies can be administered before, concurrently with, and/or subsequent to administration of an anti-ICOS antibody or immunoconjugate to the human subject. The additional antibody or antibodies can be present in the same pharmaceutical formulation as an antibody of the disclosure, and/or present in a different pharmaceutical formulation. The dose and mode of administration of an antibody of this disclosure and the dose of the additional antibody or antibodies can be the same or different, in accordance with any of the teachings of dosage amounts and modes of administration as provided in this application and as are well known in the art.

### 5.41. USE OF ANTI-ICOS ANTIBODIES IN DIAGNOSING T CELL MALIGNANCIES

The present disclosure also encompasses anti-ICOS antibodies, and formulations thereof, that immunospecifically bind to the human ICOS antigen, which anti-ICOS antibodies are conjugated to a diagnostic or detectable agent. In certain embodiments, the antibodies are anti-ICOS antibodies with enhanced effector function. Such anti-ICOS antibodies can be useful for monitoring or prognosing the development or progression of a T cell malignancy as part of a clinical testing procedure, such as determining the efficacy of a particular therapy. Such diagnosis and detection can be accomplished by coupling an anti-ICOS antibody that immunospecifically binds to the human ICOS antigen to a detectable substance including, but not limited to, various enzymes, such as but not limited to, horseradish peroxidase, alkaline phosphatase, beta-galactosidase, or acetylcholinesterase; prosthetic groups, such as but not limited to, streptavidinlbiotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, luminol; bioluminescent materials, such as but not limited to, luciferase, luciferin, and aequorin; radioactive materials, such as but not limited to iodine ($^{131}$I, $^{125}$I, $^{123}$I, $^{121}$I,), carbon ($^{14}$C), sulfur ($^{35}$S), tritium ($^{3}$H), indium ($^{115}$In, $^{113}$In, $^{112}$In, $^{111}$In,), and technetium ($^{99}$Tc), thallium ($^{201}$Ti), gallium ($^{68}$Ga, $^{67}$Ga), palladium ($^{103}$Pd), molybdenum ($^{99}$Mo), xenon ($^{133}$Xe), fluorine ($^{18}$F), $^{153}$Sm, $^{177}$Lu, $^{159}$Gd, $^{149}$Pm, $^{140}$La, $^{175}$Yb , $^{166}$Ho, $^{90}$Y, $^{47}$Sc, $^{186}$Re, $^{188}$Re, $^{142}$Pr, $^{105}$Rh, $^{97}$Ru, $^{68}$Ge, $^{57}$Co, $^{65}$Zn, $^{85}$Sr, $^{32}$P, $^{153}$Gd, $^{169}$Yb, $^{51}$Cr, $^{54}$Mn, $^{75}$Se, $^{113}$Sn, and $^{117}$Tin; positron emitting metals using various positron emission tomographies, nonradioactive paramagnetic metal ions, and molecules that are radiolabelled or conjugated to specific radioisotopes. Any detectable label that can be readily measured can be conjugated to an anti-ICOS antibody and used in diagnosing T cell malignancies. The detectable substance may be coupled or conjugated either directly to an antibody or indirectly, through an intermediate (such as, for example, a linker known in the art) using techniques known in the art. *See, e.g.,* U.S. Patent No. 4,741,900 for metal ions which can be conjugated to antibodies for use as a diagnostics according to the present disclosure. In certain embodiments, the disclosure provides for diagnostic kits comprising an anti-ICOS antibody conjugated to a diagnostic or detectable agent.

### 5.42. USE OF ANTI-ICOS ANTIBODIES IN MONITORINIG IMMUNE RECONSTITUION

The present disclosure also encompasses anti-ICOS antibodies, and formulations thereof, that immunospecifically bind to the human ICOS antigen, which anti-ICOS antibodies are conjugated to a diagnostic or detectable agent. Such anti-ICOS antibodies can be useful for monitoring immune system reconstitution following immunosuppressive therapy or bone marrow transplantation. Such monitoring can be accomplished by coupling an anti-ICOS antibody that immunospecifically binds to the human ICOS antigen to a detectable substance including, but not limited to, various enzymes, such as, but not limited to, horseradish peroxidase, alkaline phosphatase, beta-galactosidase, or acetylcholinesterase; prosthetic groups, such as, but not limited to, streptavidinlbiotin and avidin/biotin; fluorescent materials, such as, but not limited to, umbelliferone, fluorescein, fluorescein isothiocynate, rhodamine, dichlorotriazinylamine fluorescein, dansyl chloride or phycoerythrin; luminescent materials, such as, but not limited to, luminol; bioluminescent materials, such as, but not limited to, luciferase, luciferin, and aequorin; radioactive materials, such as, but not limited to, iodine ($^{131}$I, $^{125}$I, $^{123}$I, $^{121}$I,), carbon ($^{14}$C), sulfur ($^{35}$S), tritium ($^{3}$H), indium ($^{115}$In, $^{113}$In, $^{112}$In, $^{111}$In,), and technetium ($^{99}$Tc), thallium ($^{201}$Ti), gallium ($^{68}$Ga, $^{67}$Ga), palladium ($^{103}$Pd), molybdenum ($^{99}$Mo), xenon ($^{133}$Xe), fluorine ($^{18}$F), $^{153}$Sm, $^{177}$Lu, $^{159}$Gd, $^{149}$Pm, $^{140}$La, $^{175}$Yb , $^{166}$Ho, $^{90}$Y, $^{47}$Sc, $^{186}$Re, $^{188}$Re, $^{142}$Pr, $^{105}$Rh, $^{97}$Ru, $^{68}$Ge, $^{57}$Co, $^{65}$Zn, $^{85}$Sr, $^{32}$P, $^{153}$Gd, $^{169}$Yb, $^{51}$Cr, $^{54}$Mn, $^{75}$Se, $^{113}$Sn, and $^{117}$Tin; positron-emitting metals using various positron-emission tomographies, nonradioactive paramagnetic metal ions, and molecules that are radiolabelled or conjugated to specific radioisotopes. Any detectable label that can be readily measured can be conjugated to an anti-ICOS antibody and used in diagnosing an autoimmune disease or disorder. The detectable substance may be coupled or conjugated either directly to an antibody or indirectly, through an intermediate (such as, for example, a linker known in the art) using techniques known in the art. *See, e.g.,* U.S. Patent No. 4,741,900 for metal ions which can be conjugated to antibodies for use as a diagnostics according to the present disclosure. In certain embodiments, the disclosure provides for diagnostic kits comprising an anti-ICOS antibody conjugated to a diagnostic or detectable agent.

**5.43.** USE OF ANTI-ICOS ANTIBODIES IN DIAGNOSING AUTOIMMUNE DISEASES OR DISORDERS

The present disclosure also encompasses anti-ICOS antibodies, and formulations thereof, that immunospecifically bind to the human ICOS antigen, which anti-ICOS antibodies are conjugated to a diagnostic or detectable agent. In certain embodiments, the antibodies are anti-ICOS antibodies with enhanced effector function. Such anti-ICOS antibodies can be useful for monitoring or prognosing the development or progression of an autoimmune disease or disorder as part of a clinical testing procedure, such as determining the efficacy of a particular therapy. Such diagnosis and detection can be accomplished by coupling an anti-ICOS antibody that immunospecifically binds to the human ICOS antigen to a detectable substance including, but not limited to, various enzymes, such as but not limited to, horseradish peroxidase, alkaline phosphatase, beta-galactosidase, or acetylcholinesterase; prosthetic groups, such as but not limited to, streptavidinlbiotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, luminol; bioluminescent materials, such as but not limited to, luciferase, luciferin, and aequorin; radioactive materials, such as but not limited to iodine ($^{131}$I, $^{125}$I, $^{123}$I, $^{121}$I,), carbon ($^{14}$C), sulfur ($^{35}$S), tritium ($^{3}$H), indium ($^{115}$In, $^{113}$In, $^{112}$In, $^{111}$In,), and technetium ($^{99}$Tc), thallium ($^{201}$Ti), gallium ($^{68}$Ga, $^{67}$Ga), palladium ($^{103}$Pd), molybdenum ($^{99}$Mo), xenon ($^{133}$Xe), fluorine ($^{18}$F), $^{153}$Sm, $^{177}$Lu, $^{159}$Gd, $^{149}$Pm, $^{140}$La, $^{175}$Yb, $^{166}$Ho, $^{90}$Y, $^{47}$Sc, $^{186}$Re, $^{188}$Re, $^{142}$Pr, $^{105}$Rh, $^{97}$Ru, $^{68}$Ge, $^{57}$Co, $^{65}$Zn, $^{85}$Sr, $^{32}$P, $^{153}$Gd, $^{169}$Yb, $^{51}$Cr, $^{54}$Mn, $^{75}$Se, $^{113}$Sn, and $^{117}$Tin; positron emitting metals using various positron emission tomographies, noradioactive paramagnetic metal ions, and molecules that are radiolabelled or conjugated to specific radioisotopes. Any detectable label that can be readily measured can be conjugated to an anti-ICOS antibody and used in diagnosing an autoimmune disease or disorder. The detectable substance may be coupled or conjugated either directly to an antibody or indirectly, through an intermediate (such as, for example, a linker known in the art) using techniques known in the art. *See, e.g.,* U.S. Patent No. 4,741,900 for metal ions which can be conjugated to antibodies for use as a diagnostics according to the present disclosure. In certain embodiments, the disclosure provides for diagnostic kits comprising an anti-ICOS antibody conjugated to a diagnostic or detectable agent.

**5.44.** Kits

The disclosure provides a pharmaceutical pack or kit comprising one or more containers filled with a liquid formulation of the disclosure. In one embodiment, a container filled with a liquid formulation of the disclosure is a prefilled syringe. In a specific embodiment, the liquid formulations of the disclosure comprise antibodies (including antibody fragments thereof) recombinantly fused or chemically conjugated to another moiety, including but not limited to, a heterologous protein, a heterologous polypeptide, a heterologous peptide, a large molecule, a small molecule, a marker sequence, a diagnostic or detectable agent, a therapeutic moiety, a drug moiety, a radioactive metal ion, a second antibody, and a solid support. The disclosure also provides a pharmaceutical pack or kit comprising in one or more first containers a liquid formulation of the disclosure and in one or more second containers one or more other prophylactic or therapeutic agents useful for the prevention, management or treatment of a disease or disorder, for example, a disease or disorder associated with or characterized by aberrant expression and/or activity of ICOS, a disease or disorder associated with or characterized by aberrant expression and/or activity of ICOS receptor, an autoimmune disease or disorder, an inflammatory disease or disorder, a T cell proliferative disease or disorder, a T cell malignancy, transplant rejection, graft versus host disease, or one or more symptoms thereof. In a specific embodiment, the liquid formulations of the disclosure are formulated in single dose vials as a sterile liquid containing 10 mM histidine buffer at pH 6.0, 80 mM NaCl, 4% trehalose and 0.02% Polysorbate 80. The formulations of the disclosure may be supplied in 3 cc USP Type I borosilicate amber vials (West Pharmaceutical Serices - Part No. 6800-0675) with a target volume of 1.2 mL. Optionally associated with such container(s) can be a notice in the form prescribed by a governmental agency regulating the manufacture, use or sale of pharmaceuticals or biological products, which notice reflects approval by the agency of manufacture, use or sale for human administration. In another embodiment, a formulation of the disclosure may be supplied in a pre-filled syringe.

In one embodiment, a container filled with a liquid formulation of the disclosure is a pre-filled syringe. Any pre-filled syringe known to one of skill in the art may be used in combination with a liquid formulation of the disclosure. Pre-filled syringes that may be used are described in, for example, but not limited to, PCT Publications WO05032627, WO08094984, WO9945985, WO03077976, US Patents US6792743, US5607400, US5893842, US7081107, US7041087, US5989227, US6807797, US6142976, US5899889, US Patent Publications US20070161961A1, US20050075611A1, US20070092487A1, US20040267194A1, US20060129108A1. Pre-filled syringes may be made of various materials. In one embodiment a pre-filled syringe is a glass syringe. In another embodiment a pre-filled syringe is a plastic syringe. One of skill in the art understands that the nature and/or quality of the materials used for manufacturing the syringe may influence the stability of a protein formulation stored in the syringe. For example, it is understood that silicon based lubricants deposited on the inside surface of the syringe chamber may affect particle formation in the protein formulation. In one embodiment, a pre-filled syringe comprises a silicone based lubricant. In one embodiment, a pre-filled syringe comprises baked on silicone. In another embodiment, a pre-filled syringe is free from silicone based lubricants. One of skill in the art also understands that small amounts of contaminating elements leaching into the formulation from the syringe barrel, syringe tip cap, plunger or stopper may also influence stability of the formulation. For example, it is understood that tungsten introduced during the manufacturing process may adversely affect formulation stability. In one embodiment, a pre-filled syringe may comprise tungsten at a level above 500 ppb. In another embodiment, a pre-filled syringe is a low tungsten syringe. In another embodiment, a pre-filled syringe may comprise tungsten at a level between about 500 ppb and about 10 ppb, between about 400 ppb and about 10 ppb, between about 300 ppb and about 10 ppb, between about 200 ppb and about 10 ppb, between about 100 ppb and about 10 ppb, between about 50 ppb and about 10 ppb, between about 25 ppb and about 10 ppb.

The present disclosure provides kits that can be used in the above methods. In one embodiment, a kit comprises a liquid formulation of the disclosure, in one or more containers. In another embodiment, a kit comprises a liquid formulation of the disclosure, in one or more containers, and one or more other prophylactic or therapeutic agents useful for the prevention, management or treatment of a disease or disorder associated with or characterized by aberrant expression and/or activity of ICOS, a disease or disorder associated with or characterized by aberrant expression and/or activity of ICOS receptor, an autoimmune disease or disorder, an inflammatory disease or disorder, a T cell proliferative disease or disorder, a T cell malignancy, transplant rejection, graft versus host disease, or one or more symptoms thereof. In a specific embodiment, the antibody (including antibody fragments thereof) included in said liquid formulations is an antigen-binding fragment. The kit may further comprise instructions for preventing, treating and/or managing a disorder (*e.g.,* using the liquid formulations of the disclosure alone or in combination with another prophylactic or therapeutic agent), as well as side effects and dosage information for method of administration.

### 5.45.Articles of Manufacture

The present disclosure also encompasses a finished packaged and labeled pharmaceutical product. This article of manufacture includes the appropriate unit dosage form in an appropriate vessel or container such as a glass vial, pre-filled syringe or other container that is hermetically sealed. The unit dosage form is provided as a sterile particulate free solution comprising an anti-ICOS antibody that is suitable for parenteral administration.

In one embodiment, the unit dosage form is suitable for intravenous, intramuscular, intranasal, oral, topical or subcutaneous delivery. Thus, the disclosure encompasses sterile solutions suitable for each delivery route.

As with any pharmaceutical product, the packaging material and container are designed to protect the stability of the product during storage and shipment. Further, the products of the disclosure include instructions for use or other informational material that advise the physician, technician or patient on how to appropriately prevent or treat the disease or disorder in question. In other words, the article of manufacture includes instruction means indicating or suggesting a dosing regimen including, but not limited to, actual doses, monitoring procedures, and other monitoring information.

Specifically, the disclosure provides an article of manufacture comprising packaging material, such as a box, bottle, tube, vial, container, pre-filled syringe, sprayer, insufflator, intravenous (i.v.) bag, envelope and the like; and at least one unit dosage form of a pharmaceutical agent contained within said packaging material, wherein said pharmaceutical agent comprises a liquid formulation containing an antibody. The packaging material includes instruction means which indicate that said antibody can be used to prevent, treat and/or manage one or more symptoms associated with a disease or disorder associated with or characterized by aberrant expression and/or activity of ICOS, a disease or disorder associated with or characterized by aberrant expression and/or activity of ICOS receptor, an autoimmune disease or disorder, an inflammatory disease or disorder, a T cell proliferative disease or disorder, a T cell malignancy, transplant rejection, graft versus host disease, or one or more symptoms thereof by administering specific doses and using specific dosing regimens as described herein.

The disclosure also provides an article of manufacture comprising packaging material, such as a box, bottle, tube, vial, container, pre-filled syringe, sprayer, insufflator, intravenous (i.v.) bag, envelope and the like; and at least one unit dosage form of each pharmaceutical agent contained within said packaging material, wherein one pharmaceutical agent comprises a liquid formulation containing an antibody that specifically binds to ICOS and the other pharmaceutical agent comprises a prophylactic or therapeutic agent other than an antibody that specifically binds to ICOS, and wherein said packaging material includes instruction means which indicate that said agents can be used to prevent, treat and/or manage one or more symptoms associated with a disease or disorder associated with or characterized by aberrant expression and/or activity of ICOS, a disease or disorder associated with or characterized by aberrant expression and/or activity of ICOS receptor, an autoimmune disease or disorder, an inflammatory disease or disorder, a T cell proliferative disease or disorder, a T cell malignancy, transplant rejection, graft versus host disease, or one or more symptoms thereof by administering specific doses and using specific dosing regimens as described herein.

The present disclosure provides that the adverse effects that may be reduced or avoided by the methods of the disclosure are indicated in informational material enclosed in an article of manufacture for use in preventing, treating and/or managing one or more symptoms associated with an autoimmune disorder, an inflammatory disorder, a malignancy or an infection. Adverse effects that may be reduced or avoided by the methods of the disclosure include, but are not limited to, vital sign abnormalities (fever, tachycardia, bardycardia, hypertension, hypotension), hematological events (anemia, lymphopenia, leukopenia, thrombocytopenia), headache, chills, dizziness, nausea, asthenia, back pain, chest pain (chest pressure), diarrhea, myalgia, pain, pruritus, psoriasis, rhinitis, sweating, injection site reaction, and vasodilatation.

Further, the information material enclosed in an article of manufacture described herein can indicate that foreign proteins may also result in allergic reactions, including anaphylaxis, or cytosine release syndrome. The information material should indicate that allergic reactions may exhibit only as mild pruritic rashes or they may be severe such as erythroderma, Stevens-Johnson syndrome, vasculitis, or anaphylaxis. The information material should also indicate that anaphylactic reactions (anaphylaxis) are serious and occasionally fatal hypersensitivity reactions. Allergic reactions including anaphylaxis may occur when any foreign protein is injected into the body. They may range from mild manifestations such as urticaria or rash to lethal systemic reactions. Anaphylactic reactions occur soon after exposure, usually within 10 minutes. Patients may experience paresthesia, hypotension, laryngeal edema, mental status changes, facial or pharyngeal angioedema, airway obstruction, bronchospasm, urticaria and pruritus, serum sickness, arthritis, allergic nephritis, glomerulonephritis, temporal arthritis, or eosinophilia.

**5.46.** Specific embodiments

1. A sterile, stable aqueous formulation comprising an antibody that specifically binds human ICOS, wherein the antibody comprises an Fc region having complex N-glycoside-linked sugar chains in which fucose is not bound to N-acetylglucosamine in the reducing end in the sugar chain.

2. The formulation of embodiment 1, wherein said antibody was not subjected to lyophilization.

3. The formulation of embodiment 1, wherein said antibody is from an immunoglobulin type selected from the group consisting of IgA, IgE, IgM, IgD, IgY and IgG.

4. The formulation of embodiment 1, wherein said antibody is of the IgG1, IgG2, IgG3, or IgG4 human isotype.

5. The formulation of embodiment 1, wherein said antibody is a murine antibody, a chimeric antibody, a humanized antibody or a human antibody.

6. The formulation of any one of embodiments 1 to 5, wherein said antibody comprises a heavy chain variable sequence of SEQ ID NO: 7.

7. The formulation of any one of embodiments 1 to 5, wherein said antibody comprises a light chain variable sequence of SEQ ID NO: 2.

8. The formulation of any one of embodiments 1 to 5, wherein said antibody comprises a heavy chain variable sequence of SEQ ID NO: 7 and a light chain variable sequence of SEQ ID NO: 2.

9. The formulation of any one of embodiments 1 to 5, wherein said antibody comprises a heavy chain sequence of SEQ ID NO: 6 and a light chain sequence of SEQ ID NO: 1.

10. The formulation of any one of embodiments 1 to 9, wherein the concentration of said antibody is at least about 1 mg/ml, at least about 2 mg/ml, at least about 3 mg/ml, at least about 4 mg/ml, at least about 5 mg/ml, at least about 10 mg/ml, at least about 15 mg/ml, at least about 20 mg/ml, at least about 25 mg/ml, at least about 30 mg/ml, at least about 40 mg/ml, at least about 50 mg/ml, at least about 60 mg/ml, at least about 70 mg/ml, at least about 80 mg/ml, at least about 90 mg/ml, at least about 100 mg/ml, at least about 120 mg/ml, at least about 150 mg/ml, at least about 160 mg/ml, at least about 180 mg/ml, at least about 200 mg/ml, at least about 250 mg/ml, or at least about 300 mg/ml.

11A. The formulation of any one of embodiments 1 to 9, wherein the concentration of said antibody is at least about 1 mg/ml.

11B. The formulation of any one of embodiments 1 to 9, wherein the concentration of said antibody is at least about 2 mg/ml.

11C. The formulation of any one of embodiments 1 to 9, wherein the concentration of said antibody is at least about 3 mg/ml.

11D. The formulation of any one of embodiments 1 to 9, wherein the concentration of said antibody is at least about 4 mg/ml.

11E. The formulation of any one of embodiments 1 to 9, wherein the concentration of said antibody is at least about 5 mg/ml.

11F. The formulation of any one of embodiments 1 to 9, wherein the concentration of said antibody is at least about 10 mg/ml.

11G. The formulation of any one of embodiments 1 to 9, wherein the concentration of said antibody is at least about 20 mg/ml.

11H. The formulation of any one of embodiments 1 to 9, wherein the concentration of said antibody is at least about 50 mg/ml.

111. The formulation of any one of embodiments 1 to 9, wherein the concentration of said antibody is at least about 100 mg/ml.

12. The formulation of any one of embodiments 1 to 9, wherein the concentration of said antibody is at least about 125 mg/ml.

13. The formulation of any one of embodiments 1 to 9, wherein the concentration of said antibody is least about 150 mg/ml.

14. The formulation of any one of embodiments 1 to 9, wherein the concentration of said antibody is at least about 175 mg/ml.

15. The formulation of any one of embodiments 1 to 9, wherein the concentration of said antibody is at least about 200 mg/ml

16A. The formulation of any one of embodiments 1 to 9, wherein the concentration of said antibody is between about 1 mg/ml and about 50 mg/ml.

16B. The formulation of any one of embodiments 1 to 9, wherein the concentration of said antibody is between about 1 mg/ml and about 20 mg/ml.

16C. The formulation of any one of embodiments 1 to 9, wherein the concentration of said antibody is between about 5 mg/ml and about 15 mg/ml.

16D. The formulation of any one of embodiments 1 to 9, wherein the concentration of said antibody is between about 90 mg/ml and about 250 mg/ml.

17. The formulation of any one of embodiments 1 to 9, wherein the concentration of said antibody is of between about 110 mg/ml and about 250 mg/ml.

18. The formulation of any one of embodiments 1 to 17, wherein said formulation further comprises at least about one buffering component.

19. The formulation of any one of embodiments 1 to 18, wherein said formulation further comprises at least about one excipient.

20. The formulation of embodiments 18 or 19, wherein said buffering component is selected from the group consisting of histidine, citrate, phosphate, glycine, and acetate.

21. The formulation of embodiments 18 or 19, wherein said buffering component is histidine.

22. The formulation of embodiment 21, wherein said histidine is at a concentration from about 1 nM to about 200 nM.

23. The formulation of embodiment 21, wherein said histidine is at a concentration from about 1 nM to about 50 nM.

24. The formulation of embodiment 21, wherein said histidine is at a concentration from about 5 nM to about 20 nM.

25. The formulation of embodiment 21, wherein said histidine is at a concentration of about 10 nM, about 15 nM or about 20 nM.

26. The formulation of embodiment 19, wherein said excipient is a saccharide.

27. The formulation of embodiment 26, wherein said saccharide is a disaccharide.

28. The formulation of embodiment 27, wherein said disaccharide is trehalose or sucrose.

29. The formulation of embodiment 27, wherein said disaccharide is trehalose.

30. The formulation of embodiment 29, wherein said trehalose is at a concentration from about 1% to about 40%.

31. The formulation of embodiment 29, wherein said trehalose is at a concentration from about 2% to about 20%.

32. The formulation of embodiment 29, wherein said trehalose is at a concentration from about 2% to about 10%.

33. The formulation of embodiment 29, wherein said trehalose is at a concentration of about 2%, about 4% or about 8%.

34. The formulation of embodiment 19, wherein said excipient is a salt.

35. The formulation of embodiment 34, wherein said salt is sodium chloride.

36. The formulation of embodiment 35, wherein said sodium chloride is at a concentration from about 50 mM to about 200 mM.

37. The formulation of embodiment 35, wherein said sodium chloride is at a concentration of about 70 mM, about 80 mM, about 90 mM, about 100 mM, about 120 mM, or about 150 mM.

38. The formulation of embodiment 19, wherein said excipient is a surfactant.

39. The formulation of embodiment 38, wherein said surfactant is a polysorbate.

40. The formulation of embodiment 39, wherein said polysorbate is polysorbate 20 or polysorbate 80.

41. The formulation of embodiment 39, wherein said polysorbate is polysorbate 80.

42. The formulation of embodiment 41, wherein said polysorbate 80 is at a concentration from about 0.001% to about 2%.

43. The formulation of embodiment 41, wherein said polysorbate 80 is at a concentration of about 0.01%, about 0.02%, about 0.04% or about 0.08%.

44. The formulation of any one of embodiments 1 to 43, wherein said formulation has a pH of between about 5.5 and about 6.5.

45. The formulation of any one of embodiments 1 to 43, wherein said formulation has a pH of about 6.0.

46. The formulation of any one of embodiments 1 to 45, wherein said formulation is isotonic.

47. The formulation of any one of embodiments 1 to 46, wherein said formulation is stable upon storage at 40°C for at least about 4 weeks.

48. The formulation of any one of embodiments 1 to 46, wherein said formulation is stable upon storage at 5°C for at least about 3 months.

49. The formulation of any one of embodiments 1 to 46, wherein said formulation is stable upon storage at 5°C for at least about 12 months.

50. The formulation of any one of embodiments 1 to 46, wherein said antibody retains at least about 80% of binding ability to a human ICOS polypeptide compared to a reference antibody representing the antibody prior to the storage at 40°C for at least about 4 weeks.

51. The formulation of any one of embodiments 1 to 46, wherein said antibody retains at least about 80% of binding ability to a human ICOS polypeptide compared to a reference antibody representing the antibody prior to the storage at 5°C for at least about 3 months.

52. The formulation of any one of embodiments 1 to 46, wherein said antibody retains at least about 80% of binding ability to a human ICOS polypeptide compared to a reference antibody representing the antibody prior to the storage at 5°C for at least about 12 months.

53. The formulation of any one of embodiments 1 to 46, wherein said antibody retains at least about 90% of binding ability to a human ICOS polypeptide compared to a reference antibody representing the antibody prior to the storage at 40°C for at least about 4 weeks.

54. The formulation of any one of embodiments 1 to 46, wherein said antibody retains at least about 90% of binding ability to a human ICOS polypeptide compared to a reference antibody representing the antibody prior to the storage at 5°C for at least about 3 months.

55. The formulation of any one of embodiments 1 to 46, wherein said antibody retains at least about 90% of binding ability to a human ICOS polypeptide compared to a reference antibody representing the antibody prior to the storage at 5°C for at least about 12 months.

56. The formulation of any one of embodiments 1 to 46, wherein said antibody retains at least about 95% of binding ability to a human ICOS polypeptide compared to a reference antibody representing the antibody prior to the storage at 40°C for at least about 4 weeks.

57. The formulation of any one of embodiments 1 to 46, wherein said antibody retains at least about 95% of binding ability to a human ICOS polypeptide compared to a reference antibody representing the antibody prior to the storage at 5°C for at least about 3 months.

58. The formulation of any one of embodiments 1 to 46, wherein said antibody retains at least about 95% of binding ability to a human ICOS polypeptide compared to a reference antibody representing the antibody prior to the storage at 5°C for at least about 12 months.

59. The formulation of any one of embodiments 1 to 46, wherein said antibody is susceptible to aggregation, or fragmentation.

60. The formulation of any one of embodiments 1 to 46, wherein less than about 2% of said antibody forms an aggregate upon storage at 40°C for at least about 4 weeks as determined by as determined by HPSEC.

61. The formulation of any one of embodiments 1 to 46, wherein less than about 2% of said antibody forms an aggregate upon storage at 5°C for at least about 3 months as determined by HPSEC.

62. The formulation of any one of embodiments 1 to 46, wherein less than about 2% of said antibody forms an aggregate upon storage at 5°C for at least about 12 months as determined by HPSEC.

63. The formulation of any one of embodiments 1 to 46, wherein less than about 5% of said antibody is fragmented upon storage at 40°C for at least about 4 weeks as determined by RP-HPLC.

64. The formulation of any one of embodiments 1 to 46, wherein less than about 5% of said antibody is fragmented upon storage at 5°C for at least about 3 months as determined by RP-HPLC.

65. The formulation of any one of embodiments 1 to 46, wherein less than about 5% of said antibody is fragmented upon storage at 5°C for at least about 12 months as determined by RP-HPLC.

66. The formulation of any one of embodiments 1 to 65, wherein said formulation is an injectable formulation.

67. The formulation of embodiment 66, wherein said formulation is suitable for intravenous, subcutaneous, or intramuscular administration.

68. The formulation of embodiment 67, wherein said formulation is suitable for intravenous administration and the antibody or antibody fragment concentration is from about 20 mg/ml to about 40 mg/ml.

69. The formulation of embodiment 67, wherein said formulation is suitable for subcutaneous administration and the antibody or antibody fragment concentration is from about 70 mg/ml to about 250 mg/ml.

70. The formulation of any one of embodiments 1 to 65, wherein said formulation is suitable for aerosol administration.

71. A pharmaceutical unit dosage form suitable for parenteral administration to a human which comprises an antibody formulation of any one of embodiments 1 to 65 in a suitable container.

72. The pharmaceutical unit dosage form of embodiment 71, wherein the antibody formulation is administered intravenously, subcutaneously, or intramuscularly.

73. A pharmaceutical unit dosage form suitable for aerosol administration to a human which comprises an antibody formulation of any one of embodiments 1 to 65 in a suitable container.

74. The pharmaceutical unit dosage of embodiment 73, wherein the antibody formulation is administered intranasally.

75. A sealed container containing the formulation of any one of embodiments 1 to 74.

76. A kit comprising the formulation of any one of embodiments 1 to 74.

77. A method of treating an autoimmune disease or disorder in a human, comprising administering to a human in need thereof a therapeutically-effective amount of the formulation of any one of embodiments 1 to 74.

78. The method of embodiment 77, wherein the autoimmune disease or disorder is SLE or scleroderma.

79. A method of treating or preventing rejection in a human transplant patient, comprising administering to a human in need thereof a therapeutically-effective amount of the formulation of any one of embodiments 1 to 74.

80. A method of treating a T cell malignancy in a human comprising administering to a human in need thereof a therapeutically-effective amount of the formulation of any one of embodiments 1 to 74.

81. A method of treating an inflammatory disease or disorder in a human, comprising administering to a human in need thereof a therapeutically-effective amount of the formulation of any one of embodiments 1 to 74.

82. The method of embodiment 81, wherein the inflammatory disease or disorder is myositis.

83. The method of embodiment 82, wherein the myositis is inclusion-body myositis (IBM), polymyositis (PM) or dermatomyositis (DM).

84. A method of depleting ICOS expressing T cells in a human patient comprising administering to a human in need thereof a therapeutically-effective amount of the formulation of any one of embodiments 1 to 74.

85. The method of embodiment 84, wherein the depletion substantially persists for at least about 1, at least about 2, at least about 3 or at least about 4 weeks following the administration of the antibody.

86. The method of embodiment 84, wherein at least about 95% of the T cells are depleted.

87. The method of embodiment 84, wherein the ICOS expressing T cell is a memory T cell.

88. The method of embodiment 84, wherein the ICOS expressing T cell is a circulating T cell.

89. A method of disrupting germinal center architecture in a secondary lymphoid organ of a primate, comprising administering an effective amount of the formulation of any one of embodiments 1 to 74.

90. The method of embodiment 89, wherein the primate is a non-human primate.

91. A method of depleting germinal center B cells from a secondary lymphoid organ of a primate comprising administering an effective amount of the formulation of any one of embodiments 1 to 74.

92. The method of embodiment 91, wherein the primate is a non-human primate.

93. The method of embodiment 91, wherein the primate is a human.

94. The method of embodiment 91, wherein the depletion substantially persists for at least about 1, at least about 2, at least about 3 or at least about 4 weeks following the administration of the antibody.

95. A method of depleting circulating class switched B cells in a primate comprising administering an effective amount of the formulation of any one of embodiments 1 to 74.

96. The method of embodiment 95, wherein the primate is a non-human primate.

97. The method of embodiment 95, wherein the primate is a human.

98. The method of embodiment 95, wherein the depletion substantially persists for at least about 1, at least about 2, at least about 3 or at least about 4 weeks following the administration of the antibody.

99. The method of embodiment 95, wherein at least about 95% of the circulating class switched B cells are depleted.

100. A sterile, stable aqueous formulation comprising an anti-human ICOS antibody, and further comprising histidine, sodium chloride, trehalose or polysorbate 80, wherein the antibody comprises a heavy chain sequence of SEQ ID NO:6, a light chain sequence of SEQ ID NO:1 and an Fc region having complex N-glycoside-linked sugar chains in which fucose is not bound to N-acetylglucosamine in the reducing end in the sugar chain.

101. A sterile, stable aqueous formulation comprising an anti-human ICOS antibody, and further comprising histidine, sodium chloride, trehalose and polysorbate 80, wherein the antibody comprises a heavy chain sequence of SEQ ID NO: 6, a light chain sequence of SEQ ID NO: 1 and an Fc region having complex N-glycoside-linked sugar chains in which fucose is not bound to N-acetylglucosamine in the reducing end in the sugar chain.

102A. The formulation of embodiment 101, wherein said formulation comprises between about 1 mg/ml and about 20 mg/ml of the anti-human ICOS antibody, between about 1 mM and about 100 mM histidine, between about 1% and about 40% trehalose and about between about 0.001% and about 5% polysorbate 80 and wherein the pH of said formulation is between about 5 and about 7.

102B. The formulation of embodiment 101, wherein said formulation comprises between about 50 mg/ml and about 150 mg/ml of the anti-human ICOS antibody, between about 1 mM and about 100 mM histidine, between about 1% and about 40% trehalose and about between about 0.001% and about 5% polysorbate 80 and wherein the pH of said formulation is between about 5 and about 7.

103A. The formulation of embodiment 101, wherein said formulation comprises between about 5 mg/ml and about 15 mg/ml of the anti-human ICOS antibody, between about 5 mM and about 25 mM histidine, between about 2% and about 15% trehalose and between about 0.005% and about 1% polysorbate 80 and wherein the pH of said formulation is between about 5.5 and about 6.5.

103B. The formulation of embodiment 101, wherein said formulation comprises between about 80 mg/ml and about 120 mg/ml of the anti-human ICOS antibody, between about 5 mM and about 25 mM histidine, between about 2% and about 15% trehalose and between about 0.005% and about 1% polysorbate 80 and wherein the pH of said formulation is between about 5.5 and about 6.5.

104A. The formulation of embodiment 101, wherein said formulation comprises about 10 mg/ml of the anti-human ICOS antibody, about 10 mM histidine, about 4% trehalose and about 0.02% polysorbate 80 and wherein the pH of said formulation is about 6.

104B. The formulation of embodiment 101, wherein said formulation comprises about 100 mg/ml of the anti-human ICOS antibody, about 10 mM histidine, about 4% trehalose and about 0.02% polysorbate 80 and wherein the pH of said formulation is about 6.

105. The formulation of any one of embodiments 101 to 104, wherein said formulation is isotonic.

106. The formulation of any one of embodiments 101 to 104, wherein said formulation is stable upon storage at 40°C for at least about 4 weeks.

107. The formulation of any one of embodiments 101 to 104, wherein said formulation is stable upon storage at 5°C for at least about 3 months.

108. The formulation of any one of embodiments 101 to 104, wherein said formulation is stable upon storage at 5°C for at least about 12 months.

109. The formulation of any one of embodiments 101 to 104, wherein said antibody retains at least about 80% of binding ability to a human ICOS polypeptide compared to a reference antibody representing the antibody prior to the storage at 40°C for at least about 4 weeks.

110. The formulation of any one of embodiments 101 to 104, wherein said antibody retains at least about 80% of binding ability to a human ICOS polypeptide compared to a reference antibody representing the antibody prior to the storage at 5°C for at least about 3 months.

111. The formulation of any one of embodiments 101 to 104, wherein said antibody retains at least about 80% of binding ability to a human ICOS polypeptide compared to a reference antibody representing the antibody prior to the storage at 5°C for at least about 12 months.

112. The formulation of any one of embodiments 101 to 104, wherein said antibody retains at least about 90% of binding ability to a human ICOS polypeptide compared to a reference antibody representing the antibody prior to the storage at 40°C for at least about 4 weeks.

113. The formulation of any one of embodiments 101 to 104, wherein said antibody retains at least about 90% of binding ability to a human ICOS polypeptide compared to a reference antibody representing the antibody prior to the storage at 5°C for at least about 3 months.

114. The formulation of any one of embodiments 101 to 104, wherein said antibody retains at least about 90% of binding ability to a human ICOS polypeptide compared to a reference antibody representing the antibody prior to the storage at 5°C for at least about 12 months.

115. The formulation of any one of embodiments 101 to 104, wherein said antibody retains at least about 95% of binding ability to a human ICOS polypeptide compared to a reference antibody representing the antibody prior to the storage at 40°C for at least about 4 weeks.

116. The formulation of any one of embodiments 101 to 104, wherein said antibody retains at least about 95% of binding ability to a human ICOS polypeptide compared to a reference antibody representing the antibody prior to the storage at 5°C for at least about 3 months.

117. The formulation of any one of embodiments 101 to 104, wherein said antibody retains at least about 95% of binding ability to a human ICOS polypeptide compared to a reference antibody representing the antibody prior to the storage at 5°C for at least about 12 months.

118. The formulation of any one of embodiments 101 to 104, wherein said antibody is susceptible to aggregation or fragmentation.

119. The formulation of any one of embodiments 101 to 104, wherein less than about 2% of said antibody forms an aggregate upon storage at 40°C for at least about 4 weeks as determined by as determined by HPSEC.

120. The formulation of any one of embodiments 101 to 104, wherein less than about 2% of said antibody forms an aggregate upon storage at 5°C for at least about 3 months as determined by HPSEC.

121. The formulation of any one of embodiments 101 to 104, wherein less than about 2% of said antibody forms an aggregate upon storage at 5°C for at least about 12 months as determined by HPSEC.

122. The formulation of any one of embodiments 101 to 104, wherein less than about 5% of said antibody is fragmented upon storage at 40°C for at least about 4 weeks as determined by RP-HPLC.

123. The formulation of any one of embodiments 101 to 104, wherein less than about 5% of said antibody is fragmented upon storage at 5°C for at least about 3 months as determined by RP-HPLC.

124. The formulation of any one of embodiments 101 to 104, wherein less than about 5% of said antibody is fragmented upon storage at 5°C for at least about 12 months as determined by RP-HPLC.

125. The formulation of any one of embodiments 101 to 104, wherein said formulation is clear and colorless upon storage at 5°C for at least about 3 months as determined by visual inspection.

126. The formulation of any one of embodiments 101 to 104, wherein said formulation is clear and colorless upon storage at 5°C for at least about 12 months as determined by visual inspection.

127. The formulation of any one of embodiments 101 to 126, wherein said formulation is an injectable formulation.

128. The formulation of embodiment 127, wherein said formulation is suitable for intravenous, subcutaneous, or intramuscular administration.

129. The formulation of embodiment 128, wherein said formulation is suitable for intravenous administration.

130. The formulation of embodiment 128, wherein said formulation is suitable for subcutaneous administration.

131. The formulation of any one of embodiments 101 to 126, wherein said formulation is suitable for aerosol administration.

132. A process for the preparation of a formulation according to any one of embodiments 101 to 126, comprising:

   a) concentrating the anti-human ICOS antibody solution to between about 10 mg/ml and about 50 mg/ml;
   b) diafiltering said concentrated antibody with a solution comprising histidine.

133. The process of embodiment 132 further comprising:

   (c) concentrating the antibody diafiltered with a solution comprising histidine to between about 50 mg/ml and about 250 mg/ml;
   (d) admixing the concentrated antibody solution with at least about one solution comprising at least about one excipient.

134. A method for stabilizing an anti-human ICOS antibody comprising combining said antibody with histidine-HCl, sodium chloride, trehalose and polysorbate 80 at a pH of 6, wherein the antibody comprises a heavy chain sequence of SEQ ID NO: 6, a light chain sequence of SEQ ID NO: 1 and an Fc region having complex N-glycoside-linked sugar chains in which fucose is not bound to N-acetylglucosamine in the reducing end in the sugar chain.

135. The method of embodiment 134, wherein the antibody concentration is between about 80 mg/ml and about 120 mg/ml.

136. A pharmaceutical unit dosage form suitable for parenteral administration to a human which comprises an antibody formulation of any one of embodiments 101 to 131 in a suitable container.

137. The pharmaceutical unit dosage form of embodiment 136, wherein the antibody formulation is administered intravenously, subcutaneously, or intramuscularly.

138. A pharmaceutical unit dosage form suitable for aerosol administration to a human which comprises an antibody formulation of any one of embodiments 101 to 131 in a suitable container.

139. The pharmaceutical unit dosage of embodiment 138, wherein the antibody formulation is administered intranasally.

140. A sealed container containing the formulation of any one of embodiments 101 to 131.

141. A kit comprising the formulation of any one of embodiments 101 to 131.

142. A method of treating an autoimmune disease or disorder in a human, comprising administering to a human in need thereof a therapeutically-effective amount of the formulation of any one of embodiments 101 to 131.

143. The method of embodiment 142, wherein the autoimmune disease or disorder is SLE or scleroderma.

144. A method of treating or preventing rejection in a human transplant patient, comprising administering to a human in need thereof a therapeutically-effective amount of the formulation of any one of embodiments 101 to 131.

145. A method of treating a T cell malignancy in a human comprising administering to a human in need thereof a therapeutically-effective amount of the formulation of any one of embodiments 101 to 131.

146. A method of treating an inflammatory disease or disorder in a human, comprising administering to a human in need thereof a therapeutically-effective amount of the formulation of any one of embodiments 101 to 131.

147. The method of embodiment 180, wherein the inflammatory disease or disorder is myositis.

148. The method of embodiment 147, wherein the myositis is inclusion-body myositis (IBM), polymyositis (PM) or dermatomyositis (DM).

149. A method of depleting ICOS expressing T cells in a human patient comprising administering to a human in need thereof a therapeutically-effective amount of the formulation of any one of embodiments 101 to 131.

150. The method of embodiment 149, wherein the depletion substantially persists for at least about 1, at least about 2, at least about 3 or at least about 4 weeks following the administration of the antibody.

151. The method of embodiment 149, wherein at least about 95% of the T cells are depleted.

152. The method of embodiment 149, wherein the ICOS expressing T cell is a memory T cell.

153. The method of embodiment 149, wherein the ICOS expressing T cell is a circulating T cell.

154. A method of disrupting germinal center architecture in a secondary lymphoid organ of a primate, comprising administering an effective amount of the formulation of any one of embodiments 101 to 131.

155. The method of embodiment 149, wherein the primate is a non-human primate.

156. A method of depleting germinal center B cells from a secondary lymphoid organ of a primate comprising administering an effective amount of the formulation of any one of embodiments 101 to 131.

157. The method of embodiment 156, wherein the primate is a non-human primate.

158. The method of embodiment 156, wherein the primate is a human.

159. The method of embodiment 156, wherein the depletion substantially persists for at least about 1, at least about 2, at least about 3 or at least about 4 weeks following the administration of the antibody.

160. A method of depleting circulating class switched B cells in a primate comprising administering an effective amount of the formulation of any one of embodiments 101 to 131.

161. The method of embodiment 194, wherein the primate is a non-human primate.

162. The method of embodiment 194, wherein the primate is a human.

163. The method of embodiment 194, wherein the depletion substantially persists for at least about 1, at least about 2, at least about 3 or at least about 4 weeks following the administration of the antibody.

164. The method of embodiment 194, wherein at least about 95% of the circulating class switched B cells are depleted.

165. The formulation of any one of embodiments 1 to 70 or 101 to 131, wherein said formulation is a pharmaceutically acceptable formulation.

166. The formulation of any one of embodiments 66, 67, 69, 127, 128 or 130 wherein the formulation is in a pre-filled syringe.

167. The formulation of embodiment 166, wherein the pre-filled syringe comprises a needle.

168. The formulation of embodiment 166, wherein the pre-filled syringe is a plastic syringe or a glass syringe.

169. The formulation of embodiment 166, wherein the pre-filled syringe is a plastic syringe.

170. The formulation of embodiment 168, wherein the pre-filled syringe is a glass syringe.

171. The formulation of the embodiment 166, wherein the pre-filled syringe is made of materials that are substantially free from tungsten.

172. The formulation of embodiment 166, wherein the pre-filled syringe is substantially free from silicone.

173. The formulation of embodiment 166, wherein the pre-filled syringe does not comprise a silicone based lubricant.

174. The formulation of embodiment 166, wherein the pre-filled syringe comprises a plunger having a fluoropolymer resin disk.

175. The pharmaceutical unit dosage of any one of embodiments 71, 72, 136 or 137, wherein the suitable container is a pre-filled syringe.

176. The pharmaceutical unit dosage of embodiment 172, wherein the pre-filled syringe comprises a needle.

177. The pharmaceutical unit dosage of embodiment 172, wherein the pre-filled syringe is a plastic syringe or a glass syringe.

178. The pharmaceutical unit dosage of embodiment 178, wherein the pre-filled syringe is a plastic syringe.

179. The pharmaceutical unit dosage of embodiment 178, wherein the pre-filled syringe is a glass syringe.

180. The pharmaceutical unit dosage of the embodiment 172, wherein the pre-filled syringe is made of materials that are substantially free from tungsten.

181. The pharmaceutical unit dosage of embodiment 172, wherein the pre-filled syringe is substantially free from silicone.

182. The pharmaceutical unit dosage of embodiment 172, wherein the pre-filled syringe does not comprise a silicone based lubricant.

183. The pharmaceutical unit dosage of embodiment 172, wherein the pre-filled syringe comprises a plunger having a fluoropolymer resin disk.

184. The sealed container of any one of embodiments 75 or 140, wherein the sealed container is in a pre-filled syringe.

185. The sealed container of embodiment 184, wherein the pre-filled syringe comprises a needle.

186. The sealed container of embodiment 184, wherein the pre-filled syringe is a plastic syringe or a glass syringe.

187. The sealed container of embodiment 186, wherein the pre-filled syringe is a plastic syringe.

188. The sealed container of embodiment 186, wherein the pre-filled syringe is a glass syringe.

189. The sealed container of the embodiment 184, wherein the pre-filled syringe is made of materials that are substantially free from tungsten.

190. The sealed container of embodiment 184, wherein the pre-filled syringe is substantially free from silicone.

191. The sealed container of embodiment 184, wherein the pre-filled syringe does not comprise a silicone based lubricant.

192. The sealed container of embodiment 184, wherein the pre-filled syringe comprises a plunger, wherein the plunger comprises a fluoropolymer resin disk.

193. The kit of any one of embodiments 76 or 141, wherein the kit comprises a pre-filled syringe.

194. The kit of embodiment 193, wherein the pre-filled syringe comprises a needle.

195. The kit of embodiment 193, wherein the pre-filled syringe is a plastic syringe or a glass syringe.

196. The kit of embodiment 195, wherein the pre-filled syringe is a plastic syringe.

197. The kit of embodiment 195, wherein the pre-filled syringe is a glass syringe.

198. The kit of the embodiment 193, wherein the pre-filled syringe is made of materials that are substantially free from tungsten.

199. The kit of embodiment 193, wherein the pre-filled syringe is substantially free from silicone.

200. The kit of embodiment 193, wherein the pre-filled syringe does not comprise a silicone based lubricant.

201. The kit of embodiment 193, wherein the pre-filled syringe comprises a plunger, wherein the plunger comprises a fluoropolymer resin disk.

202. A pre-filled syringe containing a sterile, stable aqueous formulation comprising an anti-human ICOS antibody, and further comprising histidine, sodium chloride, trehalose or polysorbate 80, wherein the antibody comprises a heavy chain sequence of SEQ ID NO: 6, a light chain sequence of SEQ ID NO: 1 and an Fc region having complex N-glycoside-linked sugar chains in which fucose is not bound to N-acetylglucosamine in the reducing end in the sugar chain.

203. The pre-filled syringe of embodiment 202, wherein the pre-filled syringe comprises a needle.

204. The pre-filled syringe of embodiment 202, wherein the pre-filled is sealed.

205. The pre-filled syringe of embodiment 202, wherein the pre-filled syringe is a plastic syringe or a glass syringe.

206. The pre-filled syringe of embodiment 211, wherein the pre-filled syringe is a plastic syringe.

207. The pre-filled syringe of embodiment 211, wherein the pre-filled syringe is a glass syringe.

208. The pre-filled syringe of embodiment 202, wherein the pre-filled syringe is made of materials that are substantially free from tungsten.

209. The pre-filled syringe of embodiment 202, wherein the pre-filled syringe is substantially free from silicone.

210. The pre-filled syringe of embodiment 202, wherein the pre-filled syringe does not comprise a silicone based lubricant.

211. The pre-filled syringe of embodiment 202, wherein the pre-filled syringe comprises a plunger having a fluoropolymer resin disk.

212. The pre-filled syringe of embodiment 202, wherein the syringe is a plastic syringe substantially free from silicone and tungsten comprising a plunger having a fluoropolymer resin disk.

213. The pre-filled syringe of any one of embodiments 202-212, wherein said formulation comprises the anti-human ICOS antibody, histidine, sodium chloride, trehalose and polysorbate 80.

214. The pre-filled syringe of embodiment 202, wherein said formulation comprises between about 50 mg/ml and about 150 mg/ml of the anti-human ICOS antibody, between about 1 mM and about 100 mM histidine, between about 10 mM and about 200 mM NaCl, between about 1% and about 40% trehalose and between about 0.001% and about 5% polysorbate 80 and wherein the pH of said formulation is between about 5 and about 7.

215. The pre-filled syringe of embodiment 202, wherein said formulation comprises between about 80 mg/ml and about 120 mg/ml of the anti-human ICOS antibody, between about 1 mM and about 50 mM histidine, between about 50 mM and about 150 mM NaCl, between about 1% and about 20% trehalose and between about 0.005% and about 1% polysorbate 80 and wherein the pH of said formulation is between about 5.5 and about 6.5.

216. The pre-filled syringe of embodiment 202, wherein said formulation comprises about 100 mg/ml of the anti-human ICOS antibody, about 10 mM histidine, about 80 mM NaCl, about 4% trehalose and between about 0.01% and about 0.05% polysorbate 80 and wherein the pH of said formulation is about 6.

217. The pre-filled syringe of embodiment 202, wherein said formulation comprises about 100 mg/ml of the anti-human ICOS antibody, about 10 mM histidine, about 80 mM NaCl, about 4% trehalose and about 0.02% polysorbate 80 and wherein the pH of said formulation is about 6.

218. The pre-filled syringe of any one of embodiments 202 to 217, wherein said formulation is isotonic.

219. The pre-filled syringe of any one of embodiments 202 to 217, wherein said formulation is stable upon storage at 40°C for at least about 4 weeks.

220. The pre-filled syringe of any one of embodiments 202 to 217, wherein said formulation is stable upon storage at 5°C for at least about 3 months.

221. The pre-filled syringe of any one of embodiments 202 to 217, wherein said formulation is stable upon storage at 5°C for at least about 12 months.

222. The pre-filled syringe of any one of embodiments 202 to 217, wherein said antibody retains at least about 80% of binding ability to a human ICOS polypeptide compared to a reference antibody representing the antibody prior to the storage at 40°C for at least about 4 weeks.

223. The pre-filled syringe of any one of embodiments 202 to 217, wherein said antibody retains at least about 80% of binding ability to a human ICOS polypeptide compared to a reference antibody representing the antibody prior to the storage at 5°C for at least about 3 months.

224. The pre-filled syringe of any one of embodiments 202 to 217, wherein said antibody retains at least about 80% of binding ability to a human ICOS polypeptide compared to a reference antibody representing the antibody prior to the storage at 5°C for at least about 12 months.

225. The pre-filled syringe of any one of embodiments 202 to 217, wherein said antibody retains at least about 90% of binding ability to a human ICOS polypeptide compared to a reference antibody representing the antibody prior to the storage at 40°C for at least about 4 weeks.

226. The pre-filled syringe of any one of embodiments 202 to 217, wherein said antibody retains at least about 90% of binding ability to a human ICOS polypeptide compared to a reference antibody representing the antibody prior to the storage at 5°C for at least about 3 months.

227. The pre-filled syringe of any one of embodiments 202 to 217, wherein said antibody retains at least about 90% of binding ability to a human ICOS polypeptide compared to a reference antibody representing the antibody prior to the storage at 5°C for at least about 12 months.

228. The pre-filled syringe of any one of embodiments 202 to 217, wherein said antibody retains at least about 95% of binding ability to a human ICOS polypeptide compared to a reference antibody representing the antibody prior to the storage at 40°C for at least about 4 weeks.

229. The pre-filled syringe of any one of embodiments 202 to 217, wherein said antibody retains at least about 95% of binding ability to a human ICOS polypeptide compared to a reference antibody representing the antibody prior to the storage at 5°C for at least about 3 months.

230. The pre-filled syringe of any one of embodiments 202 to 217, wherein said antibody retains at least about 95% of binding ability to a human ICOS polypeptide compared to a reference antibody representing the antibody prior to the storage at 5°C for at least about 12 months.

231. The pre-filled syringe of any one of embodiments 202 to 217, wherein said antibody is susceptible to aggregation or fragmentation.

232. The pre-filled syringe of any one of embodiments 202 to 217, wherein less than about 2% of said antibody forms an aggregate upon storage at 40°C for at least about 4 weeks as determined by as determined by HPSEC.

233. The pre-filled syringe of any one of embodiments 202 to 217, wherein less than about 2% of said antibody forms an aggregate upon storage at 5°C for at least about 3 months as determined by HPSEC.

234. The pre-filled syringe of any one of embodiments 202 to 217, wherein less than about 2% of said antibody forms an aggregate upon storage at 5°C for at least about 12 months as determined by HPSEC.

235. The pre-filled syringe of any one of embodiments 202 to 217, wherein less than about 5% of said antibody is fragmented upon storage at 40°C for at least about 4 weeks as determined by RP-HPLC.

236. The pre-filled syringe of any one of embodiments 202 to 217, wherein less than about 5% of said antibody is fragmented upon storage at 5°C for at least about 3 months as determined by RP-HPLC.

237. The pre-filled syringe of any one of embodiments 202 to 217, wherein less than about 5% of said antibody is fragmented upon storage at 5°C for at least about 12 months as determined by RP-HPLC.

238. The pre-filled syringe of any one of embodiments 202 to 217, wherein said formulation is clear and colorless upon storage at 5°C for at least about 3 months as determined by visual inspection.

239. The pre-filled syringe of any one of embodiments 202 to 217, wherein said formulation is clear and colorless upon storage at 5°C for at least about 12 months as determined by visual inspection.

240. The pre-filled syringe of any one of embodiments 202 to 217, wherein said formulation is substantially free from particulates upon storage at 5°C for at least about 3 months as determined by visual inspection.

241. The pre-filled syringe of any one of embodiments 202 to 217, wherein said formulation is substantially free from particulates upon storage at 5°C for at least about 12 months as determined by visual inspection.

242. The pre-filled syringe of any one of embodiments 202 to 217, wherein said formulation is an injectable formulation.

243. The pre-filled syringe of embodiment 242, wherein said formulation is suitable for subcutaneous or intramuscular.

244. The pre-filled syringe of embodiment 243, wherein said formulation is suitable for subcutaneous administration.

245. The pre-filled syringe of any one of embodiments 202 to 244, wherein said formulation is a pharmaceutically acceptable formulation.

246. A kit comprising the pre-filled syringe of any one of embodiments 202 to 245.

Those skilled in the art will recognize, or be able to ascertain using no more than routine experimentation, many equivalents to the specific embodiments of the disclosure described herein.

Citation or discussion of a reference herein shall not be construed as an admission that such is prior art to the present disclosure.

**6. EXAMPLES**

The following section describes the development of formulations comprising an anti-human ICOS antibody. Unless stated otherwise, experimental results presented here were generated using the 136 anti-human ICOS antibody comprising a heavy chain sequence of SEQ ID NO: 6, a light chain sequence of SEQ ID NO: 1 and an Fc region having complex N-glycoside-linked sugar chains in which fucose is not bound to N-acetylglucosamine in the reducing end in the sugar chain (*see,* US Patent Application 12/116,512, filed on May 7, 2008).

**6.1.** Experimental methods

Purified anti-human ICOS antibody may be generated following standard industrial scale protocols described herein. Protein concentration may be estimated from optical density measurement at 280 nm.

Purified anti-human ICOS antibody is nanofiltered using a Planova 20N filter to remove particulate matter. Anti-human ICOS antibody formulations is prepared using Tangential Flow Filtration (TFF). The nanofiltered anti-human ICOS antibody is concentrated to approximately 25 mg/ml on a Millipore Labscale TFF device. The anti-human ICOS antibody is then 5x diafiltered into the appropriate buffer (*e.g.,* 10 mM histidine-HCL (pH 6.0), 80 mM NaCl). Once the buffer exchange is complete, the antibody is concentrated to approximately 150 mg/ml. Excipients are introduced by spiking the concentrated antibody preparation with the appropriate concentrated stock solutions. For example, a final concentration of 4% Trehalose is achieved by adding 11 ml of 10 mM histidine-HCl, 80 mM NaCl, 40% Trehalose (pH 6.0) to every 100 ml of concentrated antibody preparation. Multiple excipients may be introduced in consecutive steps. For example, a final concentration of 0.02% Polysorbate 80 is introduced after the addition of Trehalose by diluting 100 fold a 10 mM histidine-HCl (pH 6.0), 80 mM NaCl, 4% Trehalose, 2% Polysorbate 80 stock solution with the Trehalose containing antibody preparation. The final antibody concentration of is adjusted to $100 \pm 5$ mg/ml with the final formulation buffer (*e.g.,* 10 mM histidine-HCl (pH 6.0), 80 mM NaCl. 4% Trehalose, 0.02% Polysorbate 80).

The following section describes methods that may be used to characterize the formulation comprising 100 mg/ml anti-ICOS antibody in 10 mM Histidine (pH 6.0), 80 mM NaCl, 4% Trehalose, and 0.02% Polysorbate 80 in a sterile aqueous solution.

Formulation stability is tested by analyzing the physical properties of single dose aliquots stored for extended periods of time. Some aliquots are stored under temperatures recommended for clinical storage (5°C). Other aliquots are stored under elevated temperature (25°C or 40°C) to simulate the effects of very long term storage.

Additional storage conditions that may affect stability of a formulation include, but are not limited to, light intensity, light wavelength, humidity, vial composition, and stopper composition. The effect of these parameters on formulation stability may also be determined using the methods described herein.

Size exclusion chromatography may be utilized to measure the amount of antibody aggregates (e.g., dimmers) and the extent of fragmentation in the formulation. SEC may be performed using the Agilent 1100 Series High Performance Liquid Chromatography (HPLC) system as follows. Samples are diluted to 10 mg/ml. 25 μl diluted sample containing 250 ug protein is injected onto a TSK-Gel 3000 column (size 7.8 mm x 30.0 cm; Tosoh Biosciences Corporation). Protein elution profile is determined by following the eluate's optical density at 280 nm. Data analysis may be performed using ChemStation (Agilent) auto integration parameters.

Reversed Phase High Performance Liquid Chromatography (RP-HPLC) may also be used to determine the amount of antibody fragments in the formulation. RP-HPLC is performed using the Agilent 1100 Series High Performance Liquid Chromatography (HPLC) system. Samples are analysed on a PLRP-S (8 um, 4000 A, 2.0 x 150 mm) column from Michrom Bioresources. Protein elution profile is determined by following the eluate's optical density at 280 nm. Data analysis may be performed using ChemStation (Agilent) auto integration parameters.

Ion exchange chromatography (IEC) may be employed to measure charge isoform heterogeneity of in various formulations. Agilent 1100 Series High Performance Liquid Chromatography (HPLC) systems may be used for this analysis. Samples are analysed on a Propac WCX-10G (4 x 250mm) Analytical Column (Dionex). Data analysis is performed using the ChemStation (Agilent) auto integration parameters.

Visual inspection: color, clarity, and amount of particulates in a given formulation are determined by inspecting the sample with a naked eye.

6.1.1. Size exclusion chromatography (SEC)

Size exclusion chromatography may be performed to analyze the antibody formulation for the presence of antibody aggregates and fragments. The test samples are injected onto a high resolution size exclusion column (*e.g.,* G3000 SW$_{XL}$ 5 $\mu$m, 300 Å, 7.8 x 300 mm, Toso Haas). The mobile phase is 0.1 M di-sodium phosphate, 0.1 M sodium sulphate and 0.05 % sodium azide (pH 6.7), running isocratically at a flow rate of 0.25 - 1.0 mL/min. Eluted protein may be detected by UV absorbance at 280 nm and collected for further characterization. The relative amount of any protein species detected is reported as the area percent of the product peak as compared to the total area of all other detected peaks excluding the initial excluded volume peak. Peaks eluting earlier than the antibody monomer peak are recorded in the aggregate percentile, while peaks eluting later than the antibody monomer peak, but earlier than the buffer peak, are recorded in the fragment percentile. The hydrodynamic radius and molecular weight of the individual peaks may be obtained with a coupled multiangle light scattering detector.

SEC may be used to monitor antibody aggregate formation and antibody fragmentation in a formulations stored for extended time periods (*e.g.,* multiple measurements performed over 9 months). The formulation may be stored at different temperature ranges (*e.g.,* 2-8 °C, 20-24 °C and 38-42 °C). Temperature ranges above the proposed clinical storage temperature (2-8 °C) are used to stress the formulation with the goal of simulating the effects of storage beyond 9 months. The ratio of fragments and aggregates is expected to increase over time; this increase is likely to be accelerated at elevated temperatures. A finding that fragmentation and aggregation rates are constant within each temperature range would show that higher storage temperatures accurately simulate an accelerated time scale.

The logarithm of the estimated rates of fragmentation/aggregation (log(rate)) may also be determined. A finding that the log(rate) shows a linear dependence to the reciprocal of the storage temperature (1/T (K$^{-1}$) would allow the investigator to predict the rate of aggregation/fragmentation of the formulation at any temperature or, more importantly, the formulation characteristics at any time at a given temperature.

In situations where the chromatography peaks corresponding to aggregates and fragments are not be sufficiently distinct from each other, or from the monomer peak (*e.g.,* at low relative levels of aggregates/fragments), SEC may not serve as an accurate measure of fragmentation/ aggregation.

6.1.2. Analytical ultracentrifugation

Analytical ultracentrifugation (AUC) may also be used to characterize the antibody formulation for the presence of antibody aggregates and fragments. AUC is an orthogonal technique which determines the sedimentation coefficients (reported in Svedberg, S) of macromolecules in a liquid sample. Like SEC, AUC is capable of separating and detecting antibody fragments/aggregates from monomers and is further able to provide information on molecular mass. Compared to SEC, AUC eliminates the possibility of aggregate loss due to solid-phase interaction and is better able to resolve differing species of a given macromolecule.

Sedimentation velocity experiments may be performed using an analytical ultracentrifuge, for example, Beckman Optima XL-A. Test samples are diluted to an antibody concentration of 0.5 mg/ml with reference buffer (*e.g.,* 20 mM citric acid, 100 mM NaCl, 1.5% mannitol, 50 $\mu$M diethylenetriamine-pentaacetic acid, 0.02% Polysorbate 80, pH 6.0). 415 $\mu$l of the diluted antibody sample and 412 $\mu$l or the reference buffer is loaded into a 12 mm centrifuge cell in the sample and reference channels, respectively. Loaded cells are placed into an AN-50Ti analytical rotor and equilibrated to 25 °C. Samples are scanned at 280 nm with a rotor speed of 42000 rpm at full vacuum. A total of 80 scans for each cell are collected for analysis. The first scan for each sample is excluded from downstream data processing to avoid artifacts caused by meniscus.

The data is analyzed using the *c(s)* method developed by Peter Shuck at N.I.H. and the SEDFIT (version 8.8) program with implemented *c(s)*. Using the *c(s)* method, raw data scans are directly fit to a Lamm function of S in order to derive a distribution of sedimentation coefficients. The parameters used for the fitting procedure are resolution, 400; confidence interval, 0.75; grid size, 1000; partial specific volume, 0.7245; buffer density, 1.000; and buffer viscosity, 0.1002. Frictional ratio, meniscus and bottom positions are set as fitted parameters. Time independent noise is also fitted. The detected peaks are integrated and classified as follows: from 0 to 6 S, fragments; from 6 to 9 S, monomer; and from 9 to 20 S, aggregates.

AUC may be used to characterize antibody formulations with low relative levels of aggregation and fragmentation. AUC may be able to better resolve antibody fragments and aggregates from the monomer species in situations that are beyond the resolution capabilities of SEC peaks. AUC estimates of the molecular mass of an aggregate peak may also be used as an indicator of its composition (*e.g.,* dimers vs. higher multimers).

Compared to SEC, AUC may also able to better resolve differing species of a given macromolecule. It is, however, necessary to establish first the proper sample dilution rate, as the noise/signal ratio of AUC is dependent on the antibody concentration in the sample.

6.1.3. Turbidity measurement:

Protein aggregation in the antibody formulation may also be characterized by turbidity measurement. Turbidity is a measure of the amount by which the particles in a solution scatter light and, thus, may be used as a general indicator of protein aggregation or denaturation. Elevated turbidity may indicate a higher level of aggregation or an increased number/ increased size of particles.

Turbidity measurement may be performed with a turbidimeter (*e.g.,* 2100AN or 2100N, Hatch) following the manufacturer's instructions. Approximately 3 to 4 ml of formulation sample is transferred into a glass test tube and degassed for 2 minutes using an in-line vacuum system. The degassed sample is then placed into a turbidimeter (*e.g.,* 2100AN or 2100N, Hatch) sample compartment at room temperature for analysis. The turbidimeter is calibrated with STABLCAL® Stabilized Formazin Turbidity standard (Hatch) at 40, 200, 1000 and 4000 NTU (nephelometric turbidity unit) and verified by analyzing control suspensions of formazin at 3, 6, 18, 30 and 60 NTU.

6.1.4. Particle Count

The number and size of particles in a particular formulation may be determined using a particle counter (*e.g.,* Beckman Coulter Multisizer 3) according to the manufacturer's instruction.

6.1.5. Viscosity profile

Viscosities of antibody formulations may be measured using a viscometer (*e.g.,* ViscoLab 4000 Viscometer System from Cambridge Applied Systems equipped with a ViscoLab Piston (0.3055", 1-20 cP)). The viscometer is calibrated before use with the appropriate standards (*e.g.,* S6S Reference Standard from Koehler Instrument Company, Inc.). The viscometer is connected to a water bath to equilibrate the system to 20°C. Piston is checked using S6S viscosity reference standard (8.530 cP @ 20.00°C). Piston is also checked using RODI $H_2O$ (1.00 cP @ 20.0°C). The piston is cleaned and rinsed thoroughly with soap and water between measurements of each different solution type. Subsequently the system is cooled to $\leq$ 2°C. Once the system temperature is at or below 2°C, sample is loaded into the chamber and the piston is lowered into the sample. After sample is equilibrated to the temperature of the chamber, measurement is initiated. The temperature is increased at 1°C increments every 7-10 minutes to a final temperature of $\geq$ 25°C. The viscosity result is recorded immediately prior to increasing the temperature. The piston remains in motion during measurements to minimize the need for re-equilibration.

6.1.6. Differential Scanning Calorimetry

Differential Scanning Calorimetry (DSC) may be used to ascertain changes over time in the thermal stability of an antibody in a particular formulation. Thermal melting temperatures ($T_m$) are determined with a differential scanning calorimeter (*e.g.,* VP-DSC from MicroCal, LLC) following the manufacturer's instruction. In one example, VP-DSC is used at a scan rate of 1.0°C/min and with a temperature range of 25 -120°C. A filter period of 8 seconds is used along with a 5 minute pre-scan thermostating. Samples are prepared by dialysis into 25 mM Histidine-HCl, pH 6 using Pierce dialysis cups (3.5 kD). Average Mab concentrations are 500 $\mu$g/mL as determined by $A_{280}$. Melting temperatures are determined following the manufacturer's instructions using software supplied with the system.

6.1.7. Liquid Chromatography Mass Spectrometry (LC-MS)

Liquid Chromatography Mass Spectrometry (LC-MS) may be used to characterize a degradation fragment detected by SEC or AUC in the antibody formulation.

Peak SEC column fractions containing the degradation fragment are collected and digested with N-Glycosidase F, also known as PNGase F, at 37°C overnight. PNGase F is an amidase used to deglycosylate protein samples. The enzyme cleaves between the innermost GlcNAc and asparagine residues of high mannose, hybrid and complex oligosaccharides on N-linked glycoproteins. The deglycosylated samples mixed with a reducing buffer (*e.g.,* 2.5 mg/mL DTT, 6.0 M guanindine HCl, pH 8.2) and kept at 56 °C in a water bath for 60 minutes. Neat 4-vinylpyridine (*e.g.,* Aldrich Chem. Co., WI) is then added to the sample, and the reaction mixture is held at ambient temperature for 30 minutes. The deglycosylated, reduced and alkylated sample is immediately loaded onto a reversed phase column in order to separate the modified samples from the reactants.

Deglycosylated, reduced, and alkylated samples are fractionated using a reversed phase column (*e.g.,* Jupiter 5$\mu$m C4, 300 Å, 250 x 2.00 mm, Phenomenex) with a binary gradient HPLC system (Agilent 1100). Mobile phase A consists of 30% acetonitrile in water with 0.1% trifluoroacetic acid and mobile phase B consists of 50% acetonitrile in water with 0.1% trifluoroacetic acid. The samples are separated using a linear gradient of 30-50% acetonitrile in water, over 16 min. with a flow rate of approximately 200 $\mu$L/min. The column effluent is directed to a UV detector and then split 1:1, one half going through a switching valve on an Ion Trap mass spectrometer (*e.g.,* LTQ, ThermoElectro, San Jose, CA), and the remaining half to waste.

The ion-trap mass spectrometer is calibrated before the experimental run using a mixture of caffeine, L-methionyl-arginyl-phenylalanyl-alanine acetate $H_2O$, and Ultramark 162. The Electrospray Ionisation Mass Spectrometry (ESI-MS) data is acquired in positive ESI full scan mode. The BioWork deconvolution program (ThermoFinnigan) may be used to reconstruct the mass spectra and obtain the molecular masses of the peptides/proteins from their original mass spectra. The mass data subsequently is used to determine the identity of the degradation fragment.

6.1.8. Isoelectric Focusing Gel Electrophoresis

Isoelectric point measurements of may be used to ascertain the antibody's chemical stability in a given formulation. Isoelectric points are determined using a Pharmacia Biotech Multiphor 2 electrophoresis system with a multi temp 3 refrigerated bath recirculation unit and an EPS 3501 XL power supply. Pre-cast ampholine gels (Amersham Biosciences, pI range 2.5-10) are loaded with 5 $\mu$g of protein. Broad range pI marker standards (Amersham, pI range 3-10, 8 $\mu$L) are used to determine relative pI for the Mabs. Electrophoresis is performed at 1500 V, 50 mA for 105 minutes. The gel is fixed using a Sigma fixing solution (5x) diluted with purified water to 1x. Staining is performed overnight at room temperature using Simply Blue stain (Invitrogen). Destaining is carried out with a solution of 25% ethanol, 8% acetic acid and 67% purified water. Isoelectric points are determined using a Bio-Rad Densitometer relative to calibration curves of the standards.

6.1.9. Disulfide bond determination

Disulfide bond determination protocols may be used to monitor the stability of disulfide bridge crosslinks in a particular antibody formulation. Antibody samples are denatured, for example, in 10 mM phosphate buffer, 250 mM NaCl, 5 mM NEM, 6 M Guanidine, pH 7.0 at 37 °C for 1 to 3 hr. The denatured samples are diluted 6 fold with 100 mM phosphate buffer, 0.1 mM EDTA, pH 7.0, to which Endoproteinase Lys-C (*e.g.,* Roche) is added at a 1:10 enzyme to protein ratio. The reaction mixtures are incubated at 37 °C for 16 to 24 hours. In half of the reaction mixture disulfide bridges are reduced by adding 5-10 $\mu$L of 100 mM DTT followed by incubation at 37 °C for 1 hr. Lys-C digested samples are fractionated by reverse-phase HPLC (*e.g.,* Phenomenex Jupiter 5m C18 column; 250 x 2.1 mm). Eluant is analyzed by an UV-detector and an in-line LCQ or LTQ Ion Trap mass spectrometer (*e.g.,* ThermoElectron). The RP-HPLC mobile phase A is 0.1% TFA in $H_2O$ and mobile phase B is 0.1% TFA in acetonitrile. The peptides are eluted at a flow rate of 0.2 mL/min with the following step gradient: 1) 0-2 min, 5% Mobile Phase B; 2) 2-32 min, 5-20% Mobile Phase B; 3) 32-132 min, 20-40% Mobile Phase B; 4) 132-152 min, 40-60% Mobile Phase B; 5)152-155 min, 60-95% Mobile Phase B.

The ion-trap mass spectrometer is calibrated before the experimental run using a mixture of caffeine, L-methionyl-arginyl-phenylalanyl-alanine acetate $H_2O$, and Ultramark 162. The Electrospray Ionisation Mass Spectrometry (ESI-MS) data is acquired in positive ESI full scan mode. The BioWork deconvolution program (ThermoFinnigan) may be used to reconstruct the mass spectra and obtain the molecular masses of the peptides from their original mass spectra. Comparison of the mass data acquired using the DTT reduced and non-reduced samples allows the identification of the disulfide crosslinked peptides.

6.1.10. Binding affinity characterization

Binding affinity of monoclonal antibody recovered form the formulation following long term storage (e.g., 1 month at 40°C or 6 months at 5°C) may be determined by surface plasmon resonance (see, *e.g.,* Jonsson et al., Biotechniques 11(5):620-627 (1991); Johne, B., Molecular Biotechnology 9(1):65-71(1989)) using a BIAcore 3000 instrument (BIAcore, Inc., Piscataway, NJ). Antibody is captured on a Prot-G coated CM5 chip. A Prot-G coated CM5 chip with captured isotype control human-IgG (Sigma) antibody is used for reference purposes. ICOS-Fc fusion protein dissolved in HBS-EP running buffer is passed over the chip at a rate of 25 ul/min. 5 minutes of association time is followed by a 10 minute dissociation period. Independent measurements are performed by exposing the chips to different concentrations of ICOS-Fc (*e.g.* concentrations between 10 nM and 80 nM). Chips are regenerated by a 0.4 minute wash with 20 mM NaOH + 400mM NaCl at a flow rate of 100ul/min. Once the entire data set is collected, the resulting binding curves are globally fitted to a 1:1 Langmuir binding model using BIAevaluation software (BIAcore, Inc., Piscataway, NJ). This algorithm calculates both the association rate ($k_{on}$) and the dissociation rate ($k_{off}$), from which the apparent equilibrium binding constant, $K_D$, is deduced as the ratio of the two rate constants, $k_{off} / k_{on}$. A more detailed explanation of how the individual rate constants are derived can be found in the BIAevaluation Software Handbook (BIAcore, Inc., Piscataway, NJ).

**6.2.** Formulation development

The physicochemical properties of the afucosylated 136 antibody were characterized as follows. The DSC profile of the afucosylated 136 was determined as described above. DSC measurements were performed in 25 mM histidine (pH 6.0) buffer. The DSC profile of the afucosylated 136 antibody (Figure 1) is essentially identical to that of the fucosylated parent antibody.

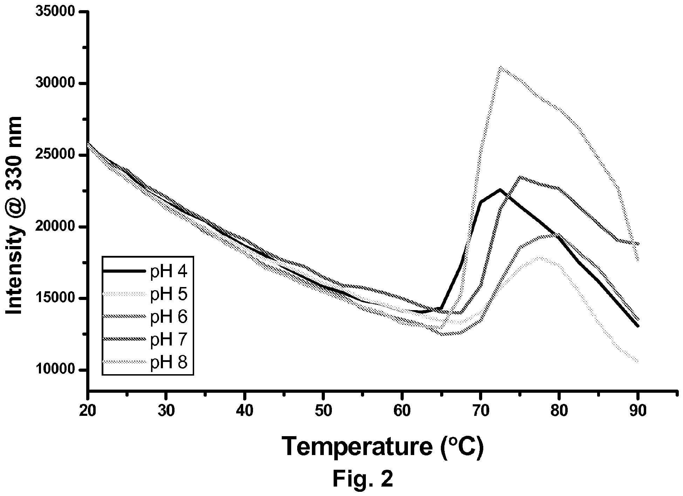

The effect of pH on the thermal stability of the 136 antibody was ascertained by measuring tryptophan fluorescence as a function of temperature at various pHs. A representative sample of the experimental results is shown in Figure 2. The 136 antibody appeared stable in the pH 5-7 range.

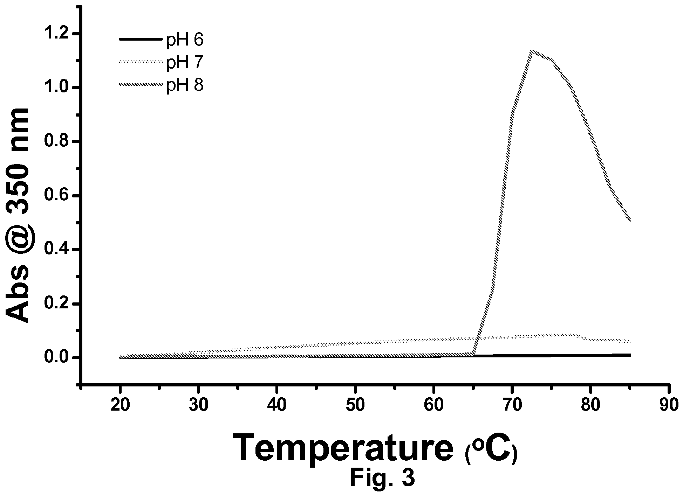

The effect of formulation pH on colloidal stability was assessed by measuring turbidity (A350 nm) of various 136 formulations as a function of temperature. A representative sample of the experimental results is shown in Figure 3. The 136 antibody appeared stable in the pH range of 4-7.

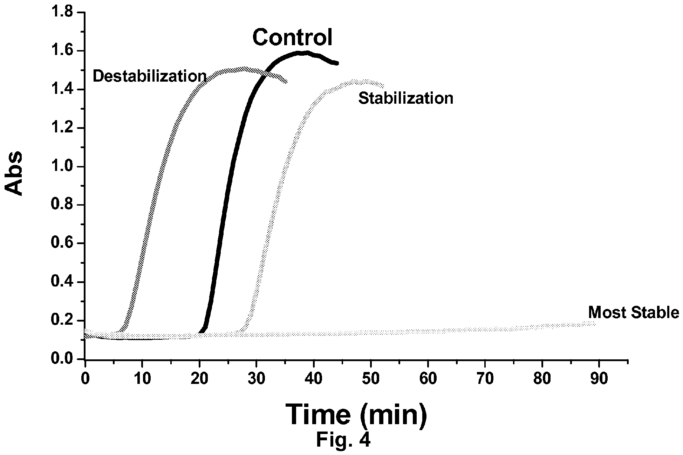

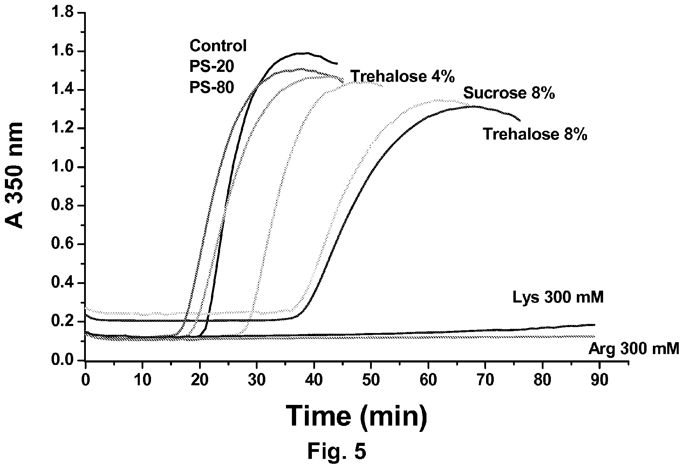

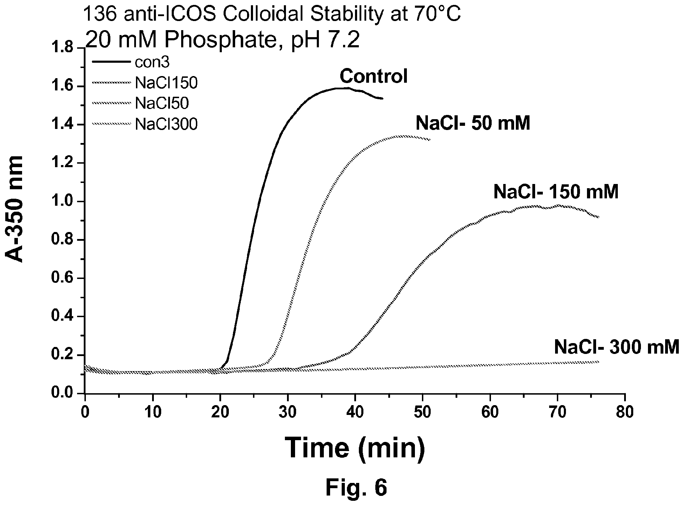

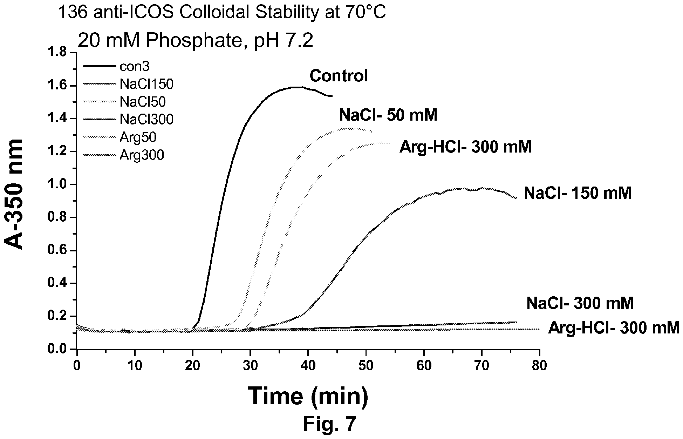

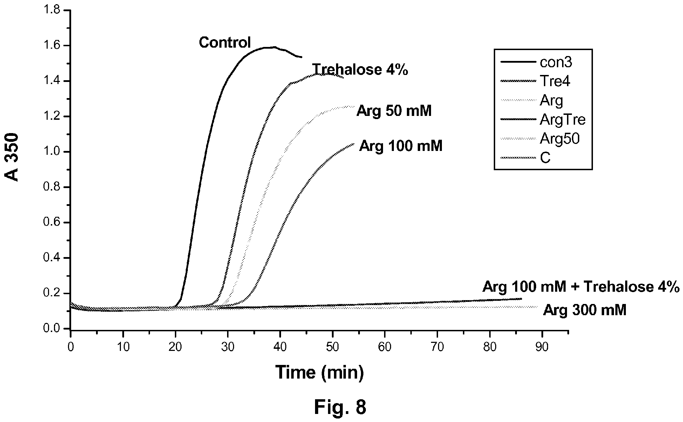

A high throughput screen was designed to identify excipients that can stabilize the 136 antibody. A metastable control buffer (20 mM phosphate at pH 7.2) was selected for the screen based on the pH sensitivity of the 136 antibody. Excipients were screened by comparing the colloidal stability of 136 in the control buffer to the colloidal stability of 136 in a buffer comprising a single additional excipient. Changes in colloidal stability were followed by measuring the turbidity (A 350 nm) of the formulation over time. Stabilizing excipients decelerate or eliminate the increase in turbidity over time while destabilizing excipients accelerate the increase in turbidity. A representative result is shown in Figure 4. Turbidity changes in a buffer comprising a stabilizing excipients were slowed down or eliminated compared to the rate of turbidity change observed in the control buffer. The high throughput screen identified histidine, citrate, arginine, lysine, sodium chloride and trehalose as stabilizing excipients for the 136 antibody (Figures 5-7). Additional experiments were performed to ascertain the effect of combinations of these excipients on 136 stability (Figure 8). The most pronounced stabilizing effect was observed with 100 mM arginine or lysine when used in combination with 4% trehalose.

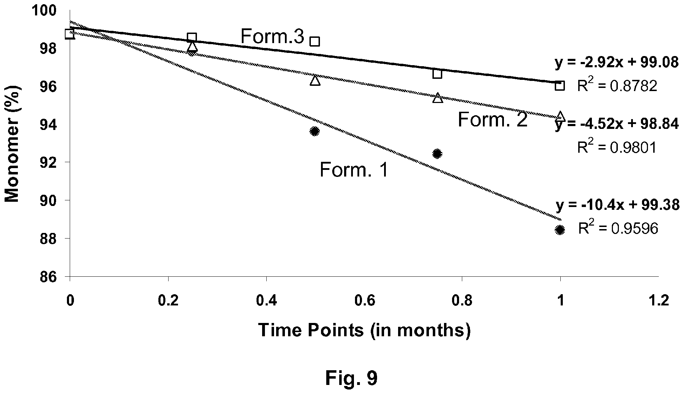

Based on the results of the high throughput screen three candidate formulations were selected for long term stability experiments. Formulation 1 comprised 10 mM histidine (pH 6.0); Formulation 2 comprised 10 mM histidine (pH 6.0) and 150 mM NaCl; Formulation 3 comprised 10 mM histidine (pH 6.0), 100 mM arginine-HCl and 4% trehalose. The stability of the 136 antibody (60 mg/ml) in these formulations was assessed by measuring the monomer concentration following storage at 40°C. Monomer concentration was determined by SEC as described herein. An example of the stability results obtained are shown in Figure 9. The results confirmed that the addition of arginine/trehalose or NaCl increased the stability of the 136 antibody. Arginine/trehalose had a stronger stabilizing effect than that of NaCl alone.

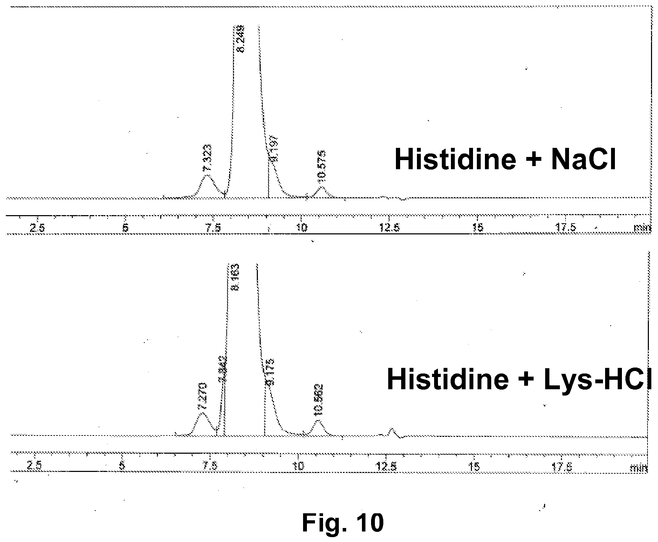

The stability of 136 antibody was also ascertained in lysine containing formulations. 136 antibody recovered form Formulation 2 (10 mM histidine (pH 6.0) and 150 mM NaCl) and Formulation 4 (10 mM histidine (pH 6.0), 100 mM lysine-HCl and 4% trehalose) following 1 month incubation at 40°C was analyzed on HP-SEC. Representative elution profiles are shown in Figure 10. The main monomer peak in the elution profile of 136 recovered form Formulation 4 displays a pronounced shoulder. The main monomer peak of the 136 antibody recovered form arginine containing formulations also displayed the same shoulder. The presence of the shoulder indicates monomer/dimer mixture formation in lysine and arginine containing formulations. Such a shoulder is not detected in the elution profile of 136 antibody recovered from Formulation 2.

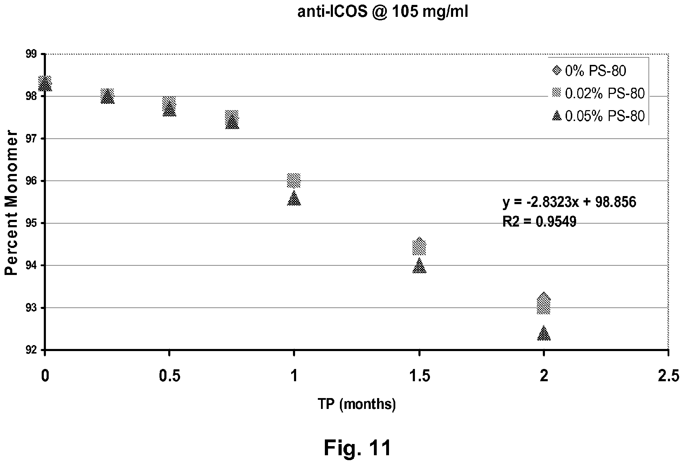

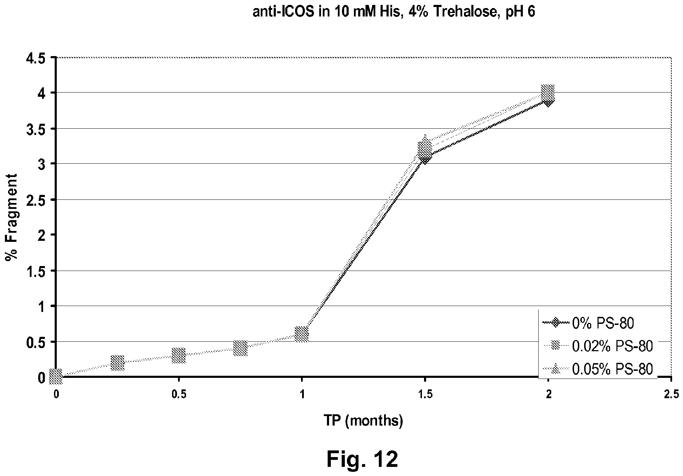

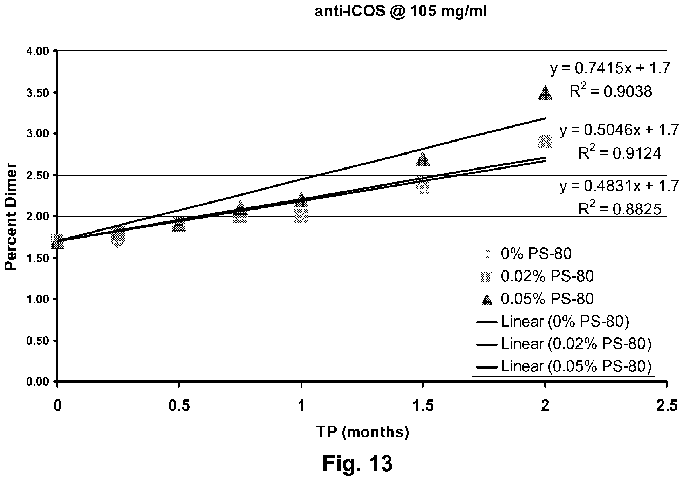

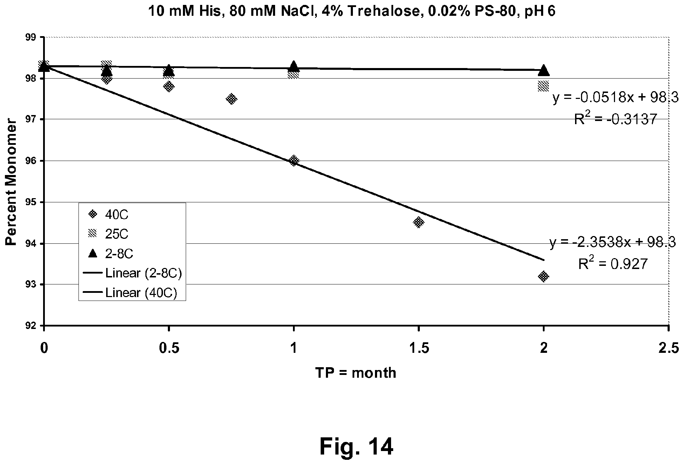

A formulation comprising 10 mM histidine (pH 6.0), 80 mM NaCl, 4 % trehalose and 0%, 0.02% or 0.05% polysorbate was selected for further stability studies. The aggregation and fragmentation profile of 136 in these formulation following storage at 5°C, 2°C or 40°C was determined using methods described herein. Representative results from these experiments are presented in Figures 11-15. Data shown demonstrates that the formulation comprising 100 mg/ml, 10 mM histidine (pH 6.0), 80 mM NaCl, 4% trehalose and 0.02% polysorbate 80 is highly stable liquid formulation.

**6.3.** Formulation stability in pre-filled syringes.

The stability of a formulation comprising 100 mg/ml antibody, 10 mM histidine, 80 mM NaCl, 4% trehalose and 0.02% PS-80 at pH 6.0 may be tested in pre-filled syringes. Stability testing is performed by loading 1 ml of the formulation into a syringe and storing the formulation-filled syringe at 5°C, 25°C or 40°C for extended periods of time. Formulation stability is analysed using the analytical methods described herein. Particle formation, a key determinant of formulation stability in pre-filled syringes, is assessed by visual inspection. Protein aggregation and/or fragmentation is assessed by subjecting the formulation recovered from the syringe to an analytical method known to one of skill (e.g., SEC).

**6.4.** Depletion of ICOS bearing T cells prevents disease in a GvH model of scleroderma.

In this study, the function of ICOS in the pathogenesis of a graft-versus-host disease (GvHD) mouse model of scleroderma (SSc) was investigated using a glycoengineered anti-mouse ICOS MAb with enhanced antibody-dependent cellular cytotoxicity (ADCC). An afucosylated rat anti-murine ICOS monoclonal antibody (IgG2a) directed against the ligand binding domain of murine ICOS was produced in a fucosyltransferase 8-deficient Chinese Hamster Ovary (CHO) producer cell line (BioWa Potelligent® Technology). The activity of the afucosylated anti-mouse ICOS MAb, was evaluated in a murine GvHD model, which recapitulates key aspects of human SSc, including inflammation, fibrosis, and vasculopathy.

Dosing of the anti-ICOS-aFuc MAb reduced severity and incidence of dermal lesions when compared to isotype control MAb and control syngeneic graft. Mean clinical scores were significantly reduced in anti-ICOS-treated groups compared to isotype control MAb-treated mice, as early as 11 days post-graft (3.4-fold, $p<0.05$), and thereafter up to 4 weeks post graft (8.1-fold, $p<0.005$). The anti-ICOS-aFuc MAb also prevented the disease-associated accumulation of TFH cells and the associated expansion of germinal center B cells and immunoglobulin secreting B cells. There were no ICOS MAb-related clinical signs or changes in body weight in animals during the study.

The results indicate that ICOS plays an important role in dermal pathology of murine GvHD-SSc, as depletion of ICOS+ T cells reduced the overall clinical disease score. The identification of dysregulated ICOS+ TFH cells in GvHD-SSc underscores their critical function in driving the generation of pathogenic B cells into germinal center B cells in secondary lymphoid tissues and in the differentiation of immunoglobulin secreting B cells in the skin. Importantly, treatment with the anti-mouse ICOS-aFuc MAb resulted in a significant reduction of the clinical signs of disease.

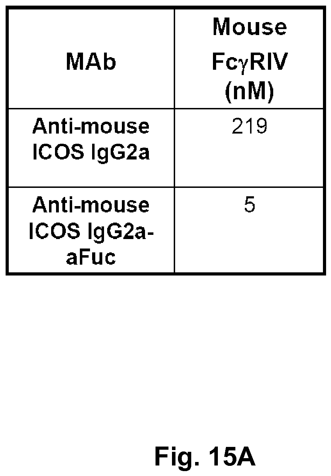

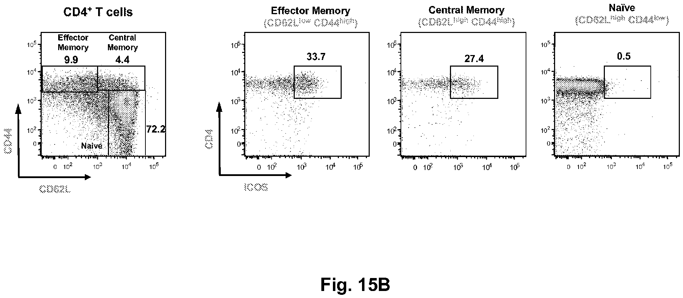

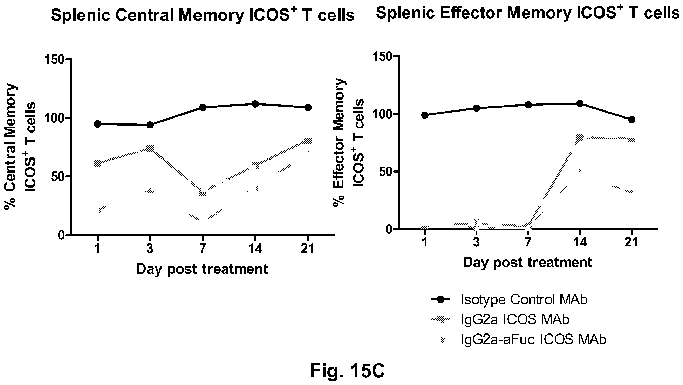

BIAcore binding affinity of the fucosylated and afucosylated anti-ICOS MAb to mouse FcgRIV is shown in Fig. 15A. Enhanced binding of ICOS MAb Fc to the FcgRIV expressed on effector cells was expected to increase antibody-dependent cellular cytotoxicity (ADCC). Figure 15B shows the immuno-phenotype characterization in the steady state of ICOS expression on naive splenic naive and T helper memory cells (central and effector). Splenocyte isolated from naive Balb/c mice were processed and stained with the indicated markers to identify the expression profile of ICOS on T helper cell subpopulations. Fucose free anti-ICOS MAb (IgG2a-aFuc) mediated more effective depletion of ICOS bearing T cells (Figure 15C). Pharmacodynamic analysis of splenic helper central and effector memory ICOS bearing T cells was determined upon one single intraperitoneal injection of the indicated anti-ICOS MAbs into naive Balb/c mice (250 μg/animal).

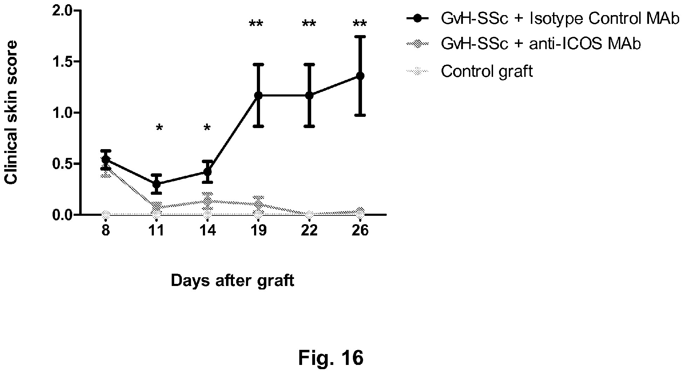

The GvH model used in this study was described in Zhou L., J Invest Dermatol, 2006, 126(2): 305-14. Balb/c hosts were grafted with T cells from a B10D2 donor. Control animals were grafted with syngenic Balb/c T cells. Antibody was administered at days 7, 14 and 21 post-graft. Administration of anti-ICOS MAb (IgG2a-aFuc) reduced graft versus host scleroderma clinical score (Figure 16). Mean clinical disease score was evaluated following biweekly treatment (starting time: day 8) with anti-mouse ICOS IG2a-aFuc or isotype control MAb (n=10). Baseline skin scores measurements were obtained on study day 6, and biweekly until study day 26, when animals were euthanized for tissue harvest. Skin was scored as follows: 0= normal; 1 = lesion <1 cm2; 2= lesion 1-2 cm2; 3 = lesion > 2 cm2. Extremities (ear, tail, paws) appearing scaly were given a score of 0.3, for a maximum total score of 3.9 per animal (*$p<0.05$, **$p<0.005$).

Anti-ICOS MAb mediated effective elimination of ICOS bearing TFH and inhibited the expansion of germinal center B cells. Figure 17 shows the immunophenotype analysis of spleen, lymph node and peripheral blood Th memory and Th memory ICOS+ cells (gated as indicated in Fig.1C) isolated from Balb/c control mice and from rag2 deficient mice treated with either anti-ICOS or isotype control MAb. Anti-ICOS therapy prevents the expansion of TFH cells (Figure 17D). While anti-ICOS MAb does not alter the overall number of total splenic B cells (CD19+) (Figure 17E), it significantly inhibited the TFH-mediated expansion of germinal center B cells (Figure 17F). Depletion of ICOS bearing T cells did not perturb the overall CD4+ (Figure 17G) and CD8+ (Figure 17H) T cell compartments.

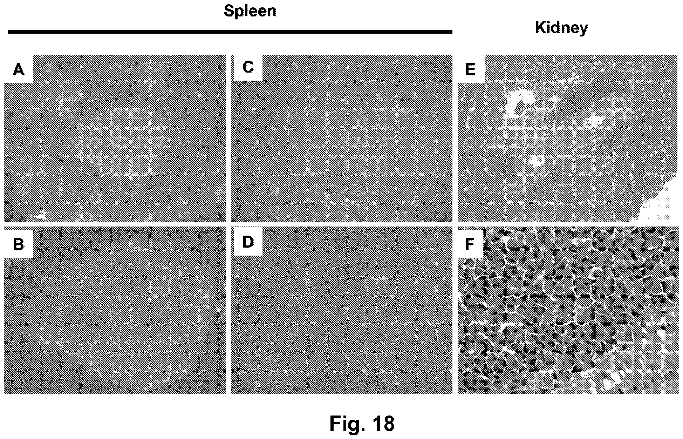

Histology of RAG2-/- spleen and kidney from an isotype control MAb treated animal and anti-ICOS MAb treated animal is shown in Figure 18. Higher magnification (x200) of the spleen demonstrates lack of germinal center formation in anti-ICOS-treated animals compared to the isotype. In the kidney, there was moderate perivascular cuffing (E) with lymphocytes admixed with fewer plasma cells (F, inset). Original magnification, x100; inset x1000.

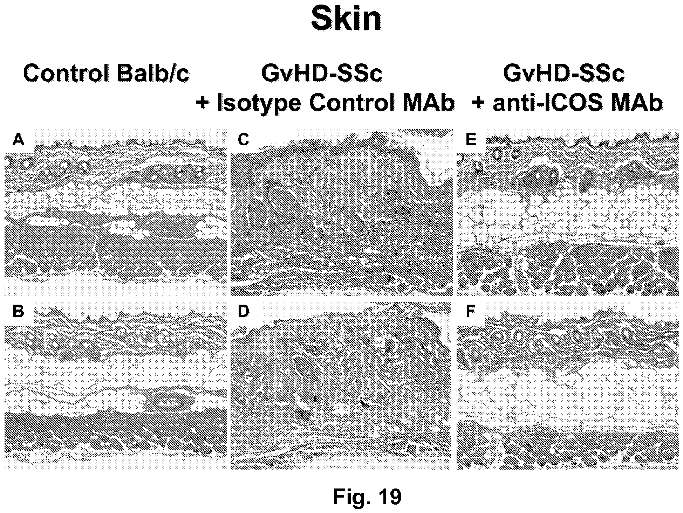

Treatment with anti-ICOS MAb significantly inhibited the development of GvHD-SSc skin pathology. Histology of back skin from either Balb/c, or RAG2-/- mice grafted with splenocytes at 4 weeks from isotype control MAb group and anti-ICOS MAb treated group is shown in Figure 19. Hematoxylin and eosin stain (Figure 19A, C and E) of skin representing 2/10 animals in the isotype group MAb demonstrates marked deep dermal inflammation with infiltration of lymphocytes, scattered neutrophils and macrophages within increased collagenous matrix. Diffusely, the epidermis is thickened with apoptosis and necrosis of individual basal cells and within the inner root sheath of hair follicles. Masson's Trichrome stain (Figure 19B, D and F) demonstrates increased immature collagen within the dermis of the isotype group. There was minimal to no skin inflammation in the anti-ICOS MAb treated animals. Original magnifications, x200.

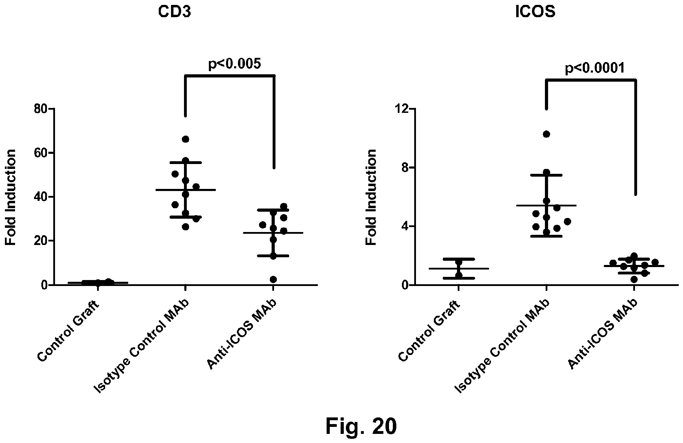

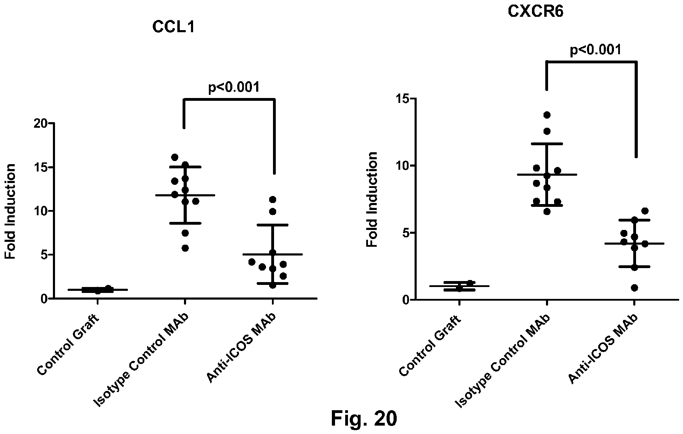

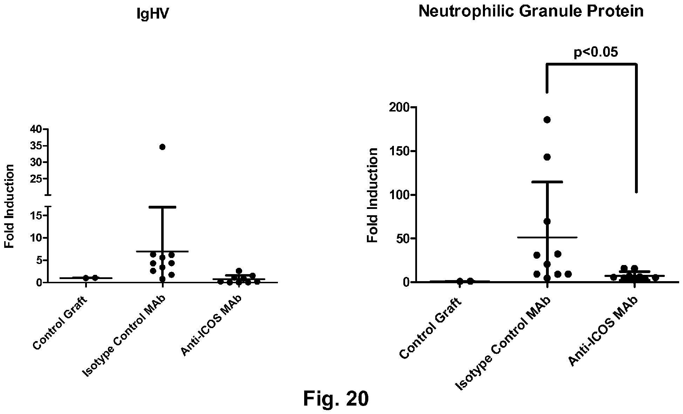

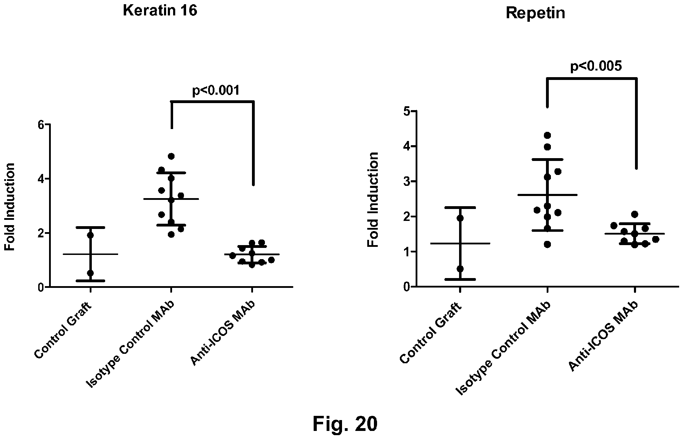

ICOS MAb treatment impacted expression of T helper- and TFH-associated genes and the autoimmune-gene fingerprint in the skin. Total RNA was extracted from skin biopsies and preamplification of cDNA and real-time PCR were prepared using the TaqMan PreAmp Master Mix Kit (Applied Biosystems). Skin RNA samples, collected from Balb/c control mice and from Rag2 knockout mice treated with anti-ICOS or isotype control MAb, were run in triplicate using TaqMan Gene Expression Assays in the BioMark 48.48 Dynamic Array chips (Fluidigm Corp). Delta-delta Cts (ΔΔCt) were calculated using the mean of the 3 reference genes (GAPDH, 18S, ACTB) and a calibrator sample, and were converted to fold expression change by the 2-ΔΔCt formula. Results are presented in Figure 20.

**6.5. Reversible self-association of the 136 anti-ICOS antibody**

Analytical ultracentrifugation analysis performed on 136 anti-ICOS antibody showed that the major peak of was broader than observed for a typical IgG. At protein concentrations of 0.1 mg/mL, 0.5 mg/mL, and 2.0 mg/mL, at 25°C in PBS, size distribution analysis showed a broadening of the peak and shift to higher S (sedimentation coefficient; Svedberg) values with increasing protein concentration, which is indicative of self-association. Sedimentation equilibrium experiments showed that the stoichiometry of the self-association best fit a monomer-trimer equilibrium model, with an association constant of $2.5 \times 10^8$ M$^{-2}$.

Dynamic Light Scattering (DLS) was used to study 136 anti-ICOS antibody self-association and to screen for conditions/excipients to prevent or minimize self association. The DLS system determines the size of a particle by first measuring the Brownian motion, which is the random movement of particles suspended in a fluid. When a coherent and monochromatic light beam passes through a colloidal dispersion, the particles scatter the light and because of Brownian motion results in time-dependent fluctuation in the scattered intensity. Analysis of the time dependence of the intensity fluctuation can yield the diffusion coefficient of the particles which is dependent on the size of the particle. Hydrodynamic radius of the particles is then calculated from the diffusion coefficient via Stokes Einstein equation.

6.5.1. Methods

Dynamic Light Scattering: The protein size distribution and hence molecular size were monitored by dynamic light scattering (DLS) using a Zetasizer Nano ZS (Malvern Instruments, Malvern, PA). This instrument incorporated noninvasive backscattering optics that could measure protein sizes in the range of 0.6 nm to 6 $\mu$m. DLS measures the time-dependent fluctuations in the intensity of scattered light due to Brownian motion of the protein molecules. The analysis of these intensity fluctuations enabled the determination of the diffusion coefficients of particles, which were mathematically converted to an average apparent hydrodynamic diameter of an equivalent sphere using the Strokes Einstein relationship. The diffusion coefficient was calculated from the time correlation function. To understand the reversible self-association of proteins, the time-dependent autocorrelation function of the photocurrent was acquired every 10 seconds, with 15-18 acquisitions for each run. The sample solution was illuminated using a 633 nm laser, and the intensity of scattered light was measured at an angle of 173 degrees. After correcting for viscosity, refractive index and absorbance, the DLS measurements provided accurate estimates of both hydrodynamic diameter and its Gaussian distribution, which was used to monitor potential self-association behavior.

Data Analysis to Calculate Percent Monomer and Trimer Fraction: Sedimentation equilibrium experiments showed that the stoichiometry of the self-association best fit a monomer-trimer equilibrium model. Hydrodynamic size obtained by DLS under various conditions was fitted to a monomer-trimer equilibrium to derive an association constant from which the mole percent of self-associated species was calculated under various conditions. Apparent size in DLS measurements was taken to be a weighted average of that of monomer (9.0 nm) and trimer (35.6 nm), multiplied by their respective fractional presence (from 0 to 1, depending on the solution conditions). As the total concentration is known, the concentration of monomer equals:

$$P_{Monomer} = P_{Total} \times 1 - \frac{[R_{H-Obs} - R_{H-Monomer}]}{[R_{H-Trimer} - R_{H-Monomer}]}$$

The concentration of trimer is then:

$$P_{Trimer} = \frac{\{[P_{Total}] - [P_{Monomer}]\}}{3}$$

And the association constant (in M$^{-2}$) is:

$$\frac{[P_{Trimer}]}{([P_{Monomer}])^3}$$

Where, $P_{Total}$ is 136 anti-ICOS antibody concentration in M, $R_{H-Obs}$ is observed hydrodynamic diameter in nm, $R_{H-Monomer}$ is hydrodynamic diameter of Monomer and $R_{H-Trimer}$ is hydrodynamic diameter of Trimer.

6.5.2. Data and Discussion

Reversible Self-Association (RSA) of proteins may result from relatively weak non-covalent protein interactions which is usually charge and hydrophobic interactions. Since the system is reversible, there will be equilibrium between the monomer and higher order forms and this equilibrium can be shifted depending on solution conditions. The following section describes the effect of changing protein concentration, pH, ionic strength and temperature on self association of 136 anti-ICOS antibody will be discussed.

6.5.2.1. Development of DLS as an effective tool to study RSA in 136 anti-ICOS antibody Correlation between DLS and AUC

Monoclonal antibodies (MW~ 150 kDa) typically have a hydrodynamic diameter of 9-12 nm and a Gaussian distribution of ~ 3 nm. A larger hydrodynamic diameter and a wider Gaussian distribution can each be indicative of self-association. Hence hydrodynamic diameter was monitored under various solution conditions to determine self association behavior of 136 anti-ICOS antibody. Before studying the effect of various solution conditions on RSA of 136 anti-ICOS antibody using DLS, we established the correlation between DLS and well established orthogonal technique (to study RSA) like AUC.

6.5.2.2.Hydrodynamic size of the 136 anti-ICOS antibody under various conditions. Effect of concentration

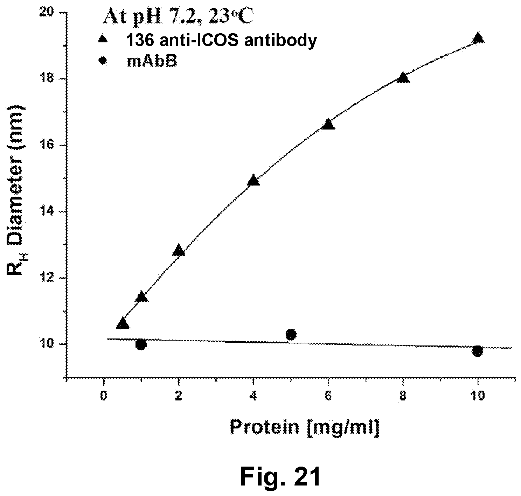

AUC analysis of the 136 anti-ICOS antibody had showed a broadening of the peak and shift to higher S (sedimentation coefficient; Svedberg) values with increasing protein concentration. In order to confirm these observations and also check if similar self-association can be picked up by DLS, the hydrodynamic diameter of the 136 anti-ICOS antibody was determined at various protein concentrations. Similar to AUC (an increase in weight average sedimentation coefficient) an increase in hydrodynamic diameter at higher protein concentration would be expected if significant self-association is present. Figure 21 shows the hydrodynamic diameter of the 136 anti-ICOS antibody over a wide range of concentrations. A concentration dependent increase in hydrodynamic diameter was observed in the wide range studied. This strongly indicates that the 136 anti-ICOS antibody is undergoing RSA at high protein concentration. Where as, a control non interacting monoclonal antibody of similar size showed no change in hydrodynamic diameter at the same concentration.

Effect of pH and Ionic strength

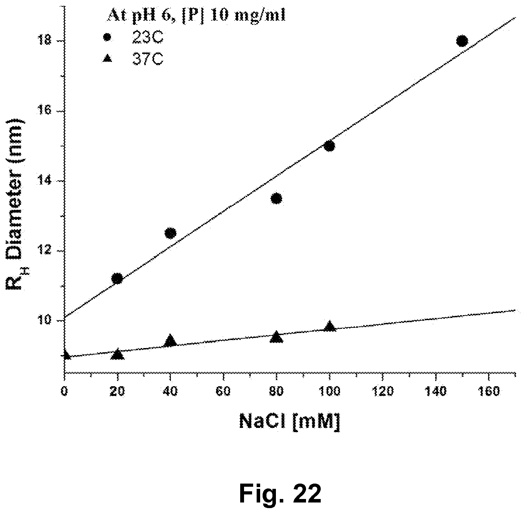

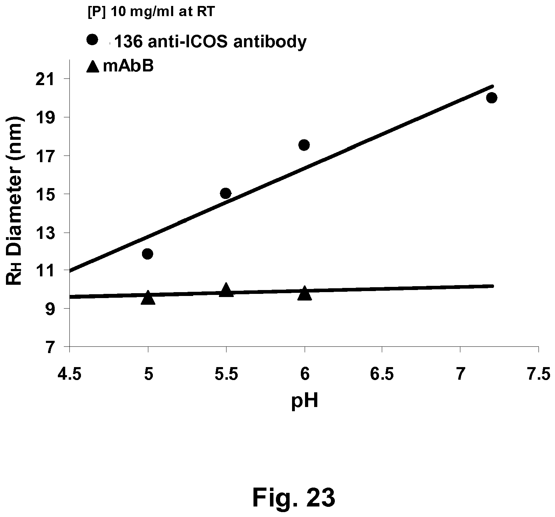

The 136 anti-ICOS antibody RSA was sensitive to increasing ionic strength (increasing NaCl concentration) and pH, suggesting the importance of charge interactions. Figure 22 shows the hydrodynamic size of the 136 anti-ICOS antibody for a fixed concentration (10 mg/ml) in presence of increasing NaCl concentrations.
DLS measurements of the 136 anti-ICOS antibody at various pH revealed that the extent of self association also increased with pH (Figure 23). Significant impact of both pH and ionic strength suggested the importance of charge interactions in the 136 anti-ICOS antibody RSA.

Effect of Temperature

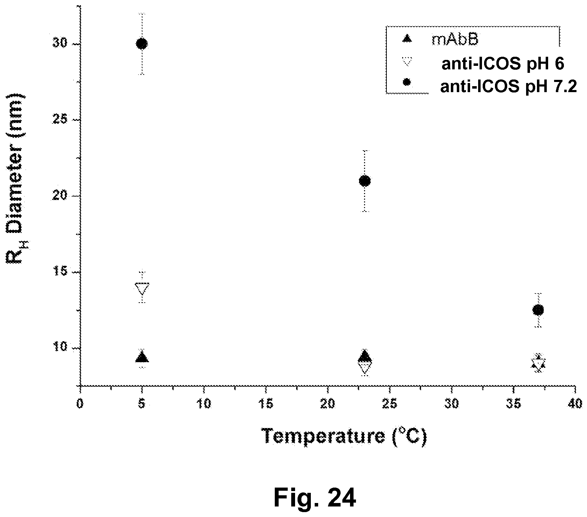

The 136 anti-ICOS antibody hydrodynamic size were determined under various temperatures to understand the effect of temperature on the 136 anti-ICOS antibody RSA. Fig 24 shows the hydrodynamic size of the 136 anti-ICOS antibody at various temperatures at pH 6 and pH 7.2 along with a control antibody (mAbB).

Kinetics of Temperature induced Dissociation

The kinetics of dilution- or temperature-induced dissociation of the 136 anti-ICOS antibody was measured using two methods: rapid dilution by static light scattering, and temperature shift by DLS. Rapid dilution experiments with single angle light scattering detection demonstrated that the rate of dissociation is rapid with a half-time lower than the detection limit of 0.1 second. The kinetics of temperature-induced dissociation of the 136 anti-ICOS antibody self-association (10 mg/mL, in PBS) as measured by DLS is presented in. With a temperature increase from 25°C to 37°C, a substantial decrease in the hydrodynamic diameter was observed within the first measurable time point of 1 minute. A similar decrease in the hydrodynamic diameter was observed upon a temperature increase from 4°C to 37°C within the first measurable time point of 3 minutes. These results indicated that temperature-induced dissociation was rapid.

6.5.2.3. Level of the 136 anti-ICOS antibody Self-association under various solution condition

Dynamic light scattering studies showed an increase in the 136 anti-ICOS antibody hydrodynamic size (indicative of self-association) at higher concentration, lower temperature, higher pH, and increased salt concentration. Also, analytical ultracentrifugation data suggested a monomer-trimer equilibrium. Therefore, the 136 anti-ICOS antibody hydrodynamic size obtained under various condition was fitted to a monomer-trimer equilibrium to derive an association constant from which the mole percent of self-associated species was calculated under various conditions. Modeling was used to determine the mole percent of self-associated 136 anti-ICOS antibody from the DLS derived association binding affinities, as a function of protein concentration.

Table 2. Mole percent of self-associated 136 anti-ICOS antibody from the DLS. Formulation buffer comprises 10 mM histidine (pH 6.0), 80 mM NaCl, 4% trehalose and 0.02% polysorbate 80.

| Protein Cone. | Buffer | Temperature | Percent Monomer |
|---|---|---|---|
| 10 | Formulation Buffer | 23° C | 80 |
| 1 | Formulation Buffer | 23° C | 99 |
| 0.01 | Formulation Buffer | 23° C | 100 |
| 10 | PBS | 37° C | 85 |
| 1 | PBS | 37° C | 99 |
| 0.01 | PBS | 37° C | 100 |

In conclusion, the reversible self-association of the 136 anti-ICOS antibody observed by analytical ultracentrifugation analysis was dependent on protein concentration and temperature. The kinetic studies indicated that rapid dissociation occurred upon dilution and at increased temperature. The reversible self-association did not induce formation of aggregates. On the basis of the rapid dilution experiments and modeling calculations, upon subcutaneous injection of the highest clinical dose (3 mg), the rapid dilution and temperature equilibration to 37°C (body temperature) will result in a primarily monomeric form of the 136 anti-ICOS antibody.

## Claims

1. A sterile, stable aqueous formulation comprising an antibody that specifically binds human ICOS, wherein said formulation comprises 10 mg/ml of the antibody, 80 mM NaCl, 10 mM histidine, 4% trehalose and 0.02% polysorbate 80 and wherein the pH of the formulation is 6; and
   wherein

   a) the antibody comprises an Fc region having complex N-glycoside-linked sugar chains in which fucose is not bound to N-acetylglucosamine in the reducing end in the sugar chain,
   b) the antibody undergoes reversible self-association in solution, wherein the reversible self-association does not induce aggregate formation, and
   c) at least 10 mole percent of the antibody exists as a trimer in PBS at 10 mg/ml antibody concentration at 37°C; and

   wherein the antibody comprises a heavy chain sequence of SEQ ID NO: 6 and a light chain sequence of SEQ ID NO: 1.

2. The formulation of claim 1, wherein said formulation is isotonic.

3. The formulation of claim 1 or claim 2, wherein said formulation is a pharmaceutically acceptable formulation.

4. The formulation of any one of claims 1 to 3, wherein said formulation is an injectable formulation, and optionally wherein the formulation is suitable for intravenous, subcutaneous, or intramuscular administration.

5. A pharmaceutical unit dosage form suitable for parenteral administration to a human which comprises an antibody formulation of any one of claims 1 to 4 in a suitable container.

6. A pre-filled syringe containing the formulation of any one of claims 1 to 4.

7. The formulation of any one of claims 1 to 4;

a) for use in treating an autoimmune disease or disorder in a human; or
b) for use in treating or preventing rejection in a human transplant patient; or
c) for use in treating an inflammatory disease or disorder in a human; or
d) for use in depleting ICOS expressing T cells in a human patient; or
e) for use in disrupting germinal center architecture in a secondary lymphoid organ of a primate.

8. The formulation for use of claim 7, wherein the autoimmune disease or disorder is SLE or scleroderma.

**Patentansprüche**

1. Sterile stabile wässrige Formulierung umfassend einen Antikörper, der spezifisch menschlichen ICOS bindet, wobei die Formulierung 10 mg/ml des Antikörpers, 80 mM NaCl, 10 mM Histidin, 4% Trehalose und 0,02% Polysorbat 80 umfasst und wobei der pH-Wert der Formulierung 6 beträgt; und wobei

a) der Antikörper eine Fc-Region mit komplexen N-Glycosid-verknüpften Zuckerketten, in denen die Fucose nicht an das N-Acetylglucosamin am reduzierenden Ende der Zuckerkette gebunden ist, umfasst,
b) der Antikörper in Lösung eine reversible Selbstassoziation durchläuft, wobei die reversible Selbstassoziation nicht Aggregatbildung induziert, und
c) mindestens 10 Molprozent des Antikörpers bei 37°C als Trimer in PBS mit einer Antikörperkonzentration von 10 mg/ml vorliegt; und

wobei der Antikörper eine Schwerkettensequenz gemäß SEQ ID NO: 6 und eine Leichtkettensequenz gemäß SEQ ID NO: 1 umfasst.

2. Formulierung nach Anspruch 1, wobei die Formulierung isotonisch ist.

3. Formulierung nach Anspruch 1 oder Anspruch 2, wobei die Formulierung eine pharmazeutisch unbedenkliche Formulierung ist.

4. Formulierung nach einem der Ansprüche 1 bis 3, wobei die Formulierung eine injizierbare Formulierung ist und gegebenenfalls wobei die Formulierung für die intravenöse, subkutane oder intramuskuläre Verabreichung geeignet ist.

5. Pharmazeutische Einzeldosisform, die für die parenterale Verabreichung an einen Menschen geeignet ist und die eine Antikörperformulierung nach einem der Ansprüche 1 bis 4 in einem geeigneten Behältnis umfasst.

6. Vorgefüllte Spritze, enthaltend die Formulierung nach einem der Ansprüche 1 bis 4.

7. Formulierung nach einem der Ansprüche 1 bis 4;

a) zur Verwendung in der Behandlung einer Autoimmunkrankheit oder -störung bei einem Menschen; oder
b) zur Verwendung in der Behandlung oder Vorbeugung von Abstoßung bei einem menschlichen Transplantationspatienten; oder
c) zur Verwendung in der Behandlung einer entzündlichen Krankheit oder Störung in einem Menschen; oder
d) zur Verwendung in der Abreicherung von ICOSexprimierenden T-Zellen in einem menschlichen Patienten; oder
e) zur Verwendung in der Störung der Keimzentrenarchitektur in einem sekundären Lymphoidorgan eines lymphatischen Organs eines Primaten.

8. Formulierung zur Verwendung nach Anspruch 7, wobei es sich bei der Autoimmunkrankheit oder -störung um SLE oder Sklerodermie handelt.

**Revendications**

1. Formulation aqueuse stable, stérile comprenant un anticorps qui se lie spécifiquement à ICOS humain, ladite formulation comprenant 10 mg/ml de l'anticorps, NaCl 80 mM, histidine 10 mM, 4 % de tréhalose et 0,02 % de polysorbate 80 et le pH de la formulation est 6 ; et dans laquelle

   a) l'anticorps comprend une région Fc ayant des chaînes de sucre N-glycosidiques complexes dans lesquelles le fucose n'est pas lié à la N-acétylglucosamine dans l'extrémité réductrice de la chaîne de sucre,
   b) l'anticorps subit une auto-association réversible en solution, l'auto-association réversible n'induisant pas la formation d'agrégats, et
   c) au moins 10 pour cent en moles de l'anticorps existe sous la forme d'un trimère dans PBS à une concentration d'anticorps de 10 mg/ml à 37 °C ; et

   dans laquelle l'anticorps comprend une séquence de chaîne lourde de SEQ ID NO: 6 et une séquence de chaîne légère de SEQ ID NO: 1.

2. Formulation selon la revendication 1, ladite formulation étant isotonique.

3. Formulation selon la revendication 1 ou la revendication 2, ladite formulation étant une formulation pharmaceutiquement acceptable.

4. Formulation selon l'une quelconque des revendications 1 à 3, ladite formulation étant une formulation injectable, et la formulation étant facultativement appropriée pour administration intraveineuse, sous-cutanée ou intramusculaire.

5. Forme pharmaceutique unitaire adaptée pour administration parentérale à un humain, qui comprend une formulation d'anticorps selon l'une quelconque des revendications 1 à 4 dans un récipient adapté.

6. Seringue préremplie contenant la formulation selon l'une quelconque des revendications 1 à 4.

7. Formulation selon l'une quelconque des revendications 1 à 4 ;

   a) pour utilisation dans le traitement d'une maladie ou un trouble auto-immun chez un humain ; ou
   b) pour utilisation dans le traitement ou la prévention d'un rejet chez un patient humain greffé ; ou
   c) pour utilisation dans le traitement d'une maladie ou d'un trouble inflammatoire chez un humain ; ou
   d) pour utilisation dans la déplétion de lymphocytes T exprimant ICOS chez un patient humain ; ou
   e) pour utilisation dans la perturbation de l'architecture de centre germinatif dans un organe lymphoïde secondaire d'un primate.

8. Formulation pour utilisation selon la revendication 7, la maladie ou le trouble auto-immun étant le lupus érythémateux disséminé ou la sclérodermie.

Anti-ICOS in 25 mM Histidine, pH 6

Fig. 1

Fig. 2

Fig. 3

Fig. 4

Fig. 5

Fig. 6

136 anti-ICOS Colloidal Stability at 70°C
20 mM Phosphate, pH 7.2

Fig. 7

**Fig. 8**

Fig. 9

**Fig. 10**

anti-ICOS @ 105 mg/ml

Fig. 11

anti-ICOS in 10 mM His, 4% Trehalose, pH 6

Fig. 12

anti-ICOS @ 105 mg/ml

Fig. 13

10 mM His, 80 mM NaCl, 4% Trehalose, 0.02% PS-80, pH 6

$y = -0.0518x + 98.3$
$R^2 = -0.3137$

$y = -2.3538x + 98.3$
$R^2 = 0.927$

Legend: 40C, 25C, 2-8C, Linear (2-8C), Linear (40C)

Y-axis: Percent Monomer
X-axis: TP = month

**Fig. 14**

| MAb | Mouse FcγRIV (nM) |
|---|---|
| Anti-mouse ICOS IgG2a | 219 |
| Anti-mouse ICOS IgG2a-aFuc | 5 |

Fig. 15A

**Fig. 15B**

**Splenic Central Memory ICOS$^+$ T cells**      **Splenic Effector Memory ICOS$^+$ T cells**

Isotype Control MAb

IgG2a ICOS MAb

IgG2a-aFuc ICOS MAb

**Fig. 15C**

Fig. 16

**A** Total Helper Memory T cells
(CD3$^+$, CD4$^+$, CD44$^{high}$ CD62L$^{low}$)

**B** Total Helper Memory ICOS$^+$ T Cells
(CD3$^+$, CD4$^+$, CD44$^{high}$ CD62L$^{low}$, ICOS$^+$)

Control Graft
Isotype Control MAb
anti-ICOS MAb

**Fig. 17**

**C**

**D**

**Peripheral Blood Helper Memory ICOS⁺ T Cells**
$(CD3^+ CD4^+ CD44^{high} CD62L^{low} ICOS^+)$

$T_{FH}$
$(CD4^+ CXCR5^+ ICOS^+)$

Control Graft
Isotype Control MAb
anti-ICOS MAb

Control Graft (Day 8)
Control Graft (Day 29)
GVHD (Day 8)
Isotype Control MAb (Day 29)
Anti-ICOS MAb (Day 29)

**Fig. 17**

E

**Total B cells**
**(CD19$^+$)**

**F**

**Germinal Center B Cells**
**(CD19$^+$ GL7$^+$ CD95$^+$)**

■ Control Graft
▨ Isotype Control MAb
▦ anti-ICOS MAb

■ Control Graft
▨ Isotype Control MAb
▦ anti-ICOS MAb

**Fig. 17**

**G**

**Total CD3<sup>+</sup> CD4<sup>+</sup> T cells**

**H**

**Total CD3<sup>+</sup> CD8<sup>+</sup> T Cells**

**Fig. 17**

Spleen

Kidney

Fig. 18

# Skin

| Control Balb/c | GvHD-SSc + Isotype Control MAb | GvHD-SSc + anti-ICOS MAb |
|---|---|---|

Fig. 19

**Fig. 20**

Fig. 20

IgHV

Neutrophilic Granule Protein

p<0.05

Fig. 20

Fig. 20

Fig. 21

**Fig. 22**

**[P] 10 mg/ml at RT**

● 136 anti-ICOS antibody

▲ mAbB

Fig. 23

Fig. 24

Fig. 25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1158004 A2
- US 4816567 A
- US 5500362 A
- US 5821337 A
- US 12116512 B
- US 6685940 B
- WO 9317715 A
- WO 9208802 A
- WO 9100360 A
- WO 9205793 A
- US 4474893 A
- US 4714681 A
- US 4925648 A
- US 5573920 A
- US 5601819 A
- US 6803039 B
- GB 91O1134 W
- WO 9002809 A
- WO 9110737 A
- WO 9201047 A
- WO 9218619 A
- WO 9311236 A
- WO 9515982 A
- WO 9520401 A
- WO 9713844 A
- US 5698426 A
- US 5223409 A
- US 5403484 A
- US 5580717 A
- US 5427908 A
- US 5750753 A
- US 5821047 A
- US 5571698 A
- US 5516637 A
- US 5780225 A
- US 5658727 A
- US 5733743 A
- US 5969108 A
- WO 9222324 A
- US 5693780 A
- US 6849425 B
- US 6632926 B
- US 6294391 B
- US 6143574 A
- EP 239400 B1
- WO 9321232 A
- US 5605793 A
- US 5811238 A
- US 5830721 A
- US 5834252 A
- US 5837458 A
- US 20030118592 A
- US 200330133939 A
- WO 02056910 A
- WO 04058821 A
- WO 04003019 A
- US 6291158 B
- US 6582915 B
- US 6696245 B
- US 6593081 B
- EP 404097 A
- WO 9311161 A
- US 20040253238 A, Bogen
- WO 9316185 A
- US 560181 A
- US 95731168 B
- US 4676980 A
- WO 9404690 A
- WO 92200373 A
- EP 03089 A
- US 5624821 A
- US 6277375 B
- US 6737056 B
- WO 0158957 A
- WO 0206919 PCT
- WO 04016750 PCT
- WO 04029207 PCT
- WO 04035752 PCT
- WO 04074455 PCT
- WO 04099249 PCT
- WO 04063351 PCT
- WO 05070963 PCT
- WO 05040217 PCT
- WO 05092925 PCT
- WO 06020114 PCT
- US 5885573 A
- US 5677425 A
- US 6165745 A
- US 5869046 A
- US 6121022 A
- US 5648260 A
- US 6528624 B
- US 6194551 B
- US 6821505 B
- US 20040002587 A
- WO 9429351 A
- WO 9958572 PCT
- WO 0042072 PCT

- WO 02060919 PCT
- US 6602684 B
- US 10277370 B
- US 10113929 B
- WO 0061739A1 A1
- WO 01292246A1 A
- WO 02311140A1 A
- WO 0230954A1 A1
- WO 00061739 A
- WO EA01229125 A
- US 20030115614 A
- US 5714350 A
- US 6350861 B
- US 6946292 B
- EP 1176195 A
- WO 03035835 A
- WO 9954342 PCT
- US 7029872 B
- US 20060148035 A1
- US 20040185045 A
- WO 2004016750 A, Koenig
- WO 9958572 A, Armour
- WO 9951642 A, Idusogie
- US 6395272 B, Deo
- US 20010036459 A
- WO 0179299 A, Ravetch
- WO 2004063351 A, Stavenhagen
- US 6331415 B
- WO 8605807 A
- WO 8901036 A
- US 5122464 A
- US 6063630 A
- US 6187305 B
- US 6692737 B
- US 6361972 B
- US 6524818 B
- US 6541221 B
- US 6623958 B
- US 6091001 A
- US 5807715 A
- WO 9418318 A
- WO 2006023148 A2, Campbell
- US 5703080 A
- US 4923990 A
- US 4675187 A
- US 10983340 A
- WO 8101145 A
- WO 8807378 A
- US 4975278 A
- WO 8705330 A
- WO 02098370 A
- WO 02069904 A
- WO 9308656 A
- US 6162432 A
- WO 9823289 A
- WO 9734631 A
- WO 9315199 A
- WO 9315200 A
- WO 0177137 A
- EP 413622 A
- US 5136021 A
- US 5147638 A
- US 5223395 A
- US 5231024 A
- US 5334380 A
- US 5360716 A
- US 5426181 A
- US 5436154 A
- US 5610279 A
- US 5644034 A
- US 5656272 A
- US 5658746 A
- US 5698195 A
- US 5736138 A
- US 5741488 A
- US 5808029 A
- US 5919452 A
- US 5958412 A
- US 5959087 A
- US 5968741 A
- US 5994510 A
- US 6036978 A
- US 6114517 A
- US 6171787 B
- US 4665077 A
- US 4120649 A
- WO 9008187 A
- US 5114721 A
- WO 9011294 A
- WO 9101133 A
- EP 340109 A
- US 4741900 A
- WO 05032627 A
- WO 08094984 PCT
- WO 9945985 PCT
- WO 03077976 PCT
- US 6792743 B
- US 5607400 A
- US 5893842 A
- US 7081107 B
- US 7041087 B
- US 5989227 A
- US 6807797 B
- US 6142976 A
- US 5899889 A
- US 20070161961 A1
- US 20050075611 A1
- US 20070092487 A1
- US 20040267194 A1
- US 20060129108 A1
- WO 61113796 A
- WO 61249365 A

**Non-patent literature cited in the description**

- **WANG et al.** *J. of Parenteral Science & Technology,* 1988, vol. 42, S4-S26
- **CLELAND et al.** *Critical Reviews in Therapeutic Drug Carrier Systems,* 1993, vol. 10 (4), 307-377
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **RAVETCH ; KINET.** *Annu. Rev. Immunol.,* 1991, vol. 9, 457-92
- **CLYNES et al.** *Proc. Natl. Acad. Sci. (USA),* 1998, vol. 95, 652-656
- **GAZZANO-SANTARO et al.** *J. Immunol. Methods,* 1996, vol. 202, 163
- **DAËRON.** *Annu. Rev. Immunol.,* 1997, vol. 15, 203-234
- **CAPEL et al.** *Immunomethods,* 1994, vol. 4, 25-34
- **HAAS et al.** *J. Lab. Clin. Med.,* 1995, vol. 126, 330-41
- **GUYER et al.** *Immunol.,* 1976, vol. 117, 587
- **KIM et al.** *J. Immunol.,* 1994, vol. 24, 249
- **GRAY et al.** *J. Virol. Meth.,* 1993, vol. 44, 11-24
- **JOSEPH P. REMINGTON.** Remington's Pharmaceutical Sciences. Mack Publishing Co,
- The Merck Index. Merck & Co, 2001
- Formulation of Small Volume Parenterals. **DE LUCA ; BOYLAN.** Pharmaceutical Dosage Form: Parenteral Medications. vol. 1, 194
- Remington: The Science & Practice of Pharmacy. Lippincott Williams & Wilkins, 2005
- Physician's Desk Reference. Medical Economics, 2005
- *The United States Pharmacopeial Convention, Pharmacopeial Forum,* 2000, vol. 26 (1), 223
- **TUTT et al.** *J. Immunol.,* 1991, vol. 147, 60-69
- **KOSTELNY et al.** *J. Immunol.,* 1992, vol. 148, 1547-1553
- **MARASCO et al.** *Proc Natl Acad Sci,* 1993, vol. 90, 7889-7893
- **KABAT et al.** Sequences of Proteins of Immunological Interest. National Institutes of Health, National Technical Information Service, 1991
- **HARLOW et al.** Antibodies: A Laboratory Manual. Cold Spring Harbor Laboratory Press, 1988
- **HAMMERLING et al.** Monoclonal Antibodies and T Cell Hybridomas. Elsevier, 1981, 563-681
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BRINKMAN et al.** *J. Immunol. Methods,* 1995, vol. 182, 41-50
- **AMES et al.** *J. Immunol. Methods,* 1995, vol. 184, 177-186
- **KETTLEBOROUGH et al.** *Eur. J. Immunol.,* 1994, vol. 24, 952-958
- **PERSIC et al.** *Gene,* 1997, vol. 187, 9-18
- **BURTON et al.** *Advances in Immunology,* 1994, vol. 57, 191-280
- **MULLINAX et al.** *BioTechniques,* 1992, vol. 12 (6), 864-869
- **SAWAI et al.** *AJRI,* 1995, vol. 34, 26-34
- **BETTER et al.** *Science,* 1988, vol. 240, 1041-1043
- **MCCAFFERTY et al.** *Nature,* 1990, vol. 348, 552-554
- **MARKS et al.** *Bio/Technology,* 1992, vol. 10, 779-783
- **WATERHOUSE et al.** *Nucl. Acids. Res.,* 1993, vol. 21, 2265-2266
- **MORRISON et al.** *Proc. Natl. Acad. Sci. USA,* 1984, vol. 81, 6851
- **MORRISON et al.** *Proc. Natl. Acad. Sci. USA,* 1984, vol. 81, 6851-6855
- **HALL, T.A.** BioEdit: a user-friendly biological sequence alignment editor and analysis program for Windows 95/98/NT. *Nucl. Acids. Symp. Ser.,* 1999, vol. 41, 95-98
- **DEVEREUX J. et al.** *Nucleic Acids Res.,* 1984, vol. 12, 387-395
- **SMITH ; WATERMAN.** *Advances in Applied Mathematics,* 1981, vol. 2, 482-489
- **NEDDLEMAN ; WUNSCH.** *J. Mol. Biol.,* 1970, vol. 48, 443-354
- **KARLIN ; ALTSCHUL.** *Proc. Natl. Acad. Sci. USA,* 1990, vol. 87, 2264-2268
- **KARLIN ; ALTSCHUL.** *Proc. Natl. Acad. Sci. USA,* 1993, vol. 90, 5873-5877
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **PEARSON W.R. ; LIPMAN D.J.** *Proc. Natl. Acad. Sci. USA,* 1988, vol. 85, 2444-2448
- **HENIKOFF S. ; HENIKOFF J.G.** *Proc. Natl. Acad. Sci. USA,* 1992, vol. 89, 10915-10919
- **AMIT et al.** *Science,* 1986, vol. 233, 747-753
- **CHOTHIA et al.** *J. Mol. Biol.,* 1987, vol. 196, 901-917

- **CUNNINGHAM ; WELLS.** *Science,* 1989, vol. 244, 1081-1085
- **HAWKINS et al.** *J. Mol. Biol.,* 1992, vol. 254, 889-896
- **LOWMAN et al.** *Biochemistry,* 1991, vol. 30 (45), 10832-10837
- **KUNKEL.** *Proc. Natl. Acad. Sci. USA,* 1985, vol. 82, 488
- **HUTCHINSON, C. et al.** *J. Biol. Chem.,* 1978, vol. 253, 6551
- **SMITH.** *Ann. Rev. Genet.,* 1985, vol. 19, 423-463
- **HILL et al.** *Methods Enzymol.,* 1987, vol. 155, 558-568
- **HO et al.** *Gene,* 1989, vol. 77, 51-59
- **SARKAR et al.** *Biotechniques,* 1990, vol. 8, 404-407
- **PASTAN et al.** *Cell,* 1986, vol. 47, 641
- **GOLDENBERG et al.** *Cancer Journal for Clinicians,* 1994, vol. 44, 43
- **PATTEN et al.** *Curr. Opinion Biotechnol.,* 1997, vol. 8, 724-33
- **HARAYAMA.** *Trends Biotechnol.,* 1998, vol. 16 (2), 76-82
- **HANSSON et al.** *J. Mol. Biol.,* 1999, vol. 287, 265-76
- **LORENZO ; BLASCO.** *Biotechniques,* 1998, vol. 24 (2), 308-313
- **HOLLINGER et al.** *Proc. Natl. Acad. Sci. USA,* 1993, vol. 90, 6444-6448
- **ZAPATA et al.** *Protein Eng.,* 1995, vol. 8 (10), 1057-1062
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- **MILLSTEIN et al.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **KOSTELNY et al.** *J. Immunol.,* 1992, vol. 148 (5), 1547-1553
- **HOLLINGER et al.** *Proc. Natl Acad. Sci. USA,* 1993, vol. 90, 6444-6448
- **GRUBER et al.** *J. Immunol.,* 1994, vol. 152, 5368
- Fundamental Immunology. Lippincott-Raven, 1999
- **BRUGGEMANN et al.** *J Exp Med,* vol. 166, 1351-1361
- **WILKINSON et al.** *J Immunol Methods,* 2001, vol. 258, 183-191
- **PATEL et al.** *J Immunol Methods,* 1995, vol. 184, 29-38
- **CLYNES et al.** *Proc. Natl. Acad. Sci. USA,* 1998, vol. 95, 652-656
- **GAZZANO-SANTORO et al.** *J. Immunol. Methods,* 1996, vol. 202, 163
- **GHETIE et al.** *Nat Biotech.,* 1997, vol. 15, 637-40
- **DUNCAN et al.** *Nature,* 1988, vol. 332, 563-564
- **LUND et al.** *J. Immunol,* 1991, vol. 147, 2657-2662
- **LUND et al.** *Mol Immunol,* 1992, vol. 29, 53-59
- **ALEGRE et al.** *Transplantation,* 1994, vol. 57, 1537-1543
- **HUTCHINS et al.** *Proc Natl. Acad Sci U S A,* 1995, vol. 92, 11980-11984
- **JEFFERIS et al.** *Immunol Lett.,* 1995, vol. 44, 111-117
- **LUND et al.** *Faseb J,* 1995, vol. 9, 115-119
- **JEFFERIS et al.** *Immunol Lett,* 1996, vol. 54, 101-104
- **LUND et al.** *J Immunol,* 1996, vol. 157, 4963-4969
- **ARMOUR et al.** *Eur J Immunol,* 1999, vol. 29, 2613-2624
- **IDUSOGIE et al.** *J Immunol,* 2000, vol. 164, 4178-4184
- **REDDY et al.** *J Immunol,* 2000, vol. 164, 1925-1933
- **XU et al.** *Cell Immunol,* 2000, vol. 200, 16-26
- **IDUSOGIE et al.** *J Immunol,* 2001, vol. 166, 2571-2575
- **SHIELDS et al.** *J Biol Chem,* 2001, vol. 276, 6591-6604
- **JEFFERIS et al.** *Immunol Lett,* 2002, vol. 82, 57-65
- **PRESTA et al.** *Biochem Soc Trans,* 2002, vol. 30, 487-490
- **KUNKEL.** *Proc. Natl. Acad. Sci. USA,* 1985, vol. 82, 488-492
- **HIGUCHI.** PCR Protocols: A Guide to Methods and Applications. Academic Press, 1990, 177-183
- **WELLS et al.** *Gene,* 1985, vol. 34, 315-323
- **HIGUCHI.** PCR Technology: Principles and Applications for DNA Amplification. Stockton Press, 1989, 61-70
- **UMANA et al.** *Nat. Biotechnol,* 1999, vol. 17, 176-180
- **DAVIES et al.** *Biotechnol Bioeng,* vol. 74, 288-294
- **SHIELDS et al.** *J Biol Chem,* 2002, vol. 277, 26733-26740
- **SHINKAWA et al.** *J Biol Chem,* 2003, vol. 278, 3466-3473
- **OKAZAKI et al.** *JMB,* 2004, vol. 336, 1239-49
- **SHIELDS, R.L. et al.** *J. Biol. Chem.,* 2002, vol. 277, 26733-26740
- **UMANA et al.** *Nat. Biotech.,* 1999, vol. 17, 176-1

- **CARON et al.** *J. Exp Med.,* 1992, vol. 176, 1191-1195
- **SHOPES, B.** *J. Immunol.,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **FOECKING et al.** *Gene,* 1986, vol. 45, 101
- **COCKETT et al.** *Bio/Technology,* 1990, vol. 8 (2
- **RUTHER et al.** *EMBO,* 1983, vol. 12, 1791
- **INOUYE ; INOUYE.** *Nucleic Acids Res.,* 1985, vol. 13, 3101-3109
- **VAN HEEKE ; SCHUSTER.** *J. Biol. Chem.,* 1989, vol. 24, 5503-5509
- **LOGAN ; SHENK.** *Proc. Natl. Acad. Sci. USA,* 1984, vol. 81, 355-359
- **BITTNER et al.** *Methods in Enzymol.,* 1987, vol. 153, 51-544
- **WIGLER et al.** *Cell,* 1977, vol. 11, 223
- **SZYBALSKA ; SZYBALSKI.** *Proc. Natl. Acad. Sci. USA,* 1992, vol. 48, 202
- **LOWY et al.** *Cell,* 1980, vol. 22, 8-17
- **WIGLER et al.** *Natl. Acad. Sci. USA,* 1980, vol. 77, 357
- **O'HARE et al.** *Proc. Natl. Acad. Sci. USA,* 1981, vol. 78, 1527
- **MULLIGAN ; BERG.** *Proc. Natl. Acad. Sci. USA,* 1981, vol. 78, 2072
- **WU ; WU.** *Biotherapy,* 1991, vol. 3, 87-95
- **TOLSTOSHEV.** *Ann. Rev. Pharmacol. Toxicol.,* 1993, vol. 32, 573-596
- **MULLIGAN.** *Science,* 1993, vol. 260, 926-932
- **MORGAN ; ANDERSON.** *Ann. Rev. Biochem.,* 1993, vol. 62, 191-217
- **MAY.** *TIB TECH,* 1993, vol. 11 (5), 155-2 15
- **SANTERRE et al.** *Gene,* 1984, vol. 30, 147
- Current Protocols in Molecular Biology. John Wiley & Sons, 1993
- **KRIEGLER.** Gene Transfer and Expression, A Laboratory Manual. Stockton Press, 1990
- Current Protocols in Human Genetics. John Wiley & Sons, 1994
- **COLBERRE-GARAPIN et al.** *J. Mol. Biol.,* 1981, vol. 150, 1
- **BEBBINGTON ; HENTSCHEL.** The use of vectors based on gene amplification for the expression of cloned genes in mammalian cells in DNA cloning. Academic Press, 1987, vol. 3
- **CROUSE et al.** *Mol. Cell. Biol.,* 1983, vol. 3, 257
- **PROUDFOOT.** *Nature,* 1986, vol. 322, 562-65
- **KOHLER.** *Proc. Natl. Acad. Sci. USA,* 1980, vol. 77, 2197
- **LINDMARK et al.** *J. Immunol. Methods,* 1983, vol. 62, 1-13
- **GUSS et al.** *EMBO J.,* 1986, vol. 5, 15671575
- **PARMLEY ; SMITH.** *Gene,* 1988, vol. 73, 305-318
- **FOWLKES et al.** *BioTechniques,* 1992, vol. 13, 422-427
- Monoclonal Antibodies: Principles and Applications. Wiley-Liss, Inc, 1995
- *Chemical Immunology,* 1997, vol. 65, 88
- *Eur. J. Immunol.,* 1993, vol. 23, 1098
- *Immunology,* 1995, vol. 86, 319
- **CARON et al.** *J. Exp. Med.,* 1992, vol. 176, 1191-1195
- **SHOPES.** *J. Immunol.,* 1992, vol. 148, 2918-2922
- *Annu. Rev. Immunol.,* 2000, vol. 18, 709
- *Annu. Rev. Immunol.,* 2001, vol. 19, 275
- **BRUHNS et al.** *Clin. Invest. Med.,* 2004, vol. 27, 3D
- **DECKER et al.** *Blood (USA),* 2004, vol. 103, 2718-2725
- Remington's Pharmaceutical Sciences. Mack Publishing Co, 1995
- Goodman and Gilman's The Pharmacological Basis of Therapeutics. MacMillan Publishing Co, 1985
- **DENARDO et al.** *Clin Cancer Res,* 1998, vol. 4 (10), 2483-90
- **PETERSON et al.** *Bioconjug Chem,* 1999, vol. 10 (4), 553-7
- **ZIMMERMAN et al.** *Nucl Med Biol,* 1999, vol. 26 (8), 943-50
- **MASSEY.** *Nature,* 1987, vol. 328, 457-458
- **NEUBERGER et al.** *Nature,* 1984, vol. 312, 604-608
- **T.E. CREIGHTON.** Proteins: Structure and Molecular Properties. W.H. Freeman & Co, 1983, 79-86
- **APLIN ; WRISTON.** *CRC Crit. Rev. Biochem.,* 1981, 259-306
- Physician's Desk Reference. 2002
- **WOOLDRIDGE et al.** *Blood,* 1997, vol. 89 (8), 2994-2998
- Physicians' Desk Reference. 2006
- **FREIREICH et al.** Quantitative comparison of toxicity of anticancer agents in mouse, rat, monkey, dog, and human. *Cancer Chemotherapy Reports,* 1966, vol. 40, 219-244
- **BUERKE et al.** *J. Immunol.,* 2001, vol. 167, 5375-80
- Physician's Desk Reference. 2007
- **WILLIAMS et al.** *Proc. Natl. Acad. Sci. USA,* 1994, vol. 91, 2762-2766
- **THORBECKE et al.** *Proc. Natl. Acad. Sci. USA,* 1992, vol. 89, 7375-7379
- **KOHNO et al.** *Proc. Natl. Acad. Sci. USA,* 1990, vol. 87, 8331-8335
- **SECKINGER et al.** *Proc. Natl. Acad. Sci. USA,* 1990, vol. 87, 5188-5192

- **OSWALD et al.** *Proc. Natl. Acad. Sci. USA,* 1992, vol. 89, 8676-8680
- **ASHKENAZI et al.** *Proc. Natl. Acad. Sci. USA,* 1991, vol. 88, 10535-10539
- **TEMKIN et al.** *J Immunol,* 2002, vol. 169 (5), 2662-2669
- **VOSSELLER et al.** *Mol. Biol. Cell,* 1997, vol. 8 (5), 909-922
- **NAGAI et al.** *Biochem Biophys Res Commun,* 1995, vol. 208 (2), 576-581
- **LEUNG et al.** *N Engl J Med,* 2003, vol. 348 (11), 986-993
- **FINN et al.** *J Allergy Clin Immuno,* 2003, vol. 111 (2), 278-284
- **CORREN et al.** *J Allergy Clin Immuno,* 2003, vol. 111 (1), 87-90
- **BUSSE ; NEAVILLE.** *Curr Opin Allergy Clin Immuno,* 2001, vol. 1 (1), 105-108
- **TANG ; POWELL.** *Eur J Pediatr,* 2001, vol. 160 (12), 696-704
- **MIYAJIMA et al.** *Int Arch Allergy Immuno,* vol. 128 (1), 24-32
- **NEERVEN et al.** *Int Arch Allergy Immuno,* 2001, vol. 124 (1-3), 400
- **GILMAN et al.** Goodman and Gilman's: The Pharmacological Basis of Therapeutics. McGraw-Hill, 2001
- The Merck Manual of Diagnosis and Therapy. Merck Sharp & Dohme Research Laboratories, 1999
- Cecil Textbook of Medicine. W.B. Saunders, 1996
- **PATTI et al.** *Eur. J. Haematol.,* 1993, vol. 51, 18
- Non-Hodgkin's Lymphomas. **FREEDMAN et al.** CANCER MEDICINE. Lea & Febiger, 1993, vol. 2, 2028-2068
- REMINGTON'S PHARMACEUTICAL SCIENCES. Mack Publishing Co, 1995
- GOODMAN AND GILMAN'S THE PHARMACOLOGICAL BASIS OF THERAPEUTICS. MacMillan Publishing Co, 1985
- **WILMAN.** "Prodrugs in Cancer Chemotherapy" Biochemical Society Transactions. *615th Meeting Belfast,* 1986, vol. 14, 375-382
- Prodrugs: A Chemical Approach to Targeted Drug Delivery. **STELLA et al.** Directed Drug Delivery. Humana Press, 1985, 247-267
- **OFFNER et al.** *Science,* 1991, vol. 251, 430-432
- **GRIGGS et al.** *Lancet Oncol.,* 2001, vol. 2, 82
- **ROSEN.** *Oncologist,* 2000, vol. 5, 20
- **HINMAN et al.** *Cancer Research,* 1993, vol. 53, 3336-3342
- **LODE et al.** *Cancer Research,* 1998, vol. 58, 2925-2928
- **JONSSON et al.** *Biotechniques,* 1991, vol. 11 (5), 620-627
- **JOHNE, B.** *Molecular Biotechnology,* 1989, vol. 9 (1), 65-71
- **ZHOU L.** *J Invest Dermatol,* 2006, vol. 126 (2), 305-14

SEQUENCE LISTING

<110> MedImmune LLC Sathish, Hasige Shah, Ambarish Carlesso, Gianluca Delaney, Tracy

<120> Antibody Formulation

<130> IC320PCT

<150> 61/113,796
<151> 2008-11-12

<150> 61/249,365
<151> 2009-10-07

<160> 10

<170> PatentIn version 3.5

<210> 1
<211> 214
<212> PRT
<213> Homo sapiens

<400> 1

```
Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Arg Leu
            20                  25                  30

Leu Ala Trp Tyr Gln Gln Lys Pro Gly Lys Ala Pro Lys Leu Leu Ile
            35                  40                  45

Tyr Val Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Asn Ser Phe Pro Trp
            85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
            115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
            165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
    195                 200                 205

Phe Asn Arg Gly Glu Cys
    210
```

<210> 2
<211> 107
<212> PRT
<213> Homo sapiens

<400> 2

**104**

```
Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Arg Leu
                20                  25                  30

Leu Ala Trp Tyr Gln Gln Lys Pro Gly Lys Ala Pro Lys Leu Leu Ile
                35                  40                  45

Tyr Val Ala Ser Ser Leu Gln Ser Gly Val Pro Ser Arg Phe Ser Gly
            50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Asn Ser Phe Pro Trp
                85                  90                  95

Thr Phe Gly Gln Gly Thr Lys Val Glu Ile Lys
                100                 105
```

<210> 3
<211> 11
<212> PRT
<213> Homo sapiens

<400> 3

```
                    Arg Ala Ser Gln Gly Ile Ser Arg Leu Leu Ala
                    1               5                   10
```

<210> 4
<211> 7
<212> PRT
<213> Homo sapiens

<400> 4

```
                    Val Ala Ser Ser Leu Gln Ser
                    1               5
```

<210> 5
<211> 9
<212> PRT
<213> Homo sapiens

<400> 5

```
                    Gln Gln Ala Asn Ser Phe Pro Trp Thr
                    1               5
```

<210> 6
<211> 455

**105**

<212> PRT
<213> Homo sapiens

<400> 6

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
            35                  40                  45

Gly Trp Ile Asn Pro His Ser Gly Gly Thr Asn Tyr Ala Gln Lys Phe
        50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Thr Tyr Tyr Tyr Asp Ser Ser Gly Tyr Tyr His Asp Ala Phe
                100                 105                 110

Asp Ile Trp Gly Gln Gly Thr Met Val Thr Val Ser Ser Ala Ser Thr
```

115                     120                     125

Lys Gly Pro Ser Val Phe Pro Leu Ala Pro Ser Ser Lys Ser Thr Ser
    130             135             140

Gly Gly Thr Ala Ala Leu Gly Cys Leu Val Lys Asp Tyr Phe Pro Glu
145             150             155             160

Pro Val Thr Val Ser Trp Asn Ser Gly Ala Leu Thr Ser Gly Val His
            165             170             175

Thr Phe Pro Ala Val Leu Gln Ser Ser Gly Leu Tyr Ser Leu Ser Ser
        180             185             190

Val Val Thr Val Pro Ser Ser Ser Leu Gly Thr Gln Thr Tyr Ile Cys
    195             200             205

Asn Val Asn His Lys Pro Ser Asn Thr Lys Val Asp Lys Arg Val Glu
    210             215             220

Pro Lys Ser Cys Asp Lys Thr His Thr Cys Pro Pro Cys Pro Ala Pro
225             230             235             240

Glu Leu Leu Gly Gly Pro Ser Val Phe Leu Phe Pro Pro Lys Pro Lys
            245             250             255

Asp Thr Leu Met Ile Ser Arg Thr Pro Glu Val Thr Cys Val Val Val
        260             265             270

Asp Val Ser His Glu Asp Pro Glu Val Lys Phe Asn Trp Tyr Val Asp
    275             280             285

Gly Val Glu Val His Asn Ala Lys Thr Lys Pro Arg Glu Glu Gln Tyr
    290             295             300

Asn Ser Thr Tyr Arg Val Val Ser Val Leu Thr Val Leu His Gln Asp
305             310             315             320

Trp Leu Asn Gly Lys Glu Tyr Lys Cys Lys Val Ser Asn Lys Ala Leu
            325             330             335

Pro Ala Pro Ile Glu Lys Thr Ile Ser Lys Ala Lys Gly Gln Pro Arg
        340             345             350

Glu Pro Gln Val Tyr Thr Leu Pro Pro Ser Arg Glu Glu Met Thr Lys
        355             360             365

```
Asn Gln Val Ser Leu Thr Cys Leu Val Lys Gly Phe Tyr Pro Ser Asp
    370             375             380

Ile Ala Val Glu Trp Glu Ser Asn Gly Gln Pro Glu Asn Asn Tyr Lys
385             390             395                 400

Thr Thr Pro Pro Val Leu Asp Ser Asp Gly Ser Phe Phe Leu Tyr Ser
            405             410                 415

Lys Leu Thr Val Asp Lys Ser Arg Trp Gln Gln Gly Asn Val Phe Ser
        420             425             430

Cys Ser Val Met His Glu Ala Leu His Asn His Tyr Thr Gln Lys Ser
        435             440             445

Leu Ser Leu Ser Pro Gly Lys
    450             455
```

<210> 7
<211> 125
<212> PRT
<213> Homo sapiens

<400> 7

```
Gln Val Gln Leu Val Gln Ser Gly Ala Glu Val Lys Lys Pro Gly Ala
1               5               10                  15

Ser Val Lys Val Ser Cys Lys Ala Ser Gly Tyr Thr Phe Thr Gly Tyr
            20              25              30

Tyr Met His Trp Val Arg Gln Ala Pro Gly Gln Gly Leu Glu Trp Met
        35              40              45

Gly Trp Ile Asn Pro His Ser Gly Gly Thr Asn Tyr Ala Gln Lys Phe
    50              55              60

Gln Gly Arg Val Thr Met Thr Arg Asp Thr Ser Ile Ser Thr Ala Tyr
65              70              75                  80

Met Glu Leu Ser Arg Leu Arg Ser Asp Asp Thr Ala Val Tyr Tyr Cys
            85              90                  95

Ala Arg Thr Tyr Tyr Tyr Asp Ser Ser Gly Tyr Tyr His Asp Ala Phe
        100             105             110

Asp Ile Trp Gly Gln Gly Thr Met Val Thr Val Ser Ser
    115             120             125
```

<210> 8
<211> 5
<212> PRT
<213> Homo sapiens

<400> 8

```
              Gly Tyr Tyr Met His
              1                 5
```

<210> 9
<211> 17
<212> PRT
<213> Homo sapiens

<400> 9

```
     Trp Ile Asn Pro His Ser Gly Gly Thr Asn Tyr Ala Gln Lys Phe Gln
     1               5               10                  15


     Gly
```

<210> 10
<211> 16
<212> PRT
<213> Homo sapiens

<400> 10

```
     Thr Tyr Tyr Tyr Asp Ser Ser Gly Tyr Tyr His Asp Ala Phe Asp Ile
     1               5               10                  15
```